# EUROPEAN PATENT APPLICATION

(11) **EP 2 060 585 A2**
(43) Date of publication of application: **20.05.2009**
(21) Application number: 08171382.8
(22) Date of filing: 04.10.2002
(51) Int. Cl.: C07K 14/59, C07K 5/083, C07K 5/103, C07K 7/06, C07K 7/08, A61K 38/00

(54) **Gene regulatory peptides**

(30) Priority: 04.10.2001 EP 01203748; 21.12.2001 US 28075
(62) Divisional of application: 02763111.8
(71) Applicant: Biotempt B.V., 7958 NZ Koekange (NL)
(72) Inventor: Khan, Nisar Ahmed, 3074 AD Rotterdam (NL); Benner, Robbert, 2992 SH Barendrecht (NL)
(74) Representative: Hatzmann, Martin

(57) **Abstract**

The invention relates to the modulation of gene expression in a cell, also called gene control, in particular in relation to the treatment of a variety of diseases. The invention provides a method for modulating expression of a gene in a cell comprising providing said cell with a signalling molecule comprising a peptide or functional analogue thereof. Furthermore, the invention provides a method for identifying or obtaining a signalling molecule comprising a peptide or functional derivative or analogue thereof capable of modulating expression of a gene in a cell comprising providing said cell with a peptide or derivative or analogue thereof and determining the activity and/or nuclear translocation of a gene transcription factor.

## Description

In the United States this application is a continuation-in-part of US application 10/028,075 filed December 21, 2001.

Gene control is generally thought to occur at four levels: 1) transcription (either initiation or termination), 2) processing of primary transcripts, 3) stabilization or destabilization of mRNAs, and 4) mRNA translation. The primary function of gene control in cells is to adjust the enzymatic machinery of the cell to its nutritional, chemical and physical environment.

It is generally thought that gene expression is regulated at both the levels of transcription and translation. Modulation or regulation of gene expression requires factors called transcriptional factors. The term "gene control or regulation" also refers to the formation and use of mRNA. Although control can be exerted at a number of different molecular steps, differential transcription probably most frequently underlies the differential rate of protein synthesis in prokaryotes as well as eukaryotes. It is generally thought that activator proteins (also called transcription factors or transcriptional activators) bind to DNA and recruit the transcriptional machinery in a cell to a promotor, thereby stimulating gene expression. Further, differential processing of RNA transcripts in the cell nucleus, differential stabilization of mRNA in the cytoplasm, and differential translation of mRNA into protein are also important in eukaryotic gene control. These steps define the regulatory decisions in a transcriptional circuit and misregulation at any stage can result in a variety of diseases.
Where in unicellular organisms, be it of prokaryotic or eukaryotic origin, a cell's response to its environment is influenced by many stimuli from the outside world, reflecting the often widely variable environment of the single cell, most cells in multicellular organisms experience a fairly constant environment. Perhaps for this reason, genes that are devoted to responses to environmental changes constitute a much smaller fraction of the total number of genes in multicellular organisms than in single-cell organisms.

As said above, cells react to environmental changes, which they perceive through extracellular signals. These signals can be either physical (e.g., light, temperature, pressure and electricity) or chemical (e.g. food, hormones and neurotransmitters). Cells can both sense and produce signals. This makes it possible for them to communicate with each other. In order to achieve this, there are complex signal-sensing and -producing mechanisms in uni- and multi-cellular organisms.

Two groups of chemical signals can be distinguished: membrane-permeable and membrane-impermeable signals. The membrane-permeable signal molecules comprise the large family of steroid hormones, such as estrogens, progesterone and androgens. Steroids pass the plasma membrane and bind to specific receptors, which are localized in the cytoplasm or nucleus of the cell. After binding of the hormone, the receptor undergoes a conformational change. The receptor is then able to bind to DNA itself or to proteins which can in turn interact with DNA. In general, steroid hormones can directly regulate gene expression by means of this process. The membrane-impermeable signal molecules include acetylcholine, growth factors, extracellular matrix components, (peptide)-hormones, neuropeptides, thrombin, lysophosphatidic acid, the yeast mating factors and, for the social amoeba *Dictyostellium discoideum,* folic acid and cyclic AMP. They may be membrane-permeable in themselve but act only outside the cell, i.e. they are recognized by receptors, which are localized on the plasma membrane of the cell. The receptors are specific for one particular signal molecule or a family of closely related signal molecules. Upon binding of their ligands, these receptors transduce the signals by several mechanisms.

The most characteristic and exacting requirement of gene control on multicellular organisms is the execution of precise developmental decisions so that the right gene is activated in the right cell at the right time. These developmental decisions include not only those related to the development of an organism oer se, as for example can be seen during embryogenesis and organogenesis or in response to disease, but also relate to the differentiation or proliferation or apoptosis of those cells that merely carry out their genetic program essentially without leaving progeny behind.

Such cells, such as skin cells, precursors of red blood cells, lens cells of the eye, and antibody-producing cells, are also often regulated by patterns of gene control that serve the need of the whole organism and not the survival of an individual cell.

It is generally reasoned that there are at least three components of gene control: molecular signals, levels and mechanisms. Firstly, it is reasoned that specific signalling molecules exist to which a specific gene can respond. Secondly, control is exerted on one or more levels (i.e., the step or steps) in the chain of events leading from the transcription of DNA to the use of mRNA in protein synthesis. Thirdly, at each of those levels, specific molecular mechanisms are employed to finally exert the control over the gene to be expressed.

Many genes are regulated not by a signalling molecule that enters the cells but by molecules that bind to specific receptors on the surface of cells. Interaction between cell-surface receptors and their ligands can be followed by a cascade of intracellular events including variations in the intracellular levels of so-called second messengers (diacylglycerol, Ca²⁺, cyclic nucleotides). The second messengers in turn lead to changes in protein phosphorylation through the action of cyclic AMP, cyclic GMP, calcium-activated protein kinases, or protein kinase C, which is activated by diaglycerol.

Many of the responses to binding of ligands to cell-surface receptors are cytoplasmatic and do not involve immediate gene activation in the nucleus. Some receptor-ligand interactions, however, are known to cause prompt nuclear transcriptional activation of a specific and limited set of genes. For example, one proto-oncogene, c-fos, is known to be activated in some cell types by elevation of almost every one of the known second messengers and also by at least two growth factors, platelet-derived growth factor and epidermal growth factor. However, progress has been slow in determining exactly how such activation is achieved. In a few cases, the transcriptional proteins that respond to cell-surface signals have been characterized.

One of the clearest examples of activation of a pre-existing inactive transcription factor following a cell-surface interaction is the nuclear factor (NF)-kappaB, which was originally detected because it stimulates the transcription of genes encoding immunoglobulins of the kappa class in B-lymphocytes. The binding site for NK-kappaB in the kappa gene is well defined (see for example P.A. Baeuerle and D. Baltimore, 1988, Science 242:540), providing an assay for the presence of the active factor. This factor exists in the cytoplasm of lymphocytes complexed with an inhibitor. Treatment of the isolated complex in vitro with mild denaturing conditions dissociates the complex, thus freeing NK-kappaB to bind to its DNA site. Release of active NF-kappaB in cells is now known to occur after a variety of stimuli including treating cells with bacterial lipopolysaccharide (LPS) and extracellular polypeptides as well as chemical molecules (e.g. phobol esters) that stimulate intracellular phosphokinases. Thus a phosphorylation event triggered by many possible stimuli may account for NF-kappaB conversion to the active state. The active factor is then translocated to the cell nucleus to stimulate transcription only of genes with a binding site for active NF-kappaB.

The inflammatory response involves the sequential release of mediators and the recruitment of circulating leukocytes, which become activated at the inflammatory site and release further mediators (Nat. Med. 7:1294;2001). This response is self-limiting and resolves through the release of endogenous anti-inflammatory mediators and the clearance of inflammatory cells. The persistent accumulation and activation of leukocytes is a hallmark of chronic inflammation. Current approaches to the treatment of inflammation rely on the inhibition of pro-inflammatory mediator production and of mechanisms that initiate the inflammatory response. However, the mechanisms by which the inflammatory response resolves might provide new targets in the treatment of chronic inflammation. Studies in different experimental models of resolving inflammation have identified several putative mechanisms and mediators of inflammatory resolution. We have shown that cyclopentenone prostaglandins (cyPGs) may be endogenous anti-inflammatory mediators and promote the resolution of inflammation *in vivo*. Others have shown a temporal shift to the production of anti-inflammatory lipoxins during the resolution of inflammation. In recent years, apoptosis has been identified as an important mechanism for the resolution of inflammation *in vivo*. It has been postulated that defects in leukocyte apoptosis are important in the pathogenesis of inflammatory disease. In addition, the selective induction of apoptosis in leukocytes may offer a new therapeutic approach to inflammatory disease.

Considering that NF-kappaB is thought by many to be a primary effector of disease (A.S. Baldwin, J. Clin. Invest., 2001, 107:3-6), numerous efforts are underway to develop safe inhibitors of NF-kappaB to be used in treatment of both chronic and acute disease situations. Specific inhibitors of NF-kappaB should reduce side effects associated with drugs such as NSAIDS and glucocorticoids and would offer significant potential for the treatment of a variety of human and animal diseases. Specific diseases or syndromes where patients would benefit from NF-kappaB inhibition vary widely and range from rheumatoid arthritis, atherosclerosis, multiple sclerosis, chronic inflammatory demyelinating polyradiculoneuritis, asthma, inflammatory bowel disease, to Helicobacter pylori-associated gastritis and other inflammatory responses, and a variety of drugs that have effects on NF-kappaB activity, such as corticosteroids, sulfasalazine, 5-aminosalicylic acid, aspirin, tepoxalin, leflunomide, curcumin, antioxidants and proteasome inhibitors. These drugs are considered to be non-specific and often only applicable in high concentrations that may end up toxic for the individual treated.

Inactive cytoplasmatic forms of transcription factors can thus be activated by removal of an inhibitor, as is the case with NF-kappaB, or, alternatively, by association of two (or more) proteins, neither of which is active by itself as in the case of interferon-alpha-stimulated factor (D.E. Levy et al., 1989, Genes and Development 3:1362). After interferon-alpha attaches to its cell-surface receptor, one of the proteins is changed within a minute or less, and the two can combine. The active (combined) factor is then translocated to the cell nucleus to stimulate transcription only of genes with a binding site for the protein. Considering that interferon-alpha is a mediator of responses of the body directed at pathogens and self-antigens, modulating regulation of genes that are under influence of the interferon-alpha-stimulated factor would contribute to the treatment of a variety of human and animal diseases.

Other typical examples of signalling molecules that affect gene expression via cell-surface receptor interaction are polypeptide hormones such as insulin, glucagon, various growth factors such as EGF, VEGF, and so on.

The steroid hormones and their receptors represent one of the best understood cases that affect transcription. Because steroid hormones are soluble in lipid membranes, they can diffuse into cells. They affect transcription by binding to specific intracellular receptors that are site-specific DNA-binding molecules. Other examples of signalling molecules that enter the cell and act intra-cellularly are thyroid hormone (T₃), vitamin D and retinoic acid, and other small lipid-soluble signalling molecules that enter cells and modulate gene expression. The characteristic DNA-binding sites for the receptors for these signalling molecules are also known as response elements.

Another example of a small molecule that is involved in regulation of gene expression is ethylene, a gas that for example induces the expression of genes involved in fruit ripening. Also, small plant hormones, known as auxines and cytokinins regulate plant growth and differentiation directly by regulating gene expression.

Given the critical role of regulatory factors in gene regulation, the development of artificial or synthetic counterparts that could be used in methods to rectify errors in gene expression has been a long-standing goal at the interface of chemistry and biology.

The inventors have now unearthed an insight in the biology and physiology of the nature of regulatory factors in gene regulation in cellular organisms that allows for an unexpected fast progress in the identification and development of an artificial or synthetic compound acting as a gene regulator, and its use as new chemical entity for the production of a pharmaceutical composition or its use in the treatment of disease. The insight is herein provided that many of small peptides that are derivable by proteolytic breakdown of endogenous proteins of an organism, or that are derivable by proteolytic breakdown of proteins of a pathogen, i.e. during the presence of said pathogen in a host organism, can exert an often very specific gene regulatory activity on cells of said organism. In a particular embodiment, the present invention has major value for investigators in furthering the quality and quantity of knowledge regarding the mechanisms controlling NFκB-initiated gene expression under a variety of different conditions and circumstances.

With these insights the invention provides among others a screening method for identifying or obtaining a signaling molecule comprising a peptide or functional derivative or analogue thereof capable of modulating expression of a gene in a cell, be it in vitro or in vivo in an experimental animal such as a monkey or a small laboratory animal such as a rat or mouse, comprising providing said cell (or animal) with at least one lead peptide or derivative or analogue thereof and determining the activity and/or nuclear translocation of a gene transcription factor, in particular wherein said lead peptide is 3 to 15 amino acids long, more preferably, wherein said lead peptide is 3 to 9 amino acids long, most preferred wherein said lead peptide is 4 to 6 amino acids long.

Functional derivative or analogue herein relates to the signalling molecular effect or activity as for example can be measured by measuring nuclear translocation of a relevant transcription factor, such as NF-kappaB in an NF-kappaB assay, or AP-1 in an AP-1 assay, or by another method as provided herein. Fragments can be somewhat (i.e. 1 or 2 amino acids) smaller or larger on one or both sides, while still providing functional activity. A screening method according to the invention is also provided wherein the method further comprises determining whether said gene transcription factor regulates the transcription of a cytokine, as for example measured by detecting cytokine transcript levels or the actual presence as such in the treated call or animal, or wherein said gene transcription factor comprises a NF-kappaB/Rel protein, or by determining relative up-regulation and/or down-regulation of at least one gene of interest expressed in said cell or of a multitude of genes expressed in said cell, as easily can be done with gene chip technology or any of other methods herein explained. The invention also provides a screening method for identifying or obtaining a signaling molecule comprising a peptide or functional derivative or analogue thereof capable of modulating expression of a gene in a cell be it in vitro or in vivo in an experimental animal such as a monkey or a small laboratory animal such as a rat or mouse, comprising providing said cell (or animal) with a lead peptide or derivative or analogue thereof and determining relative up-regulation and/or down-regulation of at least one gene expressed in said cell, especially wherein said lead peptides are sufficiently small as identified herein. Such as method as provided herein for identifying or obtaining a signaling molecule comprising a peptide or functional derivative or analogue thereof capable of modulating expression of a gene in a cell may also comprise providing (be it in vitro or in vivo) a lead peptide or derivative or analogue thereof and determining binding of said peptide or derivative or analogue thereof to a factor related to gene control, such as a transcription factor, in particular wherein said transcription factor regulates the transcription of a cytokine, or determining the activity and/or nuclear translocation of a gene transcription factor in said cell provided with said peptide.
Advantageously, a screening method according to the invention is provided wherein said lead peptide is one of a member of a library of peptides or derivatives or analogues thereof, in particular, wherein said library is composed of peptides that are selected based on their occurrence in a naturally occurring protein. For investigations aimed at finding new chemical entities useful in human or veterinary therapy, based on using the lead peptide technology as provided herein, it is preferred that said protein is a mammalian protein, a human protein is most preferred. Protein sequences can be obtained from commonly available databases, such as for example are provided for the human genome. Other useful proteins from which libraries of lead peptides can be taken are those derived from pathogen proteins. For identifying peptides useful in crop cultivation, several plant protein data bases are available.
In a preferred embodiment, a screening method according to the invention is provided wherein lead peptides in said library are selected under guidance of proteolytic site prediction, such as peptides that are predicted or deemed to be recognized in a MHC context, by way of example such as the following hCG-derived peptides that are predicted to be recognized as antigenic determinants and presented in the context of HLA-molecules:
TMTRVLQGV, VLQGVLPAL, VLPALPQVVCNYRDVR, VCNYRDVRFESI, LPQVVCNYRDVRFESI. In another embodiment, a screening method is provided wherein at least one of said peptides in said library essentially overlaps with another peptide in said library, such as seen for example with VLQGVLPAL and VLPALPQVVCNYRDVR. Other sets or libraries of useful lead peptides can be designed by making the overlaps very stringent, e.g, that all but 1 or 2 amino acids overlap, such as is given here by way of example for LQGV, QGVL, GVLP, and so on, or QGVLPA, VLPALP, PALPQV, and so on. Of course, the invention aims at providing new chemical entities that act as a signaling molecule useful in modulating expression of a gene in a cell and identifiable or obtainable by employing a screening method according to the invention as provided herein. Useful signaling molecules are already provided herein as modulators of NF-kappaB/Rel protein, as detailed further on. The invention also provides use of a signaling molecule as thus provided for the production of a pharmaceutical composition for the modulation of gene expression, for example by inhibiting NF-kappaB/Rel protein activation, or its use for the production of a pharmaceutical composition for the treatment of a primate or domestic animal.

That small peptides, and even breakdown products, can have biological activity, is already known. Proteolytic breakdown products of endogenous or pathogen derived proteins are for example routinely generated by the proteasome system and presented in the context of class I or II major histocompabilility complex (MHC). Also, it has been recognized that classically known neuropeptides (also known as peptide neurotransmitters) or small peptide hormones, such as antidiuretic hormone, oxytocin, thyrotropin-releasing hormone, gonadotropin-releasing hormone, somatostatinsgastrin, cholecystokinin, substance-P, enkephalins, neurotensin, angiotensins, and derivatives or equivalents thereof have distinct biological activity which is in general mediated by cell-surface receptor interaction. Furthermore, it is now known that certain small and arginine- or lysine- or proline-rich peptides, i.e. having more than 50% of arginine, or 50% of lysine or 50% of proline, or having more than 50% arginine and lysine, or more than 50% arginine and proline, or more than 50% lysine and proline, or more than 50% arginine and lysine and proline residues, have distinct membrane-permeation properties that may result in biological activity.

However, the present invention relates to small peptides other than classically known neuropeptides or peptide hormones, and other than the above identified arginine- or lysine- or proline-rich peptides. It is preferred that the peptides of the invention and for use as lead peptide in a screening method as provided by the invention are small. A most preferred size is 4 to 6 amino acids, peptides of 2 to 3 amino acids or 7 to 9 are also very well feasible, a size of 10 to 15 amino acids is also feasible but becomes less practical for testing a method according to the invention, and peptides from 10 - 15 amino acids or larger are preferably broken down to smaller, functionally more active, fragments.

As said, the invention provides the insight that small peptides that are derivable or obtainable by proteolytic breakdown of endogenous proteins of an organism, or that are derivable or obtainable by proteolytic breakdown of proteins of a pathogen, i.e. during the presence of said pathogen in a host organism, can exert an often very specific gene regulatory activity on cells of said organism. This insight produces an immediate incentive for systematic approaches to practice or execute a method as provided herein to identify a signalling molecule, by obtaining information about the capacity or tendency of an small (oligo)peptide, or a modification or derivative thereof, (herein jointly called lead peptide) to regulate expression of a gene. Such a method as provided herein for example comprises the steps of contacting said peptide, or a modification or derivative thereof, with at least one cell and determining the presence of at least one gene product in or derived from said cell. Such a method is particularly useful when said lead peptide comprises an amino acid sequence corresponding to a fragment of a naturally occurring polypeptide. Exemplary of course herein is a method wherein said naturally occurring polypeptide comprises an hormone such as human chorionic gonadotropic hormone (hCG). However, other proteins, selected from classes as widely varying as immunoglobulins, heat shock proteins, Cys proteases, cytochrome p450 enzymes, (serine/threonine, or tyrosine) kinases, receptor proteins, protein phosphates, come to mind first when selecting polypeptide sequence from which lead peptides are designed. Other proteins can be taken as starting point as well. Specific mention deserve pathogen proteins as starting point. For example, it is worthwhile selecting such proteins from parasite pathogens, especially of those organisms that live for a certain time in a particular endobiontic relationship with their host, such as is the case with Taenia spp, leading to cysticercosis in man and animals, as with Schistosoma spp, as with malaria, or Trypanosoma, Giardia spp, Dictiocaulus spp, etc. Other pathogens that deserve attention are intracellular organisms such as found among (myco)bacteria and viruses
In one embodiment is preferred to perform such testing as provided herein systematically, based for example on a combinatorial chemistry format wherein a multitude of lead peptides (in a so-called peptide library) is tested, and promising individual lead peptides, or groups of lead peptides are further tested in subsequent rounds of testing, whereby such lead peptides can be modified to ones desire, as for example as described herein by replacement or substitution of amino acids with other (D-, or L-, non-naturally- or naturally occurring) amino acids or modifications or derivatives thereof. Especially by including such subsequent rounds of optimisation, the invention herewith provides a systemic method for identifying or obtaining a signalling molecule comprising a peptide or functional derivative or analogue thereof capable of modulating expression of a gene in a cell comprising providing said cell with a peptide or derivative or analogue thereof and determining the activity and/or nuclear translocation of a gene transcription factor and then synthesising the molecule with the desired activity.

In that way it is first possible to obtain in a systematic way information on the tendency or capacity of a leadpeptide, or a modification or derivative thereof, to regulate expression of a gene, and to improve on that capacity in subsequent rounds of lead optimalisation, until the lead compound is developed into a chemical entity useful in a pharmaceutical composition or method of treatment aimed at regulating a gene or genes under study. Provided herein is a method for obtaining information about the capacity or tendency of a lead peptide, or a modification thereof, to regulate expression of a gene comprising the steps of contacting said lead peptide, or a modification thereof, with at least one cell and determining the presence of at least one gene product derived from said cell.

As said, lead peptides can be derived from naturally occurring polypeptides such as natural protein molecules. Lead peptides containing 3 to 15 amino acids are preferably used. They may be tested in a random fashion, being derived from the proteome of the organism under study. Lead peptides may comprise overlapping amino acid sequences as well as peptides that are the result of a predicted chemical or enzymatic cleavage / digestion of a polypeptide. Lead peptides can be linear peptides as well as cyclic peptides. A naturally occurring protein can be an endogenous protein such as human chorionic gonadotropic hormone (hCG). Further, it can for example be a peptide derived from a pathogen polypeptide such as Bordetella, Yersinia, Toxoplasma gondii and African Swine Fever Virus. As said, many pathogens have evolved mechanisms to counteract or escape the host immune response by inhibiting NF-kappaB activation and suppressing the upregulation of proinflammatory cytokines. On the other hand, some viruses, including HIV-1, CMV and SV-40, take advantage of NF-kappaB as a host factor that is activated at sites of infection. A method is provided for studying host-pathogen interactions comprising determining the effect of a lead peptide derived from a polypeptide of said pathogen on the gene expression of said host.

In one embodiment, a linear scan is performed which is the systematic screening of overlapping lead peptides derived from a protein sequence with an appropriate bioassay. Such a bioassay comprises an assay for obtaining information about the capacity or tendency of a lead peptide, or a modification thereof, to regulate expression of a gene. A scan with for example a 15-mer, or a 12-mer, or a 9-mer, or a 8-mer, or a 7-mer, or a 6-mer, or a 5-mer, or a 4-mer or a 3-mer peptides can yield valuable information on the linear stretch of amino acids that form an interaction site and allows identification of lead peptides that have the capacity or tendency to regulate gene expression. Lead peptides can be modified to modulate their capacity or tendency to regulate gene expression, which can be easily assayed in an in vitro bioassay such as a reporter assay. For example, some amino acid at some position can be replaced with another amino acidsof similar or different properties. Alanine (Ala)-replacement scanning, involving a systematic replacement of each amino acid by an Ala residue, is a suitable approach to modify the amino acid composition of a lead peptide when in a search for a signaling molecule capable of modulating gene expression. Of course, such replacement scanning or mapping can be undertaken with amino acids other than Ala as well, for example with D-amino acids. In one embodiment, a peptide derived from a naturally occurring polypeptide is identified as being capable of modulating gene expression of a gene in a cell. Subsequently, various synthetic Ala-mutants of this lead peptide are produced. These Ala-mutants are screened for their enhanced or improved capacity to regulate expression of a gene compared to lead polypeptide.

Furthermore, a lead peptide, or a modification or analogue thereof, can be chemically synthesised using D- and / or L-stereoisomers. For example, a lead peptide that is a retro-inverso of an oligopeptide of natural origin is produced. The concept of polypeptide retro-inversion (assemblage of a natural L-amino acid-containing parent sequence in reverse order using D-amino acids) has been applied successfully to synthetic peptides. Retro-inverso modification of peptide bonds has evolved into a widely used peptidomimetic approach for the design of novel bioactive molecules which has been applied to many families of biologically active peptide. The sequence, amino acid composition and length of a peptide will influence whether correct assembly and purification are feasible. These factors also determine the solubility of the final product. The purity of a crude peptide typically decreases as the length increases. The yield of peptide for sequences less than 15 residues is usually satisfactory, and such peptides can typically be made without difficulty. The overall amino acid composition of a peptide is an important design variable. A peptide's solubility is strongly influenced by composition. Peptides with a high content of hydrophobic residues, such as Leu, Val, Ile, Met, Phe and Trp, will either have limited solubility in aqueous solution or be completely insoluble. Under these conditions, it can be difficult to use the peptide in experiments, and it may be difficult to purify the peptide if necessary. To achieve a good solubility, it is advisable to keep the hydrophobic amino acid content below 50% and to make sure that there is at least one charged residue for every five amino acids. At physiological pH Asp, Glu, Lys, and Arg all have charged side chains. A single conservative replacement, such as replacing Ala with Gly, or adding a set of polar residues to the N- or C-terminus, may also improve solubility. Peptides containing multiple Cys, Met, or Trp residues can also be difficult to obtain in high purity partly because these residues are susceptible to oxidation and/or side reactions. If possible, one should choose sequences to minimize these residues. Alternatively, conservative replacements can be made for some residues. For instance, Norleucine can be used as a replacement for Met, and Ser is sometimes used as a less reactive replacement for Cys. If a number of sequential or overlapping peptides from a protein sequence are to be made, making a change in the starting point of each peptide may create a better balance between hydrophilic and hydrophobic residues. A change in the number of Cys, Met, and Trp residues contained in individual peptides may produce a similar effect. In another embodiment of the invention, a lead peptide capable of modulating gene expression is a chemically modified peptide. A peptide modification includes phosphorylation (e.g on a Tyr, Ser or Thr residue), N-terminal acetylation, C-terminal amidation, C-terminal hydrazide, C-terminal methyl ester, fatty acid attachment, sulfonation (tyrosine), N-terminal dansylation, N-terminal succinylation, tripalmitoyl-S-Glyceryl Cysteine (PAM3 Cys-OH) as well as farnesylation of a Cys residue. Systematic chemical modification of a leadpeptide can for example be performed in the process of leadpeptide optimalization.

Synthetic peptides can be obtained using various procedures known in the art. These include solid phase peptide synthesis (SPPS) and solution phase organic synthesis (SPOS) technologies. SPPS is a quick and easy approach to synthesize peptides and small proteins. The C-terminal amino acid is typically attached to a cross-linked polystyrene resin via an acid labile bond with a linker molecule. This resin is insoluble in the solvents used for synthesis, making it relatively simple and fast to wash away excess reagents and byproducts.

Generally, an amino acid consists of a central carbon atom (called the a-carbon) that is surrounded by four other groups: a hydrogen, an amino group, carboxyl group, and a side chain group. The side chain group, designated R, defines the different structures of the amino acids. Certain side chains contain functional groups that can interfere with the formation of the amide bond. Therefore, it is important to mask the functional groups of the amino acid side chain. The N-terminus can be protected with a Fmoc or Boc group, which is stable in acid, but removable by base. Any side chain functional groups are protected with base stable, acid labile groups. To begin each coupling, the masking group on the resin bound amino acid/peptide is removed with 20% piperidine in N,N-dimethyl formamide (DMF). It is then rinsed and a protected amino acid is added which has been activated at its 'alpha' carboxyl group. The activation is achieved by creating the N-hydroxybenzotriazole (HOBt) ester in situ. The activated amino acid and the resin bound amico acid are allowed to react in the presence of base to form a new peptide bond. This process is repeated until the desired peptide is assembled at the resin. Once the peptide is complete, it is ready to be cleaved from the resin. This is accomplished using a mixture of trifluoroacetic acid (TFA) and scavangers. Scavangers serve to neutralize cations which are formed during the removal of the side chain protecting groups. The solution is at least 82% TFA, and the rest a mixture of phenol, thioanisol, water, ethanedithiol (EDT), and triisopropylsilane (TIS). The lead peptide on the resin is allowed to react with the cleavage mixture for several hours, which then affords the peptide in solution. It can then be precipitated and washed in tert-butyl methyl ether, and analyzed or purified as desired.

An important link in any polypeptide chain is the amide bond, which is formed by the condensation of an amine group of one amino acid and a carboxyl group of another. The replacement of key amide bonds in peptide fragments by isosteric groups has recently received considerable attention as a possible means of generating novel bio-active substances with improved stability. In one embodiment of the invention, an lead peptide comprises a synthetic molecule in which at least one amide bond has been replaced by an isosteric group such as a ketomethylene or a trans-alkene group. Classically, well-defined molecules were systematically modified and the product compounds analyzed for improved biological activity. Newer combinatorial chemistry methods allow the synthesis of a large population of similar compounds. This is generally followed by the selection, or screening, of peptides for biological activity such as the capacity to regulate gene expression. In one embodiment of the invention, lead peptides are synthesized in a random fashion using a combinatorial chemistry approach. Combinatorial chemistry, combined with recent advances in robotic screening, enables the testing of a large number of compounds in a short period of time. This technique involves the preparation of a large number of structurally related compounds either as mixtures in the same reaction vessel or individually by parallel synthesis. In this manner large pools of similar compounds can be synthesized within a short period of time. Combinatorial libraries can be prepared using both solution chemistry and by solid phase synthesis; however, solid phase synthesis allows the use of excess reagents to drive the reaction to completion and easy removal of the reagents and side-products by simple filtration of the polymeric support and washing with solvent. Therefore, solid phase synthesis offers a more attractive approach to the generation of chemical libraries for screening purposes.

Combinatorial chemistry is well suited to peptides. Lead peptide libraries can be easily synthesized using solid-phase chemistry. Sequence degeneracy can be incorporated during the synthesis using either split synthesis or parallel synthesis. In the split synthesis approach, the solid support is divided into portions prior to each coupling step. A different molecular unit (synthon), like an amino acid, is then coupled to each portion. All portions are recombined after coupling and the synthesis cycle is completed. This "split and mix" approach has the advantage of yielding a unique sequence on each support bead and variability in synthon reactivity can be corrected by varying the coupling conditions. Peptide synthesis, where variations in reactivity between amino acids are significant, requires the "split and mix" approach. Head-to-tail cyclization of peptides on the resin provides a facile route to cyclic compounds. In addition to general advantages of solid phase synthesis, such as high efficiency and easy purification, head-to-tail cyclization of peptides on polymer supports provides minimal risk of intermolecular reactions (e.g., dimerization and oligomerization), even under high concentration. This is another advantage over solution chemistry which requires high dilution conditions to avoid intermolecular side reactions of the linear peptide.

In one embodiment, a method is provided for identifying or obtaining a signaling molecule comprising a peptide or functional derivative or analogue thereof capable of modulating expression of a gene in a cell comprising providing said cell with a peptide or functional derivative or analogue thereof and determining the activity and/or nuclear translocation of a gene transcription factor.

The thus developed chemical entity can be administered and introduced in-vivo systemically, topically, or locally. The peptide, or is modification or derivative, can be administered as the entity as such or as a pharmaceutically acceptable acid- or base-addition salt, formed by reaction with an inorganic acid (such as hydrochloric acid, hydrobromic acid, perchloric acid, nitric acid, thiocyanic acid, sulfuric acid, and phosphoric acid); or with an organic acid (such as formic acid, acetic acid, propionic acid, glycolic acid, lactic acid, pyruvic acid, oxalic acid, malonic acid, succinic acid, maleic acid, and fumaric acid); or by reaction with an inorganic base (such as sodium hydroxide, ammonium hydroxide, potassium hydroxide); or with an organic base (such as mono-, di-, trialkyl and aryl amines and substituted ethanolamines). A selected peptide and any of the derived entities may also be conjugated to sugars, lipids, other polypeptides, nucleic acids and PNA; and function in-situ as a conjugate or be released locally after reaching a targeted tissue or organ.
Going back to lead peptide detection, it is for example possible to generate such a lead peptide library at a purely random basis, the library comprising small peptide fragments as test entities (herein also called lead peptides) preferably of about 3 to 15 amino acids in length (more preferably 4 to 9, or even better 4 to 6) for each and every combination of amino acids or amino acid derivatives known, and then contacting each of said lead peptides, or a modification or derivative thereof, with at least one cell, and than determining the presence of at least one gene product in or derived from said cell.
It is however preferred to start with a more selective peptide library wherein for example lead peptides are selected on the basis of their occurence in endogenous (host or pathogen) proteins sequences from which (preferably 4 to 6 amino acids long) lead peptides are predicted and then synthesized on the basis of proteolytic site prediction. Several of such models exist, it is for example preferred to use a computer model allowing the prediction of a MHC-I or MHC-II specific proteolytic breakdown sequences. In yet another example of such a peptide library, lead peptides to be tested in said library are derived from a protein sequence by selecting and synthesizing peptide fragments in an overlapping fashion from the protein in question (preferably short fragments of 4 to 5 amino acids long considering the amount of work involved when testing longer peptide sequences in an overlapping fashion), whereby the overlap can for example be 1, 2 or 3 amino acids or whereby all but 1 amino acid overlap in the consecutive sequences. Even more preferred is a method whereby the peptide library is composed of lead peptides that are derived by first selecting longer peptide sequences under guidance of proteolytic site prediction from a protein, as above, and from those longer sequences designing 4 to 6 amino acids long peptide fragments that are derived in an overlapping fashion from the predicted longer sequences.

A further non-limiting list of proteins from which peptide secquences may be derived for further testing as lead peptide includes collagen, PSG, CEA, MAGE (malanoma associated growth antigen), Thrombospondin-1, Growth factors, MMPs, Calmodulin, Olfactory receptors, Cytochrome p450, Kinases, Von Willebrand factor (coagulation factors), Vacuolar proteins (ATP sythase), Glycoprotein hormones, DNA polymerase, Dehydrogenases, Amino peptidases, Trypsin, Viral proteins (such as envelope protein), Elastin, Hibernation associated protein, Antifreeze glycoprotein, Proteases, Circumsporozoite, Nuclear receptors, Transcription actors, Cytokines and their receptors, Bacterial antigens, Nramp, RNA polymerase, Cytoskeletal proteins, Hematopoietic (neural) membrane proteins, Immunoglobulins. HLA/MHC, G-coupled proteins and their receptors, TATA binding proteins, Transferases, Zinc finger protein, Spliceosmal proteins, HMG (high mobility group protein), ROS (reactive oxygen species), superoxidases, superoxide dismutase, Proto-oncogenes/tumor suppressor genes, Apolipoproteins

A further method is provided for obtaining information about the capacity or tendency of an lead peptide, or a modification or derivative thereof, to regulate gene expression wherein information is obtained using microarray technology. Microarray technology makes use of the sequence resources created by genome sequencing projects and other sequencing efforts to answer the question, what genes are expressed in a particular cell type of an organism, at a particular time, under particular conditions. Microarrays exploit the preferential binding of complementary single-stranded nucleic acid sequences. Microarrays allow a systematic examination of the gene expression profile in cells. There are several names for this technology - DNA microarrays, DNA arrays, DNA chips, gene chips, and others. A microarray is typically a glass (or some other material) slide, onto which nucleic acid molecules, such as DNA or RNA, are attached at fixed locations (spots). There may be tens of thousands of spots on an array, each containing a huge number of identical DNA molecules (or fragments of identical molecules), of lengths from twenty to hundreds of nucleotides. For gene expression studies, each of these molecules ideally should identify one gene or one exon in the genome. The spots are either printed on the microarrays by a robot, or synthesized by photo-lithography (similarly as in computer chip productions) or by inkjet printing.
In one embodiment of the invention, microarray technology is used to determine the capacity of a peptide to control the relative upregulation and/or downregulation of at least one gene in a cell. Also, a method is provided to exploit microarray technology to determine the modulatory effect of one or more lead peptides or derivatives thereof on the up-regulation and/ or downregulation of a multitude of genes expressed in a cell. In a further embodiment, an lead peptide with the desired activity, as determined in a for example microarray, is synthesized. There are several ways how microarrays can be used to measure gene expression levels. One of the most popular microrarray applications allows to compare gene expression levels in two different samples, e.g., the same cell type in a non-treated and a treated condition. The total mRNA from the cells in two different conditions is extracted and labelled with two different dyes: for example a green dye for cells at condition 1 and a red dye for cells at condition 2 (to be more accurate, the labelling is typically done by synthesizing single stranded DNAs that are complementary to the extracted mRNA by an enzyme called reverse transcriptase). Both extracts are washed over the microarray. Labelled gene products from the extracts hybridise to their complementary sequences in the spots due to the preferential binding - complementary single stranded nucleic acid sequences tend to attract to each other and the longer the complementary parts, the stronger the attraction. The dyes enable the amount of sample bound to a spot to be measured e.g. by the level of fluorescence emitted when a fluorescent dye is excited by a laser. If the RNA from the sample in condition 1 is in abundance, the spot will be green, if the RNA from the sample in condition 2 is in abundance, it will be red. If both are equal, the spot will be yellow, while if neither are present it will not fluoresce and appear black. Thus, from the fluorescence intensities and colours for each spot, the relative expression levels of the genes in both samples can be estimated.

As is exemplified in the detailed description, microarray technology is an attractive approach to monitor the capacity of an lead peptide to upregulate or downregulate gene expression in a cell. A typical experiment comprises a series of parallel samples, each sample containing at least one cell that is provided with a different lead peptide, or a modification or derivative thereof. Also included is a control sample containing at least one cell but no lead peptide. A cell can be a primary cell, such as a peripheral blood mononuclear cell (PBMC) or a cell derived from a laboratory cell line such as a Jurkat, COS-7, MCF-7, 293T cell. At a certain time period following addition of a peptide to a sample, an aliquot is taken. From each aliquot containing a cell that is incubated during a certain time period in the presence of an lead peptide, an inventory is made of (the clusters of) induced and repressed genes using microarray technology. This time period can be 3 hours, or a shorter time period such as 2 hours or 1 hour following addition. Remarkably, even shorter time periods can be chosen to determine the modulatory effect of a peptide or derivative or analogue thereof on gene expression, like 30 minutes or even less than 20 minutes.

The detectable effect of lead peptides according to the invention on gene control is surprisingly fast when compared to other regulators of gene transcription, which typically induce detectable changes in gene expression upon prolonged incubation times in the range of several, e.g. 6-24 hours. In a preferred embodiment, a cell is first exposed to a compound known to alter the gene expression program in a cell and subsequently provided with a peptide according to the invention. Compounds include receptor agonists, receptor antagonists and other compounds known to induce altered gene transcription. Also included are compounds which mimic intracellular signals that occur during natural responses to receptor agonists or antagonists, for example a combination of ionomycin and PMA. In another embodiment, a cell is contacted with a microbial agent such as bacterial lipopolysaccharide (LPS) or even intact bacteria (e.g. *Escherichia coli, Bortadella pertussis, Staphylococcus aureus*) to induce an immune response. A typical experiment involves a series of parallel samples comprising at least one cell, wherein each sample provided with a different lead peptide, or a modification or derivative thereof, in combination with a compound known to alter the gene expression program in said cell. In another embodiment, such a compound and a peptide are added to a cell simultaneously. In yet another embodiment, a peptide or a functional derivative thereof is added to a cell prior to or after providing a cell with a compound known to alter the gene expression profile in a cell. Then, an inventory is made of induced and repressed genes using a microarray. From this inventory, the modulatory effect of a peptide on gene control can be readily determined.

Provided is also a method for identifying or obtaining a signalling molecule comprising a peptide or a functional derivative or analogue thereof capable of modulating expression of a gene in a cell comprising providing said cell with a peptide or a derivative or analogue thereof and determining the activity of a gene transcription factor. In one embodiment of the invention, a signalling molecule comprising a peptide or functional derivative or analogue thereof capable of modulating gene expression is identified using a reporter gene assay. Reporter genes are generally nucleic acid sequences encoding easily assayed proteins. They are used to replace other coding regions whose protein products are more difficult to assay. A reporter gene is fused downstream of a promotor of interest, so that transcripts initiating at the promotor proceed through the reporter gene. Commonly used reporter genes encode enzymes such as chloramphenicol acetyltransferase (CAT), beta-galactosidase, beta-glucuronidase and luciferase. Interesting reporter genes also comprise fluorescent proteins which fluoresce on irradiation with UV. These include green fluorescent protein (GFP) and spectral variants thereof, such as yellow fluorescent protein (YFP), red fluorescent protein (RFP) and cyano fluorescent protein (CFP). Reporter genes can be attached to other sequences so that only the reporter protein is made or so that the reporter protein is fused to another protein (fusion protein). Reporter genes can "report" many different properties and events: the strength of promoters, whether native or modified for reverse genetics studies; the efficiency of gene delivery systems; and the efficiency of translation initiation signals. A reporter gene construct can be transfected into a cell, e.g. a laboratory cell line, using one of the many transfection techniques known in the art including those using DEAE-dextran, calcium phosphate precipitation, adenovirus-or retrovirus-mediated gene transduction, cationic liposome transfection systems (e.g. using Lipofectin, Lipofectamine, DOTAP or Fugene reagent) and electroporation techniques. Mixing a liposomal transfection reagent with DNA results in spontaneously formed stable complexes that can directly be added to the tissue culture medium with or without serum. These complexes adhere to the cell surface, fuse with the cell membrane and release the DNA into the cytoplasm. This method of DNA transfer is very gentle, avoiding cytotoxic effects, so that cells can be transfected with high efficiency. Transfection of a cell with a reporter gene construct can be transient or stable. Provided is a method to determine the modulatory effect of an lead peptide on gene expression by exposing a transfected cell to an lead peptide according to the invention, or a mixture thereof, and assaying for reporter gene activity in said cell. The modulatory effect can be a inhibitory or a stimulatory effect. In a preferred embodiment, a reporter gene assay is used to assay for NFkappaB activity, but reporter gene assays designed to assay the activity of one or more other transcription factors may also be used. A luciferase reporter gene construct can be placed under the control of an NFkappaB-driven promotor. Transfected cells are exposed to a series of lead peptides, such as peptide fragments of different lengths derived from naturally occurring polypeptides or synthetic peptides in which amino acids are systematically replaced e.g. by Ala residues or D-amino acids. After a certain incubation time, luciferase activity is assayed to determine the effect of an lead peptide on NFkappaB activity. From this analysis, it is clear whether a peptide has any gene modulatory effect and, if so, whether this effect is inhibitory or stimulatory.
Reporter gene assays allow analysis of a large number of different samples in a relatively short time. It can easily be performed using multiwell plates such as 96-well plates. The activity of many reporter genes can be assayed using colorimetric or fluorescense detection in for example an automated plate reader. Thus, a reporter gene assay can be used in a high -throughput format. This is especially advantageous when performing several rounds in screening for gene regulatory effects of a peptide, for example in the process of lead peptide optimalization. In these types of assays, it is preferred to focus on a small number of different promotor elements. For example, cells can be transfected with a reporter gene construct for the detection of NFkappaB activity, or with a reporter gene construct for AP-1 activity or with a reporter construct designed to readily determine NFAT-1 activity. Cells can be transfected in parallel with one reporter gene construct but it also possible to provide a cell with more than one reporter gene construct. Of course, to allow discrimination between activities of the different promotors, it is preferred that each promotor construct contains a different reporter gene. In one embodiment of the invention, a cell is provided with more than one reporter gene construct to determine the effect of a peptide or a derivative or analogue thereof on transcriptional activity. For example, a cell is co-transfected with two or even three different plasmids, each containing a distinct fluorescent reporter gene fused downstream of a distinct promotor of interest. From this, the effect of said peptide on each promotor is determined. In such an experimental set-up, it is obviously preferred that reporter gene products of the co-transfected reporter constructs are easily distinguished from each other. Interesting reporter genes that can be used in co-transfection reporter gene assays include GFP or EGFP (enhanced green fluorescence protein) and spectral variants thereof, such as RFP, YFP and CFP. Following exposure of said cell to a peptide according to the invention, the activities of each fluorescent reporter gene is measured by fluorescence detection using a suitable optical filter set, like a multi-band filter set. Multi-band sets are used for multiple labelling and simultaneous viewing of multiple fluorophores. Each set of exciters, dichroics, and emitters yields isolated bands of excitation and emission energy.

To detect an effect of an lead peptide on gene expression, especially to detect an inhibitory effect, it is preferred to have a significant level of basal gene transcriptional activity in a cell. To facilitate detection of an inhibitory effect of a peptide on gene expression, a cell can be treated with a compound known to induce a profound increase in gene expression. This compound can be added before, after or at the same time at which a cell is provided with an lead peptide. For instance, a cell containing a luciferase reporter gene under the control of an NF-kappaB-driven promotor is exposed to LPS. This will induce an increase in luciferase activity compared to untreated control cells, in which there may only be a low basal level of NF-kappaB-dependent transcriptional activity. Subsequently, or simultaneously, at least one peptide suspected of being capable to modulate gene expression is added. Then, the effect of said peptide on LPS-induced luciferase activity is assayed and compared to the luciferase activity in a parallel sample comprising cells which only received LPS but no peptide.

Using a method for identifying or obtaining a signalling molecule comprising a peptide or functional derivative or analogue thereof capable of modulating expression of a gene in a cell comprising providing said cell with a peptide or derivative or analogue thereof and determining the activity and/or nuclear translocation of a gene transcription factor as provided herein furthermore allows at random testing of a multiplicity of oligo- or leadpeptides, leading to automated combinatorial chemistry formats, wherein a great many of candidate signal molecules are being tested in a (if so desired at random) pattern for their reactivity with a molecular library of synthetic peptides representing potential signal molecules allowing the rapid detection of particularly relevant molecules out of tens of thousands of (combinations of) molecules tested. In a preferred embodiment, the invention provides a method wherein said lead peptides, or at least their activities, are positionally or spatially addressable, e.g. in an array fashion, if desired aided by computer directed localisation. In an array, said pluralities are for example addressable by their positions in a grid or matrix.

Also provided herein is a method for identifying or obtaining a signalling molecule comprising a peptide or a functional derivative or analogue thereof capable of modulating expression of a gene in a cell comprising providing said cell with a lead peptide or a derivative or analogue thereof and determining the nuclear translocation of a gene transcription factor. Cytoplasm to nucleus translocation is an early measure of transcription factor activation. It is regarded to be a critical step in the coupling of extracellular stimuli to the transcriptional activation of specific target genes. Various methods are available to a person skilled in the art to analyse the subcellular localization of a transcription factor nuclear translocation of a transcription factor. Conventional techniques to analyse the presence of a transcription factor in a nuclear fraction include EMSA (electrophoretic mobility shift assay) and immunocytochemistry. It is also possible to follow the translocation of a fluorescently-tagged transcription factor, such as GFP-NFkB, using fluorescence microscopy. However, to assay a large number of samples for the ability of an lead peptide to modulate nuclear translocation of a transcription factor, it is preferred to use a less elaborate approach. For example, commercial kits ('translocation kits') have recently become commercially available (Cellomics, Inc; www.cellomics.com) which can be used to conveniently measure the cytoplasm to nucleus translocation of a transcription factor. The assay involves detection of a transcription factor by a specific primary antibody, followed by detection of said primary antibody by a fluorochrome-conjugated secondary antibody. Translocation kits are available to measure activation of various transcription factors, including NF-kappaB, STAT and ATF-2. Together with specialized software and instrumentation, a kit comprises a fully automated screen to identify compounds, such as peptides or derivatives thereof, that inhibit or induce transcriptional activation on a cell-by-cell basis. Assays are performed in standard, high-density microplates, where measurements of the rate and extent of transcription factor translocation are made in intact cells, providing biologically representative information. Kits are available in various sizes. A kit containing reagents for 480 assays can for instance be used in a phase of evaluating the gene modulatory activity of a relatively small amount (like in the range of 10 to 15 ) of peptide fragments derived from a naturally occurring polypeptide. This can result in the identification of a few leadpeptides which can modulate the activity of a gene transcription factor. In a subsequent round of screening, such a lead peptide is used for the development of more effective derivatives or homologues. In such a process of lead peptide optimalization, a kit may be used that contains reagents for 4800 individual test samples. In a further embodiment, a kit containing reagents for 19200 individual test samples is used for the screening of a library of synthetic peptides, for example for determining the modulatory effect of thousand of random peptides generated by combinatorial chemistry. A kit combines fluorescent reagents and protocols for optimized sample preparation and assays, and requires no cell lysis, purification, or filtration steps. After fixation, the plates are stable for extended periods, ranging from one week to several months, depending on the cell type and dye, when stored light-protected at 4°C.

The present invention also has a variety of other different applications and uses. Of clinical and medical interest and value, the present invention provides the opportunity to selectively control NFκB-dependent gene expression in tissues and organs in a living subject, preferably in a primate, allowing upregulating essentially anti-inflammatory responses such as IL-10, and downregulating essentially pro-inflammatory responses such as mediated by TNF-alpha, nitric oxide (NO), IL-5, IL-1beta. The invention thus provides use of a NFκB regulating peptide or derivative thereof for the production of a pharmaceutical composition for the treatment of an ischhemic event in a primate, and provides a method of treatment of an ischemic event in a primate. In one such instance as provided herein, such a subject has suffered from ischemic events or has undergone anoxia or infarction. A typical clinical instance is the myocardial infarction or chronic myocardial ischemia of heart tissue in various zones or areas of a living human subject, or, likewise a cerebrovascular infarct.
In response to a variety of pathophysiological and developmental signals, the NFkB/Rel family of transcription factors are activated and form different types of hetero- and homodimers among themselves to regulate the expression of target genes containing kappaB-specific binding sites. NF-kB transcription factors are hetero- or homodimers of a family of related proteins characterized by the Rel homology domain. They form two subfamilies, those containing activation domains (p65-RELA, RELB, and c-REL) and those lacking activation domains (p50, p52). The prototypical NFkB is a heterodimer of p65 (RELA) and p50 (NF-kB1). Among the activated NFkB dimers, p50-p65 heterodimers are known to be involved in enhancing the transcription of target genes and p50-p50 homodimers in transcriptional repression. However, p65-p65 homodimers are known for both transcriptional activation and repressive activity against target genes. KappaB DNA binding sites with varied affinities to different NFB dimers have been discovered in the promoters of several eukaryotic genes and the balance between activated NFkB homo- and heterodimers ultimately determines the nature and level of gene expression within the cell. The term "NFkB-regulating peptide" as used herein refers to a peptide or a modification or derivative thereof capable of modulating the activation of members of the NFkB/Rel family of transcription factors. Activation of NFkB can lead to enhanced transcription of target genes. Also, it can lead to transcriptional repression of target genes. NFkB activation can be regulated at multiple levels. For example, the dynamic shuttling of the inactive NFkB dimers between the cytoplasm and nucleus by IkappaB proteins and its termination by phosphorylation and proteasomal degradation, direct phosphorylation, acetylation of NFkB factors, and dynamic reorganization of NFkB subunits among the activated NFkB dimers have all been identified as key regulatory steps in NFkB activation and, consequently, in NFkB-mediated transcription processes. Thus, a NFkB-regulating peptide is capable of modulating the transcription of genes that are under the control of NFkB/Rel family of transcription factors. Modulating comprises the upregulation or the downregulation of transcription.

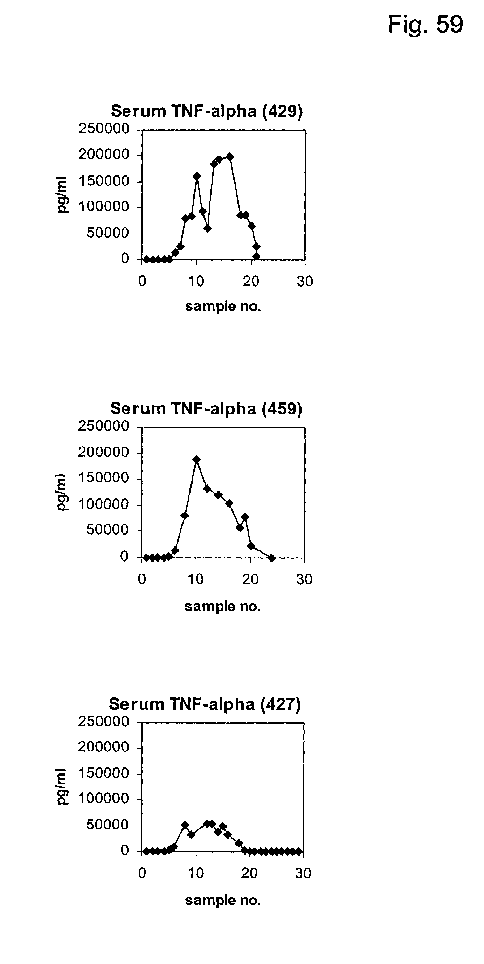

Provided also is a method for treating an acute or chronic inflammatory disease comprising administering to a subject in need of such a treatment a molecule comprising an oligopeptide, a peptide or a functional analogue thereof. Of particular importance, the present invention now provides a peptide that is capable of modulating the production of cytokines in primates. This is exemplified in Figure 59-65, showing a decrease in pro-inflammatory cytokines such as TNF-alpha, Il-1beta, IL-8, IL-6 and IL-5 and an increase in the production of the anti-inflammatory cytokine IL-10 (Fig 59-65).

Preferably, such a peptide is 3 to 15 amino acids long, and capable of modulating the expression of a gene, such as a cytokine, in a cell. In a preferred embodiment, a peptide is a signaling molecule that is capable of traversing the plasma membrane of a cell or, in other words, a peptide that is membrane-permeable. Also, a useful peptide for treating an acute or chronic inflammatory disease comprising is a peptide capable of reducing the production NO and / or TNF alpha by a cell. A reduction in the production of NO and / or TNF alpha in a cell can be achieved by inhibiting a transcription factor of the NF-kB family. This inhibition occur at several different levels, including direct binding of a peptide to a transcription factor. Also, a peptide as provided herein can inhibit the nuclear translocation of a peptide, as is shown in the detailed description.
Use of a NFkB-regulating peptide is provided, or a mixture of at least two of such peptides, for the treatment of disease that affect or is affected by the NF-kB pathway. In a preferred embodiment, a peptide according to the invention is suitably used in a strategy to modulate the production of one or more cytokines in a cell. Specifically attractive is the use of a peptide for the production of a pharmaceutical composition to inhibit the production of a cytokine in a cell, for example via the suppression of cytokine expression that is under the control of a transcription factor of the NF-kB/Rel family. For example, use of a NFkB-regulating peptide for the production of a pharmaceutical composition is provided for the treatment of sepsis. Furthermore, use of a NFkB-regulating peptide for the production of a pharmaceutical composition for the treatment of anthrax, for instance via the modulation of the production of inflammatory cytokines such as interleukin or via reducing the production of NO and / or TNF alpha in a cell. Seemingly unrelated disorders such as asthma, rheumatoid arthritis, inflammatory bowel disease, multiple sclerosis, chronic obstructive pulmonary disease, allergic rhinitis and cardiovascular disease all have inflammatory elements. As mentioned before, NF-KB is a master regulator of a broad set of inflammatory genes, including TNF, IL-1 and cell adhesion molecules, which give rise to immune-inflammatory diseases. Rheumatoid arthritis (RA) is a prototype of chronic inflammatory disease. Studies in animal models of RA demonstrated crucial involvement of NF-kB in regulation of inflammation, apoptosis, and proliferation in the arthritic synovium. Thus, NF-k.B emerges as very attractive target for therapeutic intervention in RA and other chronic inflammatory conditions. A logical way to inhibit NF-kB activation is to modulate the signaling cascades which controls transcriptional activity of NF-kB. Remarkably, as is exemplified in the detailed description, treatment of mice with a peptide according to the invention could prevent mice from development of arthritis and even profoundly decreased the severity of arthritis. Thus, the invention provides a method for treating arthritis, or another immune-inflammatory disease, comprising administering to a subject in need of such a treatment a molecule comprising an oligopeptide, a peptide or a functional analogue thereof, wherein said molecule is capable of modulating NF-kB activity, for example leading to a decreased production of NO and / or TNF alpha by a cell. In another embodiment, a NFkB-regulating peptide, or a peptide capable of regulating another type of transcription factor, is used for the production of a pharmaceutical composition for the treatment of a bone disease. In normal bone remodeling, osteoclast and osteoblast activity are coupled such that resorbed bone is entirely replaced by new bone tissue. Bone disease is often characterized by a disturbance in this balance such that there is a net increase in bone resorption over bone formation. Fully mature functional osteoclasts generally produce a variety of cytokines and growth factors that enhance osteoclast formation, activity, and/or survival. These include IL-1 a and b, IL-6, IL-11, M-CSF, and TNF-a. The invention now provides a method to control, preferably to inhibit, osteoclast differentation and maturation. Therewith, the production of cytokines by osteoclasts can be controlled. Provided is the use of a NFkB-regulating peptide for the production of a pharmaceutical composition to correct a disturbed balance between bone resorption and bone formation, for example by controlling the process of osteoclastogenesis. Such a pharmaceutical composition is advantageously applied to treat a disease which relates to increased osteoclastogenesis, such as (post-menopausal) osteoporosis and arthritis.

The present invention thus provides methods and means for specific site control of gene expression in NFκB-dependent cells involved in an ischemic event. Cardiovascular diseases are some of the leading killers of both men and women worldwide. Led predominantly by coronary heart disease and stroke, more than 60 million Americans have one or more types of cardiovascular disease including high blood pressure, congenital cardiovascular defects, and congestive heart failure. Cardiovascular disease kills more than 2,600 Americans each day and since 1900, has been the number one killer in the United States every year but 1918. Cardiovascular disease will cost the United States an estimated $329.2 billion. According to the Centers for Disease Control and Prevention (CDC), if all forms of major cardiovascular diseases were eliminated, life expectancy would rise seven years.

In a preferred embodiment, a peptide according to the invention, or a functional derivative or analogue thereof is used for the production of a pharmaceutical composition for the treatment of ischemic events. An ischemic event refers to an event in which the blood supply to a tissue is obstructed, such as stroke or myocardial infarction. Due to this obstruction, the endothelial tissue lining the affected blood vessels becomes "sticky" and begins to attract circulating white blood cells. The white cells bound to the endothelium eventually migrate into the brain or cardiac tissue, causing significant tissue destruction. Although neither acute myocardial infarction nor stroke is directly caused by inflammation, much of the underlying pathology and the damage that occurs after an acute ischemic event is caused by acute inflammatory responses during reperfusion, the restoration of blood flow to the affected organ. Thus, a method is provided herein for treating ischemic events, including cerebrovascular disease and ischemic heart failure, comprising administering to a subject in need of such a treatment a peptide acording to the invention. In particular, a method is provided to control the acute inflammatory response during reperfusion of the affected body part by administering a peptide, or a modification thereof, capable of modulating expression of a gene encoding a pro-inflammatory cytokine.

TNF-a is a pro-inflammatory and multifunctional cytokine that has been implicated in diverse pathological processes such as cancer, infection, and autoimmune inflammation. TNF-a has been recently detected in various cardiac-related illnesses including congestive heart failure, myocarditis, dilated and septic cardiomyopathy, and ischemic heart diseases. TNF mRNA and TNF-alpha protein were detected in explanted hearts from humans with dilated cardiomyopathy and ischemic heart disease, but TNF-a was not detected in nonfailing myocardium. Although the complete portfolio of signaling pathways that are common to both tumor necrosis factor receptor 1 (TNFR1) and tumor necrosis factor receptor 2 (TNFR2) is not known, it is of interest to note that a recently described zinc finger protein, termed tumor necrosis factor receptor associated factor 2 (TRAF2), has been shown to be involved with both TNFR1- and TNFR2-mediated signaling. Consequently, TRAF2-mediated signaling has been shown to activate NF-kB, with a resultant increase in the expression of the antioxidant protein manganese superoxide dismutase (MSOD). Previous studies suggested that the cytoprotective effects of TNF in the setting of myocardial ischemia were mediated through TNF-induced upregulation of MSOD. It was suggested that pro-inflammatory cytokines such as TNF may play an important role in the timing of cardiac stress response, both by providing early antiapoptotic cytoprotective signals that are responsible for delimiting cardiac injury and also by providing delayed signals that facilitate tissue repair and remodeling once myocardial damage has supervened. Given the observation that some peptides according to the invention are capable of upregulating at least one gene in a cell, the invention now provides a method to increase the expression of gene products such as MSOD and other cytoprotective NF-kB-regulated genes.

When taking ischamic heart failure as an example, a NFkappaB down-regulating peptide according to the invention can for example be introduced directly as a synthesized compound to living cells and tissues via a range of different delivery means. These include the following.
1. Intracoronary delivery is accomplished using catheter-based deliveries of synthesized peptide (or derivative) suspended in a suitable buffer (such as saline) which can be injected locally (i.e., by injecting into the myocardium through the vessel wall) in the coronary artery using a suitable local delivery catheter such as a 10mm InfusaSleeve catheter (Local Med, Palo Alto, CA) loaded over a 3.Omm x 20mm angioplasty balloon, delivered over a 0.014 inch angioplasty guidewire. Delivery is typically accomplished by first inflating the angioplasty balloon to 30 psi, and then delivering the protein through the local delivery catheter at 80 psi over 30 seconds (this can be modified to suit the delivery catheter).
2. Intracoronary bolus infusion of peptide (or derivative) synthesized previously can be accomplished by a manual injection of the substance through an Ultrafuse-X dual lumen catheter (SciMed, Minneapolis, MN) or another suitable device into proximal orifices of coronary arteries over 10 minutes.
3. Pericardial delivery of synthesized peptide (or derivative) is typically accomplished by installation of the peptide-containing solution into the pericardial sac. The pericardium is accessed via a right artrial puncture, transthoracic puncture or via a direct surgical approach. Once the access is established, the peptide material is infused into the pericardial cavity and the catheter is withdrawn. Alternatively, the delivery is accomplished via the aid of slow-release polymers such as heparinal-alginate or ethylene vinyl acetate (EVAc). In both cases, once the peptide (or derivative) is integrated into the polymer, the desired amount of peptide/polymer is inserted under the epicardial fat or secured to the myocardial surface using, for example, sutures. In addition, the peptide/polymer composition can be positioned along the adventitial surface of coronary vessels.
4. Intramyocardial delivery of synthesized peptide (orderivative) can be accomplished either under direct vision following thoracotomy or using thoracoscope or via a catheter. In either case, the peptide containing solution is injected using a syringe or other suitable device directly into the myocardium.
   Up to 2 cc of volume can be injected into any given spot and multiple locations (up to 30 injections) can be done in each patient. Catheter-based injections are carried out under fluoroscopic, ultrasound or Biosense NOGA guidance. In all cases after catheter introduction into the left ventricle the desired area of the myocardium is injected using a catheter that allows for controlled local delivery of the material.

A range of suitable pharmaceutical carriers and vehicles are known conventionally to those skilled in the art. Thus, for parenteral administration, the compound will typically be dissolved or suspended in sterile water or saline.

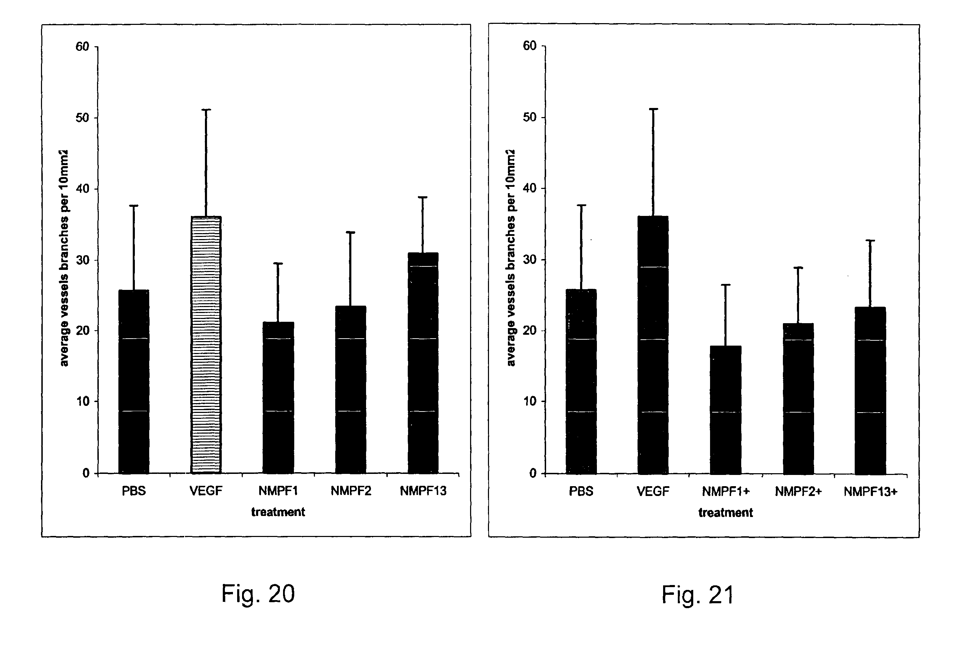

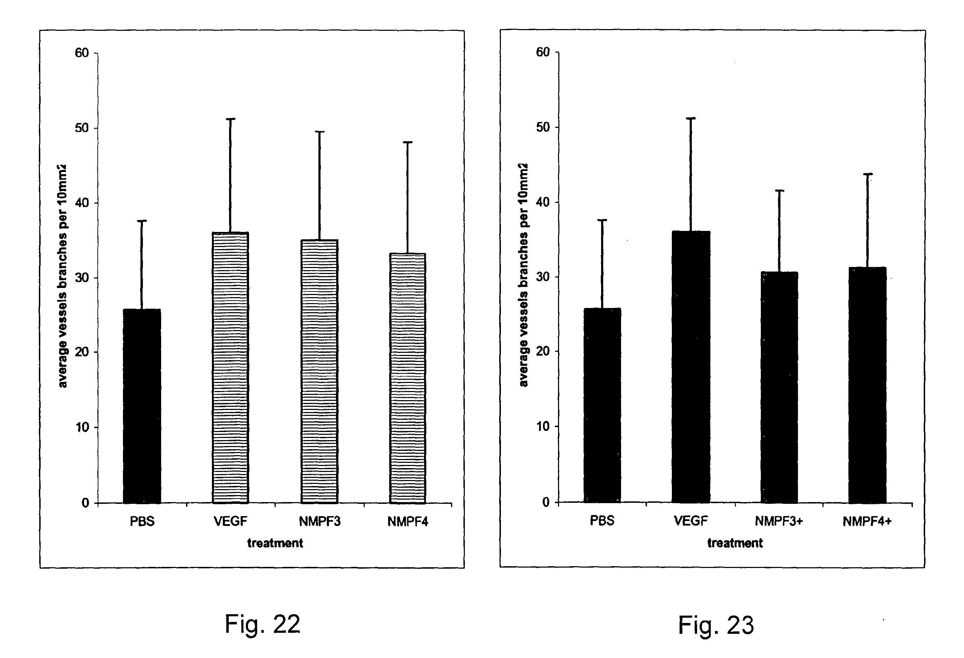

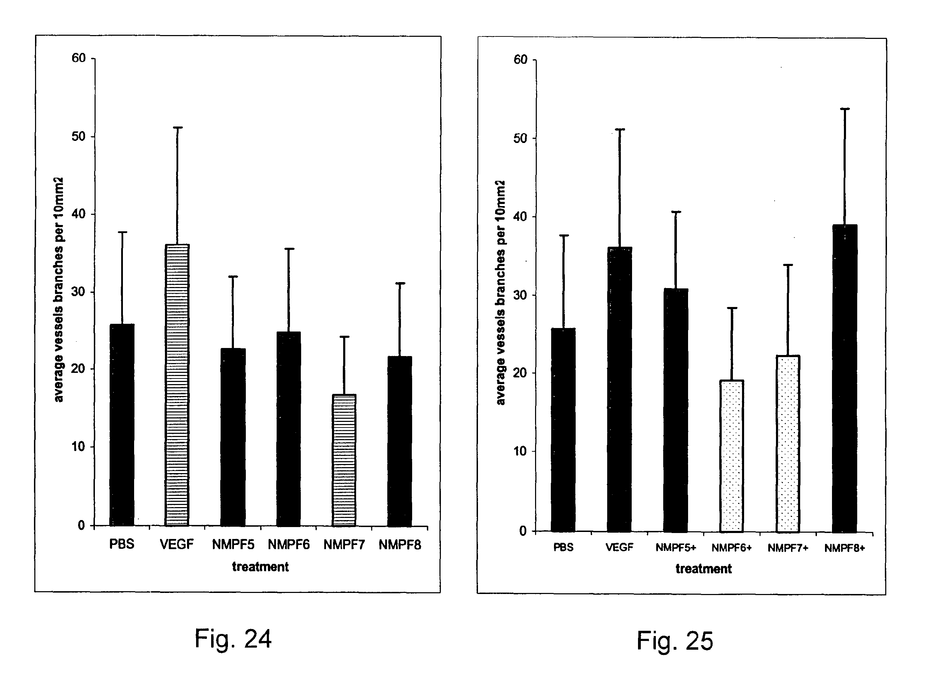

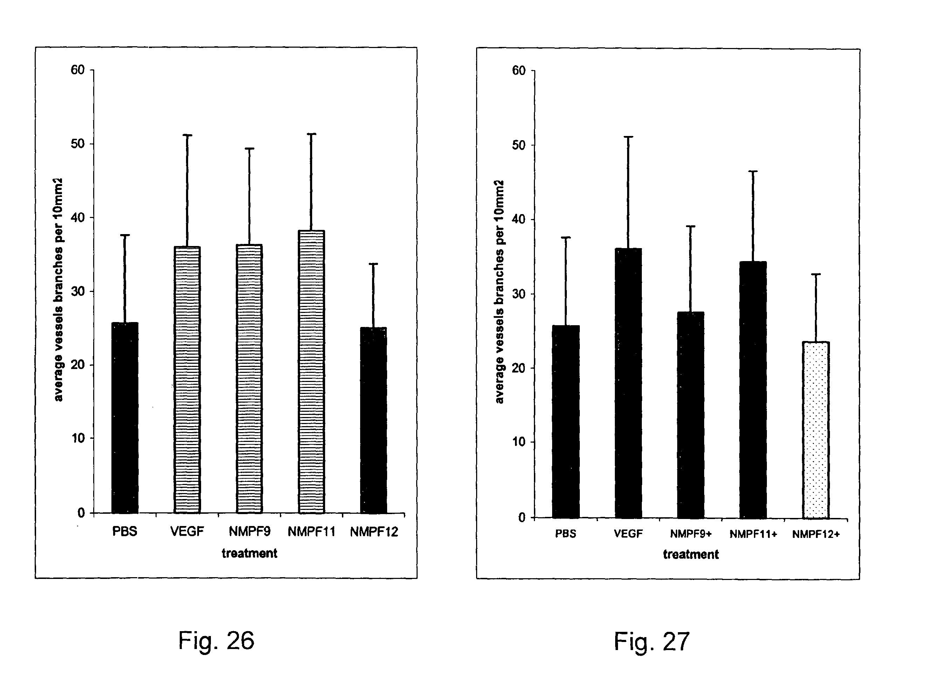

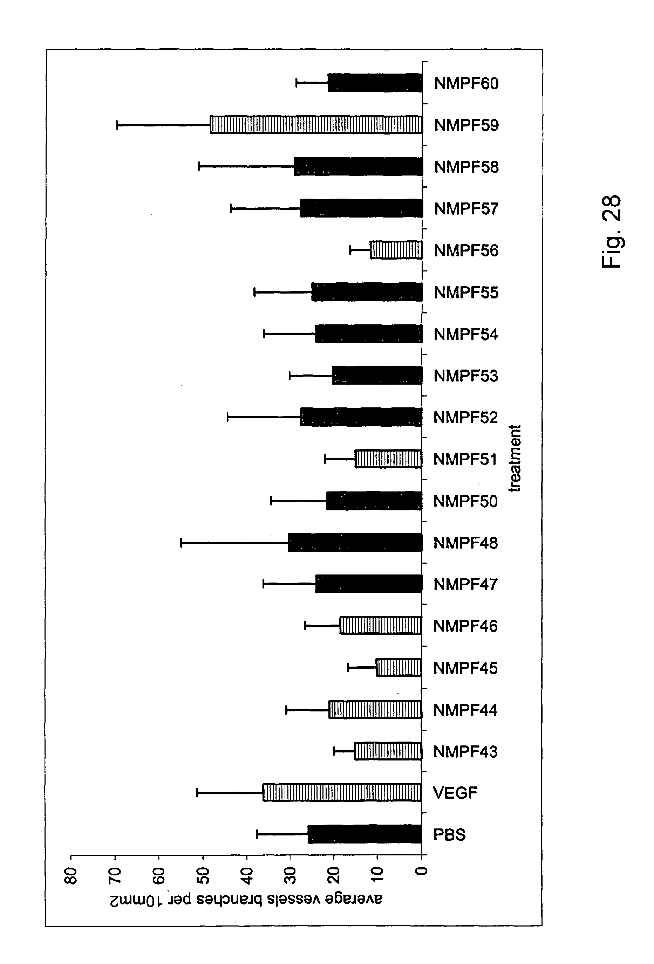

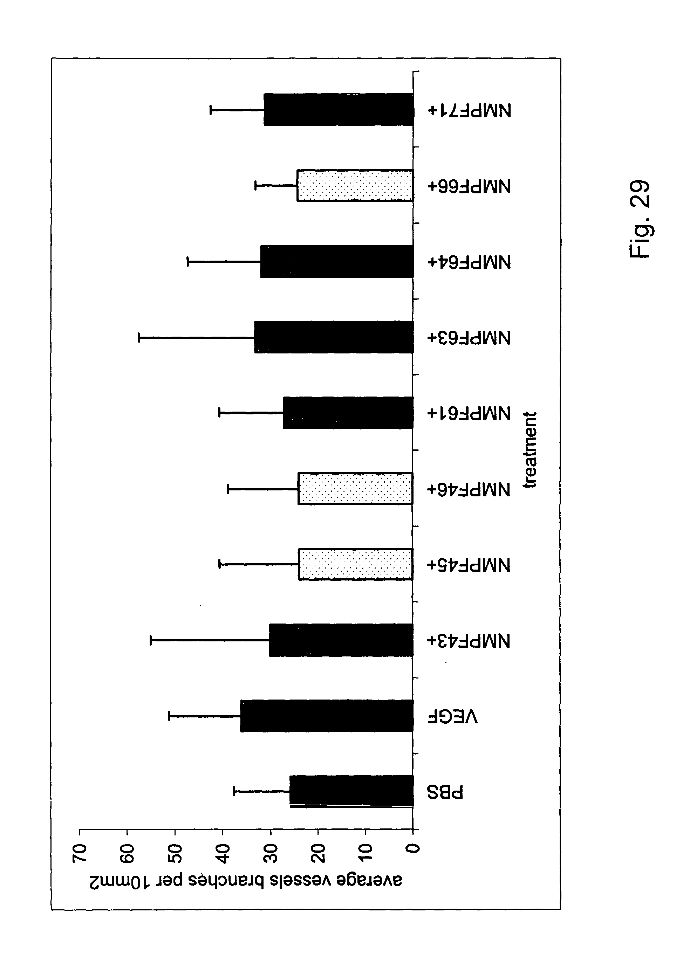

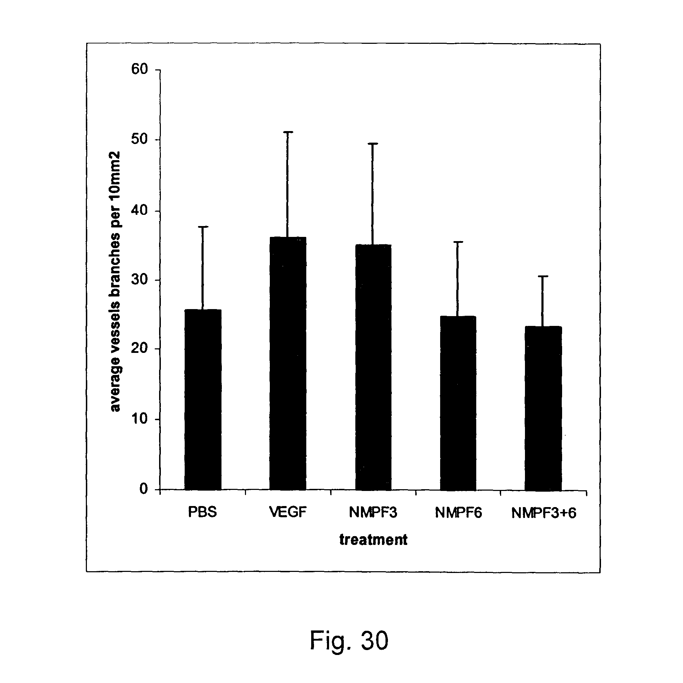

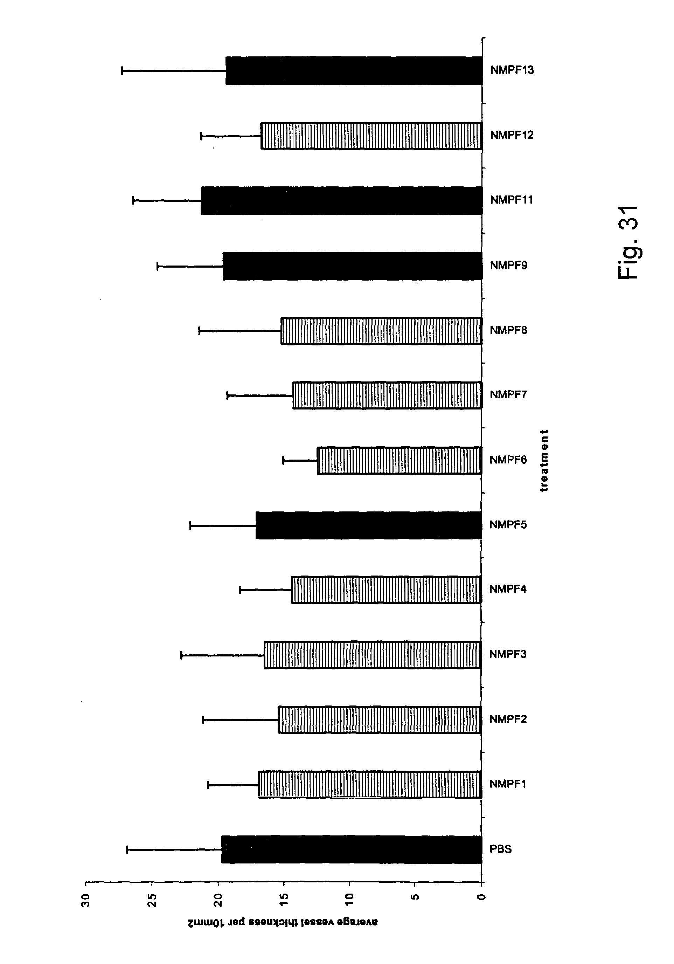

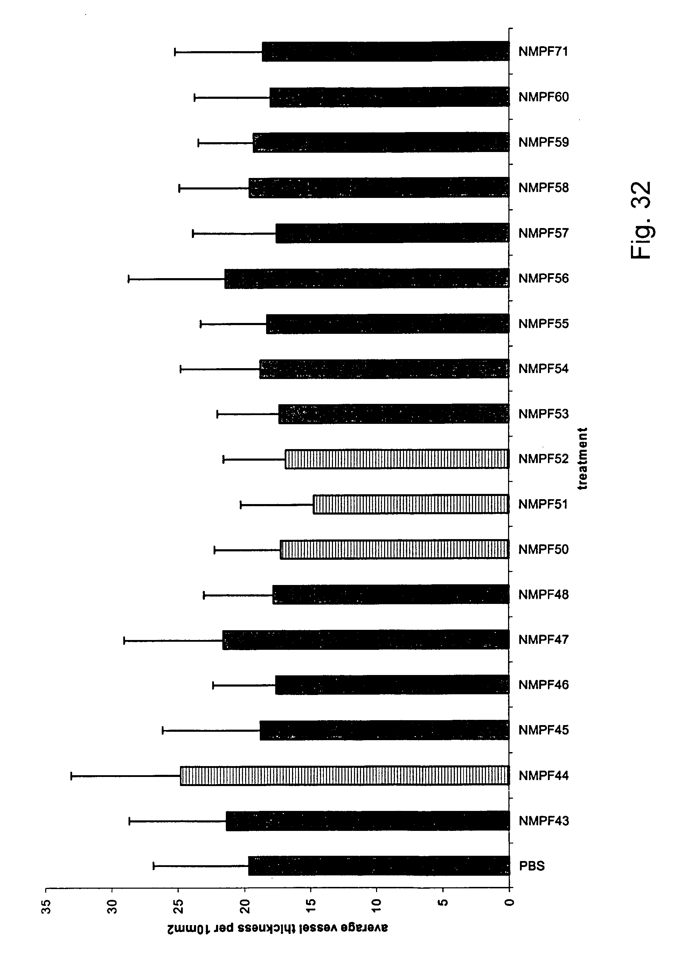

In yet another embodiment, a method is provided for modulating angiogenesis, as is illustrated in Fig 20-30 showing the effect of a peptide according to the invention on vessel branching. Also, the invention permits to modulate the process of vasculogenesis as is clearly evidenced by the ability of a peptide to control vessel thickness (Fig 31 and 32). It was reported that hCG is a potent angiogeneic factor for uterine endothelial cells *in vitro* and an important role of hCG in endometrial angogenesis was suggested. Importantly, we have now identified hCG-derived peptides that can regulate angiogenesis (see for example table 5) as well as vasculogenisis. Provided is use of a peptide according to the invention for the production of a pharmaceutical composition for the treatment of a large variety of diseases, wherein said peptide is capable of reducing the production of NO and / or TNF alpha in a cell. Diseases include, but are not limited to, septic shock, anthrax, bone disease, arthritis, and ischemic events such as cerebrovascular disease and ischemic heart failure. Also provided is a method for treating these diseases comprising administering to a subject in need of such a treatment a molecule comprising an oligopeptide, a peptide or a functional analogue thereof, wherein said molecule is capable of reducing the production NO and / or TNF alpha by a cell. Furthermore, a molecule comprising an oligopeptide, a peptide or a functional analogue thereof, comprises a molecule that is capable of modulating the production of one or more cytokines by a cell. For example, a method for treating these diseases comprises administering to a subject in need of such a treatment a NFkB-regulating peptide.

The invention for example also relates to the treatment of anthrax. Anthrax, the disease caused by the spore-forming *Bacillus anthracis (B. anthracis),* continues to be a worldwide problem among domesticated and wild herbivores in Asia and Africa and poses a worldwide threat when being used as biological weapons for biological warfare or bioterrorism. Human infections occur after contact with infected animals or contaminated animal products. Outbreaks or epidemics are a constant threat for endemic regions because spores can persist in the soil for long periods of time. Importation controls on certain animal products are necessary to prevent the establishment of anthrax where the disease is not endemic. Human anthrax is usually classified by the portal of entry into the host. Cutaneous anthrax, which accounts for the vast majority of human anthrax cases, is a localized infection with generally mild systemic symptoms and characterized by a painless papule that is surrounded by edema which can be quite extensive. The papule ulcerates by day 5 or 6 and develops into the characteristic black eschar of cutaneous anthrax. Inhalation anthrax, which occurs after inhaling airborne spores, gastrointestinal anthrax, resulting from ingestion of contaminated food, and, in some instances, untreated cutaneous anthrax are characterized by dissemination of the bacteria from the initial site of infection with development of a massive septicemia and toxemia. In inhalation anthrax, phagocytic cells transport the spores from the lung alveoli to the regional lymph nodes, where the spores germinate and bacteria multiply. The bacilli then spread into the bloodstream, where they are temporarily removed by the reticuloendothelial system. Prior to death, which occurs 2 to 5 days after infection, there is a sudden onset of acute symptoms characterized by hypotension, edema, and fatal shock due to an extensive septicemia and toxemia. Therapeutic intervention in general must be initiated early, as septicemic infections are nearly always fatal.

The invention relates to the modulation of gene expression in a cell, also called gene control, in relation to the treatment of a variety of diseases such as anthrax. As said, anthrax is a disease of animals and humans and poses a significant threat as an agent of biological warfare and terrorism. Inhalational anthrax, in which spores of *B. anthracis* are inhaled, is almost always fatal, as diagnosis is rarely possible before the disease has progressed to a point where antibiotic treatment is ineffective. The major virulence factors of B. anthracis are a poly-D-glutamic acid capsule and anthrax toxin. Anthrax toxin consists of three distinct proteins that act in concert: two enzymes, lethal factor (LF) and edema factor (EF; an adenylate cyclase); and protective antigen (PA). The PA is a four-domain protein that binds a host cell-surface receptor by its carboxy-terminal domain; cleavage of its N-terminal domain by a furin-like protease allows PA to form heptamers that bind the toxic enzymes with high affinity through homologous N-terminal domains. The complex is endocytosed; acidification of the endosome leads to membrane insertion of the PA heptamer by forming a 14-stranded beta-barrel, followed by translocation of the toxic enzymes into the cytosol by an unknown mechanism. The binary combination of PA and LF is sufficient to induce rapid death in animals when given intravenously, and certain metalloprotease inhibitors block the effects of the toxin*in vitro.* Thus, LF is a potential target for therapeutic agents that would inhibit its catalytic activity or block its association with PA. LF is a protein (relative molecular mass 90,000) that is critical in the pathogenesis of anthrax. It comprises four domains: domain I binds the mebrane-translocating component of anthrax toxin, the PA; domains II, III and IV together create a long deep groove that holds the 16-residue N-terminal tail of mitogen-activated protein kinase kinase-2 (MAPKK-2) before cleavage. Domain II resembles the ADP-ribosylating toxin from Bacillus cereus, but the active site has been mutated and recruited to augment substrate recognition. Domain III is inserted into domain II and seems to have arisen from a repeated duplication of a structural element of domain II. Domain IV is distantly related to the zinc metalloprotease family and contains the catalytic centre; it also resembles domain I. The structure thus reveals a protein that has evolved through a process of gene duplication, mutation and fusion into an enzyme with high and unusual specificity.

The MAPKK family of proteins is the only known cellular substrates of LF. Cleavage by LF near to their N termini removes the docking sequence for the downstream cognate MAP kinase. The effect of lethal toxin on tumour cells, for example, is to inhibit tumour growth and angiogenesis, most probably by inhibiting the MAPKK-1 and MAPKK-2 pathways. However, the primary cell type affected in anthrax pathogenesis is the macrophage. LF has been shown to cleave short N-terminal fragments from mitogen or extracellular signal-regulated MAPKK-1, MAPKK-2, MAPKK-3, and MAPKK-6, the upstream activators of extracellular signal-regulated kinase 1 (ERK1), ERK2, and p38. Recent data show that this results in inhibiting release, but not production, of the pro-inflammatory mediators, NO and tumor necrosis factor-alpha (TNF-alpha). In addition, high levels of lethal toxin lead to lysis of macrophages within a few hours by an unknown mechanism. Recent data suggests that this happens due to inhibition of growth-factor pathways leading to macrophage death. These observations suggest that at an early stage in infection, lethal toxin may reduce (or delay) the immune response, whereas at a late stage in infection, high titres of the bacterium in the bloodstream trigger macrophage lysis and the sudden release of high levels of NO and TNF-alpha. This may explain the symptoms before death which are characterized by the hyperstimulation of host macrophage inflammatory pathways, leading to dramatic hypotension and shock. These symptoms resemble those of LPS-induced septic shock. It is of note that LPS-nonresponder mice, such as C3H/HeJ, are also quite resistant against anthrax toxine.

The recognition sites for LF require the presence of the proline (P) residue followed by a hydrophobic residue or a glycine (G) residue, between which LF cleaves. The recognition sites further require an uncharged amino acid following the hydrophobic residue and at least one positively charged amino acid (and no negatively charged amino acid, such as Asp and Glu) within the 5 amino acids to the N-terminal side of the proline residue. Other residues in the sequence provide appropriate spacing between the critical residues or between the donor and acceptor, and thus their composition is not critical and can include any natural or unnatural amino acid.

The invention provides a method for modulating expression of a gene in a cell comprising providing the cell with a signalling molecule comprising an small peptide i.e. oligopeptide or functional analogue or derivative thereof. Such a molecule is herein also called NMPF or referenced by number. Since small peptides, and functional analogues and derivatives of such relatively short amino acid sequences, are easily synthesized these days, the invention provides a method to modulate gene expression with easily obtainable synthetic compounds such as synthetic peptides or functional analogues or derivatives thereof.

The invention also provides a method for the treatment of an inflammatory condition comprising administering to a subject in need of such treatment a molecule comprising an oligopeptide peptide or functional analogue or derivative thereof, the molecule capable of reducing production of NO by a cell, in particular wherein the molecule additionally is capable of modulating translocation and/or activity of a gene transcription factor present in a cell, especially wherein the gene transcription factor comprises a NF-kappaB/Rel protein. Advantageously, the invention provides a method wherein the modulating translocation and/or activity of a gene transcription factor allows modulation of TNF-alpha production by the cell, in particular wherein the TNF-alpha production is reduced. Considering that TNF-alpha production is central to almost all, if not all, inflammatory conditions, reducing TNF-alpha production can greatly alleviate, or mitigate, a great host of inflammatory conditions that are described herein. In particular, the invention provides a method wherein the inflammatory condition comprises an acute inflammatory condition, and it is especially useful to treat anthrax-related disease, especially when considering that with anthrax, both NO and TNF-alpha reduction will greatly mitigate the course of disease. Table 6 lists oligopeptides according to the invention that have such modulatory effect.

In particular, the invention provides a method of treatment wherein the treatment comprises administering to the subject a pharmaceutical composition comprising an oligopeptide or functional analogue or derivative thereof capable of reducing production of NO by a cell, preferably wherein the composition comprises at least two oligopeptides or functional analogues or derivatives thereof capable of reducing production of NO by a cell; examples of such combinations can be selected under guidance of table 6, whereby it suffices to select two, or more, with a desired effect, such as wherein the at least two oligopeptides are selected from the group LQGV, AQGV and VLPALP.

The invention also provides an isolated, preferably synthetic, oligopeptide or functional analogue or derivative thereof or mixture of such oligopeptides or analogues or derivatives capable of reducing production of NO by a cell. Such cell is preferably of a macrophage or DC lineage, considering the central role these cells play in the inflammatory process. The invention also provides a pharmaceutical composition comprising an oligopeptide or functional analogue or derivative according to the invention or comprising at least two oligopeptides or functional analogues or derivatives thereof capable of reducing production of NO by a cell. Furthermore, the invention provides the use of an oligopeptide or functional analogue or derivative thereof capable of reducing production of NO by a cell for the production of a pharmaceutical composition for the treatment of an inflammatory condition by the reduction of NO production by macrophages or DC in the subject to be treated.

A functional analogue or derivative of a peptide is defined as an amino acid sequence, or other sequence monomers, which has been altered such that the functional properties of the sequence are essentially the same in kind, not necessarily in amount. An analogue or derivative can be provided in many ways, for instance, through conservative amino acid substitution. Also peptidomimetic compounds can be designed that functionally or structurally resemble the original peptide taken as the starting point but that are for example composed of non-naturally occurring amino acids or polyamides. With conservative amino acid substitution, one amino acid residue is substituted with another residue with generally similar properties (size, hydrophobicity), such that the overall functioning is likely not to be seriously affected. However, it is often much more desirable to improve a specific function. A derivative can also be provided by systematically improving at least one desired property of an amino acid sequence. This can, for instance, be done by an Ala-scan and/or replacement net mapping method. With these methods, many different peptides are generated, based on an original amino acid sequence but each containing a substitution of at least one amino acid residue. The amino acid residue may either be replaced by alanine (Ala-scan) or by any other amino acid residue (replacement net mapping). This way, many positional variants of the original amino acid sequence are synthesized. Every positional variant is screened for a specific activity. The generated data are used to design improved peptide derivatives of a certain amino acid sequence.

A derivative or analogue can also for instance be generated by substitution of an L-amino acid residue with a D-amino acid residue. This substitution, leading to a peptide which does not naturally occur in nature, can improve a property of an amino acid sequence. It is for example useful to provide a peptide sequence of known activity of all D-amino acids in retro inversion format, thereby allowing for retained activity and increased half-life values. By generating many positional variants of an original amino acid sequence and screening for a specific activity, improved peptide derivatives comprising such D-amino acids can be designed with further improved characteristics.

A person skilled in the art is well able to generate analogous compounds of an amino acid sequence. This can for instance be done through screening of a peptide library. Such an analogue has essentially the same functional properties of the sequence in kind, not necessarily in amount. Also, peptides or analogues can be circularized, for example, by providing them with (terminal) cysteines, dimerized or multimerized, for example, by linkage to lysine or cysteine or other compounds with side-chains that allow linkage or multimerization, brought in tandem- or repeat-configuration, conjugated or otherwise linked to carriers known in the art, if only by a labile link that allows dissociation.

The invention also provides a signalling molecule for modulating expression of a gene in a cell comprising a small peptide or functional analogue or derivative thereof. Surprisingly, the inventors found that a small peptide acts as a signalling molecule that can modulate signal transduction pathways and gene expression. A functional analogue or derivative of a small peptide that acts as such a signalling molecule for modulating expression of one or more genes in a cell can be identified or obtained by at least one of various methods for finding such a signalling molecule as provided herein.

For example, one method as provided herein for identifying or obtaining a signalling molecule comprising a peptide or functional derivative or analogue thereof capable of modulating expression of a gene in a cell comprises providing the cell with a peptide or derivative or analogue thereof and determining the activity and/or nuclear translocation of one or more gene transcription factors. Such activity can be determined in various ways using means and/or methods honed to the specific transcription factor(s) under study. In the detailed description, it is provided to study NF-kappaB/Rel protein translocation and/or activity, but it is, of course, also easily possible to study translocation and/or activity of any other transcription factor for which such tools are available or can be designed. One such other transcription factor is for example the interferon-alpha-stimulated factor as discussed above. Other useful transcription factors to study in this context comprise c-Jun, ATF-2, Fos, and their complexes, ELK-1, EGR-1, IRF-1, IRF-3/7, AP-1, NF-AT, C/EBPs, Sp1, CREB, PPARgamma, and STAT proteins to name a few. Considering that many proteins are subject to proteolytic breakdown whereby oligopeptide fragments are generated, many already before the full protein even has exerted a function, it is hereby established that oligopeptide fragments of such proteins (of which a non-extensive list is given in the detailed description, but one can for example think of MAPKK-2 that can give rise to a peptide MLARRKPVLPALTINP, and subsequently to a peptide comprising MLARRKP or MLAR or VLPALT or VLPAL, but also of nitric oxide synthase that can give rise to peptides FPGC or PGCP, GVLPAVP, LPA, VLPAVP, or PAVP after proper proteolysis) are involved in feedback mechanisms regulating gene expression, likely by modulating the effect of transcription factors on gene expression. In addition, oligopeptide fragments of proteins (of which a non-extensive list is given in the detailed description) can also modulate the activity of extracellular components such as factor XIII (examples of oligopeptide fragments obtained from factor XIII are LQGV, LQGVVPRGV, GVVP, VPRGV, PRG, PRGV) or activated protein C (APC), thereby eventually leading to the modulation of intracellular signal transduction pathways and gene(s) expression.

As said, the invention provides active oligopeptides acting as a signalling molecule. To allow for improved bio-availability of such a signalling molecule (which is useful as a pharmacon, especially when produced artificially), the invention also provides a method for determining whether a small peptide or derivative or analogue thereof can act as a functional signalling molecule according to the invention, the method further comprising determining whether the signalling molecule is membrane-permeable, and, as explained above, after passage through a plasma membrane and not via binding with a cell-surface receptor, exerts its gene-regulatory effect. Such a signalling molecule, i.e. synthetic compound being a small peptide or functional analogue or derivative thereof as provided herein thus preferably interacts not via cell-surface-receptor mediated signalling followed by a cascade of intracellular events but has direct intracellular actitivity.

Using a method for identifying or obtaining a signalling molecule comprising a peptide or functional derivative or analogue thereof capable of modulating expression of a gene in a cell comprising providing said cell with a peptide or derivative or analogue thereof and determining the activity and/or nuclear translocation of a gene transcription factor as provided herein furthermore allows (if required at random) testing of a multiplicity of oligo-or leadpeptides, leading to automated combinatorial chemistry formats, wherein a great many of candidate signal molecules are being tested in a (if so desired at random) pattern for their reactivity with a molecular library of synthetic peptides representing potential signal molecules allowing the rapid detection of particularly relevant molecules out of tens of thousands of (combinations of) molecules tested. In a preferred embodiment, the invention provides a method wherein said leadpeptides are positionally or spatially addressable, e.g. in an array fashion, if desired aided by computer directed localisation. In an array, said pluralities are for example addressable by their positions in a grid or matrix. It is useful that such peptide fragments or oligopeptides to be tested (herein also called leadpeptides) , being at least 2 to 3 amino acids long, are no longer than about 30 amino acids, but it is preferred and most conform the apparent situation in organisms wherein these breakdown products of endogenous proteins play a regulatory role that such peptides are much smaller, e.g smaller than 16, preferably smaller than 10, even more preferably smaller than 7 amino acids, or even only 4 to 5 amino acids long.

The invention for example provides a process or method for obtaining information about the capacity or tendency of an oligopeptide, or a modification or derivative thereof, to regulate expression of a gene comprising the steps of:
a) contacting the oligopeptide, or a modification or derivative thereof, with at least one cell;
b) determining the presence of at least one gene product in or derived from the cell. It is preferred that the oligopeptide comprises an amino acid sequence corresponding to a fragment of a naturally occurring polypeptide, such as hCG, or MAPKK, or another kinase, be it of plant or animal cell, or of eukaryotic or prokaryotic origin, or a synthase of a regulatory protein in a cell, such as wherein the regulatory protein is a (pro-) inflammatory mediator, such as a cytokine. Several candidate proteins and peptide fragments are listed in the detailed description which are a first choice for such an analysis from the inventors' perspective, but the person skilled in the art and working in a specific field of interest in biotechnology shall immediately understand which protein to select for such analyses for his or her own purposes related to his or her field. The invention for example provides a process or method for obtaining information about the capacity or tendency of an oligopeptide, or a modification or derivative thereof, to regulate expression of a gene wherein said method allows (if required at random) testing of a multiplicity of oligo- or leadpeptides, leading to automated combinatorial chemistry formats, wherein a great many of candidate signal molecules are being tested in a (if so desired at random) pattern for their reactivity with a molecular library of synthetic peptides representing potential signal molecules allowing the rapid detection of particularly relevant molecules out of tens of thousands of (combinations of) molecules tested. In a preferred embodiment, the invention provides a method wherein said leadpeptides are positionally or spatially addressable, e.g. in an array fashion, if desired aided by computer directed localisation. In an array, said pluralities are for example addressable by their positions in a grid or matrix. It is useful that such peptide fragments or oligopeptides to be tested (herein also called leadpeptides) , being at least 2 to 3 amino acids long, are no longer than about 30 amino acids, but it is preferred and most conform the apparent situation in organisms wherein these breakdown products of endogenous proteins play a regulatory role that such peptides are much smaller, e.g smaller than 16, preferably smaller than 10, even more preferably smaller than 7 amino acids, or even only 4 to 5 amino acids long.

In particular, it is provided to perform a process according to the invention further including a step c) comprising determining the presence of the gene product in or derived from a cell which has not been contacted with the oligopeptide, or a modification or derivative thereof, and determining the ratio of gene product found in step b to gene product found in step c, as can easily been done with the present-day genechip technology (see for example the detailed description herein) and related methods of expression profiling known in the art.

Another method provided herein for identifying or obtaining information on a signalling molecule (or for that matter the signalling molecule itself, considering that the next step of synthesizing the molecule, generally being a short peptide, is whole within the art) comprising a peptide or functional derivative or analogue thereof capable of modulating expression of a gene in a cell comprises providing the cell with a peptide or derivative or analogue thereof and determining relative up-regulation and/or down-regulation of at least one gene expressed in the cell. The up-regulation can classically be studied by determining via for example Northern or Western blotting or nucleic acid detection by PCR or immunological detection of proteins whether a cell or cells make more (in the case of up-regulation) or less (in the case of down-regulation) of a gene expression product such as mRNA or protein after the cell or cells have been provided with the peptide or derivative or analogue thereof. Of course, various methods of the invention can be combined to better analyze the functional analogue of the peptide or derivative or analogue under study. Furthermore, relative up-regulation and/or down-regulation of a multitude or clusters of genes expressed in the cell can be easily studied as well, using libraries of positionally or spatially addressable predetermined or known relevant nucleic acid sequences or unique fragments thereof bound to an array or brought in an array format, using for example a nucleic acid library or so-called gene chip expression analysis systems. Lysates of cells or preparations of cytoplasma and/or nuclei of cells that have been provided with the peptide or derivative or analogue under study are then contacted with the library and relative binding of for example mRNA to individual nucleic acids of the library is then determined, as further described herein in the detailed description.

A functional analogue or derivative of a small peptide that can act as a signalling molecule for modulating expression of a gene in a cell can also be identified or obtained by a method for identifying or obtaining a signalling molecule comprising an oligopeptide or functional derivative or analogue thereof capable of modulating expression of a gene in a cell comprising providing a peptide or derivative or analogue thereof and determining binding of the peptide or derivative or analogue thereof to a factor related to gene control. Such a factor related to gene control can be any factor related to transcription (either initiation or termination), processing of primary transcripts, stabilization or destabilization of mRNAs, and mRNA translation.

Binding of a peptide or derivative or analogue thereof to such a factor can be determined by various methods known in the art. Classically, peptides or derivatives or analogues can be (radioactively) labelled and binding to the factor can be determined by detection of a labelled peptide-factor complex, such as by electrophoresis, or other separation methods known in the art. However, for determining binding to such factors, array techniques, such as used with peptide libraries, can also be employed, comprising providing a multitude of peptides or derivatives or analogues thereof and determining binding of at least one of the peptides or derivatives or analogues thereof to a factor related to gene control.

In a preferred embodiment, the factor related to gene control comprises a transcription factor, such as an NF-kappaB-Rel protein or another transcription factor desired to be studied. When binding of a functional analogue according to the invention to such factor has been established, it is, of course, possible to further analyze the analogue by providing a cell with the peptide or derivative or analogue thereof and determining the activity and/or nuclear translocation of a gene transcription factor in the cell, and/or by providing a cell with the peptide or derivative or analogue thereof and determining relative up-regulation and/or down-regulation of at least one gene expressed in the cell.

The invention thus provides a signalling molecule useful in modulating expression of a gene in a cell and/or useful for reducing NO production by a cell and identifiable or obtainable by employing a method according to the invention. Useful examples of such a signalling molecule can be selected from the group of oligopeptides LQG, AQG, LQGV, AQGV, LQGA, VLPALPQVVC, VLPALP, ALPALP, VAPALP, ALPALPQ, VLPAAPQ, VLPALAQ, LAGV, VLAALP, VLPALA, VLPALPQ, VLAALPQ, VLPALPA, GVLPALP, GVLPALPQ, LQGVLPALPQVVC, VVCNYRDVRFESIRLPGCPRGVNPVV SYAVALSCQCAL, RPRCRPINATLAVEK, EGCPVCITVNTTICAGYCPT, SKAPPPSLPSPSRLPGPS, SIRLPGCPRGVNPVVS, LPGCPRGVNPVVS, LPGC, MTRV, MTR, VVC, QVVC and functional analogues or derivatives thereof.

A preferred size of a signalling molecule according to the invention is at most 30 - 40 amino acids, although much smaller molecules, in particular of oligopeptide size, have been shown to be particularly effective. Surprisingly, the invention provides here the insight that gene expression can be modulated or regulated by small peptides, which are most likely breakdown products of larger polypeptides such as chorionic gonadotrophin (CG) and growth hormones or growth factors such as fibroblast growth factor, EGF, VEGF, RNA 3' terminal phosphate cyclase and CAP18. In principle, such regulating peptide sequences can be derived from a part of any protein of polypeptide molecule produced by prokaryotic and/or eukaryotic cells, and the invention provides the insight that breakdown products of polypeptides, preferably oligopeptides at about the sizes as provided herein, are naturally involved as signalling molecules in modulation of gene expression. In particular, as signalling molecule, a (synthetic) peptide is provided obtainable or derivable from beta-human chorionic gonadotrophin (beta-hCG), preferably from nicked beta-HCG. It was thought before that breakdown products of nicked-beta hCG were involved in immunomodulation (PCT International Patent Application WO99/59671) or in the treatment of wasting syndrome (PCT International Patent Application WO97/49721) but a relationship with modulation of gene expression was not forwarded in these publications. Of course, such an oligopeptide, or functional equivalent or derivative thereof, is likely obtainable or derivable from other proteins that are subject to breakdown or proteolysis and that are close to a gene regulatory cascade. Preferably, the peptide signalling molecule is obtained from a peptide having at least 10 amino acids such as a peptide having an amino acid sequence MTRVLQGVLPALPQVVC, SIRLPGCPRGVNPVVS, VVCNYRDVRFESIRLPGCPRGVNPVVSYAVALSCQCAL, RPRCRPINATLAVEKEGCPVCITVNTTICAGYCPT, CALCRRSTTDCGGPKDHPLTC, SKAPPPSLPSPSRLPGPS, CRRSTTDCGGPKDHPLTC, TCDDPRFQDSSSSKAPPPSLPSPSRLPGPSDTPILPQ.

Not wishing to be bound by theory, it is postulated herein that an unexpected mode of gene regulation has been uncovered. Polypeptides, such as endogenous CG, EGFetc., but also polypeptides of pathogens such as viral, bacterial or protozoal polypeptides, are subject to breakdown into distinct oligopeptides, for example by intracellular proteolysis. Distinct proteolytic enzymes are widely available in the cell, for example in eukaryotes in the lysosomal or proteasomal system. Some of the resulting breakdown products are oligopeptides of 3 to 15, preferably 4 to 9, most preferably 4 to 6, amino acids long that are surprisingly not without any function or effect to the cell, but as demonstrated herein may be involved, possibly via a feedback mechanism in the case of breakdown of endogenous polypeptides, as signalling molecules in the regulation of gene expression, as demonstrated herein by the regulation of the activity or translocation of a gene transcription factor such as NF-kappaB by for example peptides LQGV, VLPALPQVVC, LQGVLPALPQ, LQG, GVLPALPQ, VLPALP, VLPALPQ, GVLPALP, VVC, MTRV, and MTR. Synthetic versions of these oligopeptides as described above, and functional analogues or derivatives of these breakdown products, are herein provided to modulate gene expression in a cell and be used in methods to rectify errors in gene expression or the treatment of disease. Oligopeptides such as LQG, AQG, LQGV, AQGV, LQGA, VLPALP, ALPALP, VAPALP, ALPALPQ, VLPAAPQ, VLPALAQ, LAGV, VLAALP, VLPALA, VLPALPQ, VLAALPQ, VLPALPA, GVLPALP, GVLPALPQ, LQGVLPALPQVVC, SIRLPGCPRGVNPVVS, SKAPPPSLPSPSRLPGPS, LPGCPRGVNPVVS, LPGC, MTRV, MTR, VVC, or functional analogues or derivatives (including breakdown products) of the longer sequences thereof, are particularly effective.

By using the insight as expressed herein, in a preferred embodiment, the invention provides a method for modulating expression of a gene in a cell comprising providing the cell with a signalling molecule comprising an oligopeptide or functional analogue or derivative thereof wherein the signalling molecule is membrane-permeable in that it enters the cell. Most small peptides as described herein already have an inherent propensity to become intracellularly involved, but signalling molecules as provided herein can also be provided with additional peptide sequences, such as arginine- or lysine-rich stretches of amino acids, that allow for improved internalization across a lipid bilayer membrane, and may possibly be cleaved off later by internal proteolytic activity.

In a preferred embodiment, the invention provides a method for modulating expression of a gene in a cell comprising providing the cell with a signalling molecule comprising a small peptide (amino acid sequence) or functional analogue or derivative thereof, wherein the signalling molecule modulates NF-kappaB/Rel protein conversion or translocation. As said, NF-κB was originally identified as a gene transcription factor that bound to an enhancer element in the gene for the Igκ light chain and was believed to be B cell-specific. However, subsequent studies revealed that NF-kappaB/Rel proteins are ubiquitously expressed and play a central role as transcription factor in regulating the expression of many genes, particularly those involved in immune, inflammatory, developmental and apoptotic processes. NF-κB related gene transcription factors can be activated by different stimuli such as microbial products, proinflammatory cytokines, T-and B-cell mitogens, and physical and chemical stresses. NF-κB in turn regulates the inducible expression of many cytokines, chemokines, adhesion molecules, acute phase proteins, and antimicrobial peptides.

NF-κB represents a group of structurally related and evolutionarily conserved gene transcription factors. So far, five mammalian NF-κB proteins named Rel (c-Rel), RelA (p65), RelB, NF-kappa-B1 (p50 and its precursor p105), and NF-Kappa-B2 (p52 and it precursor p100) have been described. NF-κB proteins can exist as homo- or heterodimers, and although most NF-κB dimers are activators of transcription, the p50/p50 and p52/p52 homodimers often repress the transcription of their target genes. In Drosophila, three NF-κB homologs named Dorsal, Dif, and Relish have been identified and characterized. Structurally, all NF-κB/Rel proteins share a highly conserved NH₂-terminal Rel homology domain (RHD) that is responsible for DNA binding, dimerization, and association with inhibitory proteins known as IκBs. In resting cells, NF-κB/Rel dimers are bound to IκBs and retained in an inactive form in the cytoplasm. Like NF-κB, IkBs are also members of a multigene family containing seven known mammalian members including IκBα, IκBβ, Iκbγ, IκBε, Bcl-3, the precursor Rel-proteins, p100 and p105, and one *Drosophila* IκB named Cactus. The IκB family is characterized by the presence of multiple copies of ankyrin repeats, which are protein-protein interaction motifs that interact with NF-κB via the RHD. Upon appropriate stimulation, IκB is phosphorylated by IκB kinases (IKKs), polyubiquitinated by a ubiquitin ligase complex, and degraded by the 26S proteosome. Consequently, NF-κB is released and translocates into the nucleus to initiate gene expression.

NF-κB related transcription factors regulate the expression of a wide variety of genes that play critical roles in innate immune responses. Such NF-κB target genes include those encoding cytokines (e.g., IL-1, IL-2, IL-6, IL-12, TNF-α, LTα, LTβ, and GM-CSF), adhesion molecules (e.g., ICAM, VCAM, endothelial leukocyte adhesion molecule [ELAM]), acute phase proteins (e.g., SAA), and inducible enzymes (e.g., iNOS and COX-2). In addition, it has been demonstrated recently that several evolutionary conserved antimicrobial peptides, e.g., β-defensins, are also regulated by NF-κB, a situation similar to *Drosophila.* Besides regulating the expression of molecules involved in innate immunity, NF-κB also plays a role in the expression of molecules important for adaptive immunity, such as MHC proteins, and the expression of critical cytokines such as IL-2, IL-12 and IFN-γ. Finally NF-κB plays an important role in the overall immune response by affecting the expression of genes that are critical for regulating the apoptotic process, such as c-IAP-1 and c-IAP-2, Fas ligand, c-myc, p53, and cyclin D1.

Under normal conditions, NF-kappaB is rapidly activated upon microbial and viral invasion, and this activation usually correlates with resistance of the host to infection. However, persistent activation of NF-kappaB may lead to the production of excessive amounts of pro-inflammatory mediators such as IL-12 and TNF-alpha, resulting in tissue damage, as in insulin-dependent diabetes mellitus, atherosclerosis, Crohn's disease, organ failure, and even death of the host, as in bacterial infection-induced septic shock. It is interesting to note that in order to survive in the host, certain pathogens, such as Schistosoma japonica, Bordetella, Yersinia, Toxoplasma gondii and African Swine Fever Virus have evolved mechanisms to counteract or escape the host system by inhibiting NF-kappaB activation. On the other hand, some viruses, including HIV-1, CMV and SV-40, take advantage of NF-kappaB as a host factor that is activated at sites of infection.

Furthermore, the invention provides a method to explore alterations in gene expression in antigen-presenting cells such as dendritic cells in response to microbial exposure by analyzing a gene-expression profile of dendritic cells in response to microorganisms such as for example bacteria such as *Escherichia coli,* or other pathogenic bacteria, fungi or yeasts such as *Candida albicans*, viruses such as influenza virus and the effect of (simultaneous) treatment of these diseases with a signalling molecule according to the invention. For example, human monocyte-derived dendritic cells are cultured with one or more pathogens for 1-36 hours, and gene expression is analyzed using an oligonucleotide array representing a (be it large or small) set of genes. When the pathogens regulate the expression of a core set of a distinct number of genes, these genes may be classified according to their kinetics of expression and function. Generally, within 4 hours of pathogen exposure, genes associated with pathogen recognition and phagocytosis will be down-regulated, whereas genes for antigen processing and presentation are up-regulated 8 hours post-exposure. Treatment of such dendritic cells with a signalling molecule according to the invention (be it simultaneous or before or after the treatment of the cells with the pathogen) allows studying the effect a signalling molecule according to the invention has on the effect a pathogen has on an antigen-presenting cell.

In short, the invention surprisingly provides a signalling molecule capable of modulating expression of a gene in a cell, the molecule being a short peptide, preferably of at most 30 amino acids long, or a functional analogue or derivative thereof. In a much preferred embodiment, the peptide is an oligopeptide of from about 3 to about 15 amino acids long, preferably 4 to 12, more preferably 4 to 9, most preferably 4 to 6 amino acids long, or a functional analogue or derivative thereof. Of course, such signalling molecule can be longer, for example by extending it (N- and/or C-terminally), with more amino acids or other side groups, which can for example be (enzymatically) cleaved off when the molecule enters the place of final destination. Such extension may even be preferable to prevent the signalling molecule from becoming active in an untimely fashion; however, the core or active fragment of the molecule comprises the aforementioned oligopeptide or analogue or derivative thereof. Such a peptide according to the invention exerts its biological function by regulating gene expression in an other way than a classically known membrane-impermeable signalling molecule acts, such as acetylcholine, growth factors, extracellular matrix components, (peptide)-hormones, neuropeptides, thrombin, i.e. not by cell-surface receptor mediated signalling.

In particular, the invention provides a modulator of NF-kappaB/Rel protein activation comprising a signalling molecule according to the invention. Such modulators are widely searched after these days. Furthermore, the invention provides use of a signalling molecule according to the invention for the production of a pharmaceutical composition for the modulation of gene expression.

Also, the invention provides a method for the treatment of bone disease such as osteoporosis comprising administering to a subject in need of such treatment a molecule comprising an oligopeptide peptide or functional analogue thereof, the molecule capable of modulating production of NO and/or TNF-alpha by a cell. Such a method of treatment is particularly useful in post-menopausal women that no longer experience the benefits of being provided with a natural source of several of the signalling molecules as provided herein, as physiologically derived from hCG and its breakdown products. Furthermore, the invention provides a method for the treatment of an inflammatory condition associated with TNF-alpha activity of fibroblasts, such as seen with chronic arthritis or synovitis, comprising administering to a subject in need of such treatment a molecule comprising an oligopeptide peptide or functional analogue thereof wherein the molecule is capable of modulating translocation and/or activity of a gene transcription factor present in a cell, in particular of the NF-kappaB factor. Such a treatment can be achieved by systemic administration of a signalling molecule according to the invention, but local administration in joints, bursae or tendon sheaths is provided as well. The molecule can be selected from table 6 or identified in a method according to the invention. It is preferred when the treatment comprises administering to the subject a pharmaceutical composition comprising an oligopeptide or functional analogue thereof also capable of reducing production of NO by a cell, for example, wherein the composition comprises at least two oligopeptides or functional analogues thereof, each capable of reducing production of NO and/or TNF-alpha by a cell, in particular wherein the at least two oligopeptides are selected from the group LQGV, AQGV and VLPALP.

Furthermore, the invention provides use of an oligopeptide or functional analogue thereof capable of reducing production of NO and/or TNF-alpha by a cell for the production of a pharmaceutical composition for the treatment of an inflammatory condition or a post-meno-pausel condition, or a bone disease such as osteoporosis, or for the induction of weight loss. The term "pharmaceutical composition" as used herein is intended to cover both the active signalling molecule alone or a composition containing the signalling molecule together with a pharmaceutically acceptable carrier, diluent or excipient. Acceptable diluents of an oligopeptide as described herein in the detailed description are for example physiological salt solutions or phosphate buffered salt solutions. In one embodiment of the present invention, a signal molecule is administered in an effective concentration to an animal or human systemically, e.g. by intravenous, intra-muscular or intraperitoneal administration. Another way of administration comprises perfusion of organs or tissue, be it *in vivo* or *ex vivo*, with a perfusion fluid comprising a signal molecule according to the invention. Topical administration, e.g. in ointments or sprays, may also apply, e.g. in inflammations of the skin or mucosal surfaces of for example mouth, nose and/or genitals. Local administration can occur in joints, bursae, tendon sheaths, in or around the spinal cord at locations where nerve bundles branch off, at the location of hernias, in or around infarcted areas in brain or heart, etc. The administration may be done as a single dose, as a discontinuous sequence of various doses, or continuously for a period of time sufficient to permit substantial modulation of gene expression. In the case of a continuous administration, the duration of the administration may vary depending upon a number of factors which would readily be appreciated by those skilled in the art.

The administration dose of the active molecule may be varied over a fairly broad range. The concentrations of an active molecule which can be administered would be limited by efficacy at the lower end and the solubility of the compound at the upper end. The optimal dose or doses for a particular patient should and can be determined by the physician or medical specialist involved, taking into consideration well-known relevant factors such as the condition, weight and age of the patient, etc.

The active molecule may be administered directly in a suitable vehicle, such as e.g. phosphate-buffered saline (PBS) or solutions in alcohol or DMSO. Pursuant to preferred embodiments of the present invention, however, the active molecule is administered through a single dose delivery using a drug-delivery system, such as a sustained-release delivery system, which enables the maintenance of the required concentrations of the active molecule for a period of time sufficient for adequate modulation of gene expression. A suitable drug-delivery system would be pharmacologically inactive or at least tolerable. It should preferably not be immunogenic nor cause inflammatory reactions, and should permit release of the active molecule so as to maintain effective levels thereof over the desired time period. A large variety of alternatives are known in the art as suitable for purposes of sustained release and are contemplated as within the scope of the present invention. Suitable delivery vehicles include, but are not limited to, the following: microcapsules or microspheres; liposomes and other lipid-based release systems; viscous instillates; absorbable and/or biodegradable mechanical barriers and implants; and polymeric delivery materials, such as polyethylene oxide/polypropylene oxide block copolymers, polyesters, cross-linked polyvinylalcohols, polyanhydrides, polymethacrylate and polymethacrylamide hydrogels, anionic carbohydrate polymers, etc. Useful delivery systems are well known in the art.

A highly suitable formulation to achieve the active molecule release comprises injectable microcapsules or microspheres made from a biodegradable polymer, such as poly(dl-lactide), poly(dl-lactide-co-glycolide), polycaprolactone, polyglycolide, polylactic acid-co-glycolide, poly(hydroxybutyric acid), polyesters or polyacetals. Injectable systems comprising microcapsules or microspheres having a diameter of about 50 to about 500 micrometers offer advantages over other delivery systems. For example, they generally use less active molecules and may be administered by paramedical personnel. Moreover, such systems are inherently flexible in the design of the duration and rate of separate drug release by selection of microcapsule or microsphere size, drug loading and dosage administered. Further, they can be successfully sterilized by gamma irradiation.

The design, preparation and use of microcapsules and microspheres are well within the reach of persons skilled in the art and detailed information concerning these points is available in the literature. Biodegradable polymers (such as lactide, glycolide and caprolactone polymers) may also be used in formulations other than microcapsules and microspheres; e.g. premade films and spray-on films of these polymers containing the active molecule would be suitable for use in accordance with the present invention. Fibers or filaments comprising the active molecule are also contemplated as within the scope of the present invention.

Another highly suitable formulation for a single-dose delivery of the active molecule in accordance with the present invention involves liposomes. The encapsulation of an active molecule in liposomes or multilamellar vesicles is a well-known technique for targeted drug delivery and prolonged drug residence. The preparation and use of drug-loaded liposomes is well within the reach of persons skilled in the art and well documented in the literature.

Yet another suitable approach for single-dose delivery of an active molecule in accordance with the present invention involves the use of viscous installates. In this technique, high molecular weight carriers are used in admixture with the active molecule, giving rise to a structure which produces a solution with high viscosity. Suitable high molecular weight carriers include, but are not limited to, the following: dextrans and cyclodextrans; hydrogels; (cross-linked) viscous materials, including (cross-linked) viscoelastics; carboxymethylcellulose; hyaluronic acid; and chondroitin sulfate. The preparation and use of drug-loaded viscous instillates is well known to persons skilled in the art.

Pursuant to yet another approach, the active molecule may be administered in combination with absorbable mechanical barriers such as oxidized regenerated cellulose. The active molecule may be covalently or non-covalently (e.g., ionically) bound to such a barrier, or it may simply be dispersed therein.

A pharmaceutical composition as provided herein is particularly useful for the modulation of gene expression by inhibiting NF-kappaB/Rel protein activation.

NF-kappaB/Rel proteins are a group of structurally related and evolutionarily conserved proteins (Rel). Well known are c-Rel, RelA (p65), RelB, NF-kappaB1 (p50 and its precursor p105), and NF-kappaB2 (p52 and its precursor p100). Most NF-kappaB dimers are activators of transcription; p50/p50 and p52/p52 homodimers repress the transcription of their target genes. All NF-kappaB/Rel proteins share a highly conserved NH2-terminal Rel homology domain (RHD). RHD is responsible for DNA binding, dimerization, and association with inhibitory proteins known as IkappaBs. In resting cells, NF-kappaB/Rel dimers are bound to IkappaBs and retained in an inactive form in the cytoplasm. IkappaBs are members of a multigene family (IkappaBalpha, IkappaBbeta, IkappaBgamma, IkappaBepsilon, Bcl-3, and the precursor Rel-proteins, p100 and p105. Presence of multiple copies of ankyrin repeats interact with NF-kappaB via the RHD (protein-protein interaction. Upon appropriate stimulation, IkappaB is phosphorylated by IkappaB Kinase (IKKs), polyubiquitinated by ubiquitin ligase complex, and degraded by the 26S proteosome. NF-kappaB is released and translocates into nucleus to initiate gene expression.

NF-kappaB regulation of gene expression includes innate immune responses: such as regulated by cytokines IL-1, IL-2, IL-6, IL-12, TNF-alpha, LT-alpha, LT-beta, GM-CSF; expression of adhesion molecules (ICAM, VCAM, endothelial leukocyte adhesion molecule [ELAM]), acute phase proteins (SAA), iInducible enzymes (iNOS and COX-2) and antimicrobial peptides (beta-defensins). For adaptive immunity, MHC proteins IL-2, IL-12 and IFN-alpha are regulated by NF-kappaB. Regulation of overall immune response includes the regulation of genes critical for regulation of apoptosis (c-IAP-1 and c-IAP-2, Fas Ligand, c-myc, p53 and cyclin D1.

Considering that NF-kappaB and related transcription factors are cardinal pro-inflammatory transcription factors, and considering that the invention provides a signalling molecule, such as an oligopeptide and functional analogues or derivatives thereof that are capable of inhibiting NF-kappaB and likely also other pro-inflammatory transcription factors, herein also called NF-kappaB inhibitors, the invention provides a method for modulating NF-kappaB activated gene expression, in particular for inhibiting the expression and thus inhibiting a central pro-inflammatory pathway.

The consequence of this potency to inhibit this pro-inflammatory pathway is wide and far-reaching. The invention for example provides a method to mitigate or treat inflammatory airway disease such as asthma. Generally, asthma patients show persistent activation of NF-kappaB of cells lining the respiratory tract. Providing these patients, for example, by aerosol application, with a signalling molecule according to the invention, such as LQGV or AQGV or MTRV or functional analogue or derivative thereof, will alleviate the inflammatory airway response of these individuals by inhibiting NF-kappaB activation of the cells. Such compositions can advantageously be made with signalling molecules that are taken up in liposomes.

As said, inflammation involves the sequential activation of signalling pathways leading to the production of both pro- and anti-inflammatory mediators. Considering that much attention has focused on pro-inflammatory pathways that initiate inflammation, relatively little is known about the mechanisms that switch off inflammation and resolve the inflammatory response. The transcription factor NF-kB is thought to have a central role in the induction of pro-inflammatory gene expression and has attracted interest as a new target for the treatment of inflammatory disease. However NF-kB activation of leukocytes recruited during the onset of inflammation is also associated with pro-inflammatory gene expression, whereas such activation during the resolution of inflammation is associated with the expression of anti-inflammatory genes and the induction of apoptosis. Inhibition of NF-kB during the resolution of inflammation protracts the inflammatory response and prevents apoptosis. This shows that NF-kB has an anti-inflammatory role *in vivo* involving the regulation of inflammatory resolution. The invention provides a tool to modulate the inflammation at the end phase, a signalling molecule or modulator as provided herein allows the modulation of the NF-kappaB pathway at different stages of the inflammatory response *in vivo*, and in a particular embodiment, the invention provides a modulator of NF-kappaB for use in the resolution of inflammation, for example through the regulation of leukocyte apoptosis. Useful oligopeptides can be found among those that accelerate shock.

The invention also provides a method to mitigate or treat neonatal lung disease, also called chronic lung disease of prematurity, a condition often seen with premature children who develop a prolonged pulmonary inflammation or bronchopulmonary dysplasia. Treating such premature children with an NF-kappaB inhibitor, such as oligopeptide LQGV, or functional analogue or derivative thereof, as provided herein allows such lung conditions to be prevented or ameliorated as well.

Recent advances in bone biology provide insight into the pathogenesis of bone diseases. The invention also provides a method of treatment of a post-menopausal condition such as osteoporosis comprising modulation and inhibition of osteoclast differentiation and inhibiting TNF-alpha induced apoptosis of osteoblasts, thereby limiting the dissolve of bone structures, otherwise so prominent in post-menopausal women that have no longer a natural source of hCG and thus lack the modulatory effect of the signal molecules that are derived of hCG as shown herein. The invention thus also provides a method of treatment of a bone disease, such as osteoporosis (which is often, but not exclusively, seen with post-menopausal women). Furthermore, NO and TNF-alpha modulators as provided herein inhibit the inflammatory response and bone loss in periodontitis. Furthermore, considering that there is a correlation between TNF-alpha activity and severity of clinical manifestations in ankylosing spondylitis, the invention provides the treatment of spondylitis by use of a signalling molecule as provided herein.

Furthermore, considering that an important pathogenic component in the development of insulin-dependent diabetes mellitus (type 1) comprises over-activation of the NF-kappaB pathway as seen in dendritic cells, treatment with an NF-kappaB inhibitor according to the invention will lead to reduced symptoms of diabetes, or at least to a prolonged time to onset of the disease. Particularly effective oligopeptide signalling molecules according to the invention in this context are GVLPALPQ, LQGV MTRV, VLPALPQVVC, VLPALP, VLPALPQ, LPGCPRGVNPVVS, LPGC, VVCNYRDVRFESIRLPGCPRGVNPVVSYAVALSCQCAL, and CPRGVNPVVS, which were shown herein to postpone onset of diabetes in an Non-obese Diabetic Mouse (NOD). Another approach to treatment of diabetes, in particular insulin -independent diabetes (type 2), comprises inhibition of the PPARgamma cascade with an oligopeptide signalling molecule or functional analogue or derivative thereof.

Another use that is provided relates to a method for combating or treating auto-immune disease. A non-limiting list of immune diseases includes:

Hashimoto's thyroiditis, primary myxoedema thyrotoxicosis, pernicious anaemia, autoimmune atrophic gastritis, Addison's disease, premature menopause, insulin-dependent diabetes mellitus, stiff-man syndrome, Goodpasture's syndrome, myasthenia gravis, male infertility, pemphigus vulgaris, pemphigoid, sympathetic ophthalmia, phacogenic uveitis, multiple sclerosis, autoimmune haemolytic anaemia, idiopathic thrombocytopenic purpura, idiopathic leucopenia, primary biliary cirrhosis, active chronic hepatitis, cryptogenic cirrhosis, ulcerative colitis, Sjögren's syndrome, rheumatoid arthritis, dermatomyositis, polymyositis, scleroderma, mixed connective tissue disease, discoid lupus erythematosus, and systemic lupus erythematosus.

Another use that is provided relates to a method for combating or treating infections caused by microorganisms, in particular those infections that are caused by micro-organisms that activate the NF-kappaB pathway during infections.

Such microorganisms are manifold, including bacteria, viruses, fungi, and protozoa, but other pathogens (e.g. worms) can have the same effect. Activation of the NFkappaB pathway by a microbial infection in general occurs via activation of the Toll-like receptor pathway. The invention provides a method to modulate and in particular to inhibit parts of gene expression that are related to the inflammatory responses of an organism that are generally activated through one of the Toll-like receptor pathways.

Toll-like receptor-mediated NF-kappaB activation is central in recognition of pathogens by a host. Such recognition of pathogens generally occurs through germline-encoded molecules, the so-called pattern recognition receptors (PRRs). These PRRs recognize widespread pathogen-associated molecular patterns (PAMPs). The pattern recognition receptors are expressed as either membrane-bound or soluble proteins. They include CD14, beta2-integrins (CD11/CD18), C-type lectins, macrophage scavenger receptors, complement receptors (CR1/CD35, CR2/CD21) and Toll-like receptors (TLRs). TLRs are distinguished from other PRRs by their ability to recognize and discriminate between different classes of pathogens. TLRs represent a family of transmembrane proteins that have an extracellular domain comprising multiple copies of leucine-rich repeats (LRRs) and a cytoplasmic Toll/IL-1R (TIR) motif that has significant homology to the intracellular signalling domain of the type I IL-1 receptor (IL-1RI). Therefore, TLRs are thought to belong to the IL-1R superfamily.

Pathogen-associated molecular patterns (PAMPS) are not expressed by hosts but are components of the pathogenic micro-organism. Such PAMPS comprise bacterial cell wall components such as lipopolysaccharides (LPS), lipoproteins (BLP), peptidoglycans (PGN), lipoarabinomannan (LAM), lipoteichoic acid (LTA), DNA containing unmethylated CpG motifs, yeast and fungal cell wall mannans and beta-glucans, double-stranded RNA, several unique glycosylated proteins and lipids of protozoa, and so on.

Recognition of these PAMPS foremost provides for differential recognition of pathogens by TLRs. For example, TLR2 is generally activated in response to BLPs, PGNs of gram-positive bacteria, LAM of mycobacteria, and mannans of yeasts, whereas TLR4 is often activated by LPS of gram-negative bacteria and LTA of gram-negative bacteria; also a secreted small molecule MD-2 can account for TLR4 signalling.

Several oligopeptides capable of modulating gene expression according to the invention have earlier been tested, both *ex vivo* and *in vivo*, and in small animals, but a relationship with modulation of gene expression was not brought forward. A beneficial effect of these oligopeptides on LPS-induced sepsis in mice, namely the inhibition of the effect of the sepsis, was observed. Immunomodulatory effects with these oligopeptides have been observed *in vitro* and in *ex vivo* such as in T-cell assays showing the inhibition of pathological Th1 immune responses, suppression of inflammatory cytokines (MIF), increase in production of anti-inflammatory cytokines (IL-10, TGF-beta) and immunomodulatory effects on antigen-presenting cells (APC) like dendritic cells, monocytes and macrophages.

Now that the insight has been provided that distinct synthetic oligopeptides or functional analogues or derivatives thereof, for example those that resemble breakdown products which can be derived by proteolysis from endogenous proteins such as hCG, can be used to modulate gene expression, for example by NF-kappaB inhibition, such oligopeptides find much wider application. Release of active NF-kappaB in cells is now known to occur after a variety of stimuli including treating cells with bacterial lipopolysaccharide (LPS) and the interaction with a Toll-like receptor (see for example Guha and Mackman, Cell. Sign. 2001, 13:85-94). In particular, LPS stimulation of dendritic cells, monocytes and macrophages induces many genes that are under the influence of activation by transcription factors such as NF-kappaB, p50, EGR-1, IRF-1 and others that can be modulated by a signalling molecule according to the invention. Considering that LPS induction of EGR-1 is required for maximal induction of TNF-alpha, it is foreseen that inhibition of EGR-1 considerably reduces the effects of sepsis seen after LPS activation. Now knowing the gene modulatory effect of the signalling molecules such as oligopeptides as provided herein allows for rational design of signal molecule mixtures that better alleviate the symptoms seen with sepsis. One such mixture, a 1:1:1 mixture of LQGV, AQGV and VLPALP was administered to primates in a gram-negative induced rhesus monkey sepsis model for prevention of septic shock and found to be effective in this primate model. Accordingly, the invention provides a pharmaceutical composition for the treatment of sepsis in a primate and a method for the treatment of sepsis in a primate comprising subjecting the primate to a signalling molecule according to the invention, preferably to a mixture of such signalling molecules. Administration of such a signalling molecule or mixture preferably occurs systematically, e.g. by intravenous or intraperitoneal administration. In a further embodiment, such treatment also comprises the use of for example an antibiotic, however, only when such use is not contraindicated because of the risk of generating further toxin loads because of lysis of the bacteria subject to the action of those antibiotics in an individual thus treated.

Other use that is contemplated relates to a method for combating or treating viral infections, in particular those infections that are caused by viruses that activate the NF-kappaB pathway during infections. Such virus infections are manifold; classical examples are hepatitis B virus-induced cell transformation by persistent activation of NF-kappaB. Use of a signalling molecule according to the invention is herein provided to counter or prevent this cell transformation.

Other disease where persistent NF-kappaB activation is advantageously inhibited by a signalling molecule according to the invention is a transplantation-related disease such as transplantation-related immune responses, graft-versus-host-disease, in particular with bone-marrow transplants, acute or chronic xeno-transplant rejection, and post-transfusion thrombocytopenia.

Another case where persistent NF-kappaB activation is advantageously inhibited by a signalling molecule according to the invention is found in the prevention or mitigation of ischemia-related tissue damage seen after infarcts, seen for example *in vivo* in brain or heart, or *ex vivo* in organs or tissue that is being prepared or stored in preparation of further use as a transplant. Ischemia-related tissue damage can now be mitigated by perfusing the (pre)ischemic area with a signalling molecule according to the invention that inhibits NF-kappaB activation. Examples of conditions where ischemia (also called underperfusion) plays a role include eclampsia which can be ascribed to focal cerebral ischemia resulting from vasoconstriction, consistent with the evidence of changes detected by new cerebral imaging techniques. The liver dysfunction intrinsic to the HELLP (hemolysis, elevated liver enzymes, and low platelet count) syndrome could also be attributed to the effects of acute underperfusion. Other conditions of ischemia are seen after coronary occlusion, leading to irreversible myocardial damage produced by prolonged episodes of coronary artery occlusion and reperfusion *in vivo*, which has already been discussed in PCT/NL01/00259 as well.

Now that the insight has been provided that distinct synthetic oligopeptides, for example those that resemble breakdown products which can be derived by proteolysis from endogenous proteins such as hCG, can be used to modulate gene expression, for example by NF-kappaB inhibition, the oligopeptides find much wider application. For example, the invention provides a method for perfusing a transplant with a perfusing fluid comprising at least one signalling molecule according to the invention; ischemic or pre-implantation damage due to activation of NF-kappaB in the transplant can then be greatly diminished, allowing a wider use of the transplants.

The invention provides a signalling molecule useful in modulating expression of a gene in a cell. Several examples of the use of such a signalling molecule for the production of a pharmaceutical composition for the treatment of medical or veterinary conditions are herewith given. In one embodiment, the invention provides such use in the treatment of an immune-mediated disorder, in particular of those cases whereby a central role of NF-kappaB/Rel proteins in the immune response is found. However as said, modulating gene expression via modulating activity of other transcription factors, such as AP-1 or PPARgamma, and others is also provided, now that the gene modulating role of signalling molecules such as the oligopetides or analogues or derivatives thereof is understood. As also said, now knowing that oligopeptides, likely breakdown products, play such a central role in modulation of gene expression, the invention provides straightforward ways for identifying further gene expression modulating oligopeptides, and provides synthetic versions of these, and analogues and derivatives thereof for use in a wide variety of disorders and for use in the preparation of a wide variety of pharmaceutical compositions. Examples of such treatment and useful pharmaceutical compositions are for example found in relation to conditions wherein the immune-mediated disorder comprises chronic inflammation, such as diabetes, multiple sclerosis or acute or chronic transplant rejection, in particular in those cases whereby antigen-presenting cells (APC's ) or dendritic cells (DCs) are enhanced by (overactive) and persistent NF-kappaB expression or wherein the immune-mediated disorder comprises acute inflammation, such as septic or anaphylactic shock or acute transplant rejection. Other immune-mediated disorders that can be treated with a pharmaceutical composition comprising a signalling molecule according to the invention comprise auto-immune disease, such as systemic lupus erythematosus or rheumatoid arthritis (in particular by inhibiting IL-8 and/or IL-15 production by inhibiting NF-kappaB activity on the expression of these genes), allergy, such as asthma or parasitic disease, overly strong immune responses directed against an infectious agent, such as a virus or bacterium (in particular responses that include rapid hemorrhagic disease caused by infection with organisms such as Yersinia pestis, Ebola-virus, Staphylococcus aureus (e.g. in cases of tampon-disease), bacterial (such as meningococcal) or viral meningitis and/or encephalitis, and other life-threatening conditions). Such overly strong responses are seen with for example pre-eclampsia, recurrent spontaneous abortions (RSA) or preterm parturition or other pregnancy-related disorders. Especially with forms of eclampsia/pre-eclampsia that are associated with genetically programmed increased production of tumour-growth factor beta-1, treatment according to the invention is recommended. Also, in situations where RSA is likely attributable to increased IL-10 levels during pregnancy, or to increased TNF-alpha activity, for example due to the presence of an unfavourable allele, in particular of a G to A polymorphism in the promotor of the gene encoding TNF-alpha, treatment with a pharmaceutical composition as provided herein is recommended. Treatment directed at such pregnancy-related immune disorders is herein also provided by inhibiting NF-kappaB activity directed at activating natural killer (NK) cell activity. Also, LPS-induced reduced fertility, or abortions, seen in pregnant sows can be reduced by applying a signalling molecule or method as provided herein.

Such use in treatment of an immune-mediated disorder preferably comprises regulating relative ratios and/or cytokine activity of lymphocyte, dendritic or antigen-presenting cell subset-populations in a treated individual, in particular wherein the subset populations comprise Th1 or Th2, or DC1 or DC2 cells. Other embodiments of the invention comprise use of a signalling molecule according to the invention for the manufacture of a medicament for modulating a cardiovascular or circulatory disorder, such as coronary arterial occlusion and also in a pregnancy related cardiovascular or circulatory disorder.

Furthermore, the invention provides a pharmaceutical composition for modulating a cardiovascular or circulatory disorder, in particular a pregnancy related cardiovascular or circulatory disorder, comprising a signalling molecule according to the invention or mixtures thereof. Such a composition finds its use in a method for modulating a cardiovascular or circulatory disorder, in particular a pregnancy related cardiovascular or circulatory disorder, comprising subjecting an animal (in particular a mammal) to treatment with at least one signalling molecule according to the invention. Non-pregnancy related disorders that are for example related to hypercholesterolemia are susceptible to treatment with a signalling molecule according to the invention as well. For example, apolipoprotein E (apo E) deficiency is associated with a series of pathological conditions including dyslipidemia, atherosclerosis, Alzheimer's disease, increased body weight and shorter life span. Inheritance of different alleles of the POLYMORPHIC *apoE* gene is responsible for 10% of the variation in plasma cholesterol in most populations. Individuals HOMOZYGOUS for one variant, *apoE2*, can develop type III dysbetalipoproteinaemia if an additional genetic or environmental factor is present. Some much rarer alleles of *apoE* produce dominant expression of this disorder in heterozygous individuals. *ApoE* is a ligand for the LDL receptor and its effects on plasma cholesterol are mediated by differences in the affinity of the LDL receptor for lipoproteins carrying variant apoE proteins. The factors that regulate *apoE* gene transcription have been investigated extensively by the expression of gene constructs in transgenic mice and involve complex interactions between factors that bind elements in the 5' promoter region, in the first intron and in 3' regions many kilobases distant from the structural gene. Deletion of the *apoE* gene is associated with changes in lipoprotein metabolism (plasma total cholesterol), HDL cholesterol, HDL/TC, and HDL/LDL ratios, esterification rate in apo B-depleted plasma, plasma triglyceride, hepatic HMG-CoA reductase activity, hepatic cholesterol content, decreased plasma homocyst(e)ine and glucose levels, and severe atherosclerosis and cutaneous xanthomatosis. The invention provides a method and a signalling molecule for the treatment of conditions that are associated with dysfunctional LDL receptors such as ApoE and other members of the apolipoprotein family. In particular, use of a signalling molecule comprising GVLPALPQ and/or VLPALP or a functional analogue or derivative thereof is preferred.

The invention also provides use of a signalling molecule for the preparation of a pharmaceutical composition or medicament and methods of treatment for various medical conditions that are other than use in the preparation of a pharmaceutical composition for the treatment of an immune-mediated disorder or a method of treatment of an immune-mediated disorder. For example, the invention provides topical application, for example in an ointment or spray comprising a signal molecule according to the invention, for the prevention or mitigation of skin afflictions, such as eczemas, psoriasis, but also of skin damage related to over-exposure to UV-light.

Also, use is contemplated in palliative control, whereby a gene related to prostaglandin synthesis is modulated such that COX2 pathways are effected.

Furthermore, the invention also provides use of a signalling molecule for the preparation of a pharmaceutical composition or medicament and methods of treatment for various medical conditions that are other than use in the preparation of a pharmaceutical composition for the treatment of wasting syndrome, such as the treatment of particular individuals that are suffering from infection with HIV or a method of treatment of wasting syndrome of such individuals.

In one embodiment, the invention provides the use of a signalling molecule according to the invention for the preparation of a pharmaceutical composition or medicament for modulating angiogenesis or vascularization, in particular during embryonal development, or after transplantation to stimulate vascularization into the transplanted organ or inhibit it in a later phase. Signalling molecules that effect angiogenesis are disclosed herein in the detailed description.

Use as provided herein also comprises regulating TNF-alpha receptor (e.g. CD27) expression on cells, thereby modulating the relative ratios and/or cytokine activity of lymphocyte, dendritic or antigen presenting cell subset-populations in a treated individual. As for example described in the detailed description, the particular oligopeptide according to the invention is capable of down-regulating CD27 expression on cells of the T-cell lineage.

Down-regulating TNF-alpha itself is also particularly useful in septic-shock-like conditions that not only display increased TNF-alpha activity but display further release of other inflammatory compounds, such as NO. NO production is a central mediator of the vascular and inflammatory response. Our results show that inflammatory cells like macrophages stimulated with an inflammatory active compound such as LPS produce large amounts of NO. However, these cells co-stimulated with most of the NMPF peptides (NMPF peptide 1 to 14, 43 to 66 and 69), even in a very low dose (1 pg/ml), inhibited production of NO. Typical septic-shock-like conditions that can preferably be treated by down-regulating TNF-alpha and NO production comprise disease conditions such as those caused by Bacillus anthracis (anthrax) and Yersinia pestis toxins or infections with these micro-organisms likely involved in bio-terrorism. Anthrax toxin is produced by Bacillus anthracis, the causative agent of anthrax, and is responsible for the major symptoms of the disease. Clinical anthrax is rare, but there is growing concern over the potential use of B. anthracis in biological warfare and terrorism. Although a vaccine against anthrax exists, various factors make mass vaccination impractical. The bacteria can be eradicated from the host by treatment with antibiotics, but because of the continuing action of the toxin, such therapy is of little value once symptoms have become evident. Thus, a specific inhibitor of the toxin's action will prove a valuable adjunct to antibiotic therapy. The toxin consists of a single receptor-binding moiety, termed "protective antigen" (PA), and two enzymatic moieties, termed "edema factor" (EF) and "lethal factor" (LF). After release from the bacteria as nontoxic monomers, these three proteins diffuse to the surface of mammalian cells and assemble into toxic, cell-bound complexes.

Cleavage of PA into two fragments by a cell-surface protease enables the fragment that remains bound to the cell, PA63, to heptamerize and bind EF and LF with high affinity. After internalization by receptor-mediated endocytosis, the complexes are trafficked to the endosome. There, at low pH, the PA moiety inserts into the membrane and mediates translocation of EF and LF to the cytosol. EF is an adenylate cyclase that has an inhibitory effect on professional phagocytes, and LF is a protease that acts specifically on macrophages, causing their death and the death of the host.

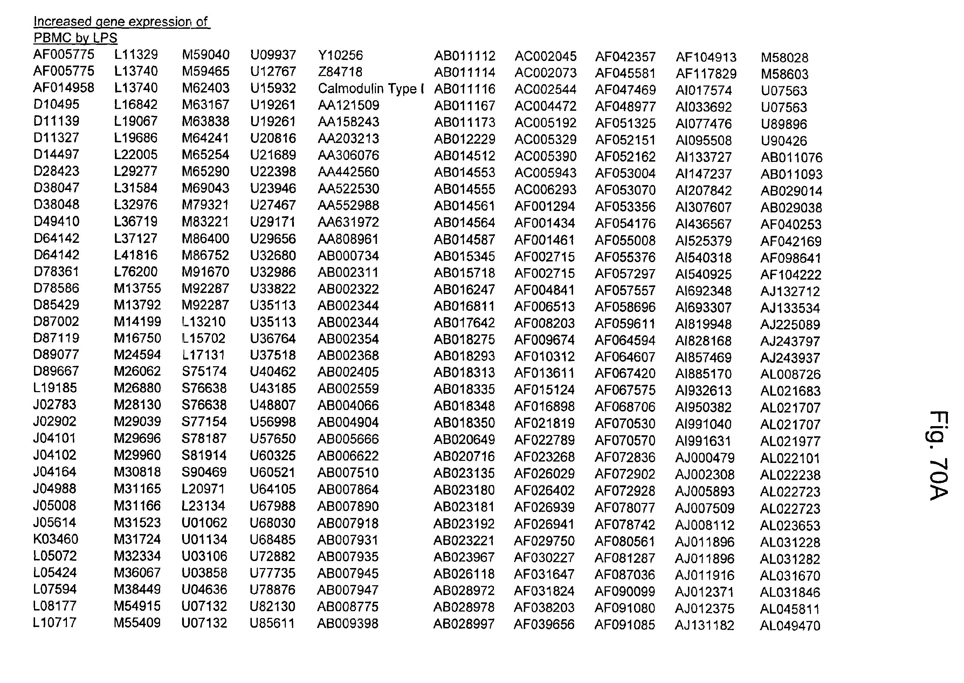

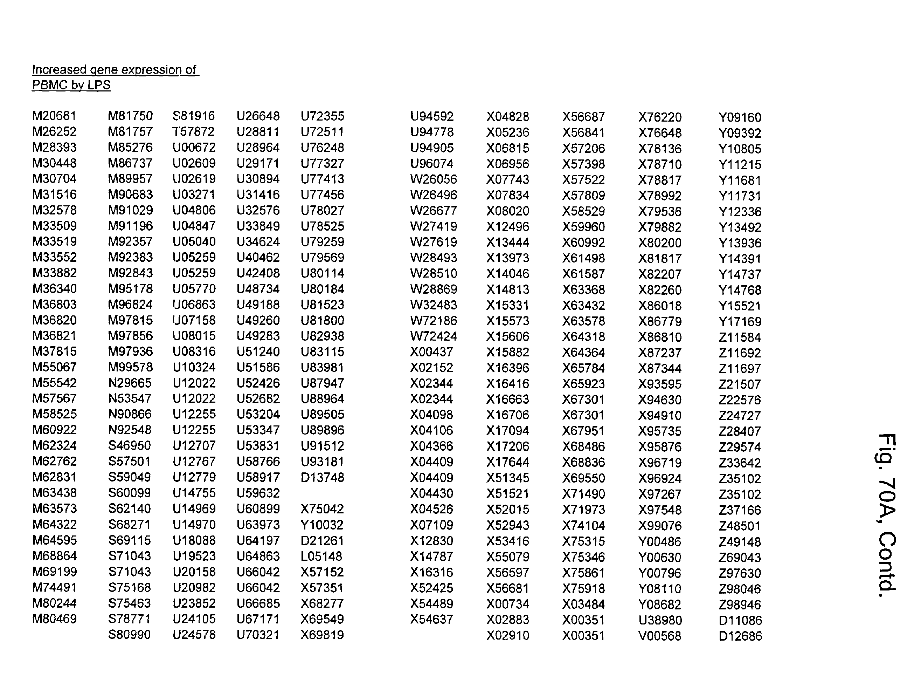

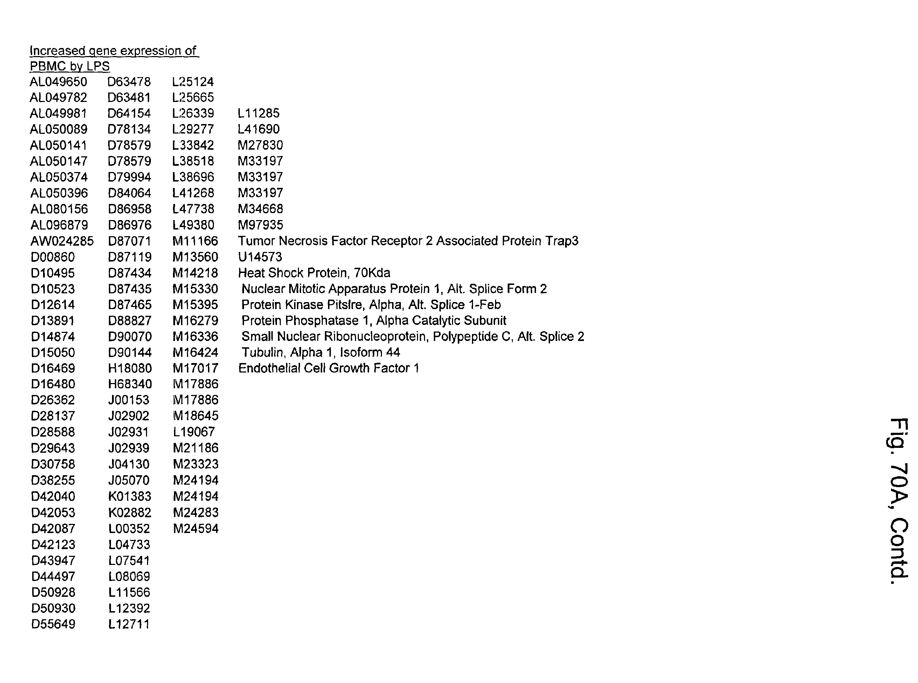

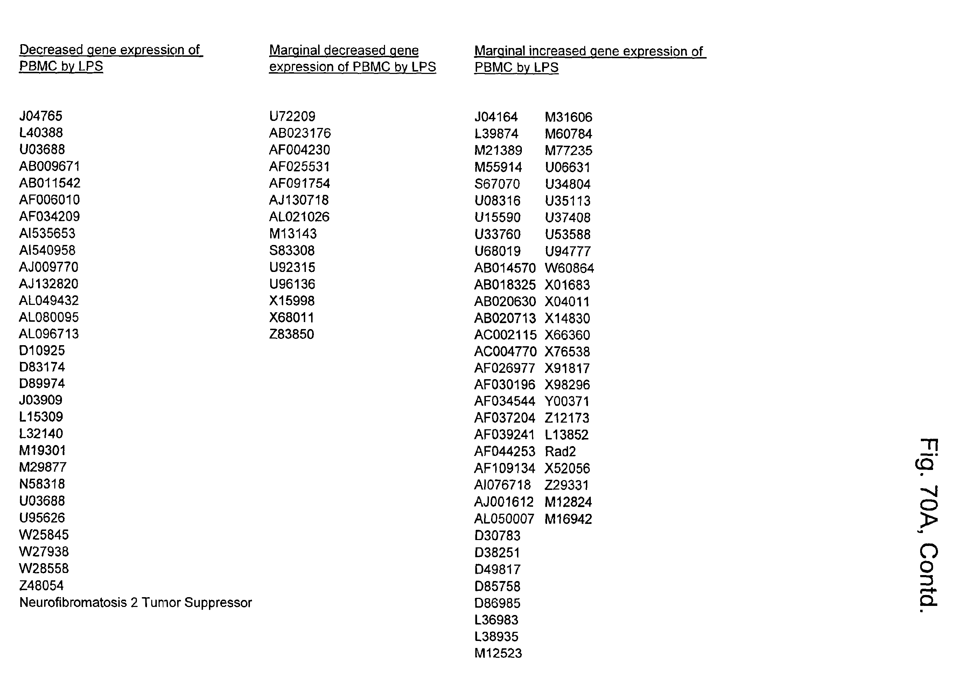

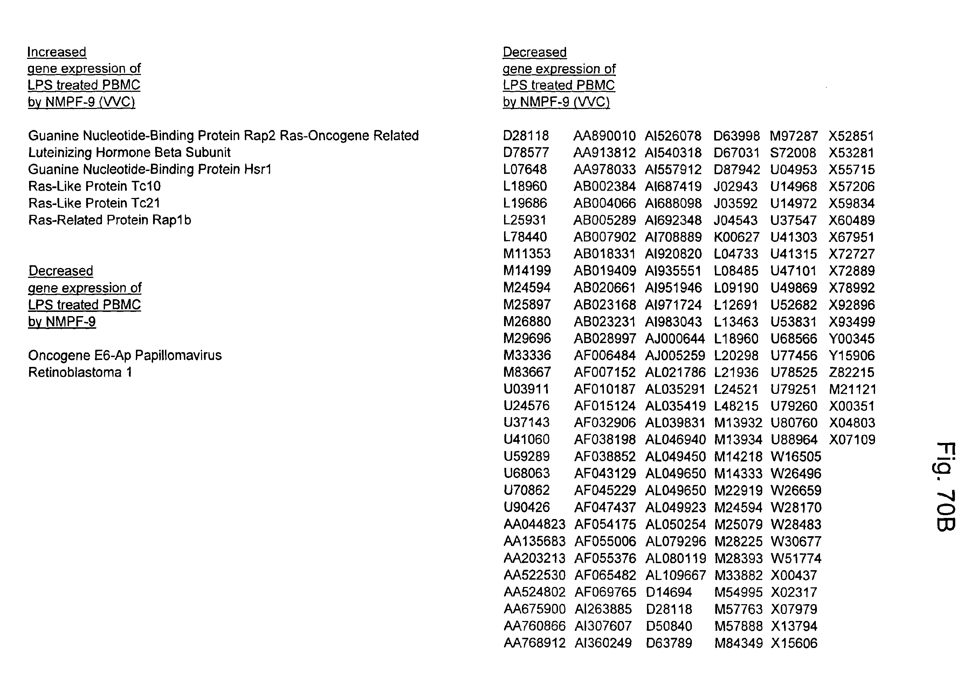

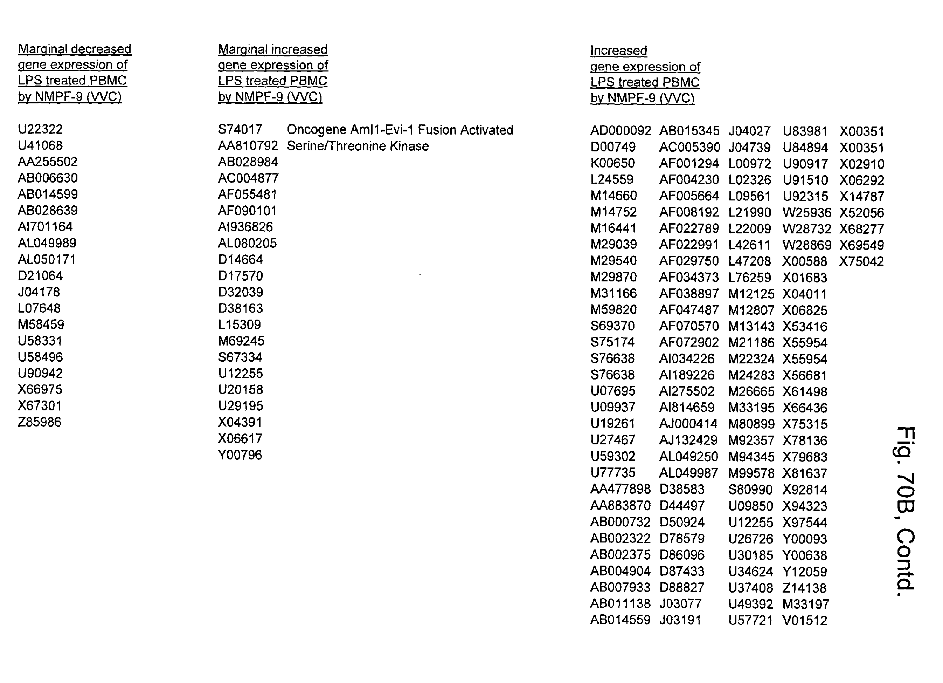

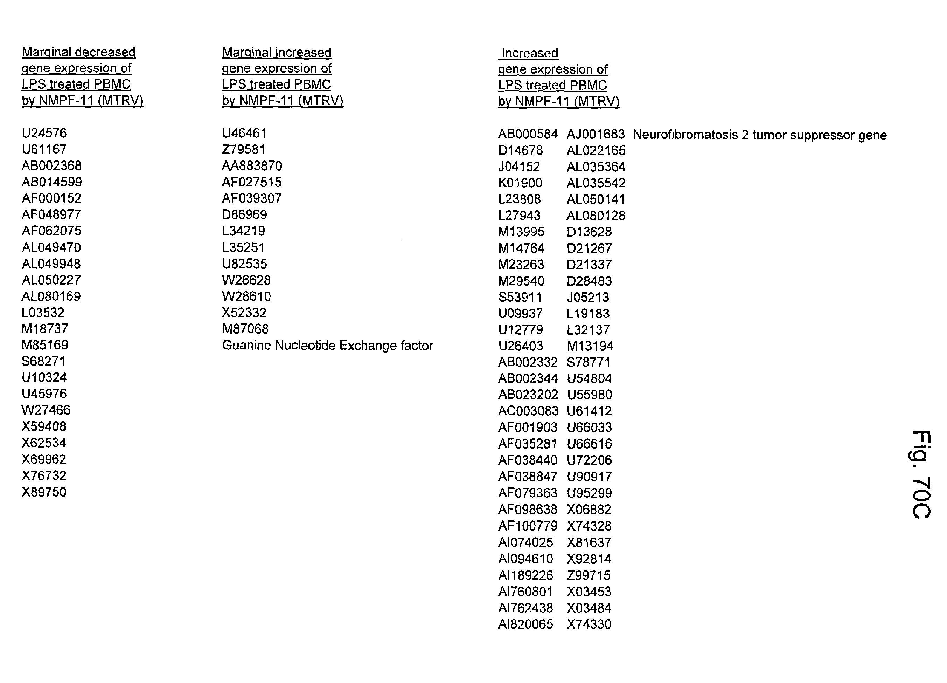

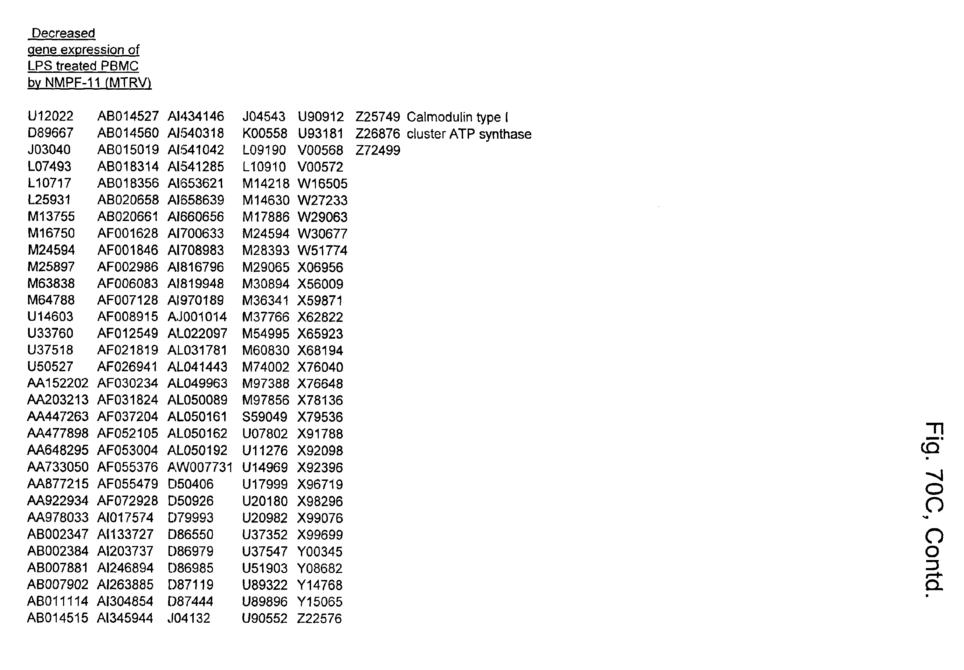

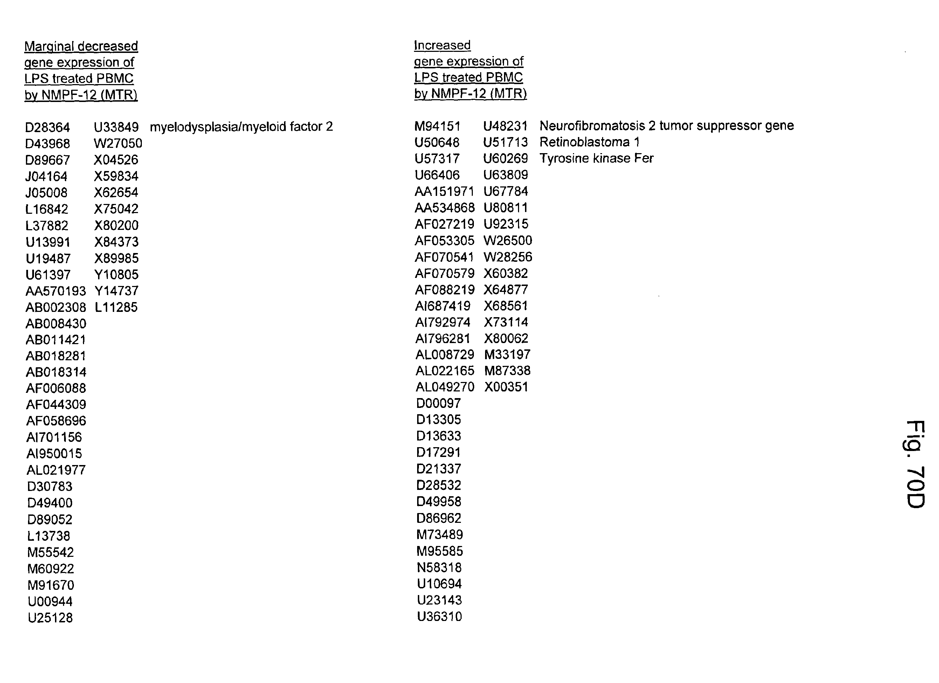

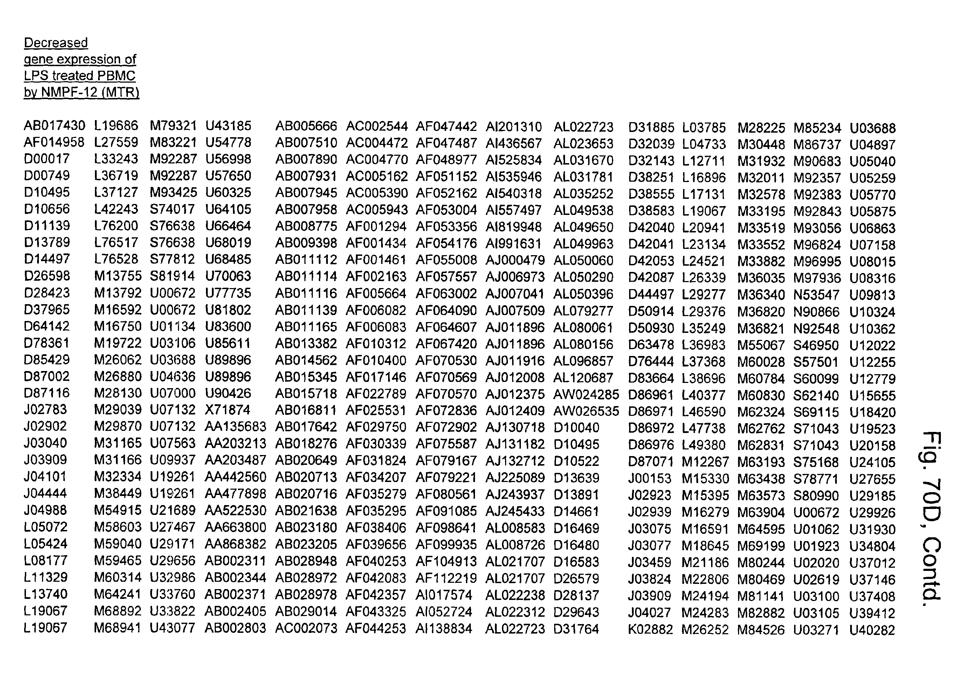

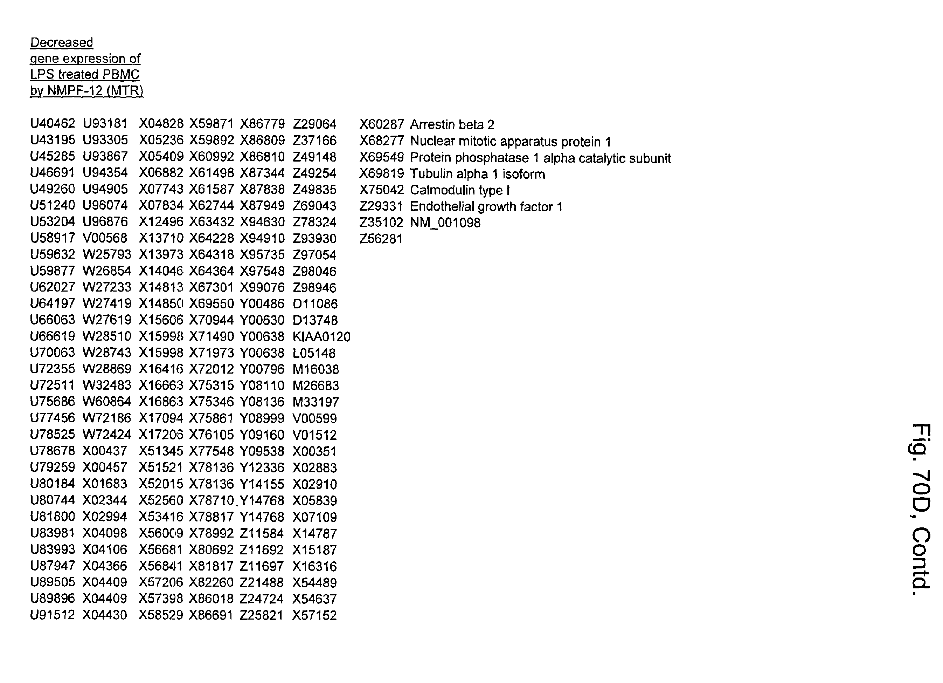

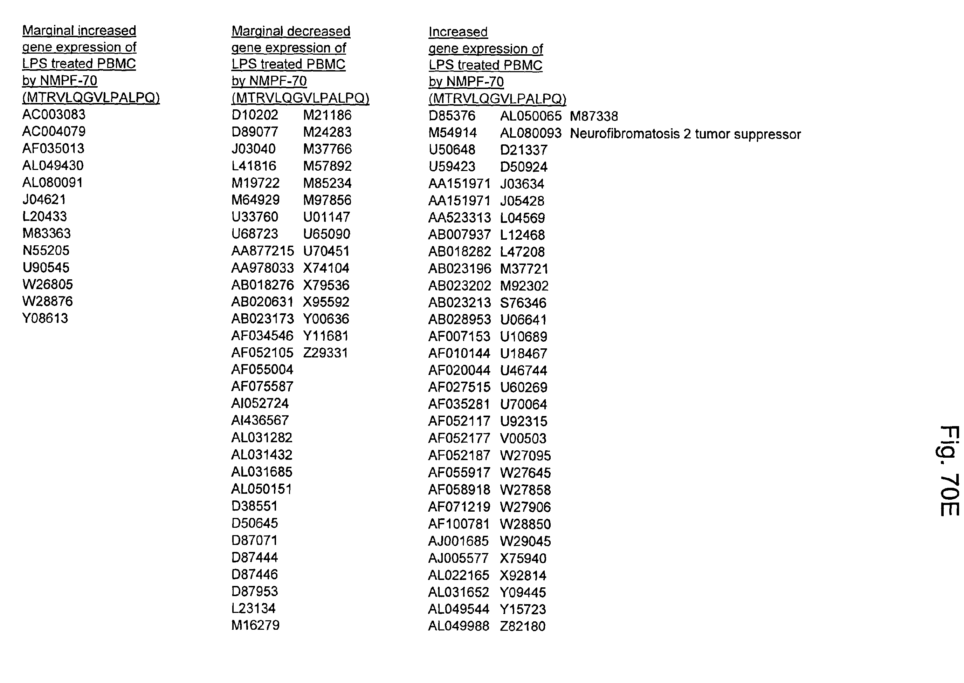

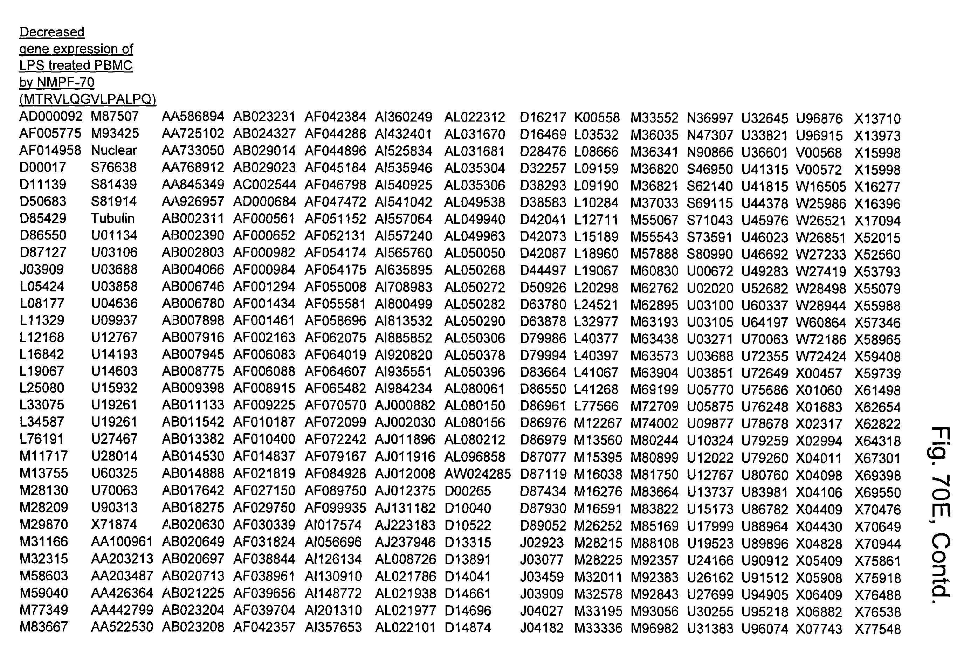

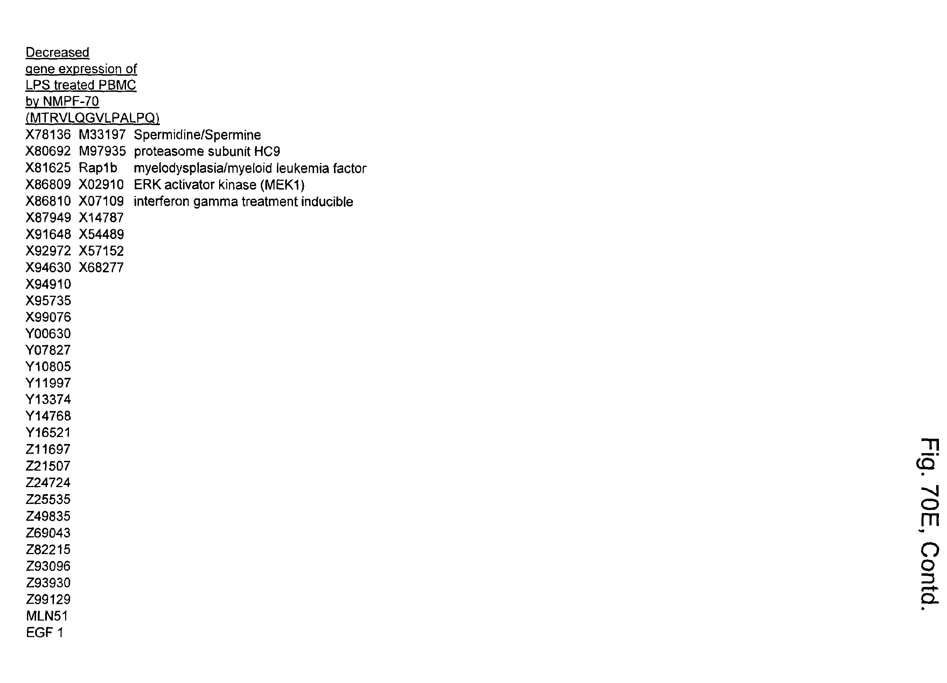

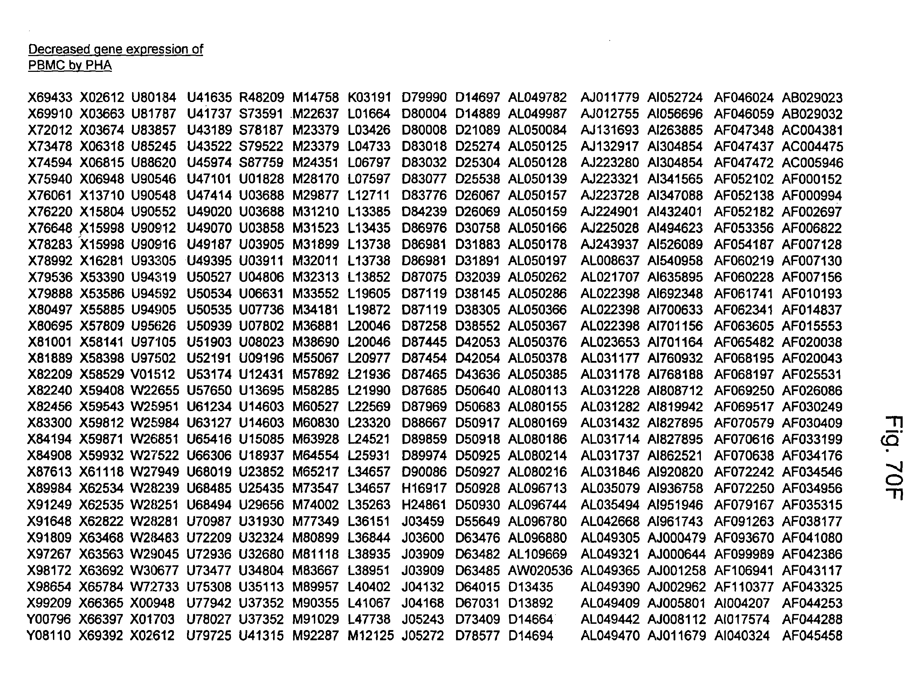

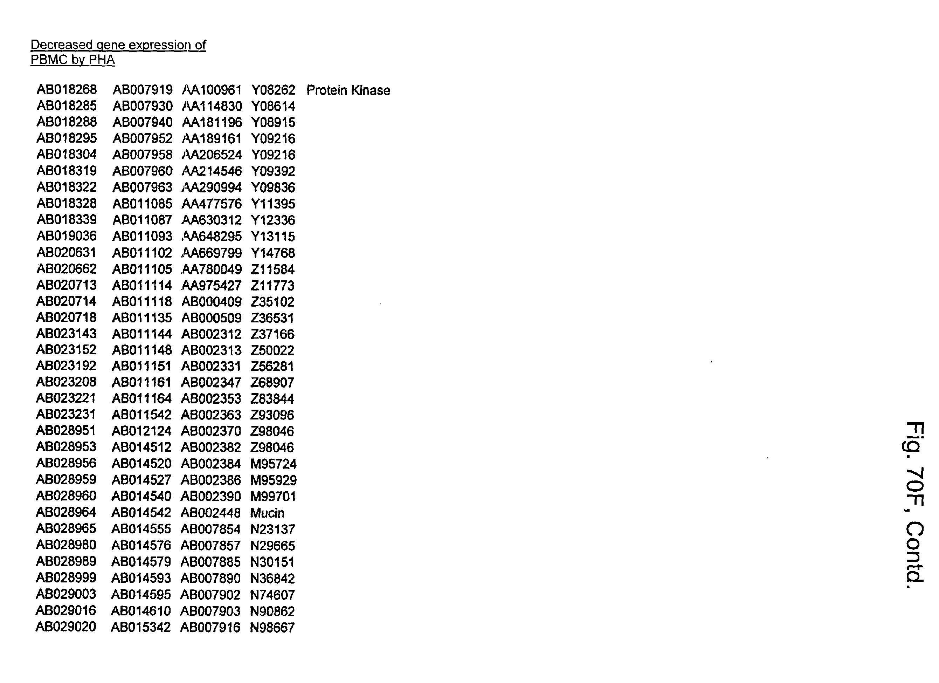

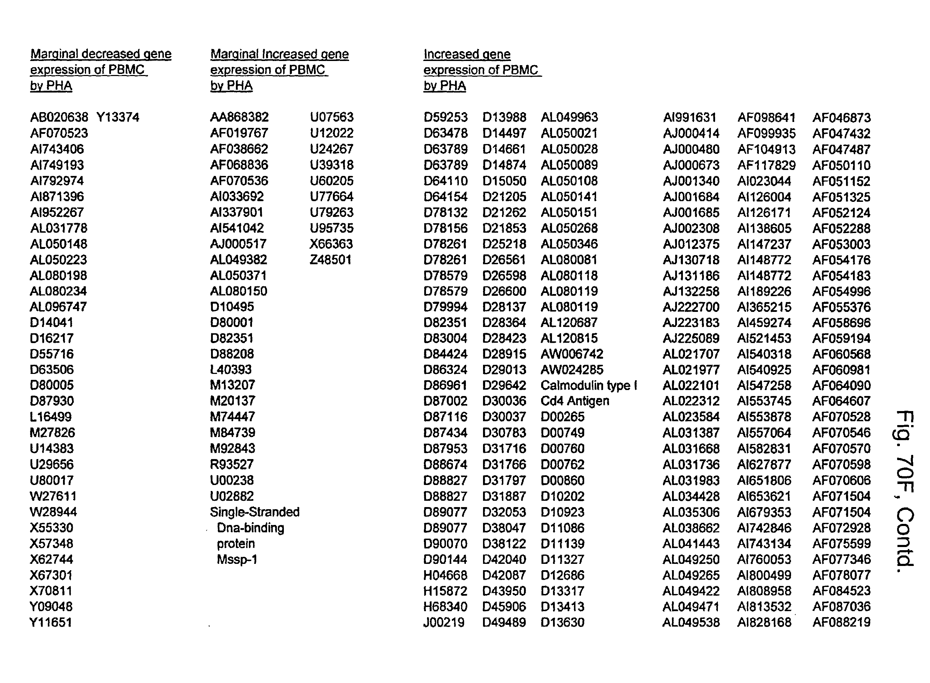

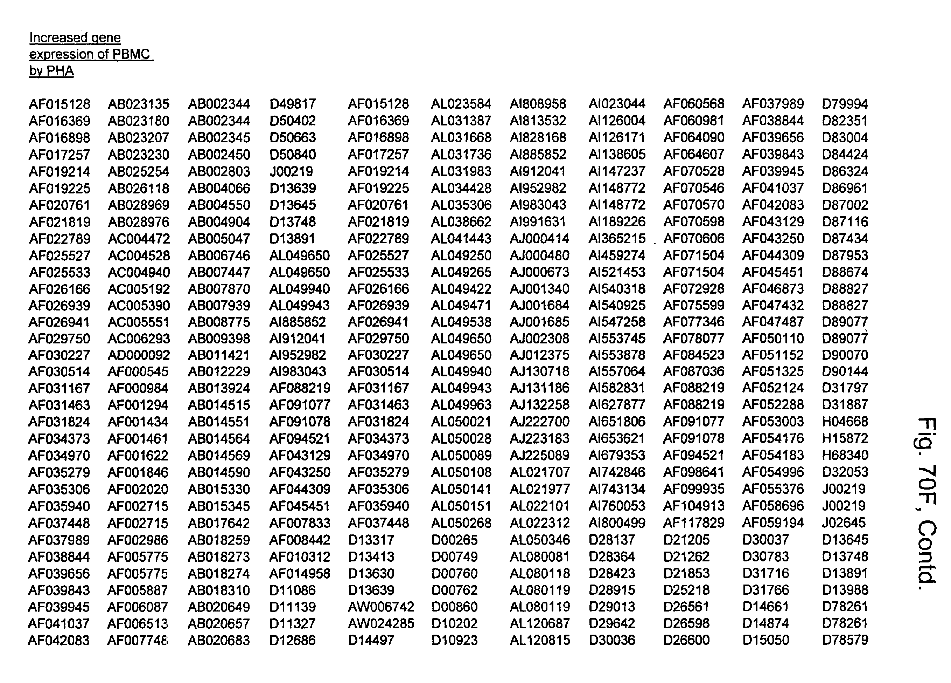

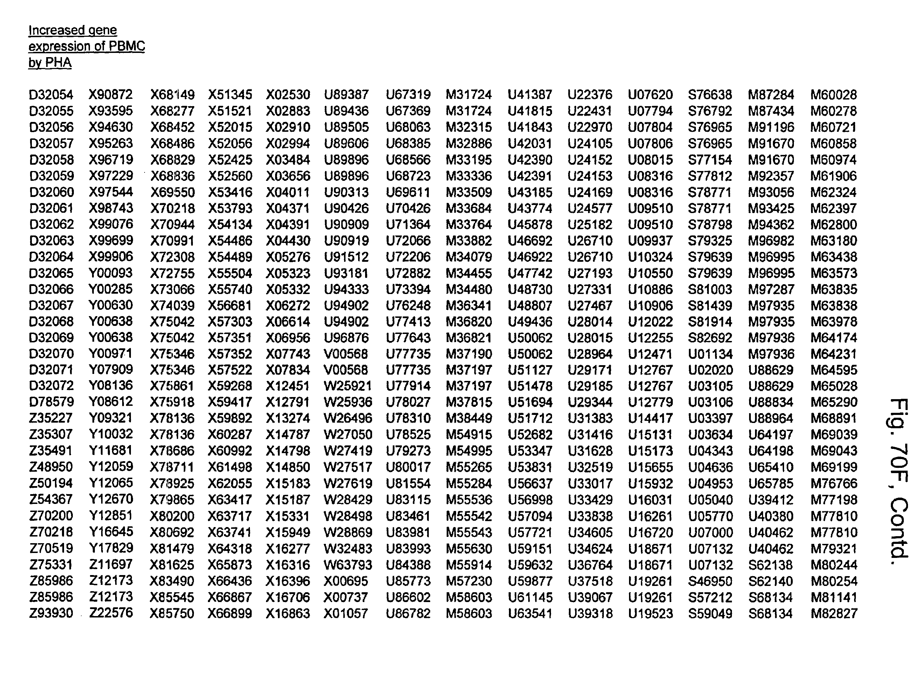

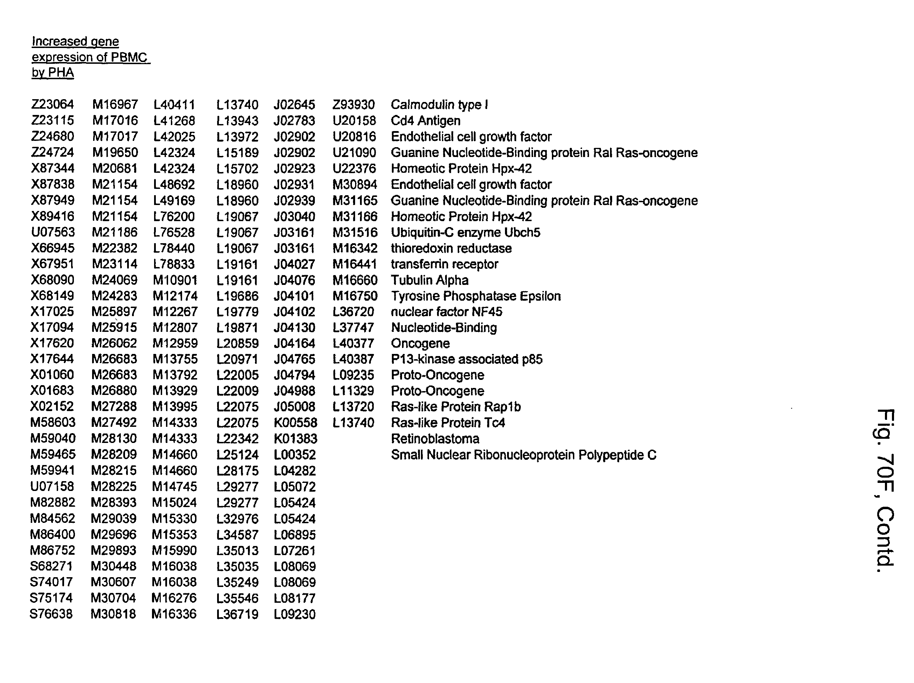

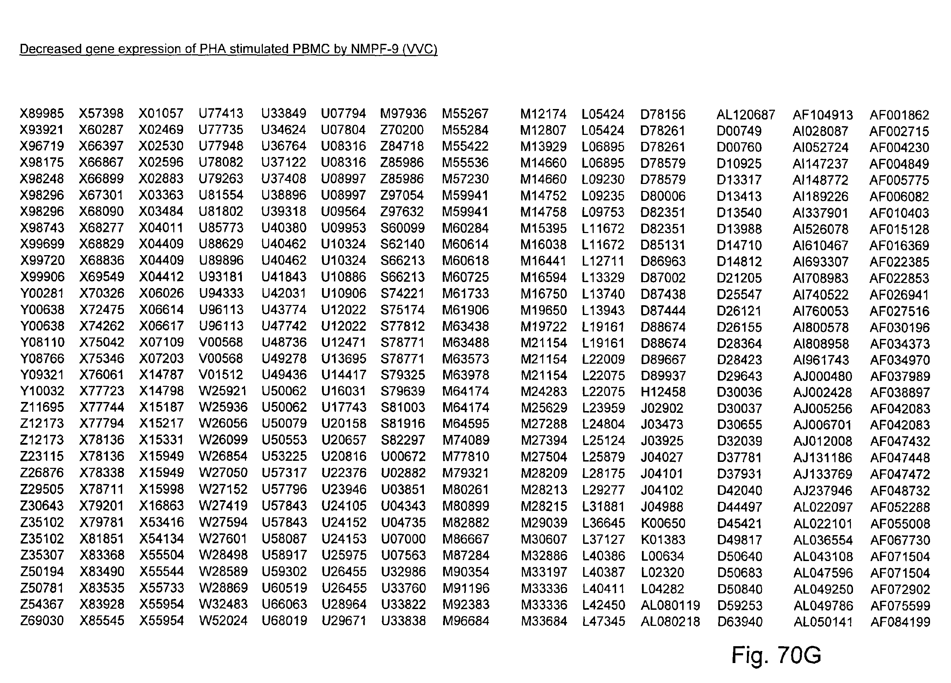

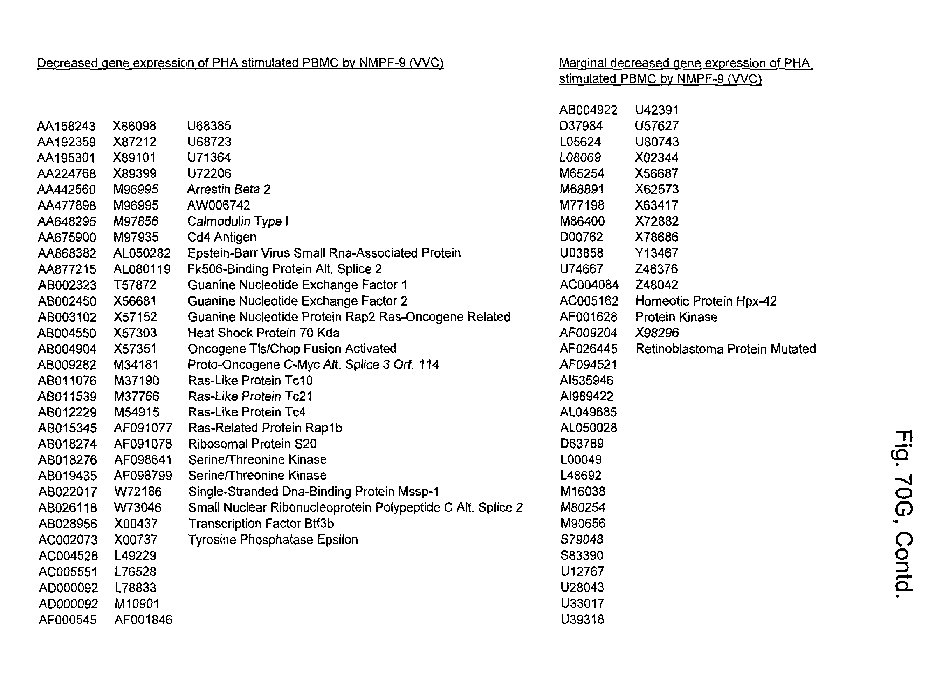

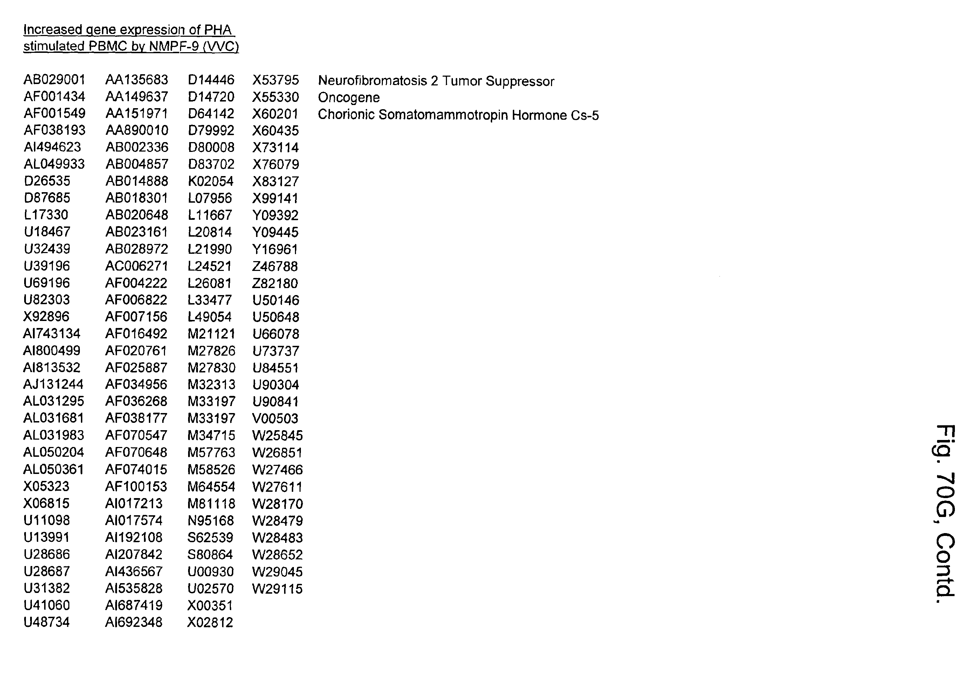

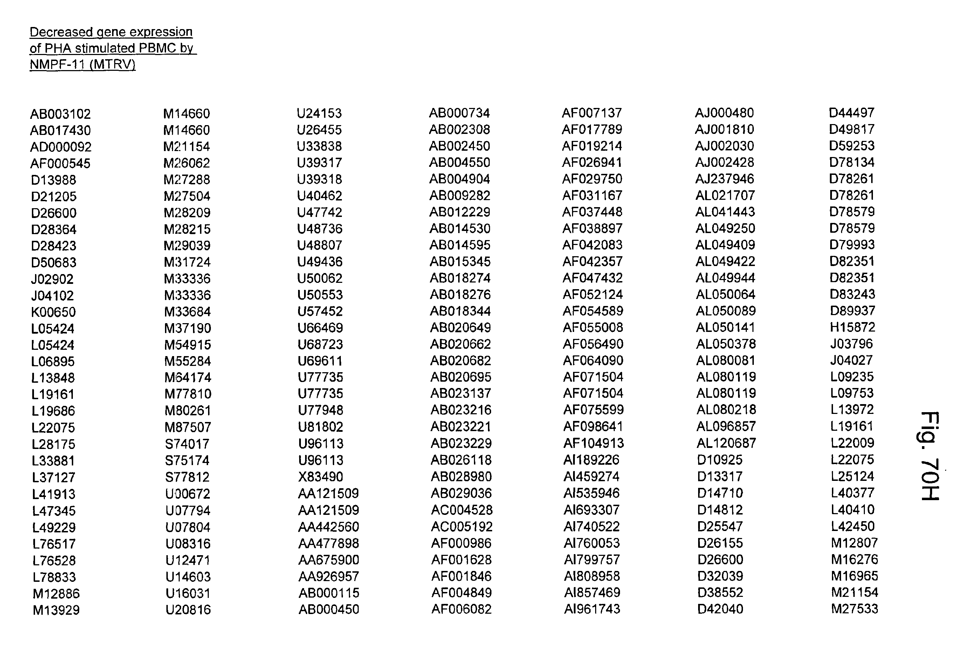

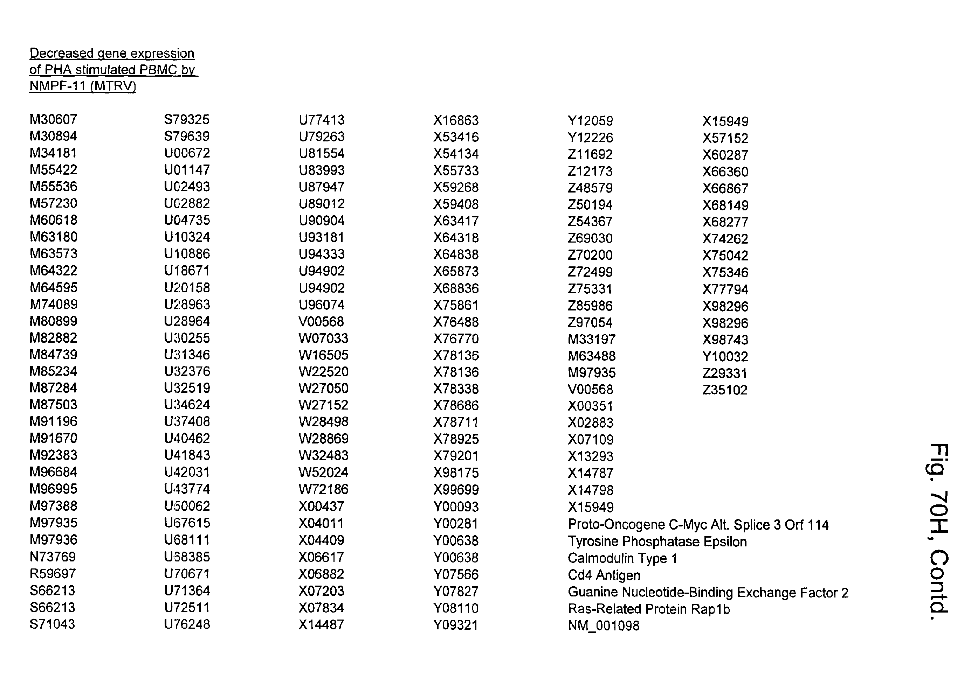

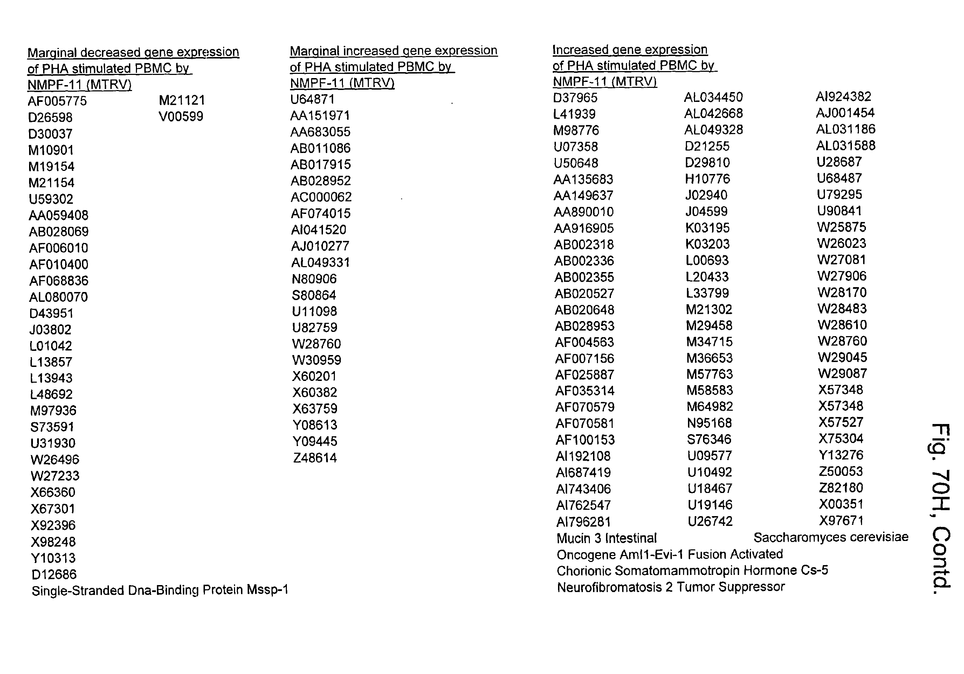

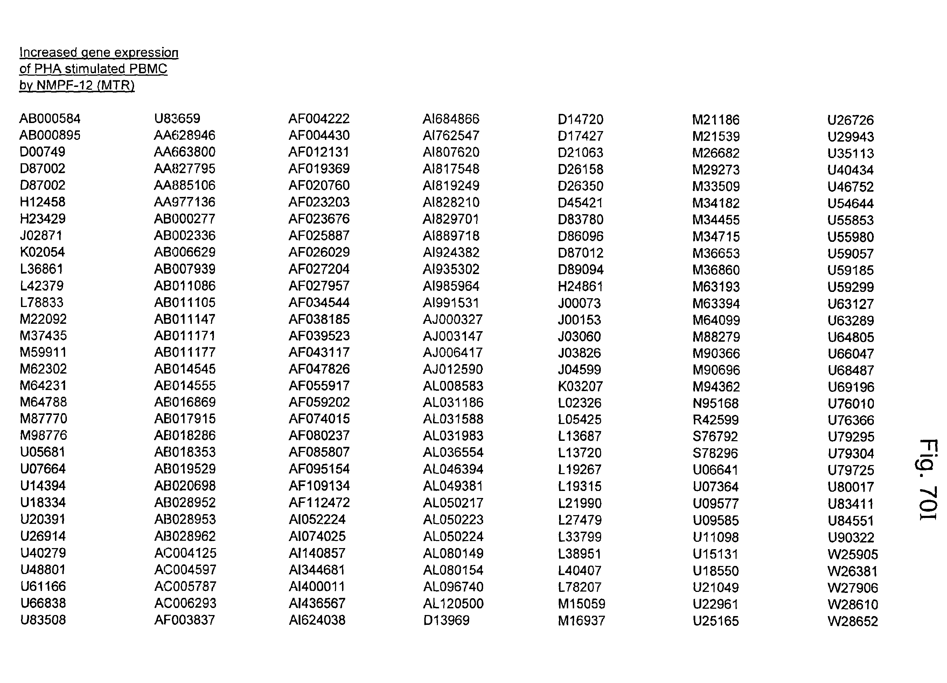

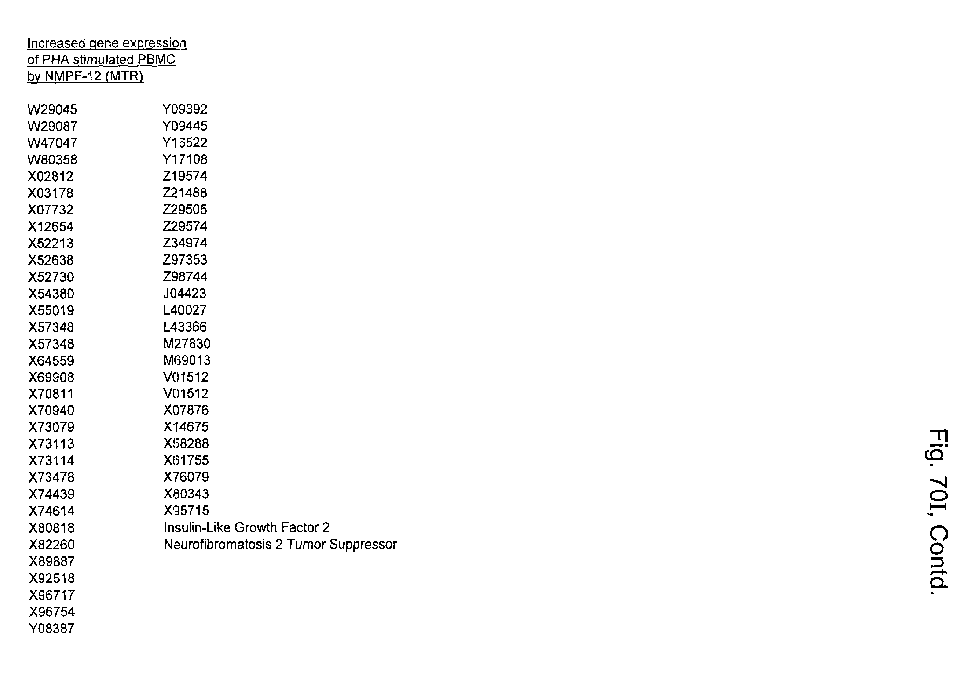

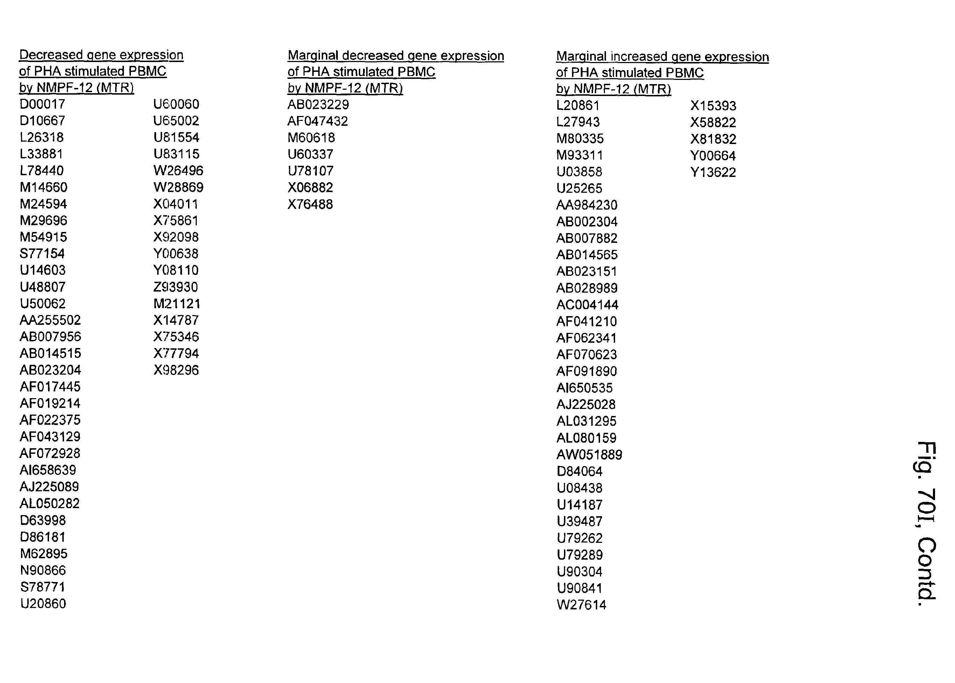

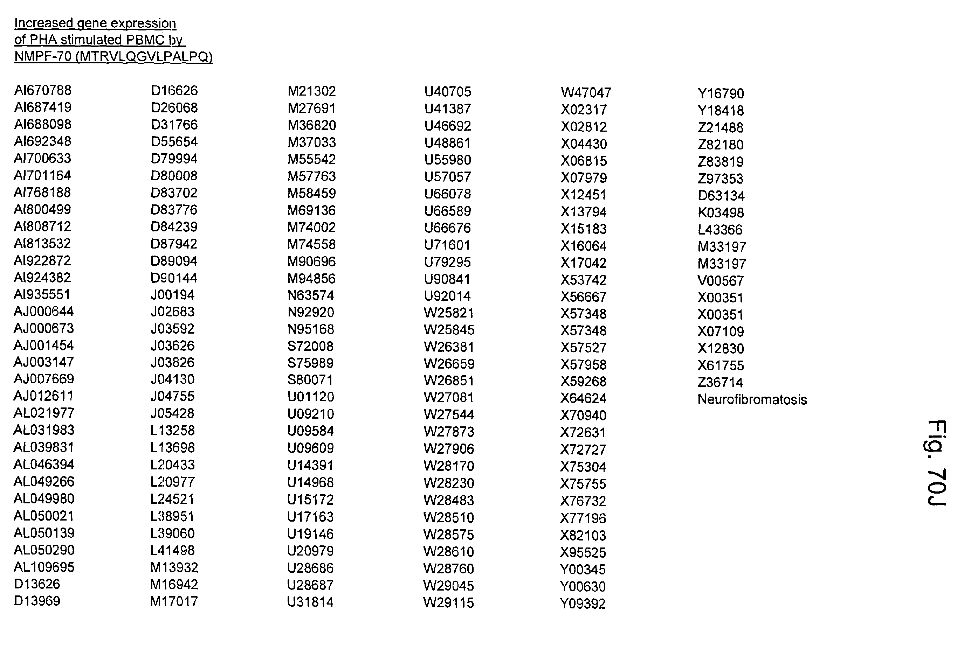

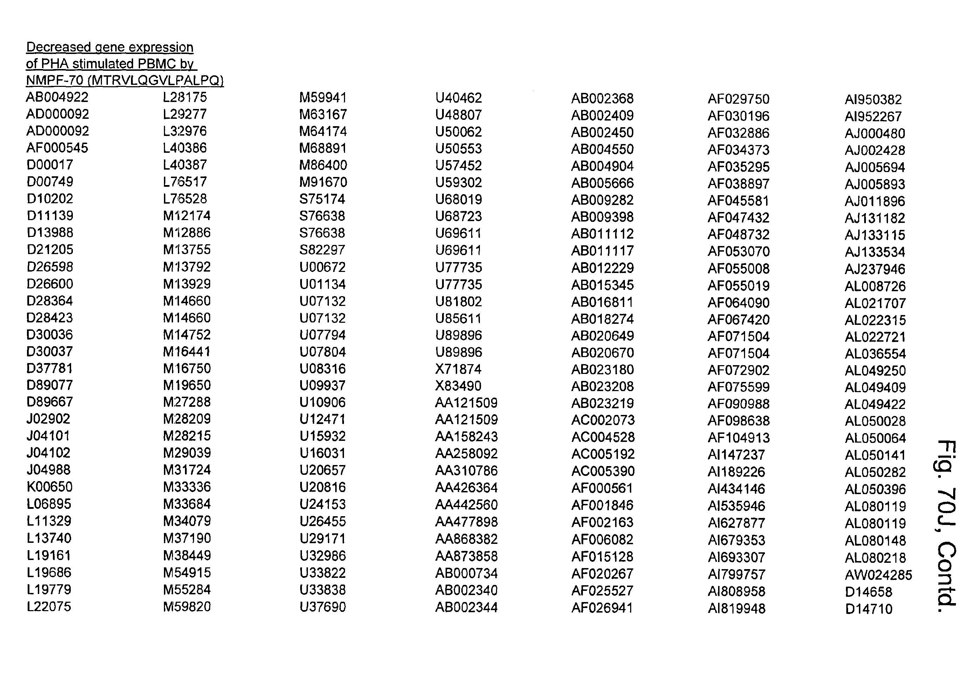

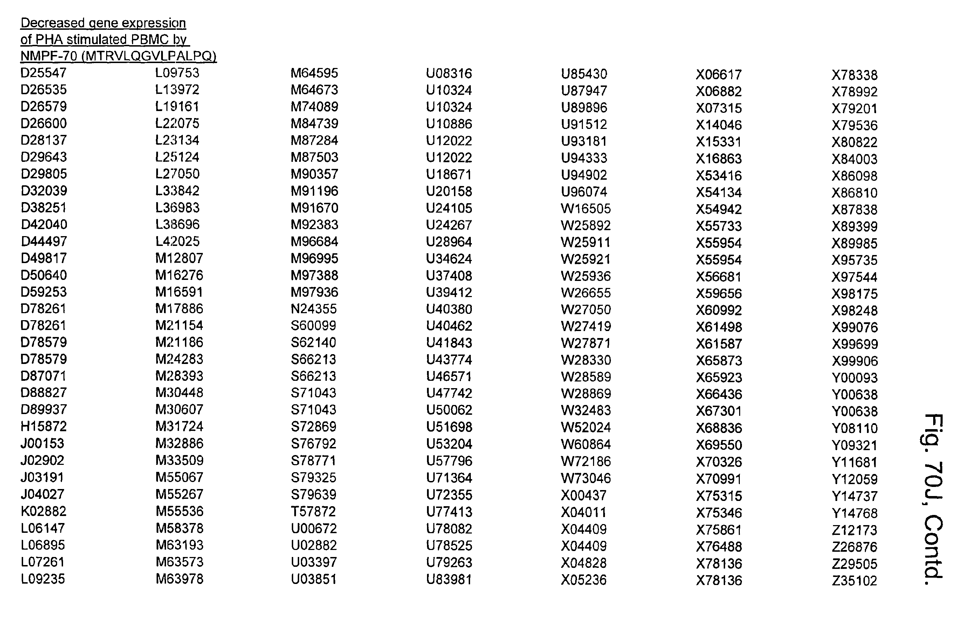

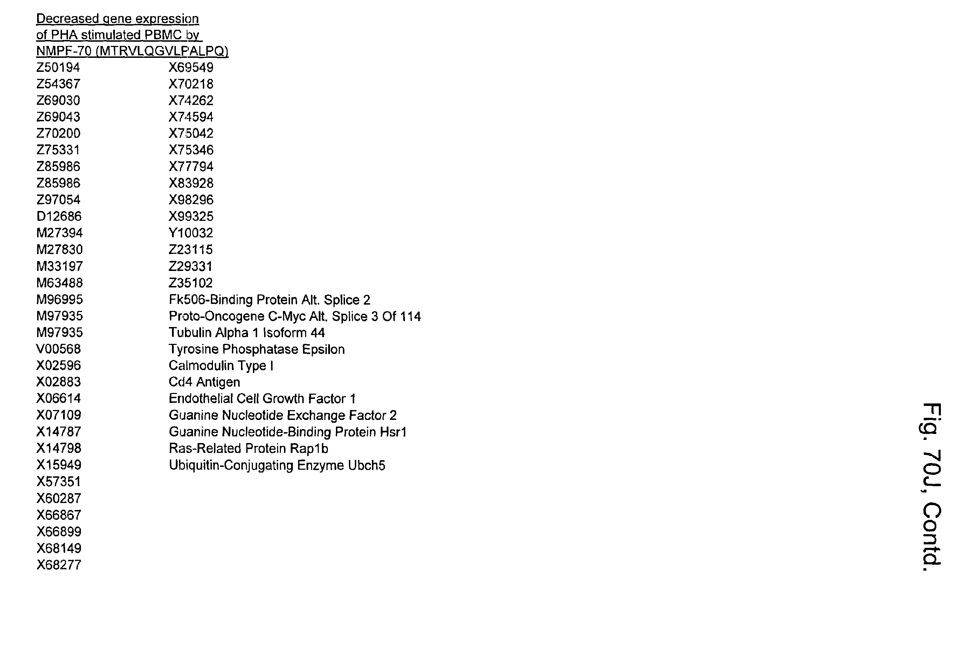

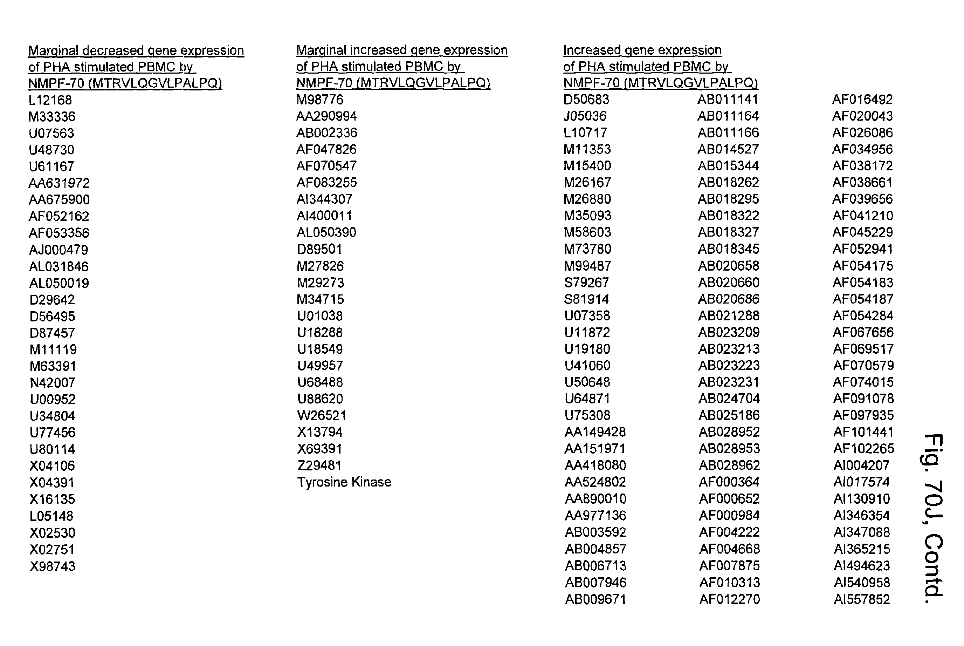

Furthermore, the invention provides use of a signalling molecule according to the invention for the production of a pharmaceutical composition for the modulation of gene expression and use in a method of treatment by modulating gene expression. Particular genes that may be modulated by particular peptides are provided herein, among others in figure 70

Down-regulating TNF-alpha itself, and/or a receptor for TNF-alpha, as is herein also provided, is also beneficial in individuals with Chagas cardiomyopathy.

Also, use of a signalling molecule according to the invention for the preparation of a pharmaceutical composition for modulation of vascularization or angiogenesis in wound repair, in particular of burns, is herein provided. Also, use of a pharmaceutical composition as provided herein is provided in cases of post-operative physiological stress, whereby not only vascularization will benefit from treatment, but the general well-being of the patient is improved as well.

Another use of a signalling molecule according to the invention comprises its use for the preparation of another pharmaceutical composition for the treatment of cancer. Such a pharmaceutical composition preferably acts via modulating and up-regulating apoptotic responses that are classically down-regulated by NF-kappaB activity. Inhibiting the activity with a signalling molecule according to the invention allows for increased cell death of tumorous cells. Another anti-cancerous activity of a signalling molecule as provided herein comprises down-regulation of c-myb, in particular in the case of hematopoietic tumors in humans. In this context, down-regulation of 14.3.3 protein is also provided.

A further use of a signalling molecule according to the invention comprises its use for the preparation of a further pharmaceutical composition for the treatment of cancer. Such a pharmaceutical composition preferably acts via modulating and down-regulating transferrine receptor availability, in particular on tumorous cells. Transferrine receptors are classically up-regulated by NF-kappaB activity. Inhibiting the activity with a signalling molecule according to the invention allows for reduced iron up-take and increased cell death of tumorous cells. In particular, erythroid and thromboid cells are susceptible to the treatment.

Yet a further use of a signalling molecule according to the invention comprises its use for the preparation of yet another pharmaceutical composition for the treatment of cancer, in particular of cancers that are caused by viruses, such as is the case with retroviral-induced malignancies and other viral-induced malignancies. Such a pharmaceutical composition preferably acts via modulating and down-regulating cell-proliferative responses that are classically up-regulated by virus-induced transcriptional or NF-kappaB activity. Inhibiting the activity with a signalling molecule according to the invention allows for decreased proliferation and increased cell death of tumorous cells. Such a pharmaceutial composition may also act via modulating angiogenic responses induced by IL-8, whereby for example inhibition of IL-8 expression via inhibition of transcription factor AP-1 or NF-kappaB expression results in the inhibition of vascularization-dependent tumor growth.

Furthermore, the invention provides the use of a signalling molecule for the preparation of a pharmaceutical composition for optimizing human or animal fertility and embryo survival, and a method for optimizing fertility and embryo survival. In particular, the invention provided for a method and composition allowing the down-regulation of TNF-alpha in the fertilized individual, optimally in combination with a composition and method for up-regulating IL-10 in the individual. Such a composition and method find immediate use in both human and veterinary medicine.

Also, the invention provides the use of a signalling molecule for the preparation of a pharmaceutical composition for modulating the body weight of an individual, in particular by modulating gene expression of a gene under influence of peroxisome proliferator-activated receptor gamma (PPARgamma) activation and lipid metabolism by applying a signalling molecule according to the invention, and a method for modulating body weight comprising providing an individual with a signalling molecule according to the invention.

A further use of a signalling molecule as provided herein lies in the modulation of expression of a gene in a cultured cell, as is for example provided herein in figure 70. Such a method as provided herein comprises subjecting a signalling molecule according to the invention to the cultured cell. Proliferation and/or differentiation of cultured cells (cells having been or being under conditions of *in vitro* cell culture known in the art) can be modulated by subjecting the cultured cell to a signalling molecule according to the invention. It is contemplated that for example research into proliferation or differentiation of cells, such as stem-cell research, will benefit greatly from understanding that a third major way of effecting gene modulation exists and considering the ease of application of synthetic peptides, and analogues or derivatives thereof.

Furthermore, it is contemplated that a signalling molecule as provided herein finds an advantageous use as a co-stimulatory substance in a vaccine, accompanying modern day adjuvants or replacing the classically used mycobacterial adjuvants, especially considering that certain mycobacteria express hCG-like proteins, of which it is now postulated that these bacteria have already made use of this third pathway found in gene modulation as provided herein by providing the host with breakdown products mimicking the signalling molecules identified herein. Treatment and use of the compositions as provided herein is not restricted to animals only, plants and other organisms are also subject to this third pathway as provided herein. Furthermore, now that the existence of such a pathway has been demonstrated, it is herein provided to make it a subject of diagnosis as well, for example to determine the gene modulatory state of a cell in a method comprising determining the presence or absence of a signalling molecule as provided herein or determining the presence or absence of a protease capable of generating such a signalling molecule from a (preferable endogenous) protein.

### BRIEF DESCRIPTION OF THE FIGURES

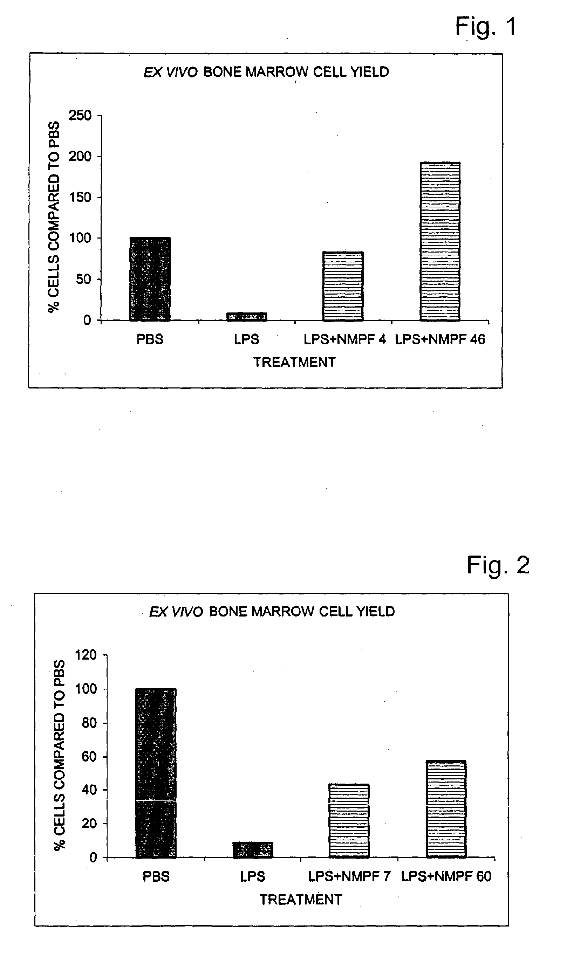

Figures 1-2. Bone marrow (BM) cell yield of treated BALB/c mice (n=6). BM cells were isolated from treated mice and cultured *in vitro* in the presence of rmGM-CSF for nine days. These figures show cell yield after nine days of culture of BM cells isolated from mice treated with PBS, LPS or LPS in combination with NMPF peptides 4, 46, 7 and 60. In these figures cell yield is expressed in relative percentage of cells compared to PBS. Each condition consists of 6 Petri dishes and results shown in these figures are representative of 6 dishes. Differences of ≥ 20% were considered significant and line bars represent significant data as compared to LPS control group. A representative experiment is shown. Findings involving all experimental conditions were entirely reproduced in 3 additional experiments.

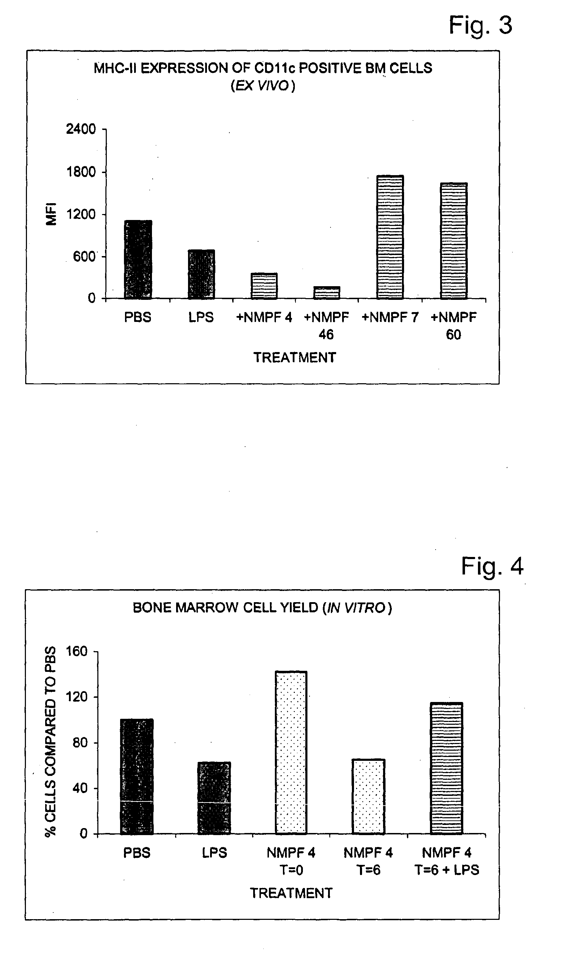

Figure 3. Effect of *in vivo* treatment on MHC-II expression on CD11c⁺ cells. Bone marrow (BM) cells were isolated from treated BALB/c mice (n=6) and cultured *in vitro* in the presence of rmGM-CSF for nine days. This figure shows MHC-II expression expressed in mean fluorescence intensity (MFI) after nine days of culturing of BM cells isolated from PBS, LPS or LPS in combination with NMPF. Each condition consists of 6 Petri dishes and results shown in these figures are representative of 6 dishes. Differences of ≥ 20% were considered significant and line bars represent significant data as compared to the LPS control group. A representative experiment is shown. Findings involving all experimental conditions were entirely reproduced in 3 additional experiments.

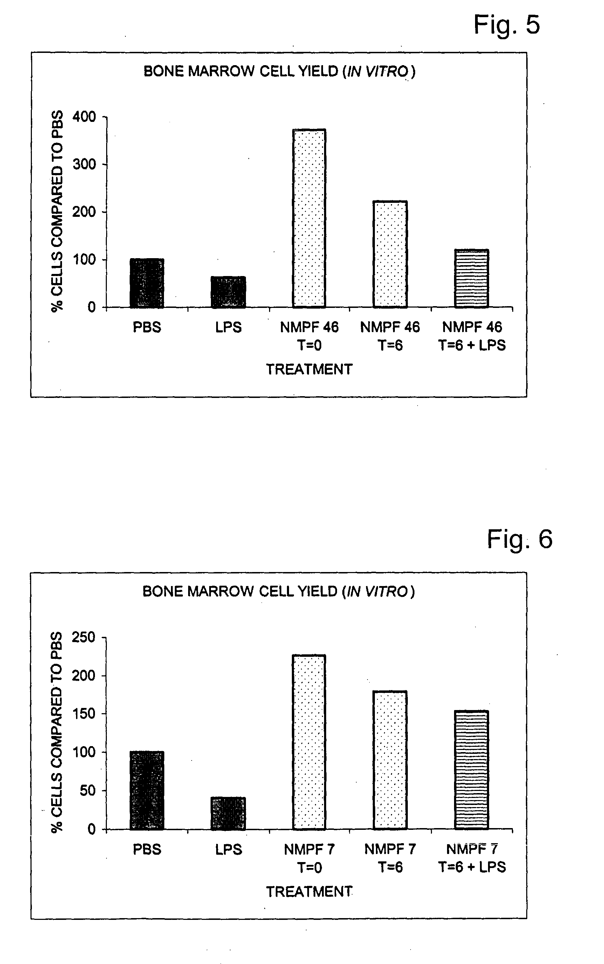

Figures 4-7. Bone marrow (BM) cell yield of *in vitro* treated BM cultures. BM cells from BALB/c mice (n=3) were cultured *in vitro* and treated with either PBS, LPS (t=6 day), NMPF 4, 7, 46, 60 (t=0 or t=6 day) or a combination of NMPF with LPS (t=6 day), in the presence of rmGM-CSF for nine days. These figures show cell yield expressed in relative percentage of cells compared to PBS after nine days of culture of BM cells. Each condition consists of 6 Petri dishes and results shown in these figures are representative of 6 dishes. Differences of ≥ 20% were considered significant. Line bars represent significant data as compared to LPS control group and dotted bars represent significant data as compared to PBS group. A representative experiment is shown. Findings involving all experimental conditions were entirely reproduced in 3 additional experiments.

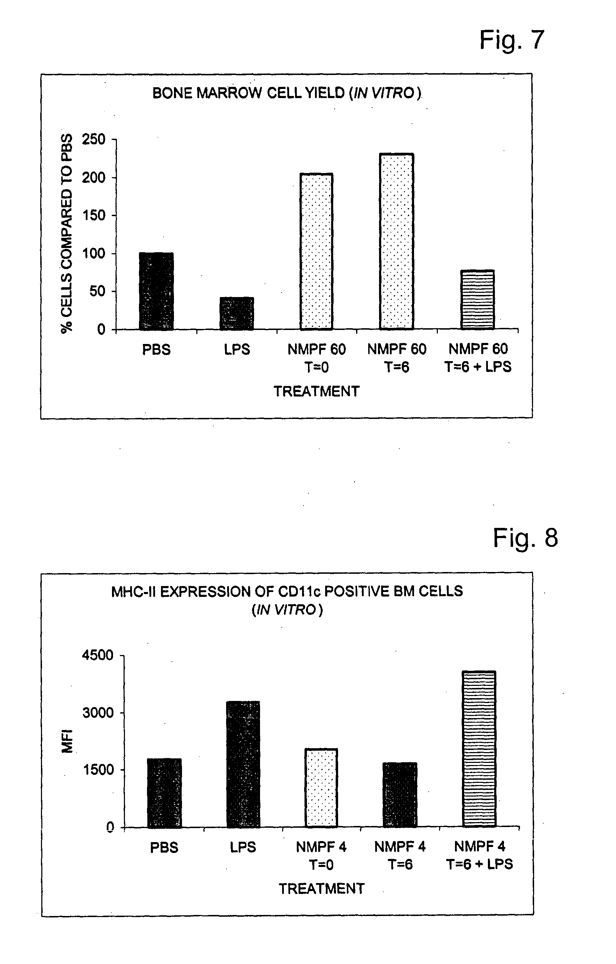

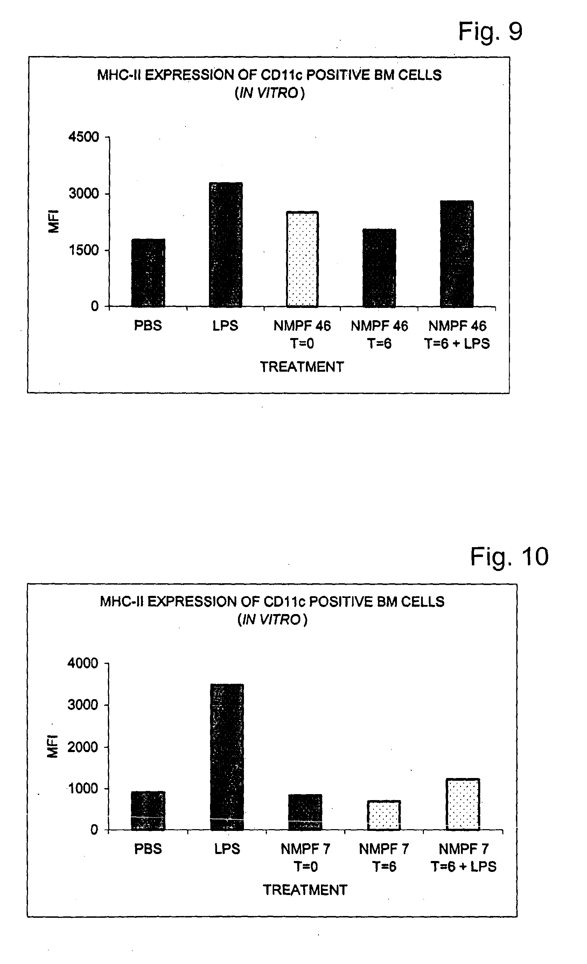

Figure 8-11. Effect of *in vitro* treatment on MHC-II expression on CD11c⁺ cells. BM cells from BALB/c mice (n=3) were cultured *in vitro* and treated with either PBS, LPS (t=6 day), NMPF 4, 7, 46, 60 (t=0 or t=6 days) or a combination of NMPF with LPS (t=6 days), in the presence of rmGM-CSF for nine days. These figures show MHC-II expressed in mean fluorescence intensity (MFI) of CD11c positive cells after nine days of culturing of BM cells. Each condition consists of 6 Petri dishes, and results shown in these figures are representative of 6 dishes. Differences of ≥ 20% were considered significant. Line bars represent significant data as compared to LPS control group and dotted bars represent significant data as compared to PBS group. A representative experiment is shown. Findings involving all experimental conditions were entirely reproduced in 3 additional experiments.

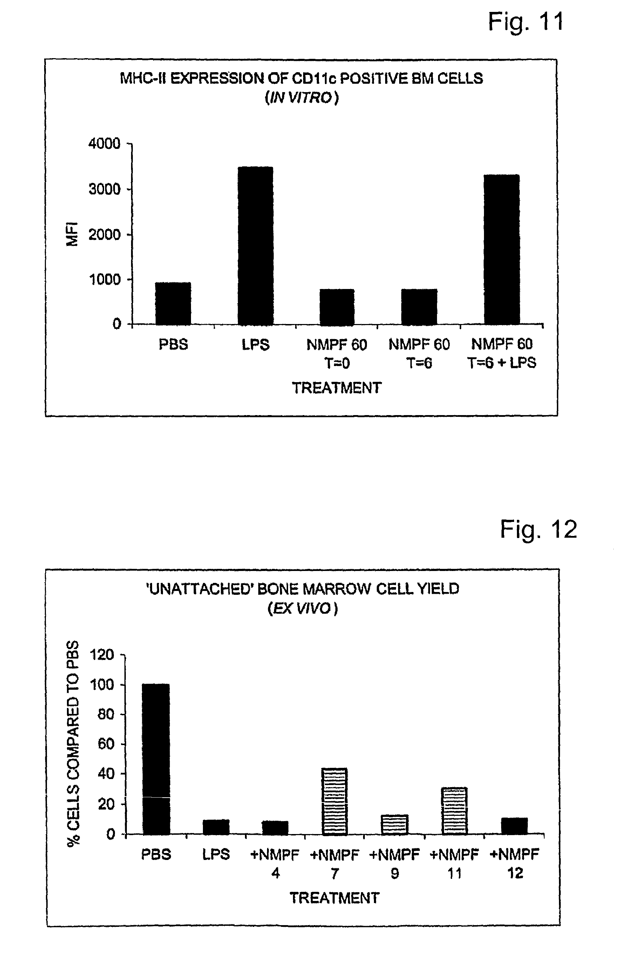

Figure 12-15. Bone marrow (BM) cell yield of treated BALB/c mice (n=6). BM cells were isolated from treated mice and cultured *ex vivo* in the presence of rmGM-CSF for nine days. These figures show cell yield after nine days of culture of BM cells in suspension (unattached) and attached to Petri dish (attached). BM cells were isolated from mice treated with PBS, LPS or LPS in combination with different NMPF peptides. In these figures cell yield is expressed in relative percentage of cells compared to PBS. Each condition consists of 6 Petri dishes and results shown here are representative of 6 dishes. Differences of ≥ 20% were considered significant and line bars represent significant data as compared to LPS control group. A representative experiment is shown. Findings involving all experimental conditions were entirely reproduced in 3 additional experiments.

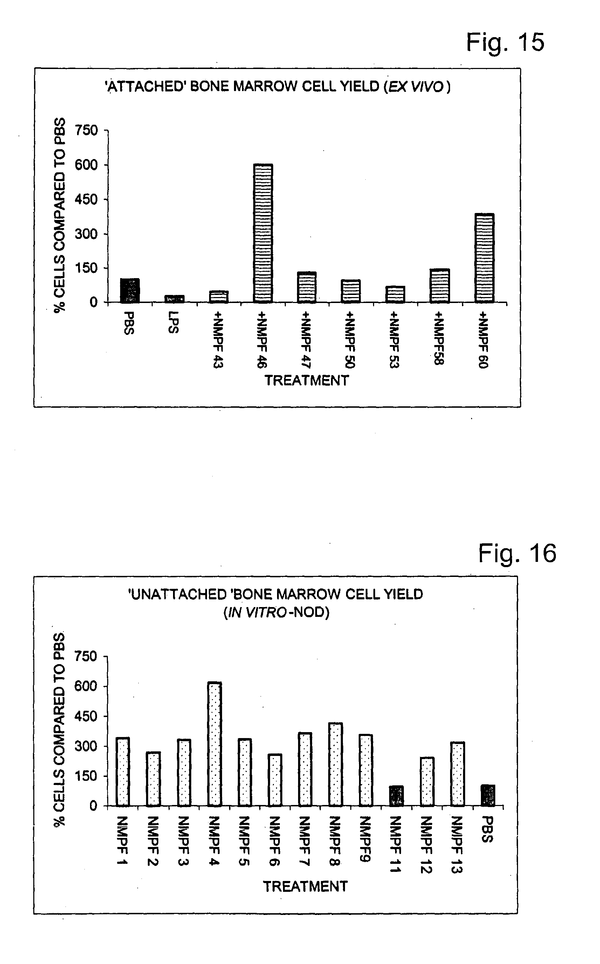

Figures 16-17. Bone marrow (BM) cell yield of *in vitro* treated BM cultures from NOD mice. BM cells from 15 week old female NOD mice (n=3) were cultured *in vitro* and treated with either PBS or NMPF in the presence of rmGM-CSF for nine days. These figures show cell yield after nine days of culture of BM cells in suspension (unattached) and attached to Petri dishes (attached). In these figures cell yield is expressed in relative percentage of cells compared to PBS. Each condition consists of 6 Petri dishes and results shown here are representative of 6 dishes. Differences of ≥ 20% were considered significant and dotted bars represent significant data as compared to PBS control group. A representative experiment is shown. Findings involving all experimental conditions were entirely reproduced in 3 additional experiments.

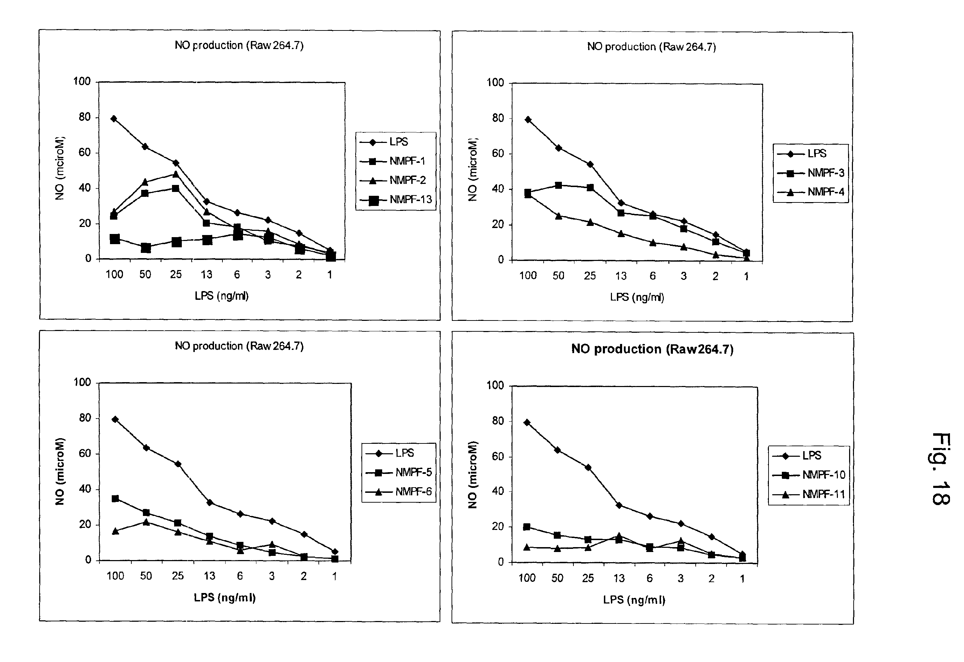

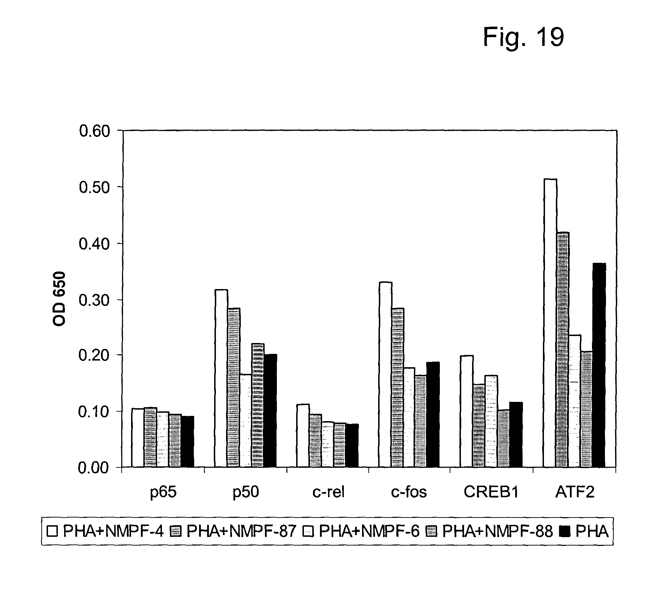

Figure 18. Effect of NMPF-1, 2, 3, 4, 5, 6, 10, 11 and 13 on LPS induced NO production (in micro molar) in macrophages (RAW264.7). This figure shows a significant inhibition of NO-production at LPS concentration ranging from 0.78 ng/ml to 100 ng/ml by 10 microgram/ml NMPF.

Figure 19.
Jurkat T cells were treated with PHA (10 microgram/ml) in the presence or absence NMPF 4 (LQGV), 87 (VGQL), 6 (VLPALP) and 88 (PLAPLV). After 3 hrs of incubation nuclear extracts were made and analyzed for transcription factors (p65, p50, c-REL, c-FOS, CREB1 and ATF2). This figure shows the effect of NMPF on these transcription factors.

Figures 20-32. In vivo treatment of fertilized chicken eggs with NMPF and the effect of NMPF on angiogenesis. Fertile chicken eggs (day 0) were treated with either PBS, NMPF, VEGF or VEGF in combination with NMPF. Ten eggs were injected for every condition. On day 8 of incubation, the embryos were removed from the eggs and were placed in a 100-mm Petri dish. The embryo and the blood vessels were photographed in vivo with the use of a microscope. Of each egg one overview picture was taken and 4 detail pictures of the blood vessels were taken. Quantification of angiogenesis (vessel branches) was accomplished by counting the number of blood vessel branches. Quantification of this vasculogenesis was accomplished by measuring the blood vessel thickness. The number of blood vessel branches and vessel thichness were measured in the pictures and were correlated to a raster (in the pictures) of 10mm² for comparison. The mean number of branches and the mean blood vessel thickness of each condition (N=10) were calculated and compared to either the PBS or VEGF controls using a Student's T-test. Line bars represent significant (p<0.05) data as compared to PBS control group and dotted bars represent significant (p<0.05) data as compared to VEGF group. Figures 20-30 show the effect of NMPF on vessel branches. Figures 31-32 show the effect of NMPF on vessel thickness.

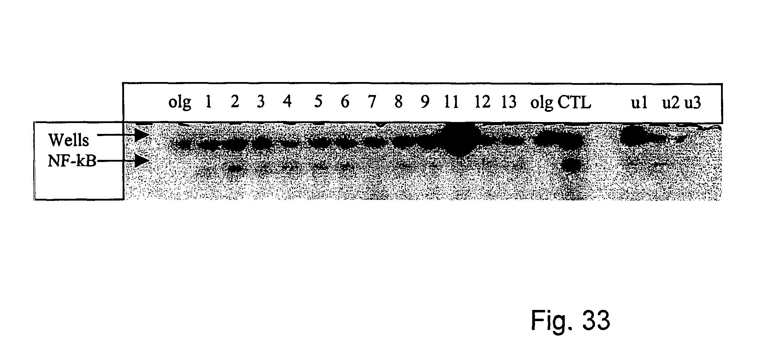

Figure 33. Detection of NF-kB via EMSA. This figure shows the presence of NF-kB in the nuclear extracts of RAW264.7 cells treated with LPS or NMPF in combination with LPS for 4 hours. Numbers 1-13 correspond to nuclear extracts from cells treated with NMPF and LPS. *CTL* corresponds to nuclear extracts from cells treated with LPS only. Specificity of the radioactively labeled NF-kB probe is shown by competition with the unlabeled oligonucleotide (u1,u2,u3) in three different concentrations (1x, 10x, 100x) with nuclear extracts of CTL and *olg* corresponding to samples containing only labeled oligonucleotide (without nuclear extract). Description: (NMPF-1)VLPALPQVVC, (NMPF-2)LQGVLPALPQ, (NMPF-3)LQG, (NMPF-4)LQGV, (NMPF-5)GVLPALPQ, (NMPF-6)VLPALP, (NMPF-7)VLPALPQ, (NMPF-8)GVLPALP, (NMPF-9)VVC, (NMPF-11)MTRV, (NMPF-12)MTR

Figure 34. HPLC chromatograph (wave length 206) in which data profile obtained from the nuclear protein extracts of LPS and LPS in combination with NMPF stimulated RAW264.7 cells are overlayed.

Figure 35. MSn analysis of NMPF-4 peptide.

Figure 36. MSn analysis of a fraction from nuclear extract of LPS stimulated RAW264.7 cells. Upper panel shows full spectrum of the fraction and lower panel shows the MS/MS spectrum of mass 413.13.

Figure 37. MSn analysis of a fraction from nuclear extract of LPS in combination with NMPF-4 stimulated RAW264.7 cells. Upper panel shows full spectrum of the fraction and lower panel shows the MS/MS spectrum of mass 416.07.

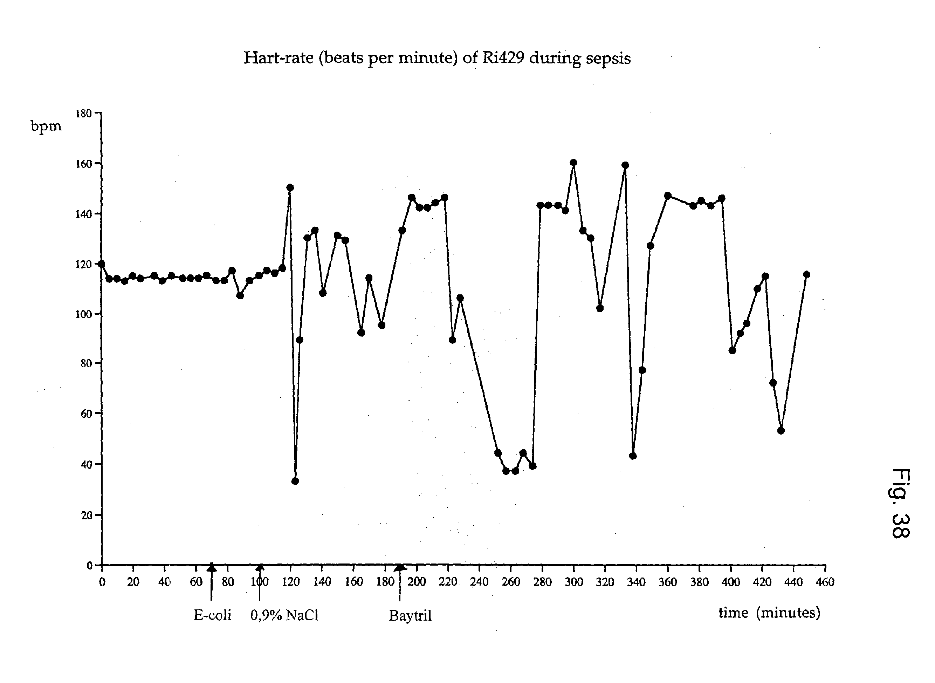

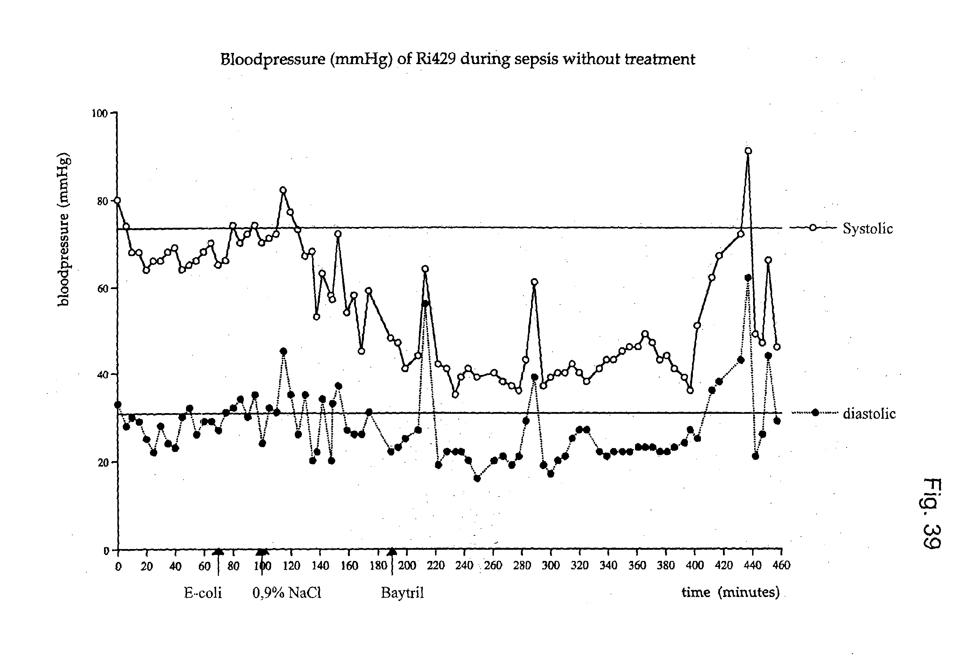

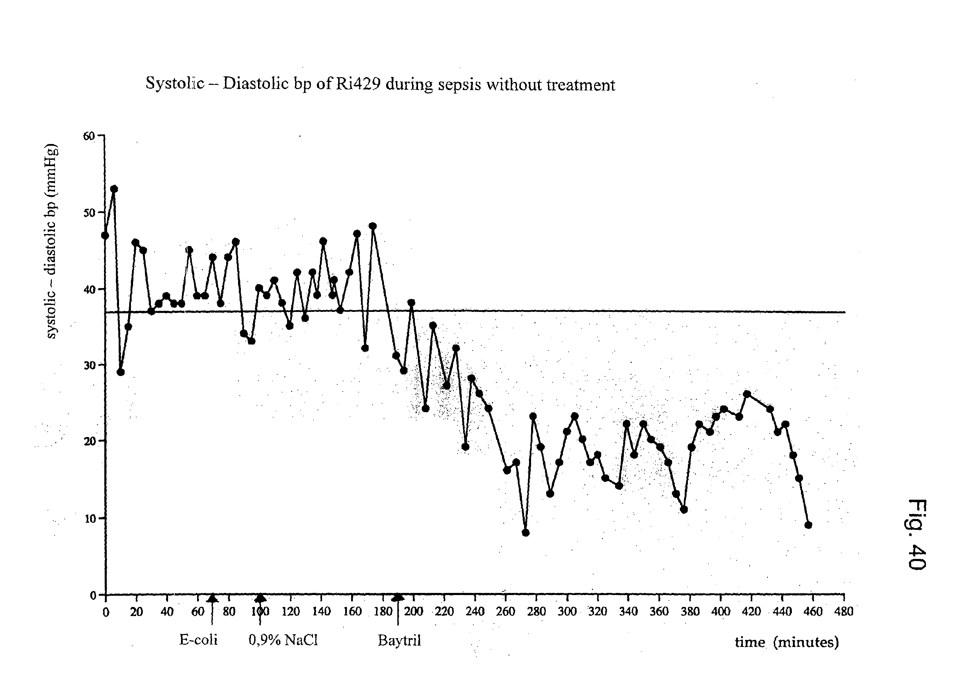

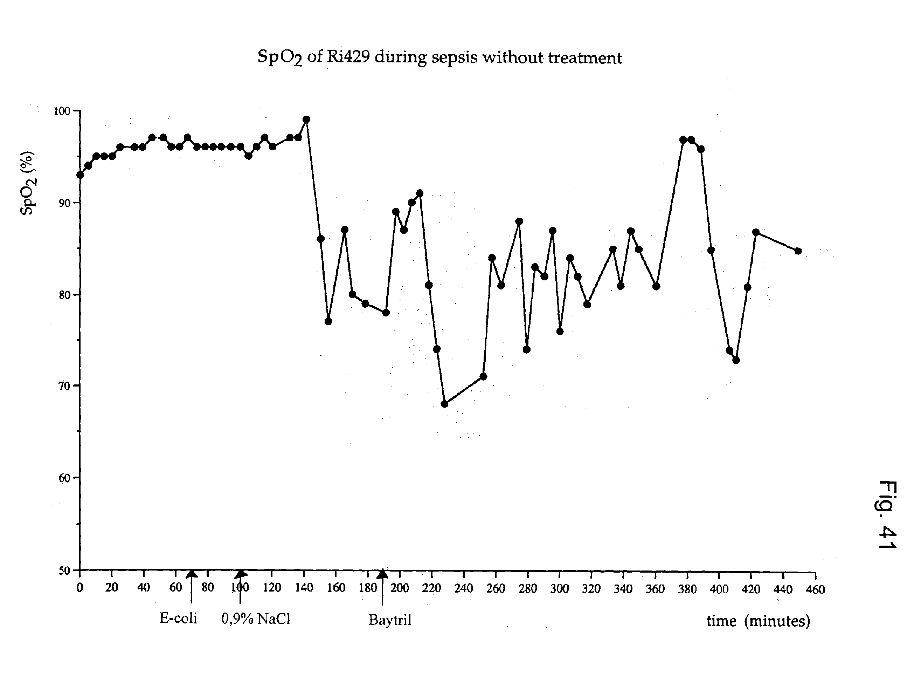

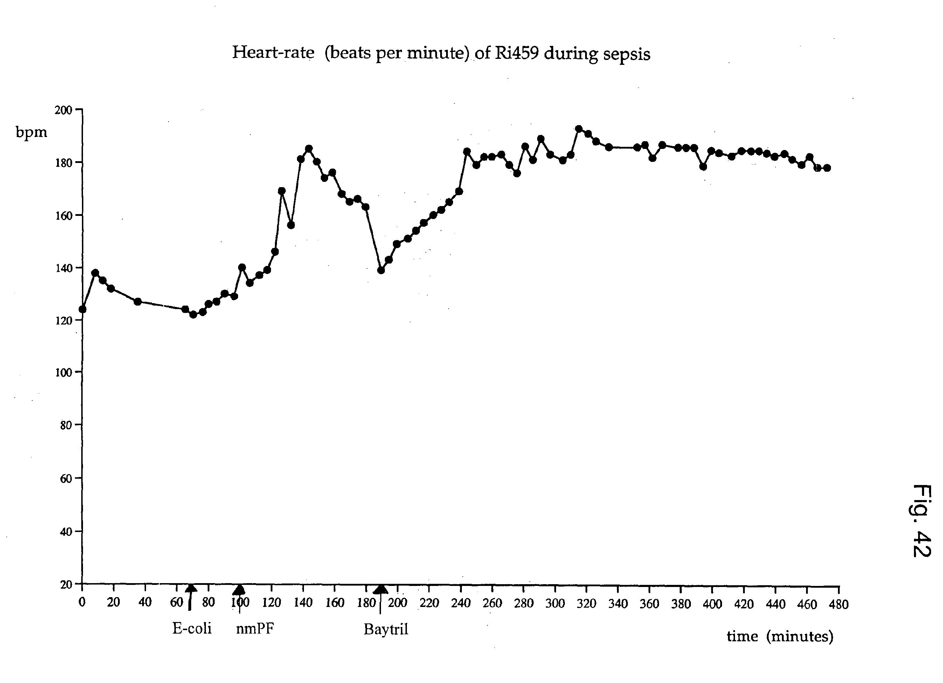

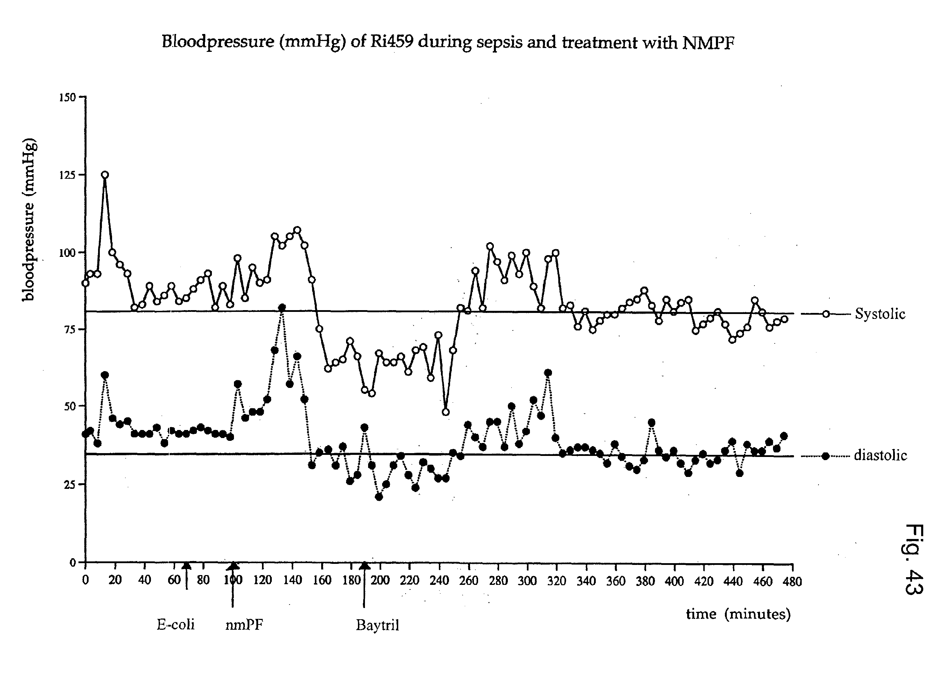

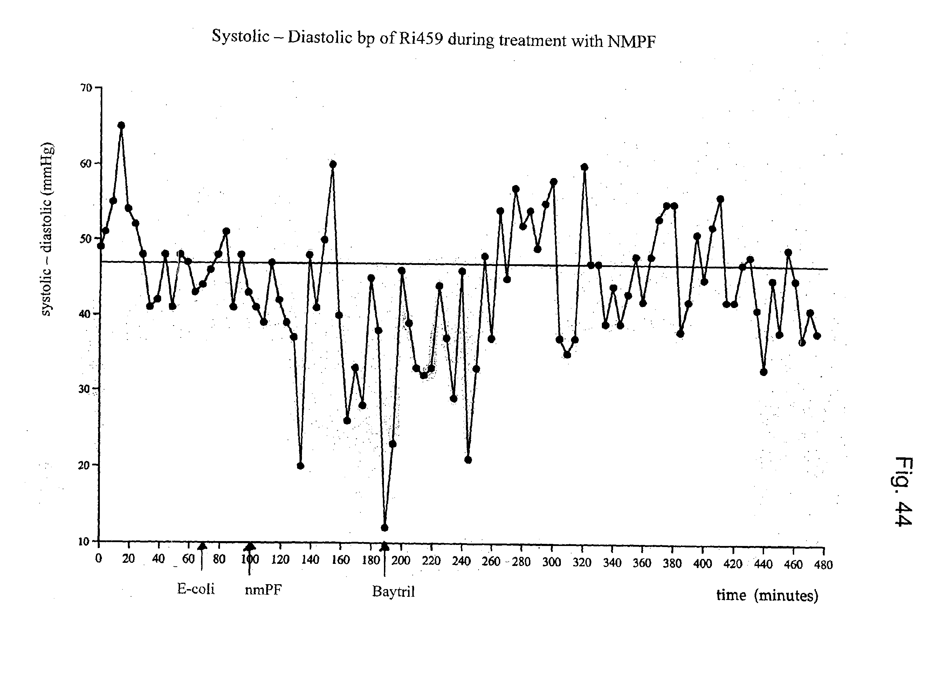

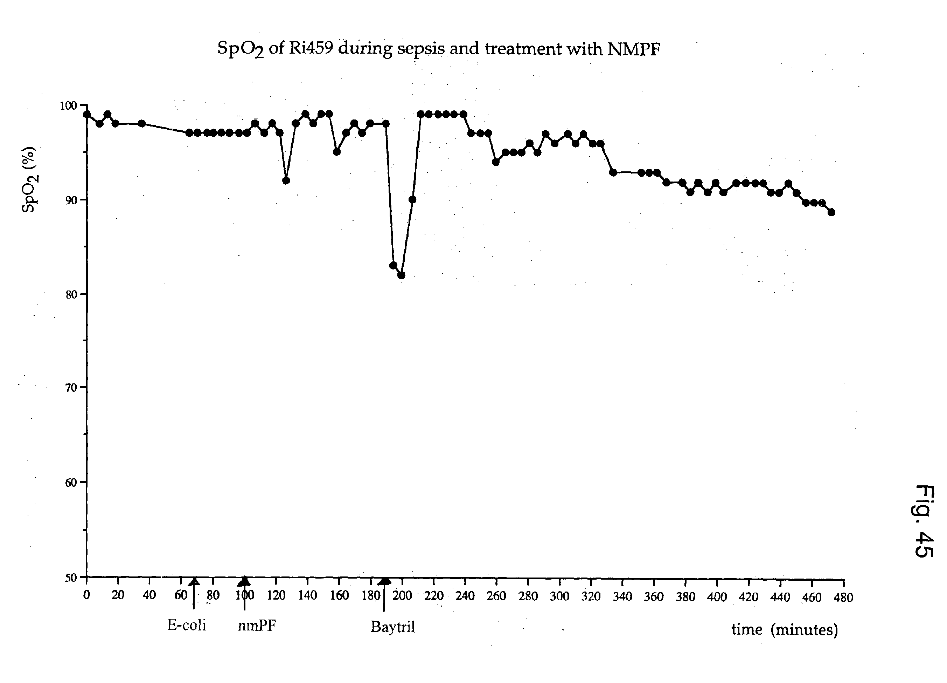

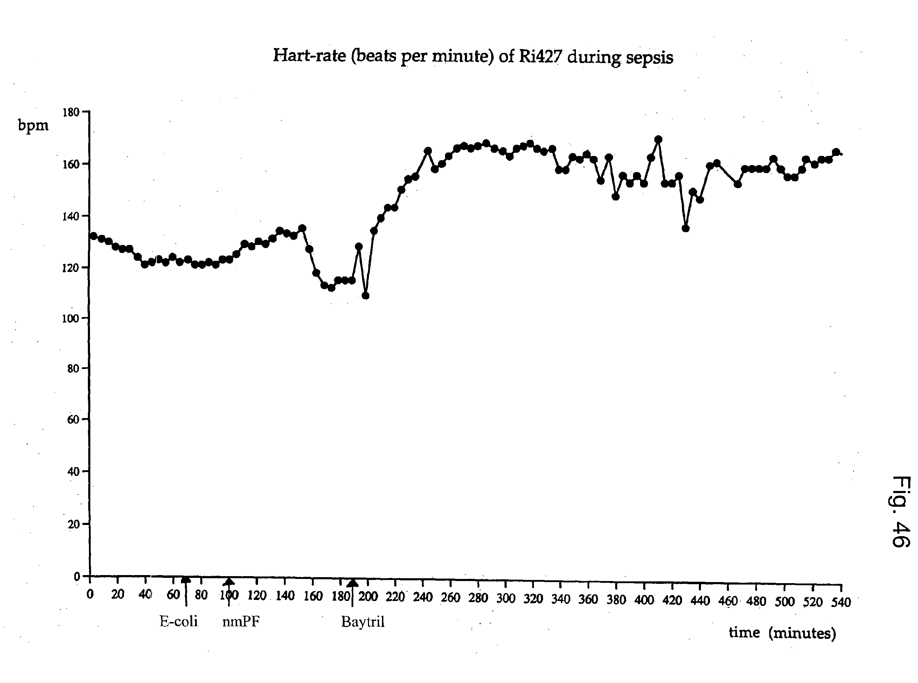

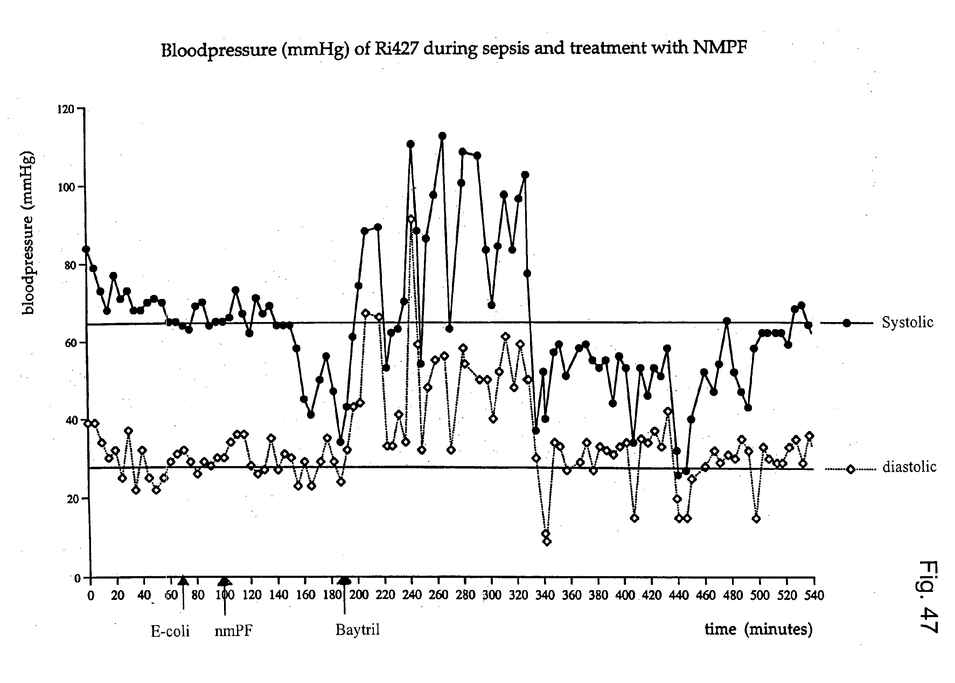

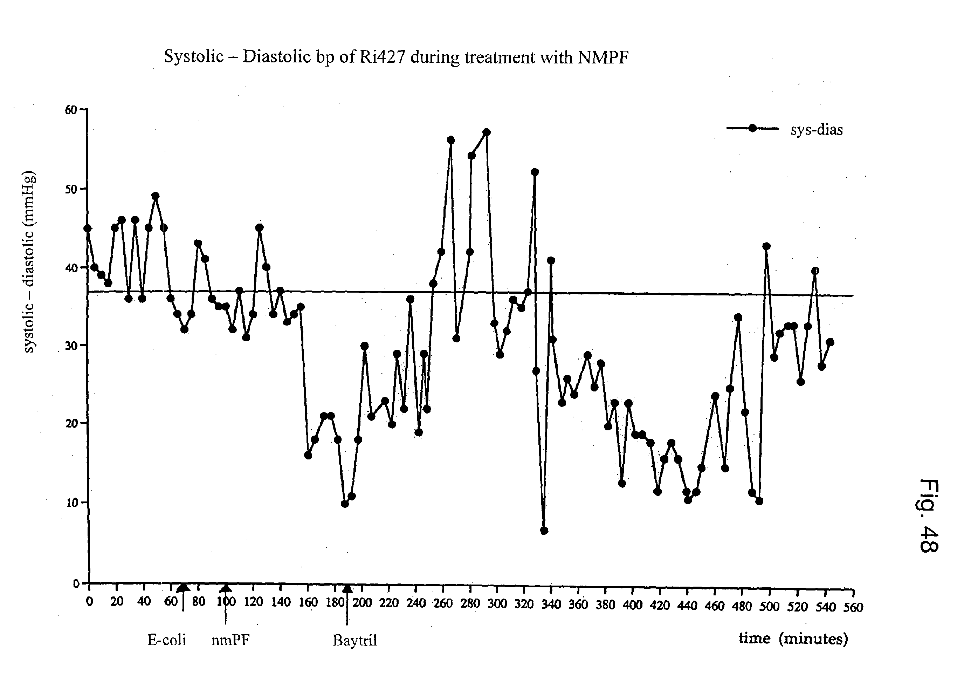

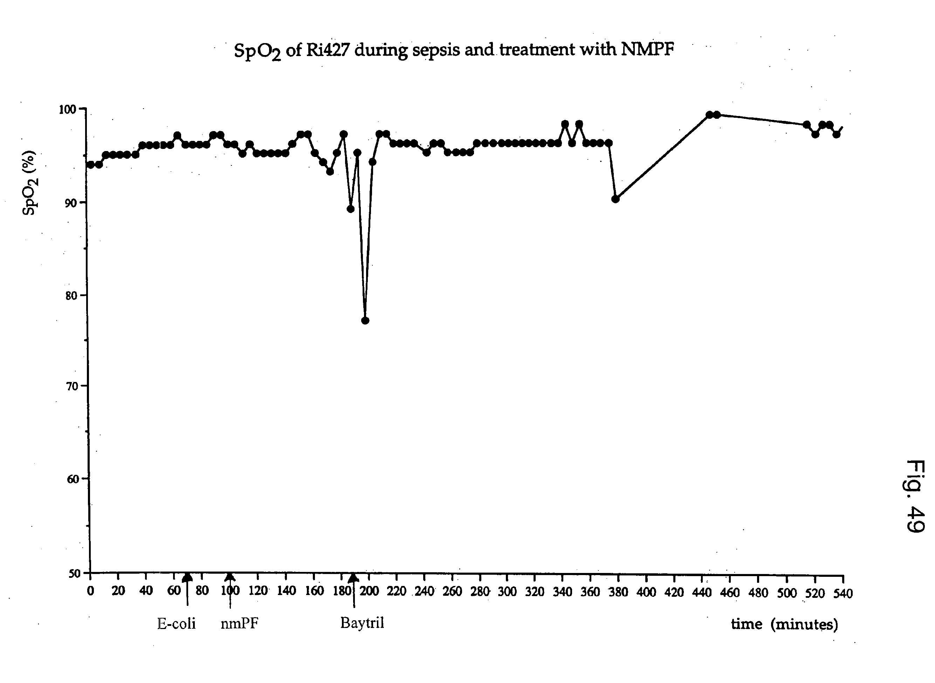

Figures 38 - 49 Effect of NMPF on septic shock syndrome in Rhesus monkeys. On the time point 70 minutes, E.coli was infused and at the end of E.coli infusion (time point 190 minutes), the antibiotic Baytril was injected. The control monkey (monkey 429) was treated with 0.9% NaC1 at the time point of 100 minutes, whereas the NMPF treated monkeys (monkey 459 and 427) received the NMPF treatment at the same time point as the control monkey. Heart rate (beats per minute), blood pressure (mmHg), difference between systolic and diastolic blood pressure and blood oxygen concentration (saturation in %) of the control monkey 429 (Figures 38-41), NMPF treated monkeys 459 (figures 42-45) and 427 (Figures 46-49) in the time (minutes) during the experiment are shown.

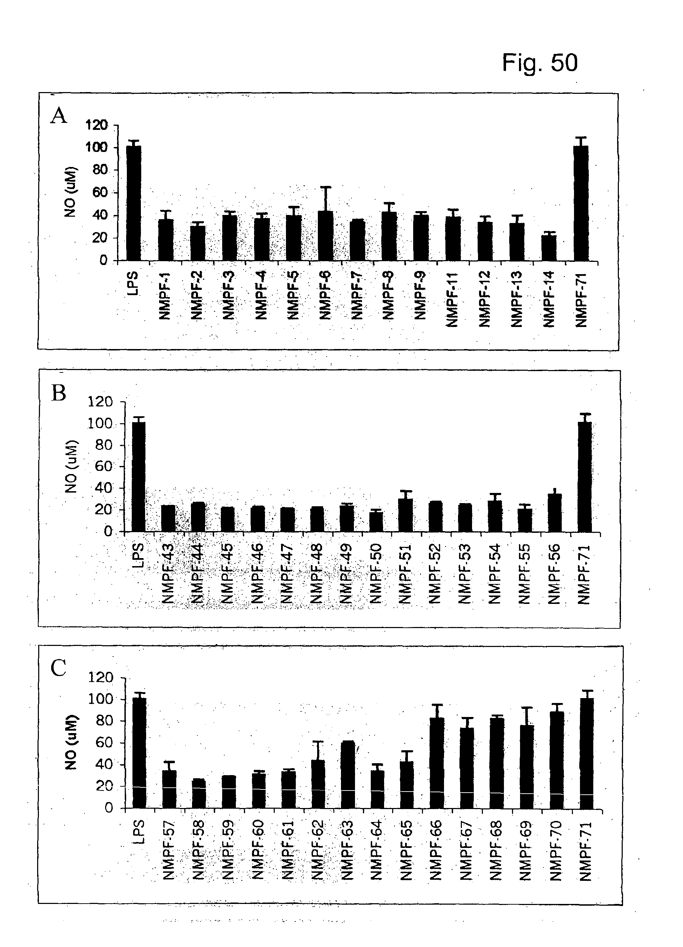

Figure 50 These figures (parts A-C) show the NO production of LPS (10 µg/ml) stimulated RAW 264.7 macrophages co-stimulated with different NMPF peptides (1 pg/ml).

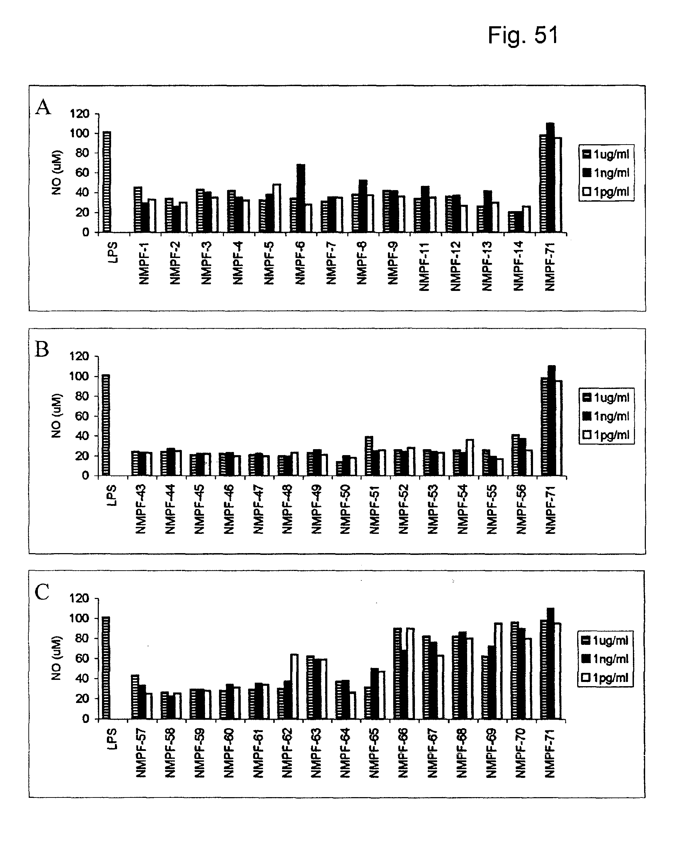

Figure 51 These figures (parts A-C) show the NO production of LPS (10 µg/ml) stimulated RAW 264.7 macrophages co-stimulated with different NMPF peptides with three different concentrations.

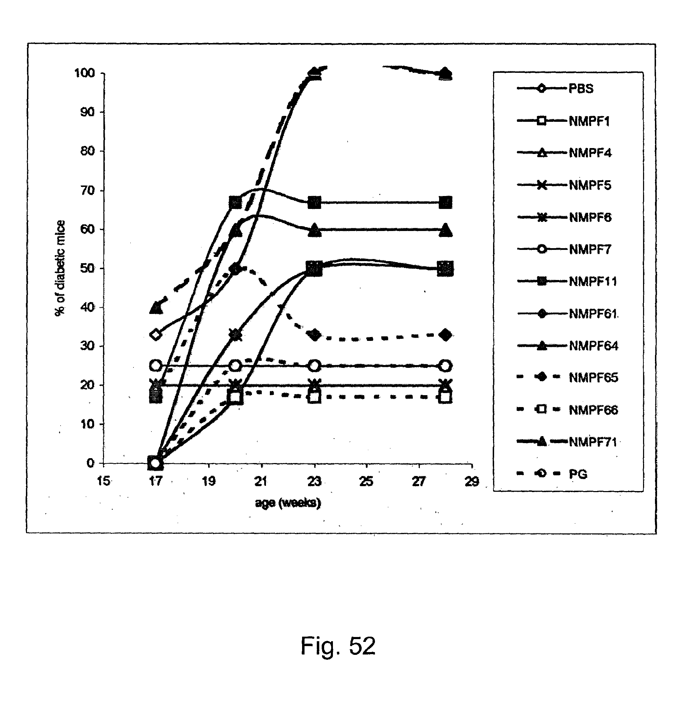

Figure 52 This figure shows the percentage of diabetic NOD mice treated for 2 weeks with the various NMPF peptides

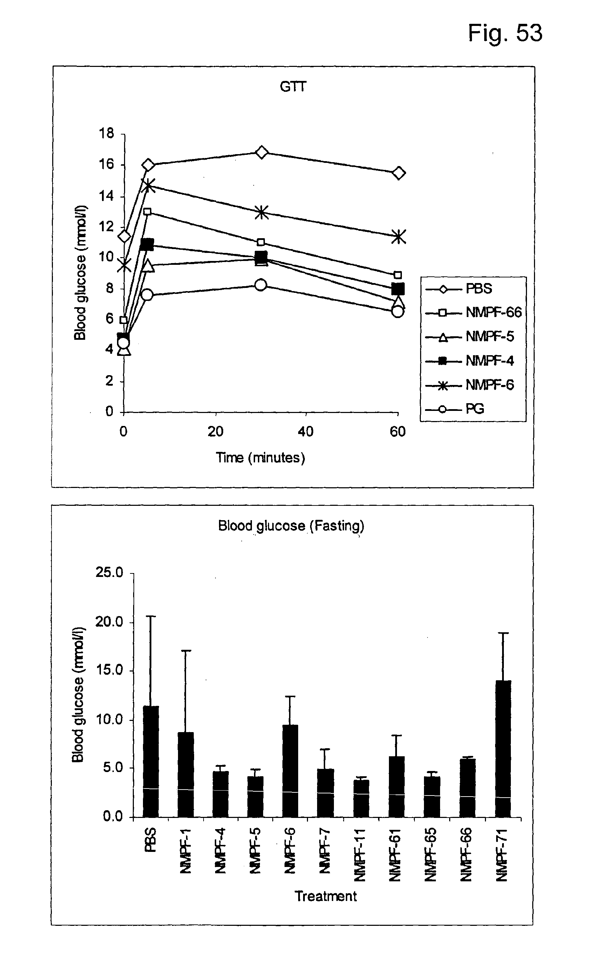

Figure 53 This figure shows the performed glucose tolerance test (GTT) in NOD mice treated with NMPF peptides (A), and fasting blood glucose levels (B).

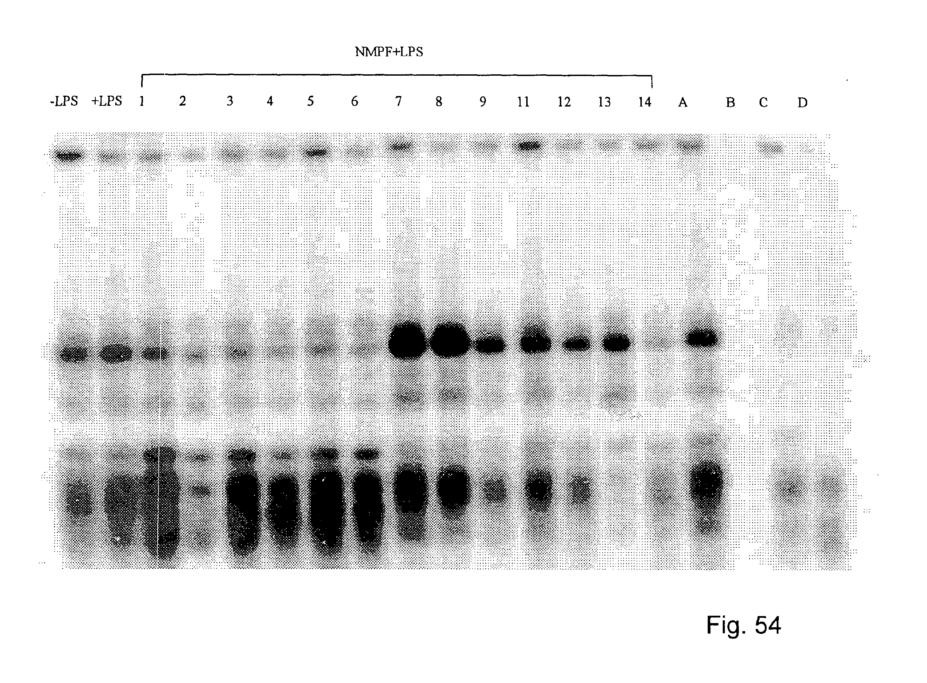

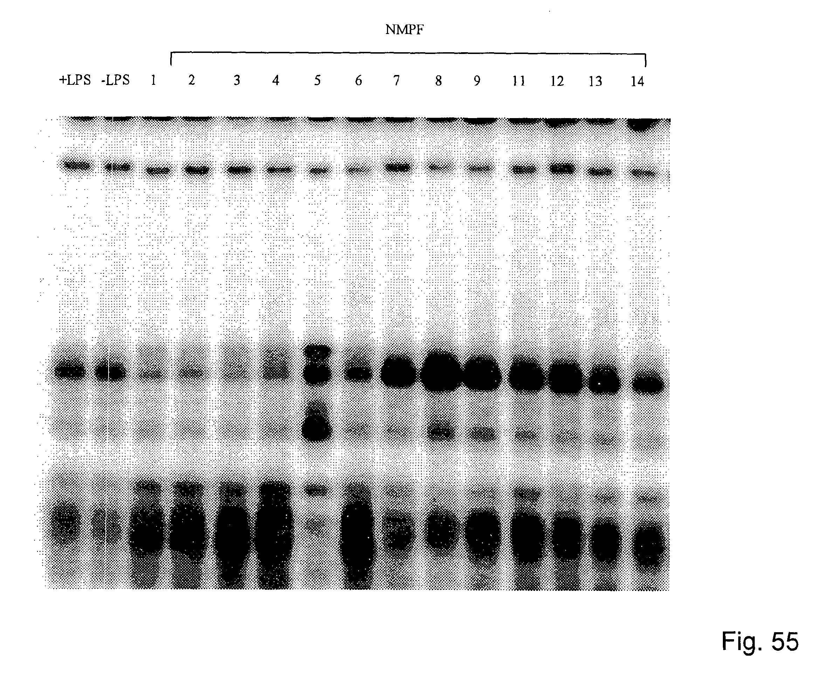

Figure 54 and 55. Semi quantitative amount of NF-kB present in the nuclear extracts of treated RAW264.7 cells. (A), NMPF peptides that show the inhibition of LPS induced translocation of NF-kB are: NMPF-1 (VLPALPQVVC), NMPF-2 (LQGVLPALPQ), NMPF-3 (LQG), NMPF-4 (LQGV), NMPF-5 (GVLPALPQ), NMPF-6 (VLPALP), NMPF-9 (VVC), NMPF-12 (MTR) and NMPF-14 (circular LQGVLPALPQVVC). NMPF peptides that promote LPS induced translocation of NF-kB are: NMPF-7 (VLPALPQ), NMPF-8 (GVLPALP) and NMPF-11 (MTRV). In this figure lane A presents one fold competition with un-label oligo, lane B presents only un-label oligo, lane C presents competition with ten fold un-label oligo and lane D presents competition with hundred fold un-label oligo. For competition labelled extracts from LPS treated cell were used (see lane '+LPS') with unlabelled oligo. Basal levels of NF-kB in the nucleus was decreased by NMPF-1 (VLPALPQVVC), NMPF-2 (LQGVLPALPQ), NMPF-3 (LQG) and NMPF-4 (LQGV) while basal levels of NF-kB in the nucleus was increased by NMPF-5 (GVLPALPQ), NMPF-7 (VLPALPQ), NMPF-8 (GVLPALP), NMPF-9 (VVC), NMPF-11 (MTRV), NMPF-12 (MTR) and NMPF-13 (LQGVLPALPQVVC) (figure 55).

Figure 56. NFκB in Splenic DCs in treated NOD mice
NOD mice were treated with either PBS or with NMPF. Hereafter, spleens were splenic DCs were isolated and analysed for nuclear NFkB p65. NOD mice treated with NMPF-3, 4, 5, 4+5, 4+6, 5+6 and 4+5+6 showed decreased levels of NFkB p65.

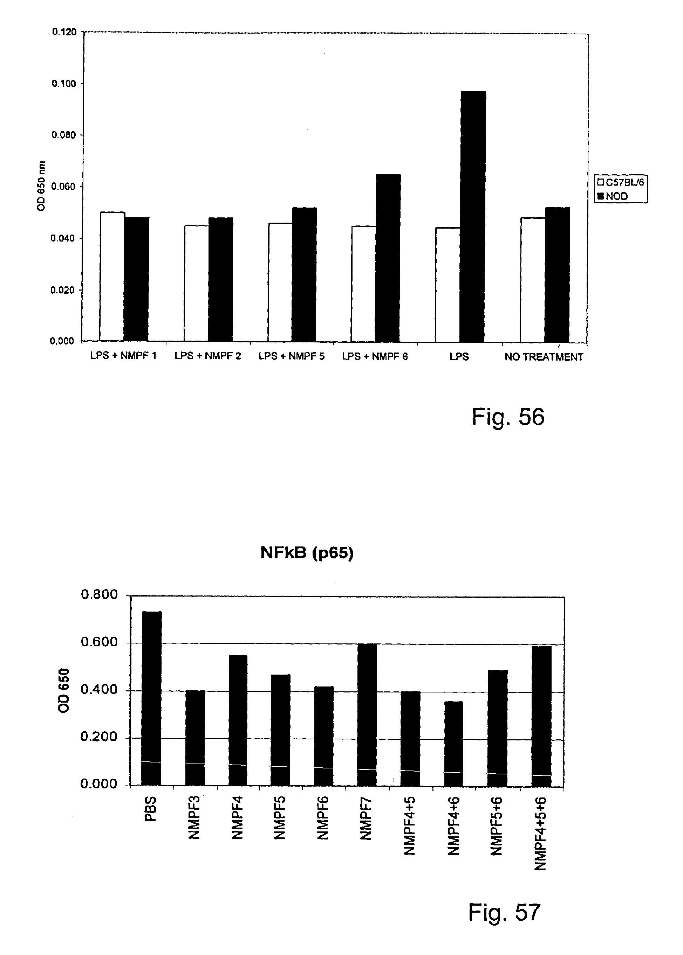

Figure 57. *NF*κ*B in Bone Marrow Derived DC's in NOD and C57bl6 Mice*
We tested in vitro the effects of NMPF on NFκB in DCs from NOD and C57BL/6 mice. Bone marrow (BM) were isolated and cultured in the presence of GM-CSF to yield BM derived DCs. The obtained DC were stimulated with LPS (5 ng/ml) and NMPF 1, 2, 5 or 6 for 30 minutes and then NFκB p65 activity was determined.
When stimulated with 5 ng/ml LPS, C57BL/6 DCs did not show an increase in NFκB activation compared to untreated DCs. However, NOD DCs did show a 2-fold increase in NFκB activation after stimulation with LPS. Effects of the NMPF on NFκB could not be detected in C57BL/6 DCs, since stimulation with LPS did not lead to NFκB activation. However, NOD DC the NMPF were able to inhibit (NMPF 1, 2, 5 and 6) LPS induced NFκB activation (figure 57). These results show the hyperresponsiveness of NFκB in NOD DCs compared to C57BL/6 DCs, and that NMPF inhibit the hyperreactive NFκB activation in NOD DCs.

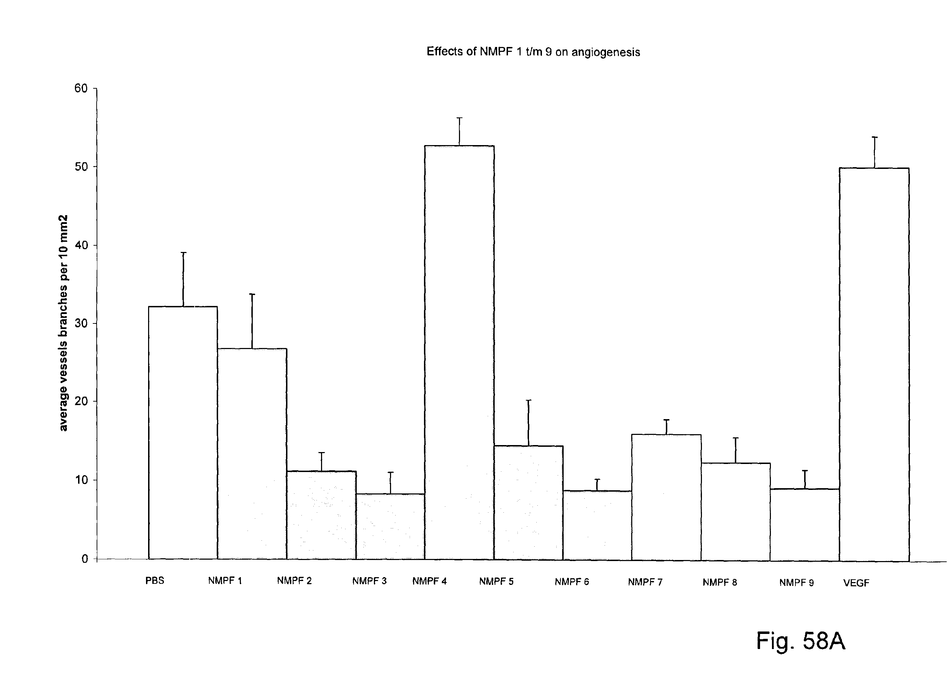

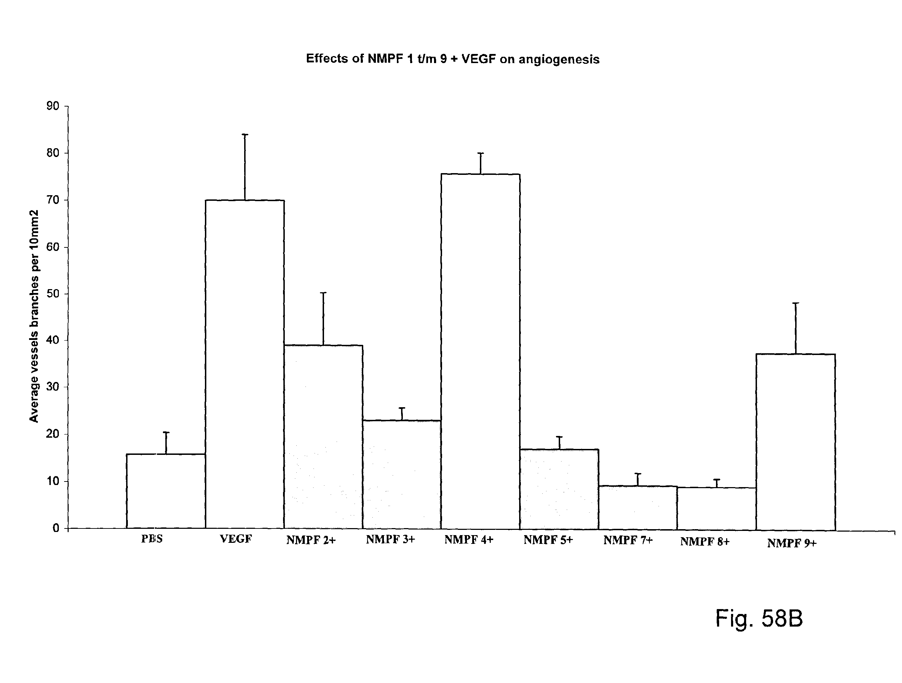

Figure 58.
This figure (fig. 58A) shows the effect on angiogenesis of NMPF 1 to 9 , which was added on day 8. NMPF 1, 2, 3, 5, 6, 7, 8 and 9 show a significant inhibition of angiogenesis compared to PBS treatment. While, NMPF-4 gives significant increase of angiogenesis. This figure also show that the treatment of VEGF on day 8 alone significantly increase angiogenesis. Figure 58B shows significant inhibition of VEGF induced angiogenesis on day 8 with NMPF 2, 3, 5, 7, 8 and 9. In addition, treatment of NMPF 1 and 6 on day 0 with VEGF significantly inhibit angiogenesis (data not shown).

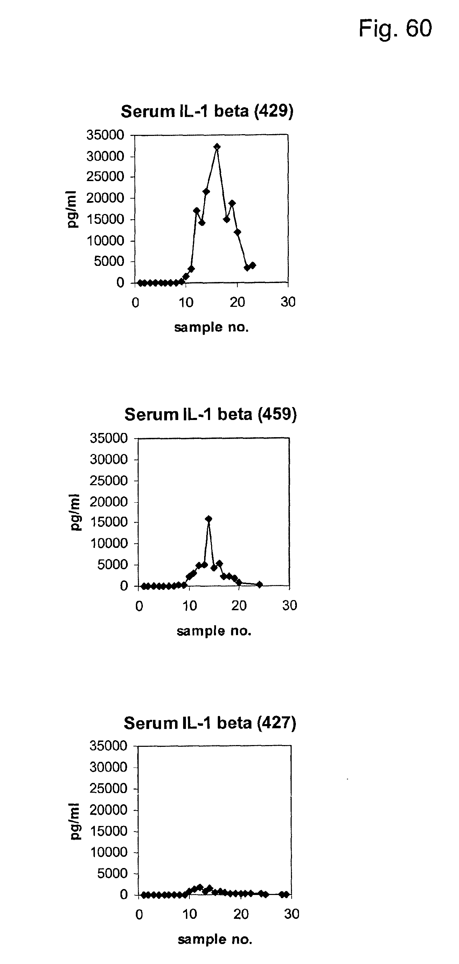

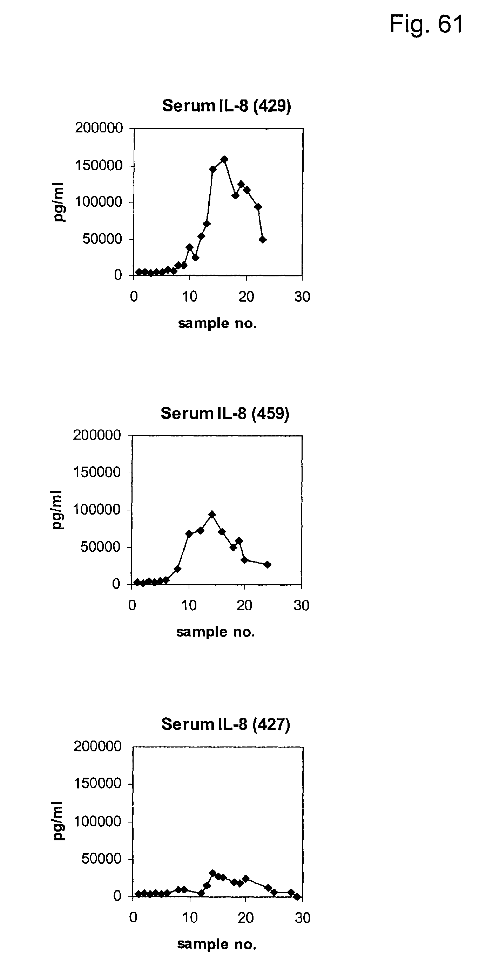

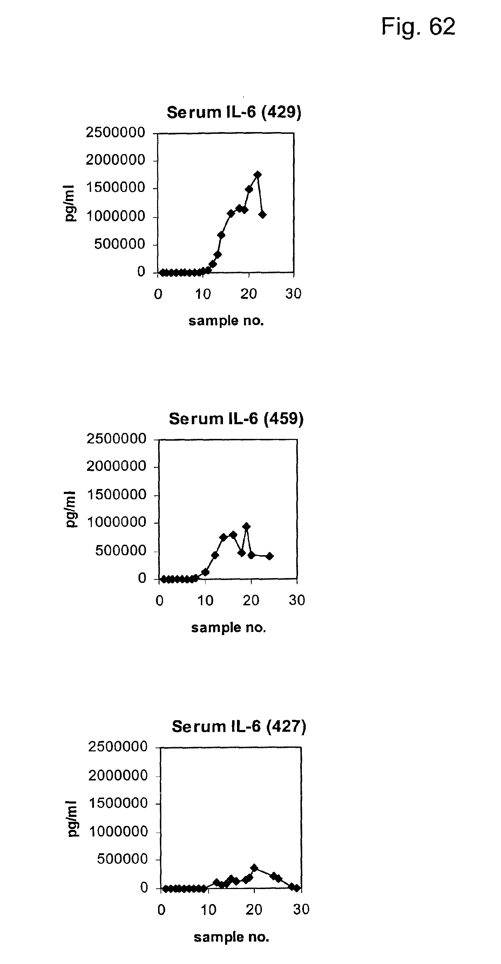

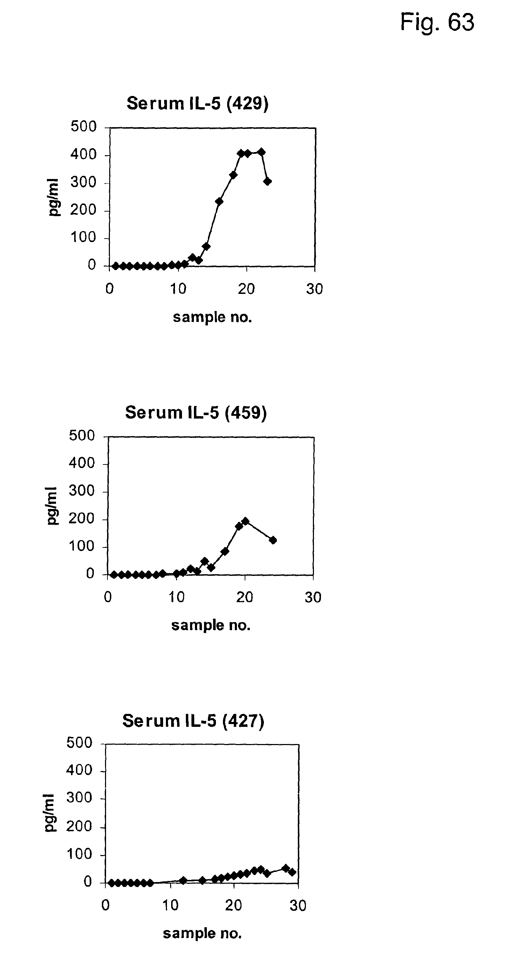

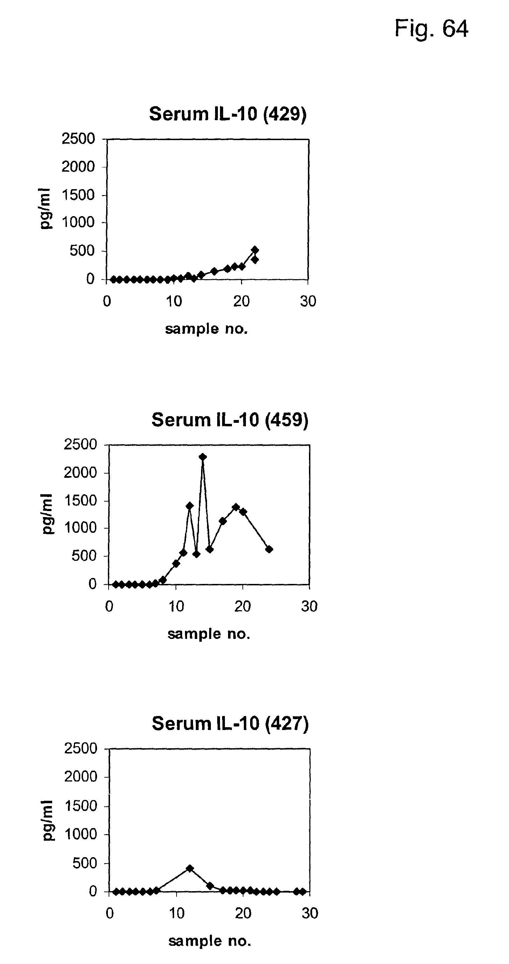

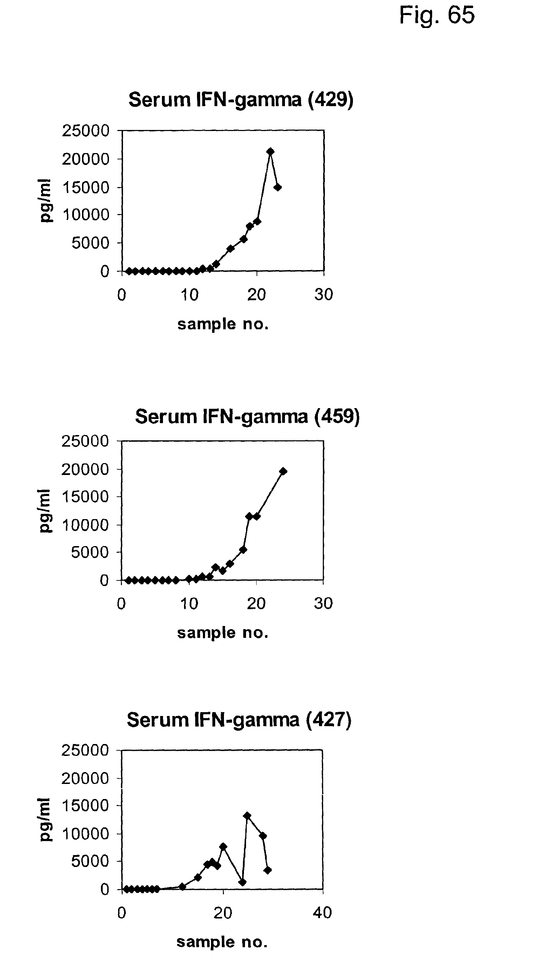

Figure 59-65. These figures show serum levels of TNF-alpha (fig. 59), IL-1beta (fig. 60), IL-8 (fig. 61), IL-6 (fig. 62), IL-5 (fig. 63), IL-10 (fig. 64) and IFN-gamma (fig. 65) of untreated monkey (429) which was sacrificed after 8 hours of E.coli injection, NMPF treated monkey (459) which was sacrificed at same time as untreated monkey and NMPF treated monkey (427) which was left alive and died after 38 hours of E. coli treatment.
Blood sample were taken at the following time points: 0, 30 (E.coli injection), 45, 60 (placebo or NMPF treatment), 75, 90, 105, 120, 135, 150 (Baytril treatment), 165, 180, 195, 210, 225, 240, 255, 270, 300, 330, 360, 390, 420, 450, 480, 510, 540, 570, 600 and 630 minutes.

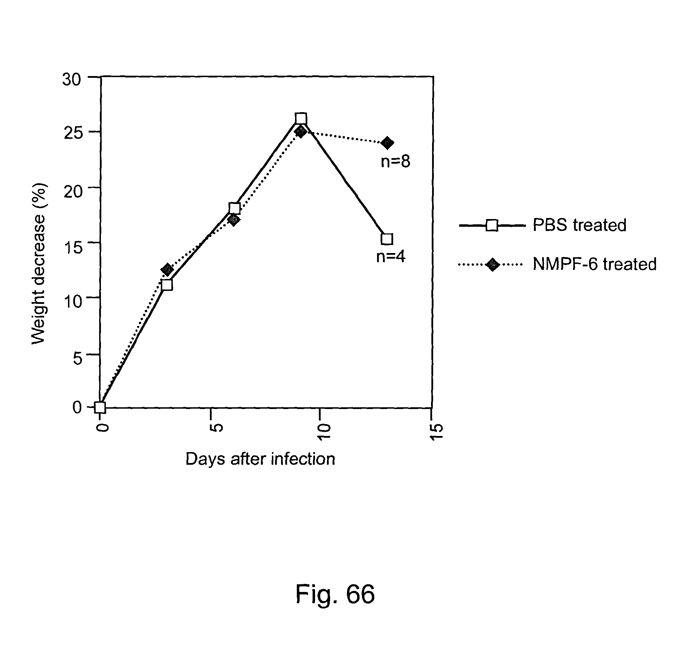

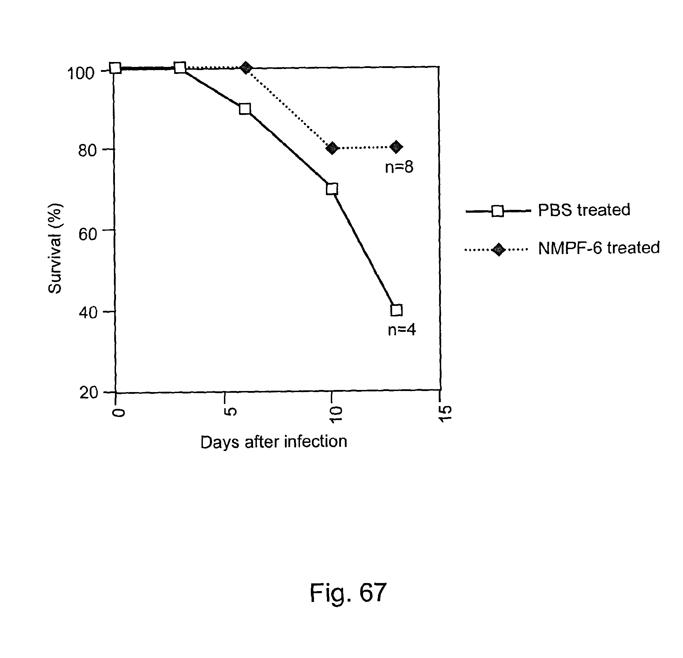

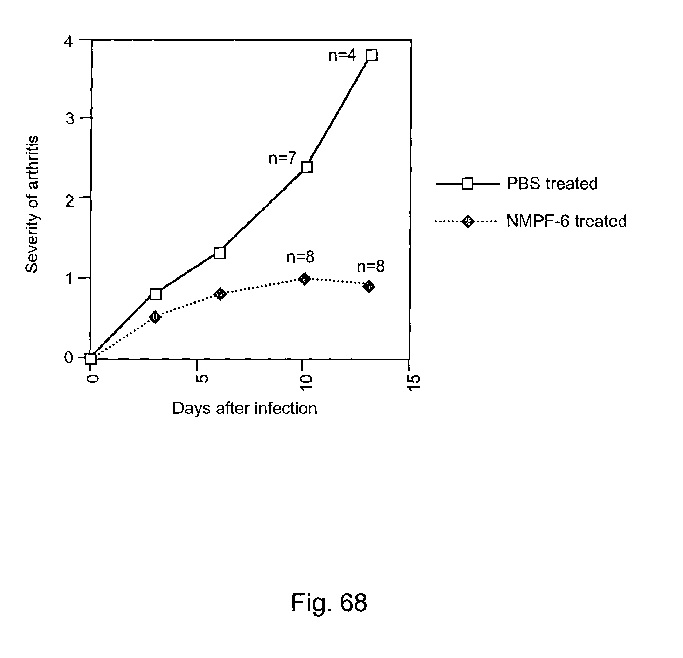

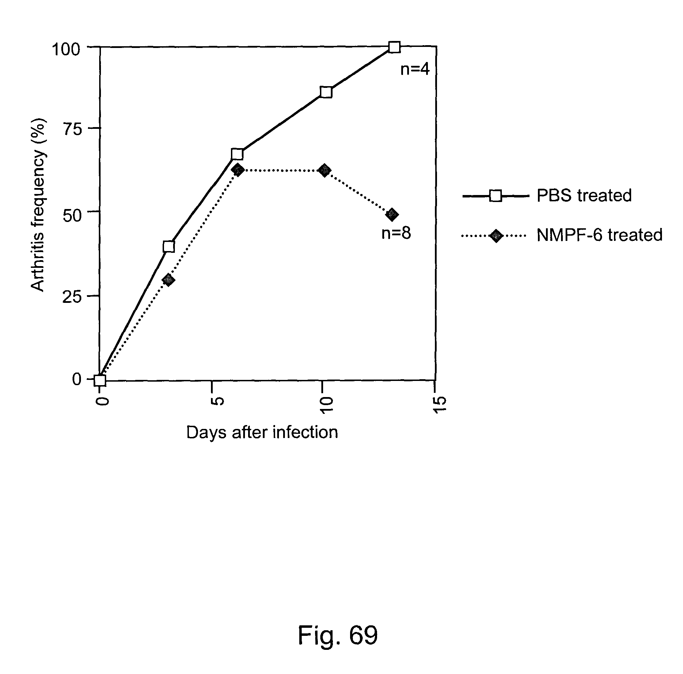

Figure 66-69. These figures show the results of a septic arthritis experiment using NMR1 mice. After i.v. injection with *S*. aureus LS-1 bacteria one group of mice were treated i.p. with PBS, 3 times per week for two weeks and other group of mice were treated i.p. with NMPF-6 (100 microgram), 3 times per week for two weeks. 10 mice were used for each group. During 13 days of follow-up period weight decrease (Fig 66), survival (Fig 67), arthritis severity (Fig 68) and arthritis severity (Fig 69) were determined.

Figure 70 lists the genes that are modulated in LPS or PHA stimulated PMBC that are further treted with a gene regulatory peptide according to the invention. 70 A to 70 E concerns LPS stimulated cells, 70 F to 70 J concern PHA stimulated cells. 70 A shows the control experiment wherein cells were only stimulated with LPS but not additionally treated with peptide, 70 B shows the effect of treatment with VVC, 70 C with MTRV, 70 D with MTR, 70 E with MTRVLQGVLPALPQ. 70 F shows the control experiment wherein cells were only stimulated with LPS but not additionally treated with peptide, 70 G shows the effect of treatment with VVC, 70 H with MTRV, 70 I with MTR, 70 J with MTRVLQGVLPALPQ. Peripheral blood mononuclear cells (PBMC) isolation. PBMC from heparinized venous blood were isolated by density gradient centrifugation of Ficoll-Hypaque (Pharmacia, Uppsala, Sweden). PBMC were washed three times and resuspended at 5.0x10e6 cells/ml in RPMI-1640 supplemented with 2mM L-glutamine, 100 IU/ml penicillin, 50 microgram/ml streptomycin, 1mM pyruvate and 10% heat-inactivated human serum. Two milliliters (10x10e6 cells) of cell suspension was plated in a 6-well plate and treated either with LPS (1 microgram/ml), LPS and NMPF (1 microgram/ml), PHA (10 microgram/ml), PHA and NMPF (1 microgram/ml) or equal volume of PBS only. Untreated PBMC and PBMC treated with LPS or LPS and NMPF were cultured for 1.5 hours, whereas untreated PBMC and PBMC treated with PHA or PHA and NMPF were cultured for 3 hours. After incubation all cells (attached and unattached) were collected and washed three times and further used in micro-array experiment. Following NMPFs were tested in this experiment: NMPF-9 (VVC), NMPF-11 (MTRV), NMPF-12 (MTR) and NMPF-70 (MTRVLQGVLPALPQ)

### DETAILED DESCRIPTION OF THE INVENTION

Cells react to environmental and intrinsic changes, which they perceive through extracellular and inter- as well as intracellular signals. The nature of these signals can be either for example physical or chemical. Moreover, different classes of molecules present in blood react to each other and induce a cascade of reactions that have direct effects on other molecules and/or eventually lead to cellular responses, for example complement system and blood coagulation proteins.

Many genes are regulated not by a signalling molecule that enters the cells but by molecules that bind to specific receptors on the surface of cells for example receptors with enzymatic activity (receptor tyrosine kinases, receptor-like protein tyrosine phosphatases, receptor serine/threonine kinases, histidine kinases, guanylyl cyclases) and receptors without enzymatic activity (cytokine receptors, integrins, G-protein-coupled receptors). Interaction between cell-surface receptors and their ligands can be followed by a cascade of intracellular events that modulate one or more intracellular-transducing proteins, including variations in the intracellular levels of so-called second messengers (diacylglycerol, Ca²⁺, cyclic nucleotides, inositol(1,4,5) trisphosphate, phosphatidylinositol(3,4,5) trisphosphate, phosphatidylinositol transfer protein (PITP)). This leads to the activation or inhibition of a so-called "effector protein". The second messengers in turn lead to changes in protein for example protein phosphorylation through the action of cyclic AMP, cyclic GMP, calcium-activated protein kinases, or protein kinases (for example AGC group serine/threonine protein kinases, CAMK group serine/threonine protein kinases, CMGC group serine/threonine kinases, protein tyrosine kinase group, or others like MEK/Ste7p). Phosphorylation by protein kinases is one of the regulatory mechanisms in signal transmission that modulate different cellular pathways such as Ras/MAPK pathway, MAP kinase pathway, JAK-STAT pathway, wnt-pathway. In many instances, this all results in altered gene expression (for example genes for the regulation of other genes, cell survival, growth, differentiation, maturation, functional activity).

Many of the responses to binding of ligands to cell-surface receptors are cytoplasmatic and do not involve immediate gene activation in the nucleus. In some instances, a pre-existing inactive transcription factor following a cell-surface interaction is activated that leads to immediate gene activation. For example, the protein NF-kappaB, which can be activated within minutes by a variety of stimuli, including membrane receptors (for example pattern recognition receptors like Toll-like receptor binding to pathogen-associated molecular patterns), inflammatory cytokines such as TNF-α, IL-1, T-cell activation signals, growth factors and stress inducers.

Our genomic experiment with NMPF peptide LQGV showed very immediate effects on signal transduction and gene regulation since the cells were treated with the peptide for only four hours. In this short period of time LQGV down-regulated at least 120 genes and up-regulated at least 6 genes in the presence of a strong stimulator (PHA/IL-2 stimulated T-cell line (PM1)), demonstrating the profound effect on signalling molecules according to the invention and modulatory effect on gene expression. The genes affected by LQGV include oncogenes, genes for transcription factors, intracellular enzymes, membrane receptors, intracellular receptors, signal transducing proteins (for example kinases) and some genes for unkown molecules. This shows that LQGV as an example of the synthetic signalling molecule (oligopeptide or functional analogue or derivative thereof) as described here, has a broad spectrum of effects at different extracellular and intracellular levels. In addition, our HPLC/MS data have shown the presence of LQGV in the nucleus of a macrophage cell line (RAW267.4) within a half hour and also indicates the direct effects on DNA level as well as at an intracellular level, which is further supported by NF-kappaB experiments. The ultimate modulatory effect of LQGV is dependent on, for example, type of the cell, differentiation and maturation status of the cell, the functional status and the presence of other regulatory molecules. This was evident by a shock experiment in which different NMPF peptides had similar or different effects on the disease. The same results were obtained with DC, fertilized chicken egg experiments, and CAO experiments; NMPF effects were dependent on type of co-stimulation (GM-CSF alone or in combination with LPS, or VEGF) and time of the treatment. Due to this, NMPF have the ability to modulate cellular responses at different levels.

The invention is further explained with the aid of the following illustrative examples.

### EXAMPLES

### MATERIAL AND METHODS

### Peptide synthesis

The peptides as mentioned in this document such as LQG, AQG, LQGV, AQGV, LQGA, VLPALP, ALPALP, VAPALP, ALPALPQ, VLPAAPQ, VLPALAQ, LAGV, VLAALP, VLPALA, VLPALPQ, VLAALPQ, VLPALPA, GVLPALP, VVCNYRDVRFESIRLPGCPRGVNPVVSYAVALSCQCAL, RPRCRPINATLAVEKEGCPVCITVNTTICAGYCPT, SKAPPPSLPSPSRLPGPS, LQGVLPALPQVVC, SIRLPGCPRGVNPVVS, LPGCPRGVNPVVS, LPGC, MTRV, MTR, and VVC were prepared by solid-phase synthesis (Merrifield, 1963) using the fluorenylmethoxycarbonyl (Fmoc)/tert-butyl-based methodology (Atherton, 1985) with 2-chlorotrityl chloride resin (Barlos, 1991) as the solid support. The side-chain of glutamine was protected with a trityl function. The peptides were synthesized manually. Each coupling consisted of the following steps: (i) removal of the alpha-amino Fmoc-protection by piperidine in dimethylformamide (DMF), (ii) coupling of the Fmoc amino acid (3 eq) with diisopropylcarbodiimide (DIC)/1-hydroxybenzotriazole (HOBt) in DMF/N-methylformamide (NMP) and (iii) capping of the remaining amino functions with acetic anhydride/diisopropylethylamine (DIEA) in DMF/NMP. Upon completion of the synthesis, the peptide resin was treated with a mixture of trifluoroacetic acid (TFA)/H₂O/triisopropylsilane (TIS) 95:2.5:2.5. After 30 minutes TIS was added until decolorization. The solution was evaporated in vacuo and the peptide precipitated with diethylether. The crude peptides were dissolved in water (50-100 mg/ml) and purified by reverse-phase high-performance liquid chromatography (RP-HPLC). HPLC conditions were: column: Vydac TP21810C18 (10 x 250 mm); elution system: gradient system of 0.1% TFA in water v/v (A) and 0.1% TFA in acetonitrile (ACN) v/v (B); flow rate 6 ml/min; absorbance was detected from 190-370 nm. There were different gradient systems used. For example for peptides LQG and LQGV: 10 minutes 100% A followed by linear gradient 0-10% B in 50 minutes. For example for peptides VLPALP and VLPALPQ: 5 minutes 5% B followed by linear gradient 1% B/minute. The collected fractions were concentrated to about 5 ml by rotation film evaporation under reduced pressure at 40°C. The remaining TFA was exchanged against acetate by eluting two times over a column with anion exchange resin (Merck II) in acetate form. The elute was concentrated and lyophilised in 28 hours. Peptides later were prepared for use by dissolving them in PBS.

### Transcription factor experiment

*Macrophage cell line*. The RAW 264.7 macrophages, obtained from American Type Culture Collection (Manassas, VA), were cultured at 37°C in 5% CO2 using DMEM containing 10% FBS and antibiotics (100 U/ml of penicillin, and 100 µg/ml streptomycin). Cells (1 x10⁶/ml) were incubated with peptide (10 µg/ml) in a volume of 2 ml. After 8 h of cultures cells were washed and prepared for nuclear extracts.

*Nuclear extracts*. Nuclear extracts and EMSA were prepared according to Schreiber et al. Methods (Schriber et al. 1989, Nucleic Acids Research 17). Briefly, nuclear extracts from peptide stimulated or nonstimulated macrophages were prepared by cell lysis followed by nuclear lysis. Cells were then suspended in 400 µl of buffer (10 mM HEPES (pH 7.9), 10 mM KCl, 0.1 mM KCL, 0.1 mM EDTA, 0.1 mM EGTA, 1 mM DTT, 0.5 mM PMSF and protease inhibitors), vigorously vortexed for 15 s, left standing at 4°C for 15 min, and centrifuged at 15,000 rpm for 2 min. The pelleted nuclei were resuspended in buffer (20 mM HEPES (pH 7.9), 10% glycerol, 400 mM NaCl, 1 mM EDTA, 1mM EGTA, 1 mM DTT, 0.5 mM PMSF and protease inhibitors) for 30 min on ice, then the lysates were centrifuged at 15,000 rpm for 2 min. The supernatants containing the solubilized nuclear proteins were stored at -70°C until used for the Electrophoretic Mobility Shift Assays (EMSA).

*EMSA*. Electrophoretic mobility shift assays were performed by incubating nuclear extracts prepared from control (RAW 264.7) and peptide treated RAW 264.7 cells with a 32P-labeled double-stranded probe (5' AGCTCAGAGGGGGACTTTCCGAGAG 3') synthesized to represent the NF-kappaB binding sequence. Shortly, the probe was end-labeled with T4 polynucleotide kinase according to manufacturer's instructions (Promega, Madison, WI). The annealed probe was incubated with nuclear extract as follows: in EMSA, binding reaction mixtures (20 µl) contained 0.25 µg of poly(dI-dC) (Amersham Pharmacia Biotech) and 20,000 rpm of 32P-labeled DNA probe in binding buffer consisting of 5 mM EDTA, 20% Ficoll, 5 mM DTT, 300 mM KCl and 50 mM HEPES. The binding reaction was started by the addition of cell extracts (10 µg) and was continued for 30 min at room temperature. The DNA-protein complex was resolved from free oligonucleotide by electrophoresis in a 6% polyacrylamide gel. The gels were dried and exposed to x-ray films.

### Apo E experiments

Apolipoprotein E (apo E) deficiency is associated with a series of pathological conditions including dyslipidemia, atherosclerosis, Alzheimer's disease, increase body weight and shorter life span. Inheritance of different alleles of the POLYMORPHIC *apoE* gene is responsible for 10% of the variation in plasma cholesterol in most populations. Individuals HOMOZYGOUS for one variant. *apoE2*, can develop type III dysbetalipoproteinaemia if an additional genetic or environmental factor is present. Some much rarer alleles of *apoE* produce dominant expression of this disorder in heterozygous individuals. ApoE, is a ligand for the LDL receptor and its effects on plasma cholesterol are mediated by differences in the affinity of the LDL receptor for lipoproteins carrying variant apoE proteins. The factors that regulate *apoE* gene transcription have been investigated extensively by the expression of gene constructs in transgenic mice and involve complex interactions between factors that bind elements in the 5' promoter region, in the first intron and in 3' regions many kilobases distant from the structural gene. Deletion of the apo E gene is associated with changes in lipoprotein metabolism [plasma total cholesterol), HDL cholesterol, HDL/TC, and HDL/LDL ratios, esterification rate in apo B-depleted plasma, plasma triglyceride, hepatic HMG-CoA reductase activity, hepatic cholesterol content, decreased plasma homocyst(e)ine and glucose levels, and severe atherosclerosis and cutaneous xanthomatosis.

### RESULTS

### NF-kB experiments

The transcription factor NF-kB participates in the transcriptional regulation of a variety of genes. Nuclear protein extracts were prepared from LPS and peptide treated RAW264.7 cells or from LPS treated RAW264.7 cells. In order to determine whether the peptide modulates the translocation of NF-kB into the nucleus, on these extracts EMSA was performed. Figure 33 shows the amount of NF-kB present in the nuclear extracts of RAW264.7 cells treated with LPS or LPS in combination with peptide for 4 hours. Here we see that indeed some peptides are able to modulate the translocation of NF-kB since the amount of labeled oligonucleotide for NF-kB is reduced. In this experiment peptides that show the modulation of translocation of NF-kB are: (NMPF-1)VLPALPQVVC, (NMPF-2)LQGVLPALPQ, (NMPF-3)LQG, (NMPF-4)LQGV, (NMPF-5)GVLPALPQ, (NMPF-6)VLPALP, (NMPF-7)VLPALPQ, (NMPF-8)GVLPALP, (NMPF-9)VVC, (NMPF-11)MTRV, (NMPF-12)MTR.

### NFkB analysis in macrophages

*Mouse macrophage cell line*: RAW 264.7 mouse macrophages were cultured in DMEM, containing 10% or 2% FBS, penicillin, streptomycin and glutamine, at 37 °C, 5% CO₂. Cells were seeded in a 12-wells plate (3x10⁶ cells/ml) in a total volume of 1 ml for 2hours and then stimulated with LPS (E. coli 026:B6; Difco Laboratories, Detroit, MI, USA) and/or NMPF (1 □g/ml). After 30 minutes of incubation plates were centrifuged and cells were collected for nuclear extracts.

*Nuclear Extracts:* Nuclear extracts and EMSA were prepared according to Schreiber et al. Method (Schriber et al. 1989, Nucleic Acids Research 17). Cells were collected in a tube and centrifuged for 5 minutes at 2000 rpm (rounds per minute) at 4°C (Universal 30 RF, Hettich Zentrifuges). The pellet was washed with ice-cold Tris buffered saline (TBS pH 7.4) and resuspended in 400 µl of a hypotonic buffer A (10 mM HEPES pH 7.9, 10 mM KCl, 0.1 mM EDTA, 0.1 mM EGTA, 1 mM DTT, 0.5 mM PMSF and protease inhibitor cocktail (Complete^{™} Mini, Roche) and left on ice for 15 minutes. Twenty five micro litre 10% NP-40 was added and the sample was centrifuged (2 minutes, 4000 rpm, 4°C). The supernatant (cytoplasmic fraction) was collected and stored at -70°C. The pellet, which contains the nuclei, was washed with 50 µl buffer A and resuspended in 50 µl buffer C (20 mM HEPES pH 7.9, 400 mM NaCl, 1 mM EDTA, 1 mM EGTA, 1 mM DTT, 0.5 mM PMSF and protease inhibitor cocktail and 10% glycerol). The samples were left to shake at 4°C for at least 60 minutes. Finally the samples were centrifuged and the supernatant (nucleic fraction) was stored at -70°C.

Bradford reagent (Sigma) was used to determine the final protein concentration in the extracts.
*EMSA:* For Electrophoretic mobility shift assays an oligonucleotide representing NF-κB binding sequence (5'-AGC TCA GAG GGG GAC TTT CCG AGA G-3') was synthesized. Hundred pico mol sense and antisense oligo were annealed and labelled with γ-³²P-dATP using T4 polynucleotide kinase according to manufacture's instructions (Promega, Madison, WI). Nuclear extract (5-7.5 µg) was incubated for 30 minutes with 75000 cpm probe in binding reaction mixture (20 microliter) containing 0.5 µg poly dI-dC (Amersham Pharmacia Biotech) and binding buffer BSB (25 mM MgCl₂, 5 mM CaCl₂, 5mM DTT and 20% Ficoll) at room temperature. The DNA-protein complex was resolved from free oligonucleotide by electrophoresis in a 4-6% polyacrylamide gel (150 V, 2-4 hours). The gel was then dried and exposed to x-ray film.

### Results

The transcription factor NF-kB participates in the transcriptional regulation of a variety of genes. Nuclear protein extracts were prepared from either LPS (1 mg/ml), NMPF (1 mg/ml) or LPS in combination with NMPF treated RAW264.7 cells. In order to determine whether the NMPF peptides modulate the translocation of NF-kB into the nucleus, on these extracts EMSA was performed. Figure 54 and 55 show the amount of NF-kB present in the nuclear extracts of treated RAW264.7 cells. Figure 54 shows that NMPF peptides are able to modulate the basal as well as LPS induced levels of NF-kB. In this experiment NMPF peptides that show the inhibition of LPS induced translocation of NF-kB are: NMPF-1 (VLPALPQVVC), NMPF-2 (LQGVLPALPQ), NMPF-3 (LQG), NMPF-4 (LQGV), NMPF-5 (GVLPALPQ), NMPF-6 (VLPALP), NMPF-9 (VVC), NMPF-12 (MTR) and NMPF-14 (circular LQGVLPALPQVVC). NMPF peptides that in this experiment promote LPS induced translocation of NF-kB are: NMPF-7 (VLPALPQ), NMPF-8 (GVLPALP) and NMPF-11 (MTRV). Basal levels of NF-kB in the nucleus was decreased by NMPF-1 (VLPALPQVVC), NMPF-2 (LQGVLPALPQ), NMPF-3 (LQG) and NMPF-4 (LQGV) while basal levels of NF-kB in the nucleus was increased by NMPF-5 (GVLPALPQ), NMPF-7 (VLPALPQ), NMPF-8 (GVLPALP), NMPF-9 (VVC), NMPF-11 (MTRV), NMPF-12 (MTR) and NMPF-13 (LQGVLPALPQVVC) (figure 55). In other experiments, NMPF-10 (QVVC) also showed the modulation of translocation of NF-kB into nucleus (data not shown).

### Effect of NMPFon DC (ex vivo/in vitro)

NOD and C57BL/6 Mice: All mice used in these studies were maintained in a pathogen-free facility at the Erasmus Medical Centre, Rotterdam (C57BL/6) or at Lucky Farm Company, Balkbrug, The Netherlands (NOD). All mice were given free access to food and water. The experiments were approved by the Animal Experiments Committee of the Erasmus Medical Centre, Rotterdam, The Netherlands.
In Vivo Treatment: 13-14 weeks old female NOD mice were used in this experiment. Ten groups of 11 (NMPF-3,4,5 and 6) or 13 (NMPF-7, 4+5, 4+6, 5+6 and 4+5+6) mice were formed at random and were treated with of either PBS (PBS group) or NMPF ( dissolved in PBS). Injected amount of each NMPF was 100 □g in a total volume of 100 □1. After 5 weeks of treatment, mice were sacrificed and spleens were isolated for DCs purification.
Isolation of Splenic DC: Spleens of PBS and NMPF treated mice were removed under aseptic conditions. Spleens were then digested with Collagenase D (Gibco BRL, life technologies) and DNase 1 in RPMI-1640 for 45 min at 37°C and single-cell suspensions were made. Erythrocytes were removed by incubating with Gey's medium for 5 min on melting ice. The cells were washed and CD11c-expressing cells were enriched using N418 magnetic beads (Miltenyi Biotec, Bisley, U.K.) and AutoMACS (Miltenyi Biotec, Bergisch Gladbach, Germany) following the manufacturer's protocol. The enriched population consisted of 80-90% CD11c⁺ and MHC II⁺ cells as determined by flow cytometric analysis. Per group obtained DC were pooled and cultured (3x106 cells/ml) in 12-well plate (Nalge Nunc International) for 2 hours and then in vitro stimulated with 1 □g/ml LPS (Sigma, E. Coli 026.B6) for 30 minutes. Cells were then lysed following the protocol described in the section nuclear extracts and analysed using the TransFactor™ kit (Clontech, BD) for NFkB (p65).
*Isolation of Bone Marrow Derived DC:* For this experiment untreated female NOD (age 13-14 weeks) and C57BL/6 (age 13-14 weeks) mice were used. Femurs and tibiae were removed and the marrow was flushed with RPMI-1640 using a syringe with a 0.45-mm needle. Clusters within the marrow suspension were disassociated by vigorous pipetting and filtrated through a 70-micrometer cell strainer. Red blood cells in suspension were lysed with Gey's medium and washed. Approximately 4⁻6×10⁷ bone marrow cells were obtained per mouse.
Upon initiation of the culture, the cell concentration was adjusted to 2 x 10⁵ cells per ml in R10 medium (RPMI-1640 medium without HEPES supplemented with 100 IU/ml penicillin, 50 mg/ml streptomycin, 1 mM pyruvate, 50 uM 2-ME, 10% v/v heat inactivated fetal calf serum (Bio Whittaker, Europe) and 20 ng/ml recombinant mouse Granulocyte Monocyte-Colony Stimulating Factor (rmGM-CSF; BioSource International, Inc., USA)). Cells were then seeded in 100 mm non-adherent bacteriological Petri dishes (Falcon) in a volume of 10 ml. For each condition six Petri-dishes were used. The cultures were placed in a 5% CO₂-incubator at 37°C. Every three days after culture initiation, 10 ml fresh R10 medium was added to each dish. Eleven days after culture initiation, non-adherent DC cells were collected and counted with a Coulter Counter (Coulter). Obtained DCs per mouse were pooled and were cultured (3x106 cells/ml) in 12-well plate (Nalge Nunc International) for 2 hours and then in vitro stimulated with 5 ng/ml LPS (Sigma, E. Coli 026.B6) or LPS and 1µg/ml NMPF (NMPF-1, 2, 5 or 6) for 30 minutes. Cells were then lysed following the protocol described in the section nuclear extracts and analysed using the TransFactor™ kit (Clontech, BD) for NFkB (p65). *Nuclear Extracts:* Nuclear extracts and EMSA were prepared according to Schreiber et al. Method (Schreiber et al. 1989, Nucleic Acids Research 17). Cells were collected in a tube and centrifuged for 5 minutes at 2000 rpm (rounds per minute) at 4°C (Universal 30 RF, Hettich Zentrifuges). The pellet was washed with ice-cold Tris buffered saline (TBS pH 7.4) and resuspended in 400 µl of a hypotonic buffer A (10 mM HEPES pH 7.9, 10 mM KCl, 0.1 mM EDTA, 0.1 mM EGTA, 1 mM DTT, 0.5 mM PMSF and protease inhibitor cocktail (Complete^{™} Mini, Roche) and left on ice for 15 minutes. Twenty five micro litre 10% NP-40 was added and the sample was centrifuged (2 minutes, 4000 rpm, 4°C). The supernatant (cytoplasmic fraction) was collected and stored at -70°C. The pellet, which contains the nuclei, was washed with 50 µl buffer A and resuspended in 50 µl buffer C (20 mM HEPES pH 7.9, 400 mM NaCl, 1 mM EDTA, 1 mM EGTA, 1 mM DTT, 0.5 mM PMSF and protease inhibitor cocktail and 10% glycerol). The samples were left to shake at 4°C for at least 60 minutes. Finally the samples were centrifuged and the supernatant (nucleic fraction) was stored at -70°C.
Bradford reagent (Sigma) was used to determine the final protein concentration in the extracts.
*Transcription factor analysis:* ELISA based TransFactor™ (Clontech, BD) kit was used for the analysis of NFκB subunit p65 according to the manufacturer's instructions.

### Results

### NFκB in Splenic DCs in treated NOD mice

It is well know hyperglycemia is correlated with higher levels of NFkB in the nucleus. NOD mice treated with NMPF-3, 4, 5, 4+5, 4+6, 5+6 and 4+5+6 showed a decreased in diabetes incidence and had lower levels of blood glucose (data not shown) as compared to PBS treated NOD mice. When nuclear extract of splenic DCs from these mice were analysed for NFkB, DCs from NMPF treated mice showed lower levels of NFkB (figure 56).

### NFκB in Bone Marrow Derived DC's in NOD and C57b16 Mice

Recently, Weaver *et al J*. *Immunol 2001* and others have described a defect in NFκB regulation in DC's from NOD mice and type 1 diabetes patients due to a hyperactive IKK. They showed that DC's derived from NOD mice were more sensitive to various forms of NFκB stimulation than DC's derived from C57BL/6 and BALB/c mice. This enhances the capacity of NOD DCs to secrete IL-12 production contributing to the development of pathogenic Th1 (Tc1) cells during the diabetogenic response. Hegazy DM et al. Genes Immun 2001, showed NFkB gene polymorphisms and susceptibility to type 1 diabetes: individuals with the A10 allele more likely to develop diabetes compared with the A14 allele. Therefore, we tested the effects of NMPF on NFκB in DCs from NOD and C57BL/6 mice. We isolated bone marrow (BM) from these mice and cultured in the presence of GM-CSF to yield BM derived DCs. We collected both adherent and non-adherent cells. The obtained cell population was first characterised by FACS using antibodies against CD11c, CD11b, MHC II (I-A^{K}) and MHC I (H-2B^{D}) (data not shown). Both in NOD and C57BL/6 mice 95% of the cells was CD11c⁺ and 90% was CD11b⁺. In NOD mice however 86% was CD11c⁺/CD11b⁺, while in C57BL/6 mice only 67% was CD11c⁺/CD11b⁺. This suggests the maturation and differentiation levels of DC's in NOD and C57BL/6 mice are not the same. We did observe a significantly (p=0.0001) lower amount of DC yield in comparison to C57BL6 mice. Hereafter, DC were stimulated with LPS (5 ng/ml) and NMPF 1, 2, 5 or 6 for 30 minutes and then NFκB activity was determined.
When stimulated with 5 ng/ml LPS, C57BL/6 DCs did not show an increase in NFκB activation compared to untreated DCs (figure 57). However, NOD DCs did show a 2-fold increase in NFκB activation after stimulation with LPS. Effects of the NMPF on NFκB could not be detected in C57BL/6 DCs, since stimulation with LPS did not lead to NFκB activation. However, NOD DC the NMPF were able to inhibit (NMPF 1, 2, 5 and 6) LPS induced NFκB activation (figure 57).
These results show the hyperresponsiveness of NFκB in NOD DCs compared to C57BL/6 DCs, and that NMPF inhibit the hyperreactive NFκB activation in NOD DCs.

### Nuclear location of peptide experiment

Reverse-phase high-performance liquid chromatography (RP-HPLC) method was used to prove the presence of synthetic oligo-peptide in the nuclear extracts. We used a Shimadzu HPLC system equipped with Vydac monomeric C18 column (column218MS54, LC/MS C18 reversed phase, 300A, 5□m, 4.6mm ID x 250mm L); elution system: gradient system of 0.01% TFA and 5% acetonitrile (CAN) in water v/v (A) and 0.01% TFA in 80% acetonitrile (ACN) v/v (B); flow rate 0.5 ml/min; absorbance was detected from 190-370 nm.
The gradient time programe was as follows:

| Time (min) | Buffer B concentration |
|---|---|
| 0.01 | 0 |
| 5.0 | 0 |
| 30.0 | 80 |
| 40.0 | 100 |
| 60.0 | 100 |
| 65.0 | 0 |
| 70.0 | 0 |

The elution time of peptide LQGV was determined by injecting 2 □g of the peptide in a separate run. Mass spectrometry (MS) analysis of fraction which contained possible NMPF-4 (LQGV) (elution time was determined by injecting the peptide in the same or separate run) was performed on LCQ Deca XP (Thermo Finnigan).

### RESULTS

### Nuclear location of peptide experiment

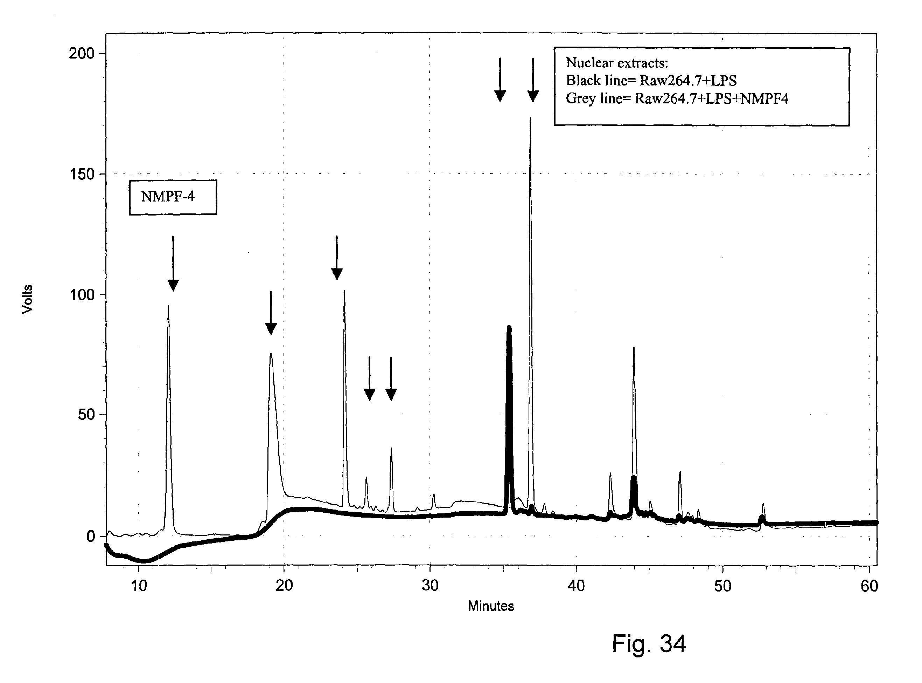

The nuclear protein extracts used in EMSA experiments were also checked for the presence of LQGV by means of HPLC and MS. Figure 34 shows HPLC chromatograph (wave length 206) in which data profile obtained from the nuclear protein extracts of LPS and LPS in combination with NMPF-4 (LQGV) stimulated RAW264.7 cells are overlayed. This figure also show the presence or absence of number of molecule signals in the nuclear extracts of oligopeptide+LPS treated cells as compared to nuclear extracts of LPS treated cells. Since HPLC profile of LQGV showed that the peptide elutes at around 12 minutes (data not shown), fraction corresponding to region 10-15 minutes was collected and analysed for the presence of this peptide in MS.

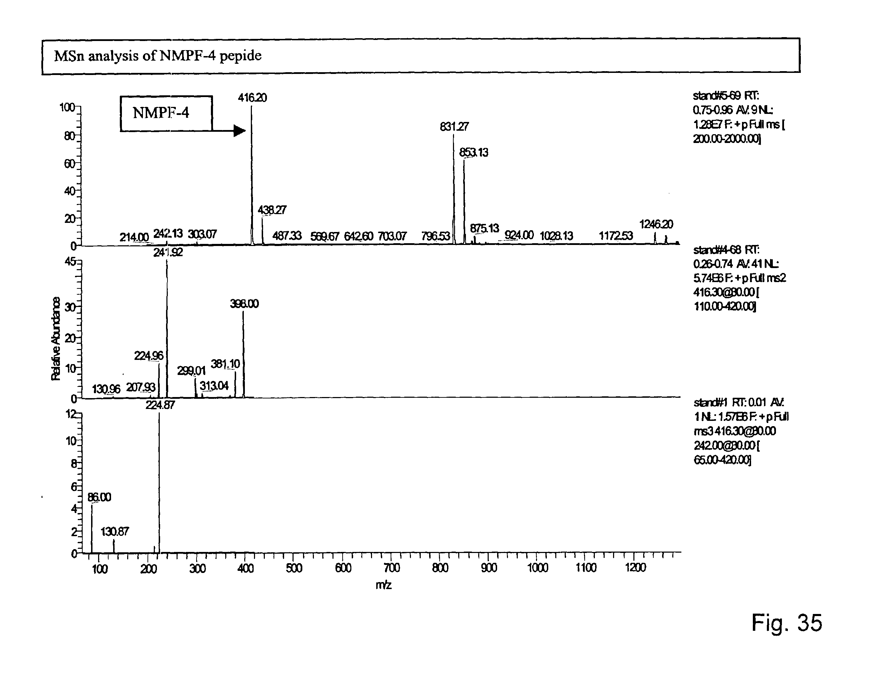

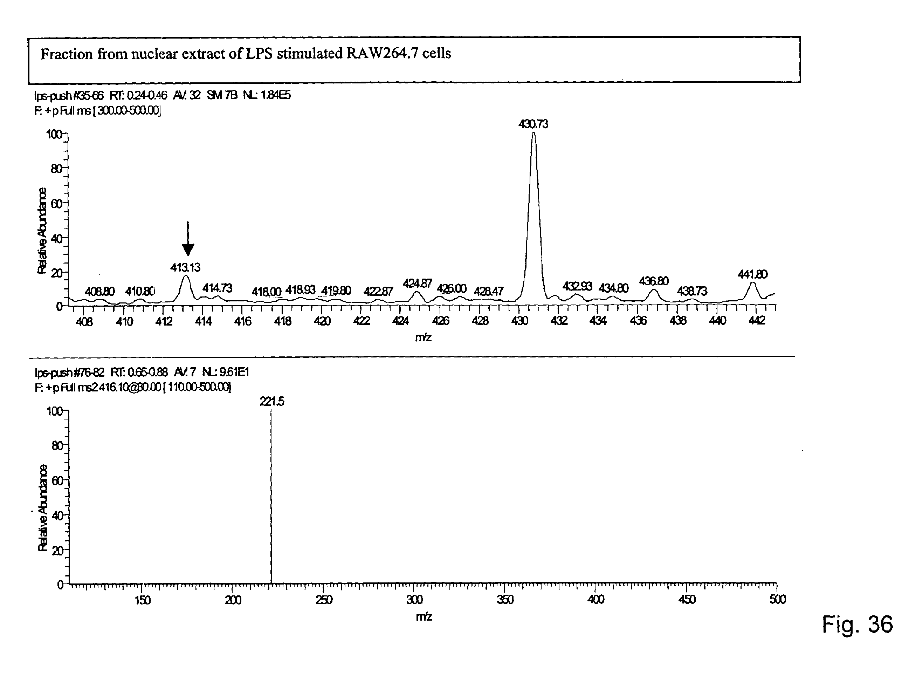

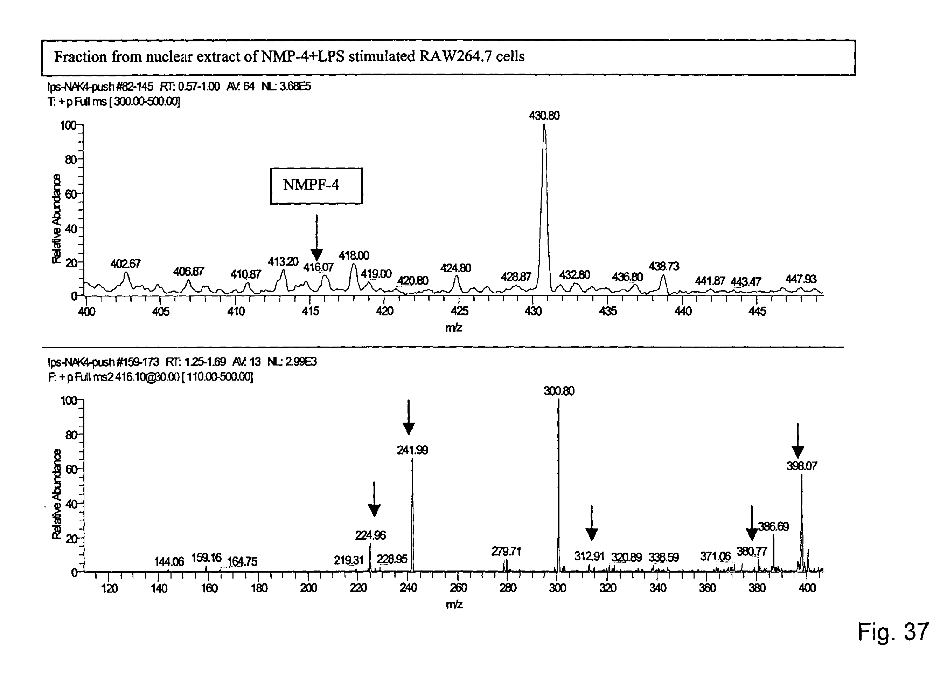

The peptide's molecular weight is around 416 Daltons. Besides 416 mass figure 35 shows some other molecular weights. This is to be explained by the high concentration of the peptide which induces the formation of dimers and sodium-adducts (m/z 416- [M+H]+, 438-[M+Na]+, 831-[2M+H]+, 853-[2M+Na]+, 1245-[3M+H]+, 1257-[3M+Na]+). Figure 36 shows the MS results of 10-15 min. fraction of nuclear extract obtained from LPS stimulated cells. These results show the absence of 416 dalton mass, while figure 37 shows the presence of 416 dalton mass of which the MSn data (figure 37) and MS-sequence confirm the presence of LQGV peptide in the nuclear protein extract obtained from LQGV+LPS stimulated RAW264.7 cells.

### Endotoxin shock model (Sepsis)

*Sepsis*. For the endotoxin model, BALB/c mice were injected i.p. with 8-9 mg/kg LPS (E. coli 026:B6; Difco Lab., Detroit, MI, USA). Control groups (PBS) were treated with PBS i.p. only. To test the effect of NMPF from different sources (synthetic, commercial hCG preparation [c-hCG]), we treated BALB/c with a dose of 300-700 IU of different hCG preparations (PG23;Pregnyl batch no. 235863, PG25; Pregnyl batch no. 255957) and with synthetic peptides (5 mg/kg) after two hours of LPS injection. In other experiments BALB/c mice were injected i.p. either with 10 mg/kg or with 11 mg/kg LPS (E. coli 026:B6; Difco Lab., Detroit, MI, USA). Subsequently mice were treated after 2 hours and 24 hours of LPS treatment with NMPF peptides.

*Semi-quantitative sickness measurements*. Mice were scored for sickness severity using the following measurement scheme:
1 Percolated fur, but no detectable behaviour differences compared to normal mice.
2 Percolated fur, huddle reflex, responds to stimuli (such as tap on cage), just as active during handling as healthy mouse.
3 Slower response to tap on cage, passive or docile when handled, but still curious when alone in a new setting.
4 Lack of curiosity, little or no response to stimuli, quite immobile.
5 Laboured breathing, inability or slow to self-right after being rolled onto back (moribund)
6 Sacrificed

### RESULTS

### Endotoxin shock model (Sepsis)

*Sepsis experiments*. To determine the effect of synthetic peptides (NMPF) in high-dose LPS shock model, BALB/c mice were injected intraperitoneally with different doses of LPS and survival was assessed daily for 5 days. In this experiment (for the LPS endotoxin model) BALB/c mice were injected i.p. with 8-9 mg/kg LPS (E. coli 026:B6; Difco Lab., Detroit, MI, USA). Control groups (PBS) were treated with PBS i.p. only. We treated BALB/c mice with a dose of 300-700 IU of different hCG preparations (PG23;Pregnyl batch no. 235863, PG25; Pregnyl batch no. 255957) or with peptides (5 mg/kg) after two hours of LPS injection.

These experiments showed (table 1.) that NMPF peptides 4, 6, 66 and PG23 inhibited shock completely (all mice had in first 24 hours sickness scores not higher than 2; shortly thereafter they recovered completely and had sickness scores of 0), while peptides 2, 3 and 7 accelerated shock (all mice had in first 24 hours sickness scores of 5 and most of them died, while the control mice treated with LPS+PBS had sickness scores of 3-4 in first 24 hours and they most of them died after 48 hours with sickness scores of 5 (17% survival rate at 72 hours). In addition, peptides 1, 5, 8, 9, 11, 12, 13, 14 and 64 showed in number of different experiments variability in effectiveness as well as in the kind (inhibitory vs accelerating) of activity. This variability is likely attributable to the rate of breakdown of the various peptides, and the different effects the various peptides and their breakdown products have *in vivo.* In addition, these experiments also showed the variability in anti-shock activity in c-hCG preparations that is likely attributable to the variation in the presence of anti-shock and shock accelerating NMPF. Visible signs of sickness were apparent in all of the experimental animals, but the kinetics and obviously the severity of this sickness were significantly different. These data are representative of at least 10 separate experiments.

In Table 2 we see the effect of ALA-replacement (PEPSCAN) in peptide LQG, LQGV, VLPALP, VLPALPQ in septic shock experiments. We conclude, that the change in even one amino acid by a neutral amino acid can lead to different activity. So, genomic differences as well as polymorphism in these peptides can regulate the immune response very precise. Derivatives of these peptides, for example (but not limited to) by addition of classical and non-classical amino acids or derivatives that are differentially modified during or after synthesis, for example benzylation, amidation, glycosylation, proteolytic cleavage, linkage to an antibody molecule or other cellular ligand etc. could also lead to a better effectiveness of the activity.

To determine whether treatment of BALB/c mice with NMPF inhibit septic shock at different stages of disease, synthetic peptides (NMPF) were injected i.p. at 2 and 24 hours after the induction of septic shock with high dose LPS (10 mg/kg).

As shown in Tables 3 and 4, control mice treated PBS after the shock induction, reached a sickness score of 5 at 14 and 24 hours, and remained so after the second injection with PBS. The survival rate in control group mice was 0% at 48 hours. In contrast to control mice, mice treated with NMPF 9, 11, 12, 43, 46, 50 and 60 reached a maximum sickness score of 2-3 at 24 hours after the induction of septic shock and further reached a maximum sickness score of 1-2 at 48 hours after the second injection of NMPF. In addition, mice treated with NMPF 5, 7, 8, 45, 53 and 58 reached a sickness score of 5 and after the second injection with NMPF all mice returned to a sickness score of 1-2 and survival rates in NMPF groups were 100%. Mice treated with NMPF 3 reached sickness scores of 3-4 and the second NMPF injection did save these mice. These experiments show that NMPF peptides have anti-shock activity at different stages of the disease and NMPF have anti-shock activity even at disease stage when otherwise irreversible damage had been done. This indicates that NMPF have effects on different cellular levels and also have repairing and regenerating capacity.

### Dendritic cells experiments

*Mice*. The mouse strain used in this study was BALB/c (Harlan, Bicester, Oxon, GB). All mice used in experiments were females between 8 and 12 weeks of age.

Mice were housed in a specific-pathogen-free facility. The Animal Use Committee at the Erasmus University Rotterdam, The Netherlands approved all studies.

*In vivo treatment*. At least six mice per group were injected intraperitonally (i.p) with LPS (10 mg/kg; Sigma). After 2 and 24 hrs of LPS induction, mice were injected i.p. with either NMPF (5 mg/kg) or Phosphate Buffered Saline (PBS), in a volume of 100 µl. LPS induced shock in this model had more than 90% mortality at 48 hrs.

*Bone marrow cell culture*. From treated mice, bone-marrow cells were isolated and cultured as follows. BALB/c mice were killed by suffocation with CO₂. The femurs and tibiae were removed and freed of muscles and tendons under aseptic conditions. The bones were placed in R10 medium (RPMI 1640, supplemented with 50 U/ml penicillin, 50 µg/ml streptomycin, 0.2 M Na-pyruvate, 2 mM glutamine, 50 µM 2-mercaptoethanol and 10% fetal calf serum (Bio Whittaker, Europe)).

The bones were then cleaned more thoroughly by using an aseptic tissue and were transferred to an ice cold mortier with 2 ml of R10 medium. The bones were crushed with a mortel to get the cells out of the bones. Cells were filtered through a sterile 100 µM filter (Beckton Dickinson Labware) and collected in a 50 ml tube (FALCON). This procedure was repeated until bone parts appeared translucent.

The isolated cells were resuspended in 10 ml of R10 and 30 ml of Geys medium was added. The cell suspension was kept on ice for 30 minutes, to lyse the red blood cells. Thereafter, the cells were washed twice in R10 medium. Upon initiation of the culture, the cell concentration was adjusted to 2 x 10⁵ cells per ml in R10 medium supplemented with 20 ng/ml recombinant mouse Granulocyte Monocyte-Colony Stimulating Factor (rmGM-CSF; BioSource International, Inc., USA) and seeded in 100 mm non-adherent bacteriological Petri dishes (Falcon). For each condition six Petri dishes were used and for further analysis, cells were pooled and analysed as described ahead. The cultures were placed in a 5% CO₂-incubator at 37°C. Every three days after culture initiation, 10 ml fresh R10 medium supplemented with rmGM-CSF at 20 ng/ml was added to each dish.

Nine days after culture initiation, non-adherent cells were collected and counted with a Coulter Counter (Coulter).

Alternatively, BM cells from untreated mice were isolated and cultured as described above and were *in vitro* treated with the following conditions: NMPF 4, NMPF 46, NMPF 7, NMPF 60 (20 µg/ml) were added to the culture either at day 0 or day 6 after culture initiation. Or LPS (1 µg/ml) was added to the culture at day 6 with or without the NMPF.

*Immunofluorescence staining*. Cells (2 x 10⁵) were washed with FACS-buffer (PBS with 1% BSA and 0.02% sodium azide), and transferred to a round-bottomed 96-well plate (NUNC). The antibodies used for staining were against MHC-II (I-A/I-E) PE and CD11c/CD18 FITC (PharMingen/Becton Dickinson, Franklin Lakes, NJ, US).

Cells were resuspended in 200 µl FACS-buffer containing both of the antibodies at a concentration of 2.5 ng/µl per antibody. Cells were then incubated for 30 min at 4°C. Thereafter, cells were washed 3 times and finally resuspended in 200 µl FACS-buffer for flow-cytometric analysis in a FACSCalibur flow cytometer (Becton Dickinson, Heidelberg, Germany). All FACS-data were analyzed with CellQuest software (Becton Dickinson, Heidelberg, Germany).

*Statistical analysis* All differences greater than 20% are considered to be significant.

### RESULTS

### Dendritic cells experiments

*Cell yield of ex vivo bone-marrow cell cultures*. To determine the *in vivo* effect of LPS and NMPF treatment on the cell yield obtained from a nine-day culture of bone-marrow with rmGM-CSF, cells were isolated from the BM of treated mice and cultured, harvested and counted as described. As shown in Figure 1 and 2, the cell yield of the bone-marrow cultures of LPS (10 mg/kg) treated mice is significantly decreased compared to PBS treated mice. Mice treated with NMPF 4, NMPF 7, NMPF 46 and NMPF 60 after LPS shock induction, had a significantly increased cell yield compared to LPS in the presence of rmGM-CSF. In addition, BM cultures from NMPF 46 treated mice gave a significantly increased cell yield even compared to the PBS group.

*Immunofluorescence staining of in vivo treated bone-marrow derived DC*. Culture of BM cells in the presence of rmGM-CSF gave rise to an increased population of cells that are positive for CD11c and MHC-II. Cells positive for these cell membrane markers are bone-marrow derived dendritic cells (DC). DC are potent antigen presenting cells (APC) and modulate immune responses. In order to determine the maturation state of myeloid derived DC, cells were stained with CD11c and MHC-II.

As shown in Figure 3, the expression of the MHC-II molecule was significantly decreased on CD11c-positive cells from LPS treated mice as compared to the PBS group. This decrease in MHC-II expression was further potentiated by the *in vivo* treatment with NMPF 4 and NMPF 46. However, treatment of mice with NMPF 7 and NMPF 60 significantly increased the expression of the MHC-II molecule even as compared to the PBS group.

*Cell yields of in vitro bone-marrow cell cultures*. To determine the effect of LPS and NMPF *in vitro* on the cell yield of a nine-day culture of bone-marrow cells, we isolated the BM cells from untreated BALB/c mice and cultured in the presence of rmGM-CSF. In addition to rmGM-CSF, cultures were supplemented with NMPF at either day 0 or day 6 with or without the addition of LPS at day 6.

As shown in Figures 4-7, there is a significant decrease in cell yield in LPS treated BM cells as compared to PBS. BM cells treated with NMPF 4, 7, 46 or 60 at time point t=0 or t=6 without LPS, showed a significant increase in cell yield as compared to the PBS group. However, BM cell cultures treated with NMPF 4 at time point t=6 showed significant decrease in cell yield as compared to the PBS group and this effect is comparable with the effect of LPS (Figure 4). In addition, BM cells treated with NMPF 4, 7, 46 or 60 at time point t=6 in combination with LPS showed a significant increase in cell yield as compared to the LPS group and even in the group of NMPF 7 the cell yield was significantly increased as compared to the PBS group.

*Immunofluorescence staining of in vitro treated bone-marrow derived DC*. To determine the maturation state of DC, CD11c positive cells were stained for MHC-II antibody. Figure 7-11 show that there is an opposite effect of LPS on MHC-II expression as compared to *in vivo* experiments, namely, MHC-II expression is significantly increased with LPS treatment *in vitro* as compared to PBS. NMPF 4 with LPS further potentiated the effect of LPS, while NMPF 7 with or without LPS (t=6), significantly inhibited the expression of MHC-II as compared to LPS and PBS, respectively. However, cells treated with NMPF 46 without LPS (t=0) showed significantly increased expression of MHC-II on CD11c positive cells. Furthermore, no significant differences were found in the group NMPF 60 with or without LPS on MHC-II expression as compared to LPS and PBS treated cells.

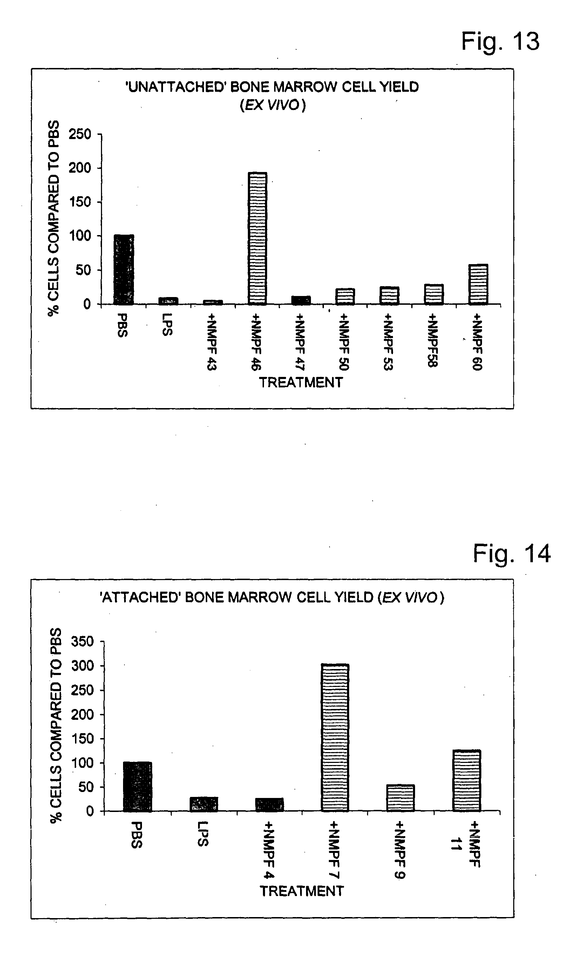

To determine the *in vivo* effect of LPS and NMPF treatment on the cell yield obtained from a nine-day culture of bone-marrow with rmGM-CSF, cells were isolated from the BM of treated mice and cultured, harvested and counted as described. The cell yield of 'attached' cells was significant increased with NMPF 4, 7, 9, 11, 43, 46, 47, 50, 53, 58 60 and even in the group of NMPF 7, 46 and 60 the cell yield was significant increased as compared to the PBS group (figure 14-15). In addition, cell yield of 'un-attached' cells was significant increased with NMPF 4, 7, 9, 11, 46, 50, 53, 58 60 and agin in the group of NMPF 46 the cell yield was significant increased as compared to the PBS group (figure 12-13).

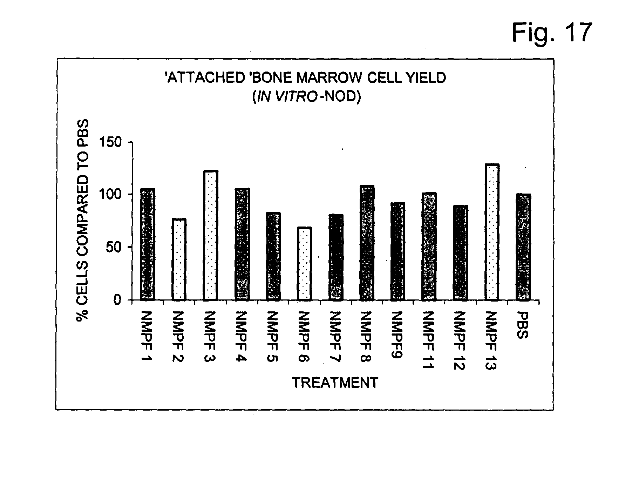

To determine the effect of LPS and NMPF *in vitro* on the cell yield of a nine-day culture of bone-marrow cells of female NOD mice, we isolated the BM cells from untreated NOD mice and cultured in the presence of rmGM-CSF. In addition to rmGM-CSF, cultures were supplemented with NMPF. In these experiments the bone-marrow cell yield of 'un-attached' cells was significant increased with NMPF 1,2,3,4,5,6,7,8,9, 12 and 13 as compared to PBS group and no effect was observed with NMPF 11 (figure 16). The 'attached' bone-marrow cells of these experiments showed different yield than the 'un-attached' cells, namely there was a significant increased in cell yield in cultures treated with NMPF 3 and 13, while cultures treated with NMPF 2 and 6 showed significant decrease in the cell yield as compared to PBS (figure 17) (more additional results are summarised in table 5).

### Coronary Artery Occlusion (CAO) experiments

*CAO induction and treatment*. NMPF have immunoregulatory effects in chronic inflammatory as well as acute inflammatory mice models. Since certain cytokines like TGF-beta1, TNF-alpha, IL-1 and ROS (reactive oxygen species) have been implicated in irreversible myocardial damage produced by prolonged episodes of coronary artery occlusion and reperfusion in vivo that leads to ischaemia and myocardial infarct, we tested the cardio-protective properties of peptides in ad libitum fed male Wistar rats (300 g). The experiments were performed in accordance with the Guiding principles in the Care and Use of Animals as approved by the Council of the American Physiological Society and under the regulations of the Animal Care Committee of the Erasmus University Rotterdam. Shortly, rats (n=3) were stabilised for 30 minutes followed by i.v. 1 ml of peptide treatment (0.5 mg/ml) in 10 minutes. Five minutes after completion of treatment, rats were subjected to a 60-min coronary artery occlusion (CAO). In the last 5 minutes of CAO, rats were again treated over 10 minutes i.v. with 1 ml of peptide (0.5 mg/ml) followed by 120 minutes of reperfusion (IP). Experimental and surgical procedures are described in detail in Cardiovascular Research 37(1998) 76-81. At the end of each experiment, the coronary artery was re-occluded and was perfused with 10 ml Trypan Blue (0.4%, Sigma Chemical Co.) to stain the normally perfused myocardium dark blue and delineate the nonstained area at risk (AR). The heart was then quickly excised and cut into slices of 1 mm from apex to base. From each slice, the right ventricle was removed and the left ventricle was divided into the AR and the remaining left ventricle, using micro-surgical scissors. The AR was then incubated for 10 min in 37°C Nitro-Blue-Tetrazolium (Sigma Chemical Co.; 1 mg per 1 ml Sorensen buffer, pH 7.4), which stains vital tissue purple but leaves infarcted tissue unstained. After the infarcted area (IA) was isolated from the noninfarcted area, the different areas of the LV were dried and weighed separately. Infarct size was expressed as percentage of the AR. Control rats were treated with PBS.

### RESULTS

### Coronary Artery Occlusion (CAO) experiments

Our CAO data showed that 15 rats in control group treated with only PBS had infarcted area of 70±2% (average±standard error) after 60-minutes of CAO followed by 2 hours of reperfusion. While rats treated with peptide VLPALP, LQGV, VLPALPQVVC, LQGVLPALPQ, LAGV, LQAV and MTRV showed infarcted area of 62±6%, 55±6%, 55±5%, 67±2%, 51±4%, 62±6% and 68±2%, respectively. Here, we see that certain peptides (such as VLPALP, LQGV, VLPALPQVVC, LAGV) have a protective effect on the area at risk for infarction. In addition, peptide LQAV showed smaller infarcted area but in some instances the area was haemorhagic infarcted. In addition NMPF-64 (LPGCPRGVNPVVS) had also protective effect (35%) in CAO experiment. It is important to note that mice treated with certain above mentioned peptides showed less viscousity of blood. Apart from immunological effect, these peptides may have also effect on blood coagulation system directly or indirectly since there is certain homology with blood coagulation factors ( for additional results of NMPF peptides see table 5.) So, in both models the circulatory system plays an important role in the pathogenesis of the disease.

### Chicken eggs experiments

*In vivo treatment of fertilised chicken eggs with NMPF*. Fertile chicken eggs (Drost Loosdrecht BV, the Netherlands) were incubated in a diagonal position in an incubator (Pas Reform BV, the Netherlands) at 37 °C and 32% relative humidity.

Solutions of NMPF peptides (1mg/ml) and VEGF were made in PBS. At least ten eggs were injected for every condition. The treatment was performed as follows: on day 0 of incubation, a hole was drilled into the eggshell to open the air cell. A second hole was drilled 10 mm lower and right from the first hole for injection. The holes in the eggshell were disinfected with jodium. The NMPF peptides (100 ug/egg) and/or VEGF (100 ng/ml) were injected in volume of 100µl. The holes in the eggshell were sealed with tape (Scotch Magic^{™} Tape, 3M) and the eggs were placed into the incubator.

*Quantification of angiogenesis*. On day 7 of incubation, the eggs were viewed under an UV lamp to check if the embryos were developing in a normal way and the dead embryos were counted. On day 8 of incubation, the embryos were removed from the eggs by opening the shell at the bottom of the eggs. The shell membrane was carefully dissected and removed. The embryos were placed in a 100-mm Petri dish. The embryo and the blood vessels were photographed (Nikon E990, Japan) *in vivo* with the use of a microscope (Zeiss Stemi SV6, Germany). One overview picture was taken and 4 detail pictures of the blood vessels were taken. Only eggs with vital embryos were evaluated.

***Data analysis*.** Quantification of angiogenesis was accomplished by counting the number of blood vessels branches. Quantification of vasculogenesis was accomplished by measuring the blood vessel thickness. The number of blood vessel branches and the blood vessel thickness were counted in the pictures (4 pictures/egg) using Corel Draw 7. Thereafter, the number of blood vessel branches and the thickness of the blood vessels were correlated to a raster of microscope (10 mm²) for comparison. The mean number of branches and the mean blood vessel thickness of each condition (n=10) were calculated and compared to the PBS control eggs using a Student's T-test.

### RESULTS

### Chicken eggs experiments

In order to determine the effect of NMPF on angiogenesis and vasculogenesis we treated fertilized chicken eggs with NMPF or NMPF in combination with VEGF as described in materials and methods section. Figures 20-30 show that NMPF 3, 4, 9 and 11 promoted angiogenesis (p<0.05), while NMPF VEGF, 7, 43, 44, 45, 46, 51 and 56 inhibited angiogenesis (p<0.05). NMPF 6, 7, 12, 45, 46 and 66 were able to inhibit angiogenesis induced by VEGF. Moreover, NMPF 6 itself did not show any effect on angiogensis, but it inhibited (p<0.05) NMPF 3 induced angiogenesis.

Figures 31-32 show that NMPF 1, 2,3, 4, 6, 7, 8, 12, 50, 51, and 52 had vasculogenesis inhibiting (p<0.05) effect, while only NMPF 44 promoted (p<0.05) vasculogenesis.

### NOD experiment

*Mice*. Female NOD mice at the age of 13-14 weeks were treated i.p. with PBS (n=6) or NMPF peptides (VLPALPQVVC, LQGV, GVLPALPQ, VLPALP, VLPALPQ, MTRV, LPGCPRGVNPVVS, CPRGVNPVVS, LPGC, MTRVLQGVLPALPQVVC, VVCNYRDVRFESIRLPGCPRGVNPVVSYAVALSCQCAL) (5 mg/kg, n=6) three times a week for 2 weeks. Every four days urine was checked for the presence of glucose (Gluketur Test; Boehringer Mannheim, Mannheim, Germany). All mice used in these studies were maintained in a pathogen-free facility. They were given free access to food and water. The experiments were approved by the Animal Experiments Committee of the Erasmus University Rotterdam. Diabetes was assessed by measurement of the venous blood glucose level using an Abbott Medisense Precision glucometer. Mice were considered diabetic after two consecutive glucose measurements ≥11 mmol/l (200 mg/dl). Onset of diabetes was dated from the first consecutive reading.

Glucose tolerance test (GTT) test was performed at 28 weeks of age in fasted mice ( n=5) by injecting 1 g/kg D-glucose intraperitoneally (i.p.). At 0 (fasting), 5, 30 and 60 minutes blood samples were collected from the tail and tested for glucose content.

### NO experiment

*Cell culture*. The RAW 264.7 murine macrophage cell line, obtained from American Type Culture Collection (Manassas, VA, USA), were cultured at 37°C in 5% CO₂ using DMEM containing 10% fetal calf serum (FCS), 50 U/ml penicillin, 50 µg/ml streptomycin, 0.2 M Na-pyruvate, 2 mM glutamine and 50 µM 2-mercaptoethanol (Bio Whittaker, Europe). The medium was changed every 2 days.

*Nitrite measurements*. Nitrite production was measured in the RAW 264.7 macrophage supernatants. The cells (7.5 x10⁵/ml) were cultured in 48-well plates in 500 microliter of culture medium. The cells were stimulated with LPS (10 microg/ml) and/or NMPF (1 pg/ml, 1 ng/ml, 1 microg/ml) for 24 hours, then the culture media were collected. Nitrite was measured by adding 100 microl of Griess reagent (Sigma) to 100 microl samples of culture medium. The OD₅₄₀ was measured using a microplate reader, and the nitrite concentration was calculated by comparison with the OD₅₄₀ produced using standard solutions of sodium nitrite in the culture medium.

### RESULTS

### NOD experiment

In order to determine whether NMPF has effect on the disease development in NOD mice, we tested NMPF on pre-diabetic female NOD mice at the age of 13-14 weeks. After only two weeks of treatment (injection of NMPF (5 mg/kg) every other day) glucosuria data of all NOD mice was analysed at the of 17 weeks. Profound anti-diabetic effect (mice negative for glucosuria) was observed in different NMPF groups as compared to PBS group, especially in NMPF groups treated with peptide VLPALPQVVC, VLPALP, MTRV, LPGCPRGVNPVVS and LPGC. In addition, impairment of the glucose tolerance test was positively correlated to insulitis, but negatively correlated to the number of functional beta cells, also this test showed that NOD mice successfully treated with NMPF were tolerant for glucose as compared to PBS group. Our results show that PBS treated NOD mice were all diabetic at the age of 23 weeks. Whereas, NOD mice treated with three times a week for two weeks with NMPF showed profound inhibition of diabetes development. The strongest anti-diabetic effects were seen with NMPF-1, 4, 5, 6, 7, 65, 66 and commercial hCG preparation (Pregnyl, Organon, Oss, The Netherlands, batch no. 235863). These mice had a low fasting blood glucose level and were tolerant for glucose (data partially shown). However, NMPF-71 showed no effect on the incidence of diabetes, while NMPF-64 and NMPF-11 had a moderate anti-diabetic effect.

### NO experiment

NO production is a central mediator of the vascular and inflammatory response. Our results show that macrophages (RAW 264.7) stimulated with LPS produce large amount of NO. However, these cells co-stimulated with most of the NMPF peptides (NMPF peptide 1 to 14, 43 to 66 and 69) even in a very low dose (1 pg/ml) inhibited the production of NO.

### Results

### ApoE experiment

The invention provides a method and a signalling molecule for the treatment of conditions that are associated with dysfunctional LDL receptors such as ApoE and other members of the apolipoprotein family. In particular, use of a signalling molecule comprising GVLPALPQ (NMPF - 5) and/or VLPALP (NMPF-6) or a functional analogue or derivative thereof is preferred. Groups of ApoE deficient mice (n=6 per group) were fed a high cholesterol food and given PBS or NMPF every other day intraperitonealy. After 2.5 weeks body weight was determined as shown in the Table below.

| Average Weight | |
|---|---|
| (g) | SD (g) p-value |
| ApoE-/- PBS 31.667 | 1.007 |
| ApoE-/- NMPF-4 31.256 | 1.496 0.536 |
| ApoE-/- NMPF-5 29.743 | 1.160 0.019 |
| Background/PBS26.760 | 1.582 10⁻⁶ |
| ApoE-/- NMPF-6 29.614 | 1.064 0.004 |

### Analysis of different peptides in data bases

Examples of different data bases in which peptides were analysed are:
Proteomics tools: Similarity searches
   BLAST data base (ExPasy, NCBI)
   SMART (EMBL)
   PATTINPROT (PBIL)
   Post-translational modification prediction
   SignalP (CBS)
   Primary structure analysis
   HLA Peptide Binding Predictions (BIMAS)
   Prediction of MHC type I and II peptide binding
   (SYFPEITHI)
   Amino acid scale representation (Hydrophobicity, other
   conformational parameters, etc.) (PROTSCALE)
   Representations of a protein fragment as a helical wheel(HelixWheel / HelixDraw)

### RESULTS

A non-extensive 1,ist of relevant oligopeptides useful for application in a method to identify signalling molecules according to the invention derivable from protein data bases.
pdb |1DE7 | 1DE7-A INTERACTION OF FACTOR XIII ACTIVATION PEPTIDE WITH ALPHA- THROMBIN
   LQGV, LQGVV, LQGVVP
pdb | 1DL6 | 1DL6-A SOLUTION STRUCTURE OF HUMAN TFIIB N-TERMINAL DOMAIN
   LDALP
pdb | 1QMH | 1QMH-A CRYSTAL STRUCTURE OF RNA 3'-TERMINAL PHOSPHATE CYCLASE, AN UBIQUITOUS ENZYME
   LQTV,VLPAL,LVLQTVLPAL
pdb | 1LYP | 1LYP CAP18 (RESIDUES 106 - 137)
   IQG, IQGL, LPKL, LLPKL
pdb | 1B90 | 1B90-A HUMAN ALPHA-LACTALBUMIN
   LPEL
pdb | 1GLU | 1GLU-A GLUCOCORTICOID RECEPTOR (DNA-BINDING DOMAIN) PARP
pdb | 2KIN | 2KIN-B KINESIN (MONOMERIC) FROM RATTUS NORVEGICUS
   MTRI
pdb | 1SMP | 1SMP-I MOL_ID: 1; MOLECULE: SERRATIA METALLO PROTEINASE; CHAIN: A
   LQKL, LQKLL, PEAP, LQKLLPEAP
pdb | 1ES7 | 1ES7-B COMPLEX BETWEEN BMP-2 AND TWO BMP RECEPTOR IA ECTODOMAINS
   LPQ,PTLP,LQPTL
pdb | 1BHX | 1BHX-F X-RAY STRUCTURE OF THE COMPLEX OF HUMAN ALPHA THROMBIN WITH THE INHIBITOR SDZ 229-357
   LQV, LQVV
pdb | 1VCB | 1VCB-A THE VHL-ELONGINC-ELONGINB STRUCTURE
   PELP
pdb | 1CQK | 1CQK-A CRYSTAL STRUCTURE OF THE CH3 DOMAIN FROM THE MAK33 ANTIBODY
   PAAP,PAAPQ,PAAPQV
pdb | 1FCB | 1FCB-A FLAVOCYTOCHROME
   LQG,
pdb | 1LDC | 1LDC-A L-LACTATE DEHYDROGENASE: CYTOCHROME C OXIDOREDUCTASE (FLAVOCYTOCHROME B=2=) (E.C.1.1.2.3) MUTANT WITH TYR 143 REPLACED BY PHE (Y143F) COMPLEXED WITH PYRUVATE
   LQG
pdb | 1BFB | 1BFB BASIC FIBROBLAST GROWTH FACTOR COMPLEXED WITH HEPARIN TETRAMER FRAGMENT
   LPAL,PALP,PALPE
pdb | 1MBF | 1MBF MOUSE C-MYB DNA-BINDING DOMAIN REPEAT 1
   LPN
pdb | 1R2A | 1R2A-A THE MOLECULAR BASIS FOR PROTEIN KINASE A
   LQG,LTELL
pdb | 1CKA | 1CKA-B C-CRK (N-TERMINAL SH3 DOMAIN) (C-CRKSH3-N) COMPLEXED WITH C3G PEPTIDE (PRO-PRO-PRO-ALA-LEU-PRO-PRO-LYS-LYS-ARG)
   PALP
pdb | 1RLQ | 1RLQ-R C-SRC (SH3 DOMAIN) COMPLEXED WITH THE PROLINE-RICH LIGAND RLP2 (RALPPLPRY) (NMR, MINIMIZED AVERAGE STRUCTURE) LPPL,PPLP
pdb | 1TNT | 1TNT MU TRANSPOSASE (DNA-BINDING DOMAIN) (NMR, 33 STRUCTURES)
   LPG, LPGL, LPK
pdb | 1GJS | 1GJS-A SOLUTION STRUCTURE OF THE ALBUMIN BINDING DOMAIN OF STREPTOCOCCAL PROTEIN G
   LAAL, LAALP
pdb | 1GBR | 1GBR-B GROWTH FACTOR RECEPTOR-BOUND PROTEIN 2 (GRB2, N-TERMINAL SH3 DOMAIN) COMPLEXED WITH SOS-A PEPTIDE (NMR, 29 STRUCTURES)
   LPKL,PKLP
pdb | 1A78 | 1A78-A COMPLEX OF TOAD OVARY GALECTIN WITH THIO-DIGALACTOSE
   VLPSIP
pdb | 1ISA | 1ISA-A IRON(II) SUPEROXIDE DISMUTASE (E.C.1.15.1.1)
   LPAL,PALP
pdb | 1FZV | 1FZV-A THE CRYSTAL STRUCTURE OF HUMAN PLACENTA GROWTH FACTOR-1 (PLGF-1), AN ANGIOGENIC PROTEIN AT 2.0A RESOLUTION PAVP, MLPAVP
pdb | 1JLI | 1JLI HUMAN INTERLEUKIN 3 (IL-3) MUTANT WITH TRUNCATION AT BOTH N- AND C-TERMINI AND 14 RESIDUE CHANGES, NMR, MINIMIZED AVERAGE
   LPC,LPCL,PCLP
pdb | 1HSS | 1HSS-A 0.19 ALPHA-AMYLASE INHIBITOR FROM WHEAT
   VPALP
pdb | 3CRX | 3CRX-A CRE RECOMBINASE/DNA COMPLEX INTERMEDIATE I
   LPA, LPAL, PALP
pdb | 1PRX | 1PRX-A HORF6 A NOVEL HUMAN PEROXIDASE ENZYME
   PTIP, VLPTIP
pdb | 1RCY | 1RCY RUSTICYANIN (RC) FROM THIOBACILLUS FERROOXIDANS VLPGFP
pdb | 1A3Z | 1A3Z REDUCED RUSTICYANIN AT 1.9 ANGSTROMS
   PGFP,VLPGFP
pdb | 1GER | 1GER-A GLUTATHIONE REDUCTASE (E.C.1.6.4.2) COMPLEXED WITH FAD
   LPALP,PALP
pdb | 1PBW | 1PBW-A STRUCTURE OF BCR-HOMOLOGY (BH) DOMAIN
   PALP
pdb | 1BBS | 1BBS RENIN (E.C.3.4.23.15)
   MPALP
AI188872 11.3 366 327 18 382 [Homo sapiens]qd27c01.x1
   Soares_placenta_8to9weeks_2NbHP8to9W
   Homo sapiens cDNA clone IMAGE:1724928 3' similar to gb:J00117 CHORIOGONADOTROPIN BETA CHAIN PRECURSOR
   (HUMAN);, mRNA sequence.; minus strand; translated
   MXRVLQGVLPALPQVVC, MXRV, MXR,
AI126906 19.8 418 343 1 418 [Homo sapiens]qb95f01.x1 Soares_fetal_heart_NbHH19W Homo sapiens cDNA clone IMAGE:1707865 3' similar to gb:J00117 CHORIOGONADOTROPIN BETA CHAIN PRECURSOR
   (HUMAN);, mRNA sequence.; minus strand; translated
   ITRVMQGVIPALPQVVC
AI221581 29.1 456 341 23 510 [Homo sapiens]qg20a03.x1
   Soares_placenta_8to9weeks_2NbHP8to9W Homo sapiens cDNA clone IMAGE:1760044 3' similar to gb:J00117 CHORIOGONADOTROPIN BETA CHAIN PRECURSOR (HUMAN);, mRNA sequence.; minus strand; translated
   MTRVLQVVLLALPQLV
Mm.42246.3 Mm.42246 101.3 837 304 28 768 GENE=Pck1 PROTSIM=pir:T24168
   phosphoenolpyruvate carboxykinase 1,
   cytosolic; translated
   KVIQGSLDSLPQAV, LDSL, LPQ
Mm.22430.1 Mm.22430 209.4 1275 157 75 1535 GENE=Ask-pending
   PROTSIM=pir:T02633 activator of S phase kinase; translated
   VLQAILPSAPQ, LQA, LQAIL, PSAP, LPS
Hs.63758.4 Hs.63758 93.8 3092 1210 51 2719 GENE=TFR2
   PROTSIM=pir:T30154 transferrin receptor 2; translated KVLQGRLPAVAQAV, LQG, LPA, LPAV
Mm.129320.2 Mm.129320 173.0 3220 571 55 2769 GENE= PROTSIM=pir:T16409 Sequence 8 from Patent WO9950284; translated
   LVQKVVPMLPRLLC, LVQ, LPRL, PMLP
Mm.22430.1 Mm.22430 209.4 1275 157 75 1535 GENE=Ask-pending PROTSIM=pir:T02633 activator of S phase kinase; translated
   VLQAILPSAPQ, LQA, LQAIL, PSAP, PSAPQ
P20155 IAC2_HUMAN Acrosin-trypsin inhibitor II precursor (HUSI-II) [SPINK2] [Homo sapiens]
   LPGCPRHFNPV, LPG, LPGC
Rn.2337.1 Rn.2337 113.0 322 104 1 327 GENE= PROTSIM=PRF:1402234A Rat pancreatic secretory trypsin inhibitor type II (PSTI-II) mRNA, complete cds; minus strand; translated
   LVGCPRDYDPV, LVG, LVGC
Hs.297775.1 Hs.297775 43.8 1167 753 31 1291 GENE= PROTSIM=sp:000268 ESTs, Weakly similar to T2D3_HUMAN TRANSCRIPTION INITIATION FACTOR TFIID 135 KDA SUBUNIT [H.sapiens]; minus strand; translated
   PGCPRG,PGCP
   Mm.1359.1 Mm.1359 PROTSTM=pir.A39743 urokinase plasmiogen activator receptor LPGCP, PGCP, LPG, LPGC
sptrembl | O56177 | O56177 ENVELOPE GLYCOPROTEIN
   VLPAAP,PAAP
sptrembl | Q9W234 | Q9W234 CG13509 PROTEIN.//arembl | AE003458 | AE003458_7 gene: "CG13509"; Drosophila melanogaster genomic scaffold
   LAGTIPATP, LAG, PATP
swiss | P81272 | NS2B HUMAN NITRIC-OXIDE SYNTHASE IIB (EC 1.14.13.39) (NOS, TYPE II B) (NOSIIB) (FRAGMENTS)
   GVLPAVP,LPA,VLPAVP,PAVP
sptrembl | O030137 | O30137 HYPOTHETICAL 17.2 KDA
   GVLPALP, PALP, LPAL
sptrembl | Q9IYZ3 | Q9IYZ3 DNA POLYMERASE
   GLLPCLP, LPC, LPCL, PCLP
sptrembl | Q9PVW5 | Q9PVW5 NUCLEAR PROTEIN NP220
   PGAP, LPQRPRGPNP, LPQ, PRGP, PNP
Hs.303116.2 PROTSIM=pir;T33097 stromal cell-derived factor 2-like1; translated
   GCPR
pdb | 1DU3 | 1DU3-A CRYSTAL STRUCTURE OF TRAIL-SDR5
   GCPRGM
pdb | 1D0G | 1D0G-R CRYSTAL STRUCTURE OF DEATH RECEPTOR 5 (DR5) BOUND
   TO APO2L/TRAIL
   GCPRGM
pdb | 1BIO | 1BIO HUMAN COMPLEMENT FACTOR D IN COMPLEX WITH ISATOIC ANHYDRIDE INHIBITOR
   LQHV
pdb | 4NOS | 4NOS-A HUMAN INDUCIBLE NITRIC OXIDE SYNTHASE WITH INHIBITOR
   FPGC,PGCP
pdb | 1FL7 | 1FL7-B HUMAN FOLLICLE STIMULATING HORMONE
   PARP,VPGC
pdb | 1HR6 | 1HR6-A YEAST MITOCHONDRIAL PROCESSING PEPTIDASE
   CPRG, LKGC
pdb | 1BFA | 1BFA RECOMBINANT BIFUNCTIONAL HAGEMAN FACTOR/AMYLASE INHIBITOR FROM
   PPGP, LPGCPREV, LPGC, PGCP, CPRE
swissnew | P01229 | LSHB HUMAN Lutropin beta chain precursor
   MMRVLQAVLPPLPQVVC, MMR, MMRV, LQA, LQAV, VLPPLP, PPLP, QVVC,
   VVC, VLPPLPQ, AVLPPLP, AVLPPLPQ
swissnew | P07434 | CGHB PAPAN Choriogonadotropin beta chain precursor
   MMRVLQAVLPPVPQVVC, MMR, MMRV, LQA, LQAG, VLPPVP, VLPPVPQ,
   QVVC, VVC, AVLPPVP, AVLPPVPQ
swissnew | Q28376 | TSHB HORSE Thyrotropin beta chain precursor
   MTRD, LPK, QDVC, DVC, IPGC, PGCP
swissnew | P95180 | NUOB MYCTU NADH dehydrogenase I chain B
   LPGC, PGCP
sptrembl | Q9Z284 | Q9Z284 NEUTROPHIL ELASTASE
   PALP,PALPS
sptrembl | Q9UCG8 | Q9UCG8 URINARY GONADOTROPHIN PEPTIDE (FRAGMENT).
   LPGGPR, LPG, LPGG, GGPR
XP_028754 growth hormone releasing hormone [Homo sapiens]
   LQRG, LQRGV, LGQL

A further non-limiting list includes collagen, PSG, CEA, MAGE (malanoma associated growth antigen), Thrombospondin-1, Growth factors, MMPs, Calmodulin, Olfactory receptors, Cytochrome p450, Kinases, Von Willebrand factor (coagulation factors), Vacuolar proteins (ATP sythase), Glycoprotein hormones, DNA polymerase, Dehydrogenases, Amino peptidases, Trypsin, Viral proteins (such as envelope protein), Elastin, Hibernation associated protein, Antifreeze glycoprotein, Proteases, Circumsporozoite, Nuclear receptors, Transcription actors, Cytokines and their receptors, Bacterial antigens, Nramp, RNA polymerase, Cytoskeletal proteins, Hematopoietic (neural) membrane proteins, Immunoglobulins. HLA/MHC, G-coupled proteins and their receptors, TATA binding proteins, Transferases, Zinc finger protein, Spliceosmal proteins, HMG (high mobility group protein), ROS (reactive oxygen species), superoxidases, superoxide dismutase, Proto-oncogenes/tumor suppressor genes, Apolipoproteins
SignalP (CBS)
   SignalP predictions: (for example)
   MTRVLQGVLPALP
   QVVC
   HLA Peptide Binding Predictions (BIMAS)
(For example)
Half time of dissociation
HLA molecule type I (A_0201): VLQGVLPAL (84)
GVLPALPQV (51)
VLPALPQVV (48)
RLPGCPRGV (14)
TMTRVLQGV (115)
scores
MHC II (H2-Ak 15 - mers) CPTMTRVLQGVLPAL 14
PGCPRGVNPVVSYAV 14
HLA-DRB1*0101 15-mersPRGVNPVVSYAVALS 29
TRVLQGVLPALPQVV 28
LQGVLPALPQVVCNY 22
HLA-DRB1*0301 (DR17)
15 - mers CPTMTRVLQGVLPAL 26
MTRVLQGVLPALPQV 21
SIRLPGCPRGVNPVV 17

**TABLE 1. Results of shock experiments in mice**

| TEST SUBSTANCE | | % SURVIVAL IN TIME | | | |
|---|---|---|---|---|---|
| (HRS) | | 0 | 16 | 40 | 72 |
| PBS | | 100 | 100 | 67 | 17 |
| PG23 | | 100 | 100 | 100 | 100 |
| PG25 | | 100 | 83 | 83 | 83 |

| PEPTIDE NMPF | | | | | |
|---|---|---|---|---|---|
| | SEQUENCE | | | | |
| 1 | VLPALPQVVC | 100 | 100 | 50 | 17 |
| 2 | LQGVLPALPQ | 100 | 67 | 0 | 0 |
| 3 | LQG | 100 | 83 | 20 | 17 |
| 4 | LQGV | 100 | 100 | 100 | 100 |
| 5 | GVLPALPQ | 100 | 100 | 80 | 17 |
| 6 | VLPALP | 100 | 100 | 100 | 100 |
| 7 | VLPALPQ | 100 | 83 | 0 | 0 |
| 8 | GVLPALP | 100 | 100 | 83 | 67 |
| 9 | VVC | 100 | 100 | 50 | 50 |
| 11 | MTRV | 100 | 100 | 67 | 50 |
| 12 | MTR | 100 | 100 | 67 | 50 |
| 13 | LQGVLPALPQVVC | 100 | 100 | 100 | 100 |
| 14 | (CYCLIC) LQGVLPALPQVVC | 100 | 83 | 83 | 83 |
| 64 | LPGCPRGVNPVVS | 100 | 100 | 100 | 100 |
| 66 | LPGC | 100 | 100 | 100 | 100 |

**TABLE 2. Additional results of shock experiments**

| NMPF SEQUENCE ID: | ANTI-SHOCK EFFECT |
|---|---|
| LQGV | +++ |
| AQGV | +++ |
| LQGA | +++ |
| VLPALP | +++ |
| ALPALP | ++ |
| VAPALP | ++ |
| ALPALPQ | ++ |
| VLPAAPQ | ++ |
| VLPALAQ | +++ |

| | SHOCK ACCELERATING EFFECT |
|---|---|
| LAGV | +++ |
| LQAV | +++ |
| VLAALP | +++ |
| VLPAAP | +++ |
| VLPALA | +++ |
| VLPALPQ | +++ |
| VLAALPQ | +++ |
| VLPALPA | +++ |

**TABLE 3. Further additional results of shock experiments**

| NMPF PEPTIDES | | | % SURVIVAL IN TIME (HRS) | | |
|---|---|---|---|---|---|
| Tx | Tx | | | | |
| | 0 | | 14 | 24 | 48 |
| PBS | 100 | | 100 | 100 | 0 |
| NMPF-3 | 100 | | 100 | 100 | 0 |
| NMPF-5 | 100 | | 100 | 100 | 100 |
| NMPF-7 | 100 | | 100 | 100 | 67 |
| NMPF-8 | 100 | | 100 | 100 | 100 |
| NMPF-9 | 100 | | 100 | 100 | 100 |
| NMPF-11 | 100 | | 100 | 100 | 100 |
| NMPF-12 | 100 | | 100 | 100 | 100 |
| NMPF-43 | 100 | | 100 | 100 | 100 |
| NMPF-45 | 100 | | 100 | 100 | 100 |
| NMPF-46 | 100 | | 100 | 100 | 100 |
| NMPF-50 | 100 | | 100 | 100 | 100 |
| NMPF-53 | 100 | | 100 | 100 | 100 |
| NMPF-58 | 100 | | 100 | 100 | 100 |
| NMPF-60 | 100 | | 100 | 100 | 100 |

**TABLE 4. Further additional results**

| NMPF PEPTIDES | | SICKNESS SCORES | | |
|---|---|---|---|---|
| Tx | Tx | | | |
| | 0 | 14 | 24 | 48 |
| PBS | 0,0,0,0,0,0 | 5,5,5,5,4,4 | 5,5,5,5,5,5 | †††††† |
| NMPF-3 | 0,0,0,0,0,0 | 3,3,3,3,3,4 | 4,4,4,4,4,4 | †††††† |
| NMPF-5 | 0,0,0,0,0,0 | 5,5,5,5,5,5 | 5,5,5,5,5,5 | 2,2,2,2,2,2 |
| NMPF-7 | 0,0,0,0,0,0 | 1,1,4,4,4,4 | 5,5,5,5,5,5 | 2,2,2,2,†† |
| NMPF-8 | 0,0,0,0,0,0 | 3,3,5,5,5,5 | 5,5,5,5,5,5 | 2,2,4,4,4,5 |
| NMPF-9 | 0,0,0,0,0,0 | 3,3,4,4,5,5 | 2,2,2,2,2,2 | 1,1,2,2,2,2 |
| NMPF-11 | 0,0,0,0,0,0 | 1,1,3,3,4,4, | 2,2,2,2,4,4 | 1,1,1,1,1,1 |
| NMPF-12 | 0,0,0,0,0,0 | 1,1,1,1,3,3 | 1,1,1,1,1,1 | 1,1,1,1,1,1 |
| NMPF-43 | 0,0,0,0,0,0 | 1,1,4,4,4,4 | 1,1,1,1,3,3 | 2,2,2,2,2,2 |
| NMPF-45 | 0,0,0,0,0,0 | 5,5,5,5,4,4 | 3,3,4,4,5,5 | 2,2,4,4,5,5 |
| NMPF-46 | 0,0,0,0,0,0 | 1,1,2,2,3,3 | 1,1,2,2,2,2 | 1,1,1,1,1,1 |
| NMPF-50 | 0,0,0,0,0,0 | 1,1,1,1,3,3 | 2,2,2,2,3,3 | 1,1,1,1,1,1 |
| NMPF-53 | 0,0,0,0,0,0 | 5,5,5,5,5,5 | 5,5,5,5,5,5 | 1,1,2,2,2,2 |
| NMPF-58 | 0,0,0,0,0,0 | 5,5,5,5,3,3 | 5,5,5,5,3,3 | 1,1,1,1,1,1 |
| NMPF-60 | 0,0,0,0,0,0 | 1,1,4,4,2,2 | 2,2,2,2,4,4 | 1,1,1,1,1,1 |

**Table 5 Summary of results of the various peptides in the various experiments. + = effects; -+ = variable effect; no entry is no effect or not yet tested when table was assembled**

| **ID** | **SEQUENCE** | **SEPSIS** | **ANGIOGENSIS** | **CAO** | **DC** | **NOD** |
|---|---|---|---|---|---|---|
| **NMPF-1** | VLPALPQVVC | -+ | | + | + | |
| **NMPF-2** | LQGVLPALPQ | -+ | | | + | |
| **NMPF-3** | LQG | -+ | + | + | + | |
| **NMPF-4** | LQGV | + | + | + | + | |
| **NMPF-5** | GVLPALPQ | -+ | | | + | |
| **NMPF-6** | VLPALP | + | + | + | + | |
| **NMPF-7** | VLPALPQ | + | + | | + | |
| **NMPF-8** | GVLPALP | -+ | | | + | |
| **NMPF-9** | VVC | + | + | | + | |
| **NMPF-10** | QVVC | | | | | |
| **NMPF-11** | MTRV | + | + | | + | + |
| **NMPF-12** | MTR | -+ | + | | + | |
| **NMPF-13** | LQGVLPALPQVVC | + | | | + | |
| **NMPF-14** | cyclic- LQGVLPALPQVVC | + | | | | |
| **NMPF-43** | AQG | + | + | | + | |
| **NMPF-44** | LAG | | + | | | |
| **NMPF-45** | LQA | + | + | | | |
| **NMPF-46** | AQGV | + | + | | + | |
| **NMPF-47** | LAGV | -+ | | + | + | |
| **NMPF-48** | LQAV | | | | | |
| **NMPF-49** | LQGA | + | | | | |
| **NMPF-50** | ALPALP | + | | | + | |
| **NMPF-51** | VAPALP | + | + | | | |
| **NMPF-52** | VLAALP | | | | | |
| **NMPF-53** | VLPAAP | + | | | + | |
| **NMPF-54** | VLPALA | | | | | |
| **NMPF-55** | ALPALPQ | + | | | | |
| **NMPF-56** | VAPALPQ | | + | | | |
| **NMPF-57** | VLAALPQ | | | | | |
| **NMPF-58** | VLPAAPQ | + | | | + | |
| **NMPF-59** | VLPALAQ | + | + | | | |
| **NMPF-60** | VLPALPA | + | | | + | |
| **NMPF-61** | VVCNYRDVRFESIRLPGCPRGVNPVVSYAVALSCQCAL | -+ | | + | | |
| **NMPF-62** | VVCNYRDVRFESIRLPGCPRGVNPVVSYAVALSCQ | | | | | |
| **NMPF-63** | SIRLPGCPRGVNPVVS | -+ | | | | |
| **NMPF-64** | LPGCPRGVNPVVS | | | + | | |
| **NMPF-65** | CPRGVNPVVS | | | | | |
| **NMPF-66** | LPGC | + | + | + | | |
| **NMPF-67** | CPRGVNP | | | | | |
| **NMPF-68** | PGCP | -+ | | | | |
| **NMPF-69** | RPRCRPINATLAVEKEGCPVCITVNTTICAGYCPT | | | | | |
| **NMPF-70** | MTRVLQGVLPALPQ | -+ | | | | |
| **NMPF-71** | MTRVLPGVLPALPQVVC | -+ | | | | |
| **NMPF-74** | CALCRRSTTDCGGPKDHPLTC | | | | | |
| **NMPF-75** | SKAPPPSLPSPSRLPGPC | | | | | |
| **NMPF-76** | TCDDPRFQDSSSSKAPPPSLPSPSRLPGPSDTPILPQ | | | | | |

### MODULATION OF NO AND/OR

**Table 6 TNF-A**

| **ID** | **SEQUENCE** | **TNF-A** | **NO** | **TNF-A and NO** |
|---|---|---|---|---|
| **NMPF-1** | VLPALPQVVC | ++ | ++++ | ++++ |
| **NMPF-2** | LQGVLPALPQ | -+ | ++++ | ++++ |
| **NMPF-3** | LQG | + | ++++ | ++++ |
| **NMPF-4** | LQGV | ++++ | ++++ | +++++++ |
| **NMPF-5** | GVLPALPQ | ++++ | ++++ | +++++++ |
| **NMPF-6** | VLPALP | ++++ | ++++ | +++++++ |
| **NMPF-7** | VLPALPQ | ++++ | ++++ | +++++++ |
| **NMPF-8** | GVLPALP | ++++ | ++++ | +++++++ |
| **NMPF-9** | VVC | ++++ | ++++ | +++++++ |
| **NMPF-10** | QVVC | ++++ | +++ | ++++ |
| **NMPF-11** | MTRV | ++++ | ++++ | ++++ |
| **NMPF-12** | MTR | ++++ | ++++ | ++++ |
| **NMPF-13** | LQGVLPALPQVVC | ++ | ++++ | ++++ |
| **NMPF-14** | cyclic- LQGVLPALPQVVC | ++ | ++++ | ++++ |
| **NMPF-43** | AQG | ++++ | ++++ | +++++++ |
| **NMPF-44** | LAG | -+ | ++++ | ++++ |
| **NMPF-45** | LQA | ++++ | ++++ | +++++++ |
| **NMPF-46** | AQGV | ++++ | ++++ | +++++++ |
| **NMPF-47** | LAGV | ++ | ++++ | ++++ |
| **NMPF-48** | LQAV | ++ | ++++ | ++++ |
| **NMPF-49** | LQGA | ++ | ++++ | ++++ |
| **NMPF-50** | ALPALP | ++++ | ++++ | +++++++ |
| **NMPF-51** | VAPALP | + | +++ | ++++ |
| **NMPF-52** | VLAALP | ++ | ++++ | ++++ |
| **NMPF-53** | VLPAAP | ++++ | ++++ | +++++++ |
| **NMPF-54** | VLPALA | + | ++++ | ++++ |
| **NMPF-55** | ALPALPQ | + | ++++ | ++++ |
| **NMPF-56** | VAPALPQ | -+ | ++++ | ++++ |
| **NMPF-57** | VLAALPQ | + | ++++ | ++++ |
| **NMPF-58** | VLPAAPQ | ++++ | ++++ | +++++++ |
| **NMPF-59** | VLPALAQ | ++ | ++++ | ++++ |
| **NMPF-60** | VLPALPA | ++++ | ++++ | +++++++ |
| **NMPF-61** | VVCNYRDVRFESIRLPGCPRGVN PVVSYAVALSCQCAL | -+ | ++++ | ++++ |
| **NMPF-62** | VVCNYRDVRFESIRLPGCPRGVN PVVSYAVALSCQ | -+ | +++ | ++++ |
| **NMPF-63** | SIRLPGCPRGVNPVVS | -+ | ++ | ++ |
| **NMPF-64** | LPGCPRGVNPVVS | ++ | ++++ | ++++ |
| **NMPF-65** | CPRGVNPVVS | ++ | +++ | +++ |
| **NMPF-66** | LPGC | +++ | ++ | +++ |
| **NMPF-67** | CPRGVNP | -+ | + | + |
| **NMPF-68** | PGCP | + | + | +++ |
| **NMPF-69** | RPRCRPINATLAVEK EGCPVCITVNTTICAGYCPT | -+ | ++ | ++ |
| **NMPF-70** | MTRVLQGVLPALPQ | -+ | + | + |
| **NMPF-71** | MTRVLPGVLPALPQVVC | -+ | -+ | -+ |
| **NMPF-74** | CALCRRSTTDCGGPKDHPLTC | -+ | ++ | + |
| **NMPF-75** | SKAPPPSLPSPSRLPGPS | + | ++ | ++ |
| **NMPF-76** | TCDDPRFQDSSSSKAPPPSLPSPS RLPGPSDTPILPQ | + | + | + |
| **NMPF-78** | CRRSTTDCGGPKDHPLTC | + | + | + |

| | | | | |
|---|---|---|---|---|
| from -+ to +++++++ indicates from barely active to very active in modulating | | | | |

### Monkey experiment

Efficacy of NMPF, here a mixture 1:1:1 of LQGV, AQGV and VLPALP, administered in a gram-negative induced rhesus monkey sepsis model for prevention of septic shock.

Overwhelming inflammatory and immune responses are essential features of septic shock and play a central part in the pathogenesis of tissue damage, multiple organ failure, and death induced by sepsis. Cytokines, especially tumour necrosis factor (TNF)-alpha interleukin (IL)-1beta, and macrophage migration inhibitory factor (MIF) have been shown to be critical mediators of septic shock. Yet, traditional anti-TNF and anti-IL-1 therapies have not demonstrated much benefit for patients with severe sepsis. We have designed peptides that block completely LPS induced septic shock in mice, even when treatment with these peptides is started up to 24 hours after LPS injection. These peptides are also able to inhibit the production of MIF. This finding provides the possibility of therapeutic use of these peptides for the treatment of patients suffering from septic shock. Since primates are evolutionary more closer to humans, we tested these peptides for their safety and effectiveness in a primate system.

**EXPERIMENTAL DESIGN**

| GROUP | EXPERIMENTAL TREATMENT | BIOTECHNIQUES | NUMBER | |
|---|---|---|---|---|
| | (independent variable, e.g. placebo treated control group) | | | |
| animal I | i.v. infusion of a lethal dose of live | Live E.coli infusion Blood sampling | N=1 | |
| | Escherichia.coli (10E10 CFU/kg) | No recovery (section) | | |
| | + antibiotics | | | |
| | + placebo treated | | | |
| animal II | i.v. infusion of a lethal dose of live | Live E.coli infusion Blood sampling | N=1 | |
| | Escherichia.coli (10E10 CFU/kg) | No recovery (section) | | |
| | + antibiotics | | | |
| | + oligopeptide ( 5mg/kg of each of 3 peptides) | | | |

Only naive monkeys were used in this preclinical study to exclude any interaction with previous treatments. The animals were sedated with ketamine hydrochloride. Animals were intubated orally and are allowed to breathe freely. The animals were kept anesthesized with O₂/N₂O/isoflurane. The animals received atropin as pre-medication for O₂/N₂O/isoflurane anesthesia. A level of surgical anesthesia was maintained during the 2 h infusion of *E.coli* and for 6 h following E.coli challenge after which the endothracheal tubes were removed and the animals were euthanized. Before bacteria were induced, a 1 hour pre-infusion monitoring of heart-rate and blood pressure was performed. Two rhesus monkeys were infused with a 10 ¹⁰CFU per kg of the Gram negative bacterium E.coli to induce a fatal septic shock. One monkey received placebo-treatment and was sacrificed within 7 hours after infusion of the bacteria without recovery from the anesthesia. The second monkey received treatment with test compound and was sacrificed at the same time point.

In a limited dose-titration experiment performed in 1991 with the same bacterium strain, the used dose proved to induce fatal shock within 8 hours. In recent experiments a 3-fold lower dose was used inducing clear clinical and pathomorphological signs of septic shock without fatal outcome.
The monkeys were kept anaesthetized throughout the observation period and sacrificed 7 hours after the start of the bacterium infusion for pathological examination. The animals underwent a gross necropsy in which the abdominal and thorac cavities were opened and internal organs examined in situ.

### Full description of the experiment with three rhesus monkeys

The study was conducted in rhesus monkeys (Maccaca mulatta). Only experimentally naive monkeys were used in study to exclude any interaction with previous treatments. Prior to the experiment the state of health of the animals was assessed physically by a veterinarian. All animals had been declared to be in good health and were free of pathogenic ecto- and endoparasites and common bacteriological infections: Yersinia pestis, Yersinia enterocolitica, Yersinia pseudotuberculosis, Shigella, Aeromonas hydrophilia, pathogenic Campylobacter species and Salmonella.

*Reagents*. The Escherichia coli strain was purchased from ATCC (E-coli; 086a: K61 serotype, ATCC 33985). In a control experiment the strain proved equally susceptible to bactericidal factors in human and rhesus monkey serum.
Prior to the experiment a fresh culture was set-up; the E.coli strain was cultured for one day, harvested and washed thoroughly to remove free endotoxin. Prior to infusion into the animal the number and viability of the bacteria was assessed. Serial dilutions of the E.coli stock were plated on BHI agar and cultured overnight at 37 degrees C. The colonies on each plate were counted and the number of colony forming units per ml were calculated. The body weight measurement of the day of the experiment was used to calculate the E.coli dose and E.coli stock was suspended in isotonic saline (N.P.B.I., Emmer-Compascuum, The Netherlands) at the concentration needed for infusion (total dose volume for infusion approximately 10 ml/kg. The E.coli suspension was kept on ice until infusion.

Antibiotic was used to synchronise the shock induction in the monkeys. Baytril (Baytril 2.5%, Bayer, Germany) was used instead of gentamycin, as the strain proved only marginally susceptible to the latter antibiotic.

Individual animals were identified by a number or letter combination tattooed on the chest.

### Experimental design.

| GROUP (number/ | EXPERIMENTAL TREATMENT | | NUMBER | SEX |
|---|---|---|---|---|
| letter or other identification | (independent variable, e.g. placebo treated control group) | | | |
| Animal I | i.v. infusion of a lethal dose of live | Live E.coli infusion Blood sampling | N=1 | F |
| | Escherichia.coli (10E10 CFU/kg) | No recovery | | |
| | + antibiotic | | | |
| | + placebo treated | | | |
| Animal II | i.v. infusion of a lethal dose of live | Live E.coli infusion Blood sampling | N=1 | F |
| | Escherichia.coli (10E10 CFU/kg) | No recovery (section) | | |
| | + antibiotic | | | |
| | + NMPF-4, 6, 46; each 5mg/kg | | | |
| Animal III | i.v. infusion of a lethal dose of live | Live E.coli infusion Blood sampling | N=1 | F |
| | Escherichia.coli (10E10 CFU/kg) | Recovery and survival | | |
| | + antibioti | | | |
| | + NMPF-4, 6, 46; each 5mg/kg | | | |

*Anesthesia*. All animals were fasted overnight prior to the experiment. On the morning of the experiment the animals were sedated with ketamine hydrochloride (Tesink, The Netherlands) and transported to the surgery. The animal was placed on its side on a temperature controlled heating pad to support body temperature. Rectal temperature was monitored using a Vet-OX 5700. The animals were intubated orally and were allowed to breathe freely. The animals were kept anesthesized using O₂/N₂O/isoflurane inhalation anaesthesia during the E.coli infusion and the 7 hour observation period following E.coli challenge after which the endothracheal tubes were removed and the animals were euthanized or allowed to recover from anesthesia. The femoral or the cephalic vein was cannulated and used for infusing isotonic saline, live E.coli and antibiotic administration. Insensible fluid loss was compensated for by infusing isotonic saline containing 2.5% glucose (Fresenius, 's Hertogenbosch, The Netherlands) at a rate of 3.3 ml/kg/hr. *Preparative actions*. During anesthesia the animals were instrumented for measurement of blood pressure (with an automatic cuff), heart rate and body temperature. Isotonic saline was infused at 3.3 ml/kg/hr to compensate for fluid loss. Femoral vessels were cannulated for infusion of E.coli and antibiotics. Temperature controlled heating pads were used to support body temperature. The monkeys were continuously monitored during E.coli challenge and for the 6 hr period following E.coli administration. After 7 hrs 2 animals (the control animal and one treated with NMPF) were sacrificed to compare the direct effect of the compound at the level of histology. The 3^{rd} animal, treated with NMPF, was allowed to recover from anaesthesia and was intensively observed during the first 12 hours after recovery followed by frequent daily observation. The decision to allow 3^{rd} animal to recover was made after consulting with the veterinarian.
*Induction of septic shock*. Before the infusion of E.coli, a 1 hr pre-infusion monitoring of heart-rate and blood pressure was performed. All three animals received an i.v. injection of E.coli 086 (k61 serotype; ATCC 33985) at a lethal dose of 10 x109 CFU/kg bodyweight. In a dose titration study with this batch performed in 1991, this bacterial dose induced lethal shock within 8 hrs after the start of the infusion. The infusion period was 2 hrs.
*Antibiotics*. Baytril was administered intravenously immediately after completion of the 2 h. E.coli infusion (i.v.; dose 9 mg/kg).
*Treatment with NMPF*. 30 minutes post-onset of E.coli infusion the animals were administered a single intravenous bolus injection a mixer of NMPF oligopeptide. The oligopeptide mixer contained the following NMPF peptides: LQGV (5 mg/kg), AQGV (5 mg/kg) and VLPALP (5 mg/kg). These NMPF peptides were dissolved in 0.9% sodium chloride for injection (N.P.B.I., Emmer Compascuum, The Netherlands).

### RESULTS

### Preliminary monkey results

An anti-shock effect of the test compound on sepsis in the monkey treated with the oligopeptide mixture , namely the inhibition of the effect of the sepsis in this early 7-hour trajectory of this primate model was observed. Immunomodulatory effects with these peptides have been observed *in vitro*/*ex vivo* such as in T-cell assays the inhibition of pathological Th1 immune responses, suppression of inflammatory cytokines (MIF), increase in production of anti-inflammatory cytokines (IL-10, TGF-beta) and immunomodulatory effects on antigen presenting cells (APC) like dendritic cells and macrophages.

The following organs were weighed and a bacterial count were performed:
kidneys
liver
lungs
lymph nodes
gross lesions

Tissues of all organs were preserved in neutral aqueous phosphate buffered 4% solution of formaldehyde. Lymphoid organs were be cryopreserved. All tissues will be processed for histopathological examination.

### Further results obtained in the three-monkey experiment

*Monkey 429(control).* Female monkey (5.66 kg) received an i.v. injection of E.coli 086 (10E10 CFU/kg). In a dose titration study with this batch performed in 1991, this bacterial dose induced lethal shock within 8 hrs after the start of the infusion. The infusion period was 2 hrs. Baytril was administered intravenously immediately after completion of the 2 h. E.coli infusion (i.v.; dose 9 mg/kg). After the E.coli injection the monkey was observed by the authorized veterinarian without knowing which of the monkey received NMPF treatment. The clinical observations were as follows: vomiting, undetectable pulse, heart arythmia, abnormalities in ECG: signs of ventricle dilatation/heart decompensation (prolonged QRS complex, extra systoles), decreased blood clotting and forced respiration. In addition, there was big fluctuation in heart rate (30-150 beats per minute), collapse of both systolic and diastolic blood pressure (35/20 mmHg) and decrease in blood oxygen concentration (80-70%). Seven hours after the start of the E.coli infusion, monkey began to vomit blood and faeces, and have convulsions. After final examination, the veterinarian did not gave permission to let this monkey awake. At this time point control monkey was euthanised. Hereafter, post mortem examination was conducted and internal organs were examined in situ. Number of internal bleedings were found by the pathologist.

*Monkey 459(NMPF).* Female monkey (5.44 kg) received an i.v. injection of E.coli 086 (10E10 CFU/kg). In a dose titration study with this batch performed in 1991, this bacterial dose induced lethal shock within 8 hrs after the start of the infusion. The infusion period was 2 hrs. 30 minutes after the initiation of E.coli infusion, NMPF was i.v. injected in a single bolus injection. Baytril was administered intravenously immediately after completion of the 2 h. E.coli infusion (i.v.; dose 9 mg/kg). After the E.coli injection this monkey was also observed by the authorized veterinarian without knowing which of the monkey received NMPF treatment. The clinical observations were as follows: normal pulse, heart sounds normal, normal ECG, higher heart-rate but otherwise stable (180 beats per minute), no hypotension (75/30 mmHg), normal blood oxygen concentration (95-85%), lungs sound normal, normal turgor. Seven hours after the start of the E.coli infusion, the clinical condition of the monkey was stable. After final examination, the veterinarian did give permission to let this monkey awake due to her stable condition. Howver, in order to be able to compare the hematological and immunological parameters between the control and NMPF treated monkey, at this time point NMPF treated monkey 459 was euthanised. Hereafter, post mortem examination was conducted and internal organs were examined in situ. No macroscopic internal bleedings were found by the pathologist.

*Monkey 427(NMPF).* Female monkey (4.84 kg) received an i.v. injection of E.coli 086 (10E10 CFU/kg). In a dose titration study with this batch performed in 1991, this bacterial dose induced lethal shock within 8 hrs after the start of the infusion. The infusion period was 2 hrs. 30 minutes after the initiation of E.coli infusion, NMPF was i.v. injected. Baytril was administered intravenously immediately after completion of the 2 h. E.coli infusion (i.v.; dose 9 mg/kg). After the E.coli injection this monkey was also observed by the authorised veterinarian without knowing which of the monkey received NMPF treatment. The clinical observations were as follows: normal pulse, heart sounds normal, normal ECG, moderately higher heart-rate but otherwise stable (160 beats per minute), no hypotension (70/30 mmHg), normal blood oxygen concentration (95-90%), lungs sound normal, normal turgor. Seven hours after the start of the E.coli infusion, the clinical condition of the monkey was stable. After final examination, the veterinarian did give permission to let this monkey wake up due to her stable condition. Monkey woke up quickly, she was alert and there was a slow disappearance of oedema.

### Summarizing comment on pathology reports

For comparative evaluation of the pathology reports special emphasis was put on those organs that showed distinct study-related alterations and of the two monkeys (429 and 459) that were euthanised or died at about seven hours in the experiment.
Liver was most severely damaged in animal 429 indicated by multifocal pronounced (up to subtotal) lobular necrotic areas with neutorphil-granulocytic demarcation. This animal showed multifocal acute hepatocytic necrosis, dissociation of hepatocytes and sinusoidal leukocytosis.
In contrast animal 459 did not show significant damage of hepatocytes as compared to the above mentioned alterations of 429 although cloudy swelling of hepatocytes very well indicated presence of damage in hepatocytic membrane functions (cell membrane as well as membranes of sub cellular compartments as endoplasmic reticulum and golgi apparatus i.o.). However disturbances in energy dependent membrane transport processes might finally be of transient nature.
Summarizing the pathomorphological features of the liver tissue of the above mentioned animals rhesus monkey 459 showed comparatively minimal alterations.
Stomach mucosa showed pronounced acute diffuse hemorrhages in animal 429 alterations that are regarded as associated with endotoxemia. In addition animal 429 had acute hemorrhages in the abdominal cavity perifocal to the uterus. No comparable findings were present in animal 459.
No comparable findings were seen in the other animal.
Comparative evaluation of adrenal glands did not show significant differences between the three animals. Adrenals of both animals presented multifocal to diffuse cortical neutrophil-granulocytic infiltrations.
Summarizing comments: Tissue damage is regarded as most severe in animal 429 while animal 459 shows quantitatively and qualitatively comparably only slight tissue alterations.

### Genomic experiment

### ANNEMIEK

PM1 T-cell line was obtained from American Type Culture Collection (Manassas, VA) and was cultured at 37°C in 5% CO2. These cells were maintained and cultured in RPMI 1640, 10% fetal bovine serum, 2 mM L-glutamine, and antibiotics penicillin and streptomycin. For genomic experiments cells (2 x10⁶/ml) were incubated with phytohemagglutinin (PHA, 10□g/ml) and IL-2 (200 IU/ml) or PHA, IL-2 and peptide LQGV (10mg/ml) in a volume of 2 ml in 6-wells plates. After 4 h of cultures 10 x10⁶ cells were washed and prepared for genechip probe arrays experiment. The genechip expression analysis was performed according to the manufacturer's instructions (Expression Analysis, Technical Manual, Affymetrix Genechip). The following major steps outline Genechip Expression Analysis: 1) Target preparation 2) Target hybridization 3) Experiment and fluidics station setup 4) Probe Array washing and staining 5) Probe array scan and 6) Data analysis.

### RESULTS

### Genomic experiment

The gene chip expression analysis revealed that due to LQGV treatment of PM1 (T-cell line) cells for 4 hours in the presence of PHA/IL-2 down-regulated at least 120 genes more than 2 fold as compared to control PM1 cells (stimulated with PHA/IL-2) only. Moreover, at least 6 genes were up-regulated more than 2 fold in peptide treated cells as compared to control cells.

Identification of down-regulated genes due to treatment with LQGV in genomics experiment. Given are the -Fold Change and Accession number(s) or description of the gene(s).
- 21.2: M11507
- 10.1: U22376
- 9.7: X68836
- 9.3: M97935
- 8.7: D30037
- 7.5: U28964
- 6.7: U10564; L23959
- 6.5: W29030
- 6.1: U08997
- 5.7: M97935
- 5.6: Y00638
- 5.3: Ras-Like Protein Tc21; X83492
- 4.8: AJ002428
- 4.7: Ras-Related Protein Rap1b
- 4.6: AL080119
- 4.5: AF047448; D14710; X59618; D28364;;AA477898
- 4.4: L19161; U48736; L43821;Ras-Like Protein Tc21;U22376
- 4.3: U18271
- 4.2: Fk506-Binding Protein, Alt. Splice 2 ;J05614
- 4.1: U08316; W28732;Y00638
- 4: AF000545
- 3.8: U08997
- 3.6: X03484; M32886; M28209
- 3.5: L34075; J04088
- 3.4: L19161;D28423;AA442560; X98248
- 3.3: AB020670; W28869; Z12830;AL021546
- 3.2: U78082; X74262
- 3.1: M64174; AI862521; W27517
- 3: D13988; AL080119; M33336; L75847; M21154; AA675900; M97936
- 2: U16720; M33336; U50079; U16720; X87212; AI740522; M21154; X00737
- 2.1: AF034956; Ras Inhibitor Inf; M27749; Ras-Like Protein Tc4; X92106; D88674; H15872; L07541; V01512; L23959; Stimulatory Gdp/Gtp Exchange Protein For C-Ki-Ras P21 And Smg P21; L13943; X78925; U78733
- 2.2: L07540; AF040958; D00596; AI659108; AF042083; W28907
- 2.3: AF073362; J04423; D59253; M21154; Proto-Oncogene C-Myc; W26787
- 2.4: L12002; M55536; S75881; S75881; AF050110; M86667
- 2.5: U17743; U90549; U31382; S81916; M64595; Serine Hydroxymethyltransferase; U88629; U72518; L14595; AB014584; AI924594; U68111; AI924594; AL009179; AF091077; M28211; Z85986; AB019435; U39318; X78711; Y09443; Z82200
- 2.6: X69549 Zinc Finger Protein, Kruppel-Like; D88357
- 2.7: D10656; M28211
- 2.8: W27594; X05360; V00568; L24804
- 2.9: L05624

Identification of Up regulated genes due to LQGV treatment. Depicted are the -Fold Change and the Accession number or description of the gene(s).
- 4.9: AF043324
- 3.3: L08096
- 2.1: AL031681; X87838
- 2.2: AW024285; D38524; L38935
- 2.5: L12711
- 2.6: AF026029
- 2.8: X70683

### Further examples of use

Examples of different receptor-intracellular signalling pathways involved in different disease pathogenesis where signalling molecules according to the invention find their use are:

LPS stimulation of antigen presenting cells (like DC, macrophages, monocytes) through different Toll-like receptors activates different signalling pathways including, MAPK pathways, ERK, JNK and p38 pathways. These pathways directly or indirectly phosphorylate and activate various transcription factors, including E1k-1, c-Jun, c-Fos, ATF-1, ATF-2, SRF, and CREB. In addition, LPS activates the IKK pathway of MyD88, IRAK, and TRAF6. TAK1-TAB2 and MEKK1-ECSIT complexes phosphorylate IKKb, which in turn phosphorylates IkBs. Subsequent degradation of IkBs permits nuclear translocation of NFkB/Rel complexes, such as p50/p65. Moreover, the P13K-Akt pathway phosphorylates and activates p65 via an unknown kinase. Some of these pathways could also be regulated by other receptor signalling molecules such as hormones/growth factor receptor tyrosine kinases (PKC/Ras/IRS pathway) and cytokine receptors (JAK/STAT pathway). In the genomic experiment with the T-cell line several of these genes appeared to be downregulated or upregulated by the peptide used (LQGV). It is now clear that other peptides in T cells and the same and other peptides in other cell types similarly downregulate or up-regulate several of these transcription factors and signalling molecules. In DC and fertilized eggs experiments NMPF had the ability to modulate growth factor (GM-CSF, VEGF) and LPS signalling. Some diseases associated with dysregulation of NF-kB and related transcription factors are: Atherosclerosis, asthma, arthritis, anthrax, cachexia, cancer, diabetes, euthyroid sick syndrome, AIDS, inflammatory bowel disease, stroke, (sepsis) septic shock, inflammation, neuropathological diseases, autoimmunity, thrombosis, cardiovascular disease, psychological disease, post surgical depression, wound healing, burn-wounds healing and neurodegenerative disorders.

PKC plays an essential role in T cell activation via stimulation of for example AP-1 and NF-kB that selectively translocate to the T cell synapse via Vav/Rac pathway. PKC is involved in a variety of immunological and non-immunological diseases as is clear from standard text books of internal medicine (examples are metabolic diseases, cancer, angiogenesis, immune mediated disorders, diabetes etc.)

LPS and ceramide induce differential multimeric receptor complexes, including CD14, CD11b, Fc-gRIII, CD36, TAPA, DAF and TLR4. This signal transduction pathway explains the altered function of monocytes in hypercholesterolemia and lipid disorders.

Oxidized low-density lipoproteins contribute to stages of the atherogenic process and certain concentrations of oxidized low-density lipoproteins induce apoptosis in macrophages through signal transduction pathways. These pathways are involved in various vascular diseases such as atherosclerosis, thrombosis etc.

Bacterial DNA is recognized by cells of the innate immune system. This recognition requires endosomal maturation and leads to activation of NF-kB and the MAPK pathway. Recently it has been shown that signaling requires the Toll like receptor 9 (TLR9) and the signalling adaptor protein MyD88. Recognition of dsRNA during viral infection seems to be dependent on intracellular recognition by the dsRNA dependent protein kinase PKR. TLRs play an essential role in the immune system and they are important in bridging and balancing innate immunity and adaptive immunity. Modulation of these receptors or their down-stream signalling pathways are important for the treatment of various immunological conditions such as infections, cancer, immune-mediated diseases, autoimmunity, certain metabolic diseases with immunological component, vascular diseases, inflammatory diseases etc.

Effect of growth factor PDGF-AA on NF-kB and proinflammatory cytokine expression in rheumatoid synoviocytes; PDGF-AA augmented NF-kB activity and mRNA expression of IL-1b, IL-8 and MIP-1a. Therefore, PDGF-AA may play an important role in progression of inflammation as well as proliferation of synoviocytes in RA.

Dendritic cell (DC) activation is a critical event for the induction of immune responses. DC activation induced by LPS can be separated into two distinct processes: first, maturation, leading to upregulation of MHC and costimulatory molecules, and second, rescue from immediate apoptosis after withdrawal of growth factors (survival). LPS induces NF-kB transcription factor. Inhibition of NF-kB activation blocked maturation of DCs in terms of upregulation of MHC and costimulatory molecules. In addition, LPS activates the extracellular signal-regulated kinases (ERK), and specific inhibition of MEK1, the kinase which activates ERK, abrogate the ability of LPS to prevent apoptosis but do not inhibit DC maturation or NF-kB nuclear translocation. This shows that ERK and NF-kB regulate different aspects of LPS induced DC activation. Our DC data and NF-kB data also show the various effects of NMPF peptide on DC maturation and proliferation in the presence or absence of LPS. NMPF peptides modulate these pathways and are novel tools for the regulation of DC function and immunoregulation. This opens new ways for the treatment of immune diseases, particularly those in which the immune system is in disbalance (DC1-DC2, Th1-Th2, regulatory cell etc.)

DC mediate NK cell activation which can result in tumour growth inhibition. DC cells and other antigen presenting cells (like macrophages, B-cells) play an essential role in the immune system and they are also important in bridging and balancing innate immunity and adaptive immunity. Modulation of these cells or their down-stream signalling pathways are important for the treatment of various immunological conditions such as infections, cancer, immune-mediated diseases, autoimmunity, certain metabolic diseases with immunological component, vascular diseases, inflammatory diseases etc. There is also evidence in the literature that mast cells play important roles in exerting the innate immunity by releasing inflammatory cytokines and recruitment of neutrophils after recognition of infectious agents through TLRs on mast cells.

In murine macrophages infected with Mycobacterium tuberculosis through JAK pathway activate STAT1 and activation of STAT1 may be the main transcription factor involved in IFN-g-induced MHC class II inhibition.

Recognition of mannose-binding lectin (MBL) through TLRs influences multiple immune mechanisms in response to infection and involved in innate immunity. Balance between innate and adoptive immunity is crucial for balanced immune system and dysregulation in immune system lead to different spectrum of diseases such as, inflammatory diseases, autoimmunity, infectious diseases, pregnancy associated diseases (like miscarriage and pre-eclampsia), diabetes, atherosclerosis and other metabolic diseases.

Nuclear factor-kappaB (NFkappaB) is critical for the transcription of multiple genes involved in myocardial ischemia-reperfusion injury. Clinical and experimental studies have shown that myocardial ischemia-reperfusion injury results in activation of the TLRs and the complement system through both the classical and the alternative pathway in myocardial infarction, atherosclerosis, intestinal ischaemia, hemorrhagic shock pulmonary injury, and cerebral infarction etc.

Peroxisome proliferator-activated receptors (PPARs) are ligand-activated transcription factors which function as regulators of lipid and lipoprotein metabolism, glucose homeostasis, influence cellular proliferation, differentiation and apoptosis and modulation of inflammatory responses. PPAR alpha is highly expressed in liver, muscle, kidney and heart, where it stimulates the beta-oxidative degradation of fatty acids. PPAR gamma is predominantly expressed in intestine and adipose tissue, where it triggers adipocyte differentiation and promotes lipid storage. Recently, the expression of PPAR alpha and PPAR gamma was also reported in cells of the vascular wall, such as monocyte/macrophages, endothelial and smooth muscle cells. The hypolipidemic fibrates and the antidiabetic glitazones are synthetic ligands for PPAR alpha and PPAR gamma, respectively. Furthermore, fatty acid-derivatives and eicosanoids are natural PPAR ligands: PPAR alpha is activated by leukotriene B4, whereas prostaglandin J2 is a PPAR gamma ligand, as well as of some components of oxidized LDL, such as 9- and 13-HODE. These observations suggested a potential role for PPARs not only in metabolic but also in inflammation control and, by consequence, in related diseases such as atherosclerosis. More recently, PPAR activators were shown to inhibit the activation of inflammatory response genes (such as IL-2, IL-6, IL-8, TNF alpha and metalloproteases) by negatively interfering with the NF-kappa B, STAT and AP-1 signalling pathways in cells of the vascular wall. Furthermore, PPARs may also control lipid metabolism in the cells of the atherosclerotic plaque. PPARs are also involved in a variety of immunological and non-immunological diseases as is clear from standard text books of internal medicine (examples are metabolic diseases, cancer, angiogenesis, immune mediated disorders, diabetes etc.)

As mentioned above the nuclear receptor PPARg is important in adipogenesis, lipid storage and involved in atherosclerosis. While expressed in adipose tissue this receptor is also expressed in macrophages and in the colon. In addition, PPARg is implicated in a number of processes such as cancer and inflammation. Moreover, microbes, via its cognate receptors, typified by the TLRs, possess the capacity to regulate PPARg dependent metabolic functions and as such illustrates the intricate interplay between the microbial flora and metabolic control in the alimentary tract.

Cyclo-oxygenase 2 (COX2), an inducible isoform of prostaglandin H synthase, which mediates prostaglandin synthesis during inflammation, and which is selectively overexpressed in colon tumours, is thought to play an important role in colon carcinogenesis. Induction of COX2 by inflammatory cytokines or hypoxia-induced oxidative stress can be mediated by nuclear factor kappa B (NF-kappaB). So, inhibition of NF-kB modulate COX pathway and this inhibition of NF-kB can be therapeutically useful in diseases in which COXs are involved, such as inflammation, pain, cancer (especially colorectal cancer), inflammatory bowel disease and others.

Neuronal subsets in normal brains constitutively express functionally competent C5a receptors. The functional role of C5a receptors revealed that C5a triggered rapid activation of protein kinase C and activation and nuclear translocation of the NF-kappa B transcription factor. In addition, C5a was found to be mitogenic for undifferentiated human neuroblastoma cells, a novel action for the C5aR. In contrast, C5a protects terminally differentiated human neuroblastoma cells from toxicity mediated by the amyloid A beta peptide. This shows that normal hippocampal neurons as well as undifferentiated and differentiated human neuroblastoma cells express functional C5a receptors. These results show the role of neuronal C5aR receptors in normal neuronal development, neuronal homeostasis, and neuroinflammatory conditions such as Alzheimer's disease.

Activation of the complement system plays also an important role in the pathogenesis of atherosclerosis. The proinflammatory cytokine interleukin (IL)-6 is potentially involved in the progression of the disease. Here the complement system induces IL-6 release from human vascular smooth-muscle cells (VSMC) by a Gi-dependent pathway involving the generation of oxidative stress and the activation of the redox sensitive transcription factors NF-kB and AP-1. Modulation of complement system is important for broad ranges of disorders such as blood disorders, infections, some metabolic diseases (diabetes), vascular diseases, transplant rejection and related disorders, autoimmune diseases, and other immunological diseases.

Different transcription factors like NF-kB and intracellular signaling molecules such as different kinases are also involved in multiple drug resistance. So, it is reasonable to believe that NMPF peptides will be effective against multiple drug resistance. Moreover, our genomic data shows that a number of genes and signalling molecules involved in tumorogenesis and metastasis are modulated. In addition since oligopeptides have also effect on angiogenesis, thus these peptides will also be used for the treatment of cancer and related diseases whereby angiogenesis requires modulation.

Proliferative diabetic retinopathy (PDR) is one of the major causes of acquired blindness. The hallmark of PDR is neovascularisation (NV), abnormal angiogenesis that may ultimately cause severe vitreous cavity bleeding and/or retinal detachment. Since NMPF peptides have angiogenesis stimulatory as well as inhibitory effects and have the ability to modulate intracellular signaling involved in growth factors (like insulin), pharmacologic therapy with certain NMPF peptides can improve metabolic control (like glucose) or blunt the biochemical consequences of hyperglycaemia (through mechanisms such as in which aldose reductase, protein kinase C (PKC), PPARs are involved). For this metabolic control or diabetes (type 2) NMPF (LQGV, VLPALP, VLPALPQ, GVLPALPQ, AQG, LAG, LQA, AQGV, VAPALP, VAPALPQ, VLPALPA, LPGC, MTR, MTRV, LQG, CRGVNPVVS are recommended. The angiogenesis in PDR could be also treated with above mentioned oligopeptides.

### Septic arthritis experiment

*Mice*. NMR1 mice were used for this experiments. Ten mice per treatment group were used in this experiment.
*Bacteria and inoculation*. S. aureus LS-1 was originally isolated from a swollen joint of spontaneously arthritic NZB/W mouse. One of the characteristics of this staphylococcal strain is that it produces large amounts of TSST-1, an exotoxin with superantigenic properties. The bacteria were cultured on blood agar for 24 hours and then reincubated on blood agar for another 24 hours. They were kept frozen at -20 degrees C in PBS (0.13 M sodium chloride, 10 mM sodium phosphate (pH 7.4)) containing 5% bovine serum albumin and 10% dimethyl sulfoxide (C2H6OS) until use. Prior to use, the bacterial solution was thawed, washed in PBS, and diluted in PBS to achieve the desired concentration of bacteria. Mice were inoculated with bacteria either in one of the tail veins (200 microlitre). Viable counts were made from the leftover bacterial solution, serially diluted, and then cultured on blood agar plates to ascertain the number of bacteria injected. After i.v. injection with bacteria one group of mice were treated i.p. with PBS, 3 times per week for two weeks and other group of mice were treated i.p. with NMPF-6 (100 micro-gram), 3 times per week for two weeks. During 13 days of follow-up period arthritis severity, mortality and weight decrease was measured.
*Clinical evaluation of arthritis*. All mice were followed up individually. The joints, i.e. finger/toe and wrist/ankle were inspected visually at regular intervals, i.e. at least every second or third day. Arthritis was defined as visible joint erythema and/or swelling of at least one joint. To evaluate the intensity of arthritis, a clinical scoring (arthritic index) was carried out using a system where macroscopic inspection yielded a score of 0 to 4 points for each limb (0, neither swelling nor erythema; 1, mild swelling and/or erythema; 2, moderate swelling and erythema; 3, marked swelling and erythema; 4, maximum swelling and erythema (moribund mice). In addition, arthritis frequency and weight was measured.

### Results

*Weight decrease* : There were no differences found between the two groups in weight decrease (Fig 66).
*Survival*. Until day 10 no major differences with respect to survival (PBS treated group: 70%, NMPF treated group:80%) was observed. At the termination of the study PBS treated mice had a survival rate of 40% and NMPF treated mice had a survival rate of 80% (Fig 67).
*Severity of arthritis*. Clear-cut differences in severity of arthritis between the groups were measured. Differences were visible from the very beginning and increased with time. At day 10 arthritic index in PBS treated mice was 2.4 and in NMPF treated mice was 1.0. At day 13 the arthritic index in PBS treated mice was 3.8 and in NMPF treated mice was 0.9 (Fig 68).
*Frequency of arthritic mice:* no difference in frequency of arthritis between day 0-6 was observed between the two groups of mice. Thereafter, continue increase of arthritis frequency for PBS treated group (100% at day 13) but decrease for NMPF treated group (50% at day 13) (Fig 69).
In conclusion, these results show that NMPF-6 (VLPALP) treatment prevents mice from development of arthritis and even profoundly decreases the severity of arthritis.

### Osteoclastogenesis Assay

A delicate balance between bone resorption and bone formation is critical for the maintenance of bone strength and integrity, wherein bone-resorbing osteoclasts and bone-forming osteoblasts play central roles. In fact, this physiologic process, termed bone remodeling, must be regulated strictly, and tipping the balance in favor of osteoclasts causes bone destruction observed in pathological conditions such as autoimmune arthritis, periodontitis, (postmenopausal) osteoporosis, Paget's disease and bone tumors
Regulation of osteoclast differentiation is an aspect central to the understanding of the pathogenesis and the treatment of bone diseases such as autoimmune arthritis and osteoporosis. Excessive signaling by RANKL (receptor activator of NF-kappaB ligand), a member of the tumor necrosis factor (TNF) family essential for osteoclastogenesis, is thought to contribute to such pathological conditions. Joint destruction because of matrix degradation and excessive bone loss characterizes inflammatory bone diseases such as osteolysis, osteoarthritis, and rheumatoid arthritis. Accumulation of inflammatory cells and their secreted products at the inflammation site attracts osteoclasts and their precursor cells, leading to further deterioration of the bone component. Tumor necrosis factor-alpha, interleukin-1 (IL-1), and RANKL (also known as OPGL and ODF), are abundant in sites of inflammation and are known to promote osteoclast recruitment, differentiation, and activation. Osteoclast differentiation *per se* requires activation of the RANK/RANKL pathway.
The role of RANKL and TNF-alpha in osteoclast (OC) formation has been established clearly. To determine the effect of NMPF osteoclast formation, we used bone marrow (BM) cells and RAW 264.7 mouse monocytes as a model system for the differentiation of multinucleated osteoclasts.
*OC Generation and Characterization:* OC were generated by culturing BM cells with recombinant soluble RANKL (20 ng/ml) and M-CSF (10 ng/ml) for 7 days with or without NMPF (10 microgram/mL). OC were also generated by culturing RAW 264.7 cells with RANKL (20 ng/ml) without M-CSF or with TNF-alpha and treated with NMPF. Both culture systems generate large numbers of TRAP⁺ multinucleated cells, which express typical OC markers. Osteoclast formation was measured by quantitating the presence of multinucleated TRAP positive cells (more than three nuclei) using cytochemical staining.
*Results:* As anticipated, we observed a marked inhibition of ligand (RANKL; MCS-F; TNF-alpha) induced osteclastogenesis, when cells were co-incubated with those peptides capable of inhibiting NF-kappaB activity. The ability of this set of peptides to inhibit osteoclast formation was observed in the BM as well as in the RAW 264.7 model systems, as evidenced by a reduced number of multinucleated, TRAP positive cells compared to control cells which had only received ligand.

## Claims

1. Use of at least one isolated peptide having an amino acid sequence chosen from the group consisting of VLPALPQVVC, LQGVLPALPQ, LQG, LQGV, GVLPALPQ,VLPALP, VLPALPQ, GVLPALP, VVC, QVVC, MTRV, MTR, LQGVLPALPQVVC, cyclic- LQGVLPALPQVVC, AQG, LAG, LQA, AQGV, LAGV, LQAV, LQGA, ALPALP, VAPALP, VLAALP, VLPAAP, VLPALA, ALPALPQ, VAPALPQ, VLAALPQ, VLPAAPQ, VLPALAQ, VLPALPA, VVCNYRDVRFESIRLPGCPRGVNPVVSYAVALSCQCAL, VVCNYRDVRFESIRLPGCPRGVNPVVSYAVALSCQ, SIRLPGCPRGVNPVVS, LPGCPRGVNPVVS, CPRGVNPVVS, LPGC, CPRGVNP PGCP,RPRCRPINATLAVEK EGCPVCITVNTTICAGYCPT, MTRVLQGVLPALPQ, CALCRRSTTDCGGPKDHPLTC, SKAPPPSLPSPSRLPGPS, TCDDPRFQDSSSSKAPPPSLPSPSRLPGPSDTPILPQ and CRRSTTDCGGPKDHPLTC
for the manufacture of a medicament for the treatment, suppression or prevention of a NFkB-regulated inflammatory disease, the disease being selected from the group consisting of asthma, inflammatory bowel disease, anthrax, bone disease, arthritis, multiple sclerosis, chronic obstructive pulmonary disease, cerebrovascular disease and allergic rhinitis.

2. Use according to claim 1, wherein the at least one peptide is selected from the group consisting of VLPALPQVVC, LQGVLPALPQ, LQG, LQGV, GVLPALPQ, VLPALP, VLPALPQ, GVLPALP, VVC, QVVC, MTRV, MTR, LQGVLPALPQVVC, cyclic- LQGVLPALPQVVC, AQG, LAG, LQA, AQGV, LAGV, LQAV, LQGA, ALPALP, VAPALP, VLAALP, VLPAAP, VLPALA, ALPALPQ, VAPALPQ, VLAALPQ, VLPAAPQ, VLPALAQ, VLPALPA, VVCNYRDVRFESIRLPGCPRGVNPVVSYAVALSCQCAL, VVCNYRDVRFESIRLPGCPRGVNPVVSYAVALSCQ and LPGCPRGVNPVVS.

3. Use according to claim 1 or 2, comprising the use of a mixture of at least two of said isolated peptides.

4. Use according to claim 3, wherein the at least two peptides are selected from the group consisting of LQGV, AQGV and VLPALP.

5. Use according to claim 1 or 2, wherein said bone disease is autoimmune arthritis, periodontitis, (postmenopausal) osteoporosis, Paget's disease, inflammatory bone disease or bone tumours.

6. Use according to claim 5, wherein the peptide has an amino acid sequence chosen from the group consisting of VLPALPQVVC, LQGVLPALPQ, LQG, LQGV, GVLPALPQ, VLPALP, VVC, MTR and cyclic LQGVLPALPQVVC.

7. Use according to claim 1 or 2, wherein said peptide is VLPALP and wherein said disease is arthritis.

8. Use of an isolated peptide having an amino acid sequence chosen from the group consisting of LQGV, GVLPALPQ, VLPALPQ, VLPALP, MTRV, LPGCPRGVNPVVS, CPRGVNPVVS and LPGC, for the manufacture of a medicament for the treatment, suppression or prevention of diabetes.

9. Use according to claim 8, wherein the peptide is chosen from the group consisting of LQGV, VLPALPQ, GVLPALPQ, VLPALP, CPRGVNPVVS and LPGC.

10. An isolated peptide having an amino acid sequence chosen from the group consisting of LQG, LQGV, VVC, MTRV, VLPALAQ, VLPALPQ, AQG, LAG, LQA, AQGV, VAPALP and VAPALPQ for the modulation of angiogenesis.

11. An isolated peptide having an amino acid sequence chosen from the group consisting of LAG, VLPALPQVVC, LQGVLPALPQ, LQG, LQGV, VLPALP, VLPALPQ, GVLPALP, MTR, ALPALP, VAPALP and VLAALP for the modulation of vasculogenesis.

12. Use of at least one isolated peptide having an amino acid sequence chosen from the group consisting of VLPALPQVVC, LQGVLPALPQ, LQG, LQGV, AQGV, MTRV, GVLPALPQ, VLPALP, VVC, MTR and cyclic LQGVLPALPQVVC for the manufacture of a medicament for the treatment, suppression or prevention of cardiac injury, wherein said treatment comprises intracoronary, pericardial or intramyocardial delivery of the at least one peptide.

13. Use according to claim 12, wherein said intracoronary delivery comprises the use of a local delivery catheter.

14. Use according to claim 12, wherein said pericardial delivery comprises installation of a peptide-containing solution into the pericardial sac or via the aid of slow-release polymers, preferably heparinal-alginate or ethylene vinyl acetate.

15. Use according to claim 12, wherein said intramyocardial delivery comprises injecting a peptide-containing solution directly into the myocardium, preferably either under direct vision following thoracotomy or using a thoracoscope or via a catheter.
